# EUROPEAN PATENT APPLICATION

(11) **EP 3 545 887 A1**
(43) Date of publication of application: **02.10.2019**
(21) Application number: 19165944.0
(22) Date of filing: 28.03.2019
(51) Int. Cl.: A61B 18/12, A61M 1/00, A61B 18/14, B01D 46/00, A61B 34/37, A61B 34/35, A61B 18/00, A61B 17/00

(54) **METHOD FOR SMOKE EVACUATION FOR SURGICAL HUB**

(30) Priority: 28.03.2018 US 201862649291 P; 28.03.2018 US 201862649294 P; 28.03.2018 US 201862649296 P; 28.03.2018 US 201862649300 P; 28.03.2018 US 201862649302 P; 28.03.2018 US 201862649307 P; 28.03.2018 US 201862649309 P; 28.03.2018 US 201862649310 P; 28.03.2018 US 201862649313 P; 28.03.2018 US 201862649315 P; 28.03.2018 US 201862649320 P; 28.03.2018 US 201862649323 P; 28.03.2018 US 201862649327 P; 28.03.2018 US 201862649333 P; 30.03.2018 US 201862650877 P; 30.03.2018 US 201862650882 P; 30.03.2018 US 201862650887 P; 30.03.2018 US 201862650898 P; 19.04.2018 US 201862659900 P; 01.05.2018 US 201862665128 P; 01.05.2018 US 201862665129 P; 01.05.2018 US 201862665134 P; 01.05.2018 US 201862665139 P; 01.05.2018 US 201862665177 P; 01.05.2018 US 201862665192 P; 28.06.2018 US 201862691219 P; 28.06.2018 US 201862691227 P; 28.06.2018 US 201862691228 P; 28.06.2018 US 201862691230 P; 28.06.2018 US 201862691251 P; 28.06.2018 US 201862691257 P; 28.06.2018 US 201862691262 P; 30.06.2018 US 201862692747 P; 30.06.2018 US 201862692748 P; 30.06.2018 US 201862692768 P; 23.08.2018 US 201862721995 P; 23.08.2018 US 201862721996 P; 23.08.2018 US 201862721999 P; 23.08.2018 US 201862721994 P; 23.08.2018 US 201862721998 P; 10.09.2018 US 201862729176 P; 10.09.2018 US 201862729177 P; 10.09.2018 US 201862729182 P; 10.09.2018 US 201862729183 P; 10.09.2018 US 201862729184 P; 10.09.2018 US 201862729185 P; 10.09.2018 US 201862729186 P; 10.09.2018 US 201862729191 P; 10.09.2018 US 201862729195 P; 25.10.2018 US 201862750529 P; 25.10.2018 US 201862750539 P; 25.10.2018 US 201862750555 P; 30.11.2018 US 201862773741 P; 30.11.2018 US 201862773742 P; 30.11.2018 US 201862773778 P; 04.12.2018 US 201816209447
(71) Applicant: Ethicon LLC, Guaynabo 00969 (PR)
(72) Inventor: YATES, David C., Cincinnati, OH Ohio 45242 (US); DANZIGER, Benjamin J., Cincinnati, OH Ohio 45242 (US); SHELTON, Frederick E., IV,, Cincinnati, OH Ohio 45242 (US); HARRIS, Jason L., Cincinnati, OH Ohio 45242 (US); ALRDIDGE, Jeffrey L., Cincinnati, OH Ohio 45242 (US)
(74) Representative: Bettridge, Paul Sebastian

(57) **Abstract**

A method for evacuating particulates from a surgical site is disclosed. The method comprises communicatively connecting a particulate evacuation module and a surgical hub, wherein the surgical hub comprises a surgical hub enclosure, and wherein the particulate evacuation module is configured to be received within the surgical hub enclosure. The method further comprises removing a particulate from the surgical site and into the particulate evacuation module, analyzing the removed particulate, and modifying an operation of the particulate evacuation module based on the analysis of the removed particulate.

## Description

### CROSS-REFERENCE TO RELATED APPLICATIONS

The present application claims priority under 35 U.S.C. § 119(e) to U.S. Provisional Patent Application No. 62/773,778, titled METHOD FOR ADAPTIVE CONTROL SCHEMES FOR SURGICAL NETWORK CONTROL AND INTERACTION, filed November 30, 2018, to U.S. Provisional Patent Application No. 62/773,728, titled METHOD FOR SITUATIONAL AWARENESS FOR SURGICAL NETWORK OR SURGICAL NETWORK CONNECTED DEVICE CAPABLE OF ADJUSTING FUNCTION BASED ON A SENSED SITUATION OR USAGE, filed November 30, 2018, to U.S. Provisional Patent Application No. 62/773,741, titled METHOD FOR FACILITY DATA COLLECTION AND INTERPRETATION, filed November 30, 2018, and to U.S. Provisional Patent Application No. 62/773,742, titled METHOD FOR CIRCULAR STAPLER CONTROL ALGORITHM ADJUSTMENT BASED ON SITUATIONAL AWARENESS, filed November 30, 2018, the disclosure of each of which is herein incorporated by reference in its entirety.

The present application claims priority under 35 U.S.C. § 119(e) to U.S. Provisional Patent Application No. 62/750,529, titled METHOD FOR OPERATING A POWERED ARTICULATING MULTI-CLIP APPLIER, filed October 25, 2018, to U.S. Provisional Patent Application No. 62/750,539, titled SURGICAL CLIP APPLIER, filed October 25, 2018, and to U.S. Provisional Patent Application No. 62/750,555, titled SURGICAL CLIP APPLIER, filed October 25, 2018, the disclosure of each of which is herein incorporated by reference in its entirety.

The present application also claims priority under 35 U.S.C. § 119(e) to U.S. Provisional Patent Application No. 62/729,183, titled CONTROL FOR A SURGICAL NETWORK OR SURGICAL NETWORK CONNECTED DEVICE THAT ADJUSTS ITS FUNCTION BASED ON A SENSED SITUATION OR USAGE, filed September 10, 2018, to U.S. Provisional Patent Application No. 62/729,177, titled AUTOMATED DATA SCALING, ALIGNMENT, AND ORGANIZING BASED ON PREDEFINED PARAMETERS WITHIN A SURGICAL NETWORK BEFORE TRANSMISSION, filed September 10, 2018, to U.S. Provisional Patent Application No. 62/729,176, titled INDIRECT COMMAND AND CONTROL OF A FIRST OPERATING ROOM SYSTEM THROUGH THE USE OF A SECOND OPERATING ROOM SYSTEM WITHIN A STERILE FIELD WHERE THE SECOND OPERATING ROOM SYSTEM HAS PRIMARY AND SECONDARY OPERATING MODES, filed September 10, 2018, to U.S. Provisional Patent Application No. 62/729,185, titled POWERED STAPLING DEVICE THAT IS CAPABLE OF ADJUSTING FORCE, ADVANCEMENT SPEED, AND OVERALL STROKE OF CUTTING MEMBER OF THE DEVICE BASED ON SENSED PARAMETER OF FIRING OR CLAMPING, filed September 10, 2018, to U.S. Provisional Patent Application No. 62/729,184, titled POWERED SURGICAL TOOL WITH A PREDEFINED ADJUSTABLE CONTROL ALGORITHM FOR CONTROLLING AT LEAST ONE END EFFECTOR PARAMETER AND A MEANS FOR LIMITING THE ADJUSTMENT, filed September 10, 2018, to U.S. Provisional Patent Application No. 62/729,182, titled SENSING THE PATIENT POSITION AND CONTACT UTILIZING THE MONO-POLAR RETURN PAD ELECTRODE TO PROVIDE SITUATIONAL AWARENESS TO THE HUB, filed September 10, 2018, to U.S. Provisional Patent Application No. 62/729,191, titled SURGICAL NETWORK RECOMMENDATIONS FROM REAL TIME ANALYSIS OF PROCEDURE VARIABLES AGAINST A BASELINE HIGHLIGHTING DIFFERENCES FROM THE OPTIMAL SOLUTION, filed September 10, 2018, to U.S. Provisional Patent Application No. 62/729,195, titled ULTRASONIC ENERGY DEVICE WHICH VARIES PRESSURE APPLIED BY CLAMP ARM TO PROVIDE THRESHOLD CONTROL PRESSURE AT A CUT PROGRESSION LOCATION, filed September 10, 2018, and to U. S. Provisional Patent Application No. 62/729,186, titled WIRELESS PAIRING OF A SURGICAL DEVICE WITH ANOTHER DEVICE WITHIN A STERILE SURGICAL FIELD BASED ON THE USAGE AND SITUATIONAL AWARENESS OF DEVICES, filed September 10, 2018, the disclosure of each of which is herein incorporated by reference in its entirety.

The present application also claims priority under 35 U.S.C. § 119(e) to U.S. Provisional Patent Application No. 62/721,995, titled CONTROLLING AN ULTRASONIC SURGICAL INSTRUMENT ACCORDING TO TISSUE LOCATION, filed August 23, 2018, to U.S. Provisional Patent Application No. 62/721,998, titled SITUATIONAL AWARENESS OF ELECTROSURGICAL SYSTEMS, filed August 23, 2018, to U.S. Provisional Patent Application No. 62/721,999, titled INTERRUPTION OF ENERGY DUE TO INADVERTENT CAPACITIVE COUPLING, filed August 23, 2018, to U.S. Provisional Patent Application No. 62/721,994, titled BIPOLAR COMBINATION DEVICE THAT AUTOMATICALLY ADJUSTS PRESSURE BASED ON ENERGY MODALITY, filed August 23, 2018, and to U.S. Provisional Patent Application No. 62/721,996, titled RADIO FREQUENCY ENERGY DEVICE FOR DELIVERING COMBINED ELECTRICAL SIGNALS, filed August 23, 2018, the disclosure of each of which is herein incorporated by reference in its entirety.

The present application also claims priority under 35 U.S.C. § 119(e) to U.S. Provisional Patent Application No. 62/692,747, titled SMART ACTIVATION OF AN ENERGY DEVICE BY ANOTHER DEVICE, filed on June 30, 2018, to U.S. Provisional Patent Application No. 62/692,748, titled SMART ENERGY ARCHITECTURE, filed on June 30, 2018, and to U.S. Provisional Patent Application No. 62/692,768, titled SMART ENERGY DEVICES, filed on June 30, 2018, the disclosure of each of which is herein incorporated by reference in its entirety.

The present application also claims priority under 35 U.S.C. § 119(e) to U.S. Provisional Patent Application No. 62/691,228, titled METHOD OF USING REINFORCED FLEX CIRCUITS WITH MULTIPLE SENSORS WITH ELECTROSURGICAL DEVICES, filed June 28, 2018, to U.S. Provisional Patent Application No. 62/691,227, titled CONTROLLING A SURGICAL INSTRUMENT ACCORDING TO SENSED CLOSURE PARAMETERS, filed June 28, 2018, to U.S. Provisional Patent Application No. 62/691,230, titled SURGICAL INSTRUMENT HAVING A FLEXIBLE ELECTRODE, filed June 28, 2018, to U.S. Provisional Patent Application No. 62/691,219, titled SURGICAL EVACUATION SENSING AND MOTOR CONTROL, filed June 28, 2018, to U. S. Provisional Patent Application No. 62/691,257, titled COMMUNICATION OF SMOKE EVACUATION SYSTEM PARAMETERS TO HUB OR CLOUD IN SMOKE EVACUATION MODULE FOR INTERACTIVE SURGICAL PLATFORM, filed June 28, 2018, to U.S. Provisional Patent Application No. 62/691,262, titled SURGICAL EVACUATION SYSTEM WITH A COMMUNICATION CIRCUIT FOR COMMUNICATION BETWEEN A FILTER AND A SMOKE EVACUATION DEVICE, filed June 28, 2018, and to U.S. Provisional Patent Application No. 62/691,251, titled DUAL IN-SERIES LARGE AND SMALL DROPLET FILTERS, filed June 28, 2018, the disclosure of each of which is herein incorporated by reference in its entirety.

The present application claims priority under 35 U.S.C. § 119(e) to U.S. Provisional Patent Application No. 62/665,129, titled SURGICAL SUTURING SYSTEMS, filed May 1, 2018, to U.S. Provisional Patent Application No. 62/665,139, titled SURGICAL INSTRUMENTS COMPRISING CONTROL SYSTEMS, filed May 1, 2018, to U.S. Provisional Patent Application No. 62/665,177, titled SURGICAL INSTRUMENTS COMPRISING HANDLE ARRANGEMENTS, filed May 1, 2018, to U.S. Provisional Patent Application No. 62/665,128, titled MODULAR SURGICAL INSTRUMENTS, filed May 1, 2018, to U.S. Provisional Patent Application No. 62/665,192, titled SURGICAL DISSECTORS, filed May 1, 2018, and to U.S. Provisional Patent Application No. 62/665,134, titled SURGICAL CLIP APPLIER, filed May 1, 2018, the disclosure of each of which is herein incorporated by reference in its entirety.

The present application also claims priority under 35 U.S.C. § 119(e) to U.S. Provisional Patent Application No. 62/659,900, titled METHOD OF HUB COMMUNICATION, filed on April 19, 2018, the disclosure of which is herein incorporated by reference in its entirety.

The present application also claims priority under 35 U.S.C. § 119(e) to U.S. Provisional Patent Application No. 62/650,898, filed on March 30, 2018, titled CAPACITIVE COUPLED RETURN PATH PAD WITH SEPARABLE ARRAY ELEMENTS, to U.S. Provisional Patent Application No. 62/650,887, titled SURGICAL SYSTEMS WITH OPTIMIZED SENSING CAPABILITIES, filed March 30, 2018, to U.S. Provisional Patent Application No. 62/650,882, titled SMOKE EVACUATION MODULE FOR INTERACTIVE SURGICAL PLATFORM, filed March 30, 2018, and to U.S. Provisional Patent Application No. 62/650,877, titled SURGICAL SMOKE EVACUATION SENSING AND CONTROLS, filed March 30, 2018, the disclosure of each of which is herein incorporated by reference in its entirety.

This application also claims the benefit of priority under 35 U.S.C. § 119(e) to U.S. Provisional Patent Application No. 62/649,302, titled INTERACTIVE SURGICAL SYSTEMS WITH ENCRYPTED COMMUNICATION CAPABILITIES, filed March 28, 2018, to U.S. Provisional Patent Application No. 62/649,294, titled DATA STRIPPING METHOD TO INTERROGATE PATIENT RECORDS AND CREATE ANONYMIZED RECORD, filed March 28, 2018, to U.S. Provisional Patent Application No. 62/649,300, titled SURGICAL HUB SITUATIONAL AWARENESS, filed March 28, 2018, to U.S. Provisional Patent Application No. 62/649,309, titled SURGICAL HUB SPATIAL AWARENESS TO DETERMINE DEVICES IN OPERATING THEATER, filed March 28, 2018, to U.S. Provisional Patent Application No. 62/649,310, titled COMPUTER IMPLEMENTED INTERACTIVE SURGICAL SYSTEMS, filed March 28, 2018, to U.S. Provisional Patent Application No. 62/649,291, titled USE OF LASER LIGHT AND RED-GREEN-BLUE COLORATION TO DETERMINE PROPERTIES OF BACK SCATTERED LIGHT, filed March 28, 2018, to U.S. Provisional Patent Application No. 62/649,296, titled ADAPTIVE CONTROL PROGRAM UPDATES FOR SURGICAL DEVICES, filed March 28, 2018, to U.S. Provisional Patent Application No. 62/649,333, titled CLOUD-BASED MEDICAL ANALYTICS FOR CUSTOMIZATION AND RECOMMENDATIONS TO A USER, filed March 28, 2018, to U.S. Provisional Patent Application No. 62/649,327, titled CLOUD-BASED MEDICAL ANALYTICS FOR SECURITY AND AUTHENTICATION TRENDS AND REACTIVE MEASURES, filed March 28, 2018, to U.S. Provisional Patent Application No. 62/649,315, titled DATA HANDLING AND PRIORITIZATION IN A CLOUD ANALYTICS NETWORK, filed March 28, 2018, to U.S. Provisional Patent Application No. 62/649,313, titled CLOUD INTERFACE FOR COUPLED SURGICAL DEVICES, filed March 28, 2018, to U.S. Provisional Patent Application No. 62/649,320, titled DRIVE ARRANGEMENTS FOR ROBOT-ASSISTED SURGICAL PLATFORMS, filed March 28, 2018, to U.S. Provisional Patent Application No. 62/649,307, titled AUTOMATIC TOOL ADJUSTMENTS FOR ROBOT-ASSISTED SURGICAL PLATFORMS, filed March 28, 2018, and to U.S. Provisional Patent Application No. 62/649,323, titled SENSING ARRANGEMENTS FOR ROBOT-ASSISTED SURGICAL PLATFORMS, filed March 28, 2018, the disclosure of each of which is herein incorporated by reference in its entirety.

This application also claims the benefit of priority under 35 U.S.C. § 119(e) to U.S. Provisional Patent Application No. 62/611,341, titled INTERACTIVE SURGICAL PLATFORM, filed December 28, 2017, to U.S. Provisional Patent Application No. 62/611,340, titled CLOUD-BASED MEDICAL ANALYTICS, filed December 28, 2017, and to U.S. Provisional Patent Application No. 62/611,339, titled ROBOT ASSISTED SURGICAL PLATFORM, filed December 28, 2017, the disclosure of each of which is herein incorporated by reference in its entirety.

### BACKGROUND

The present disclosure relates to methods for surgical systems and evacuators thereof. Surgical smoke evacuators are configured to evacuate smoke, as well as fluid and/or particulate, from a surgical site. For example, during a surgical procedure involving an energy device, smoke can be generated at the surgical site.

### SUMMARY

In one aspect the present disclosure provides a method for evacuating particulates from a surgical site. The method comprising: communicatively connecting a particulate evacuation module and a surgical hub, wherein the surgical hub comprises a surgical hub enclosure, and wherein the particulate evacuation module is configured to be received within the surgical hub enclosure; removing a particulate from the surgical site and into the particulate evacuation module; analyzing the removed particulate; and modifying an operation of the particulate evacuation module based on the analysis of the removed particulate.

In another aspect, the present disclosure provides a method for evacuating particulates from a surgical site, the method comprising: connecting a particulate evacuation module and a surgical hub, wherein the surgical hub comprises a surgical hub enclosure, and wherein the particulate evacuation module is configured to be received within the surgical hub enclosure; activating a surgical instrument within the surgical site; drawing a particulate from the surgical site into the particulate evacuation module; analyzing the particulate; and modifying an operating parameter of the particulate evacuation module based on the analysis of the particulate.

In another aspect, the present disclosure provides a method for evacuating smoke from a surgical site, the method comprising: connecting a smoke evacuation module and a surgical hub, wherein the surgical hub comprises a surgical hub enclosure, and wherein the smoke evacuation module is configured to be received within the surgical hub enclosure; inserting a component of the smoke evacuation module into the surgical site; removing smoke from the surgical site; analyzing the removed smoke; and modifying an operation of the smoke evacuation module based on the analysis of the removed smoke.

### BRIEF DESCRIPTION OF THE DRAWINGS

The features of various aspects are set forth with particularity in the appended claims. The various aspects, however, both as to organization and methods of operation, together with further objects and advantages thereof, may best be understood by reference to the following description, taken in conjunction with the accompanying drawings as follows.
FIG. 1 is perspective view of an evacuator housing for a surgical evacuation system, in accordance with at least one aspect of the present disclosure.
FIG. 2 is a perspective view of a surgical evacuation electrosurgical tool, in accordance with at least one aspect of the present disclosure.
FIG. 3 is an elevation view of a surgical evacuation tool releasably secured to an electrosurgical pencil, in accordance with at least one aspect of the present disclosure.
FIG. 4 is a schematic depicting internal components within an evacuator housing for a surgical evacuation system, in accordance with at least one aspect of the present disclosure.
FIG. 5 is a schematic of an electrosurgical system including a smoke evacuator, in accordance with at least one aspect of the present disclosure.
FIG. 6 is a schematic of a surgical evacuation system, in accordance with at least one aspect of the present disclosure.
FIG. 7 is a perspective view of a surgical system including a surgical evacuation system, in accordance with at least one aspect of the present disclosure.
FIG. 8 is a perspective view of an evacuator housing of the surgical evacuation system of FIG. 7, in accordance with at least one aspect of the present disclosure.
FIG. 9 is an elevation, cross-section view of a socket in the evacuator housing of FIG. 8 along the plane indicated in FIG. 8, in accordance with at least one aspect of the present disclosure.
FIG. 10 is a perspective view of a filter for an evacuation system, in accordance with at least one aspect of the present disclosure.
FIG. 11 is a perspective, cross-section view of the filter of FIG. 10 taken along a central longitudinal plane of the filter, in accordance with at least one aspect of the present disclosure.
FIG. 12 is a pump for a surgical evacuation system, such as the surgical evacuation system of FIG. 7, in accordance with at least one aspect of the present disclosure.
FIG. 13 is a perspective view of a portion of a surgical evacuation system, in accordance with at least one aspect of the present disclosure.
FIG. 14 is a front perspective view of a fluid trap of the surgical evacuation system of FIG. 13, in accordance with at least one aspect of the present disclosure.
FIG. 15 is a rear perspective view of the fluid trap of FIG. 14, in accordance with at least one aspect of the present disclosure.
FIG. 16 is an elevation, cross-section view of the fluid trap of FIG. 14, in accordance with at least one aspect of the present disclosure.
FIG. 17 is an elevation, cross-section view of the fluid trap of FIG. 14 with portions removed for clarity and depicting liquid captured within the fluid trap and smoke flowing through the fluid trap, in accordance with at least one aspect of the present disclosure.
FIG. 18 is a schematic of an evacuator housing of an evacuation system, in accordance with at least one aspect of the present disclosure.
FIG. 19 is a schematic of an evacuator housing of another evacuation system, in accordance with at least one aspect of the present disclosure.
FIG. 20 illustrates a smoke evacuation system in communication with various other modules and devices, in accordance with at least one aspect of the present disclosure.
FIG. 21 depicts an example flow diagram of interconnectivity between the surgical hub, the cloud, the smoke evacuation module, and/or various other modules A, B, and C, in accordance with at least one aspect of the present disclosure.
FIG. 22 illustrates a smoke evacuation system in communication with various other modules and devices, in accordance with at least one aspect of the present disclosure.
FIG. 23 illustrates one aspect of a segmented control circuit, in accordance with at least aspect of the present disclosure.
FIG. 24 discloses a method of operating a control circuit portion of the smoke evacuation module so as to energize the segmented control circuit in stages, in accordance with at least one aspect of the present disclosure.
FIG. 25 is a schematic diagram of a smoke evacuation system, in accordance with at least one aspect of the present disclosure.
FIG. 26 is a schematic diagram of a filter communication circuit of the smoke evacuation system of FIG. 25, in accordance with at least one aspect of the present disclosure.
FIG. 27 is a schematic diagram of a filter device of the smoke evacuation system of FIG. 25, in accordance with at least one aspect of the present disclosure.
FIG. 28 is a schematic of a housing of an evacuation system, in accordance with at least one aspect of the present disclosure.
FIG. 29 illustrates an electrosurgical system, in accordance with at least one aspect of the present disclosure.
FIG. 30 illustrates a return pad of the electrosurgical system of FIG. 29, in accordance with at least one aspect of the present disclosure.
FIG. 31 illustrates a plurality of electrodes of the return pad of FIG. 30, in accordance with at least one aspect of the present disclosure.
FIG. 32 illustrates an array of sensing devices of the return pad of FIG. 30, in accordance with at least one aspect of the present disclosure.
FIG. 33 illustrates a method for concurrently applying a nerve stimulation signal and electrosurgical energy to a patient, in accordance with at least one aspect of the present disclosure.
FIG. 34 is a block diagram of a computer-implemented interactive surgical system, in accordance with at least one aspect of the present disclosure.
FIG. 35 is a surgical system being used to perform a surgical procedure in an operating room, in accordance with at least one aspect of the present disclosure.
FIG. 36 is a surgical hub paired with a visualization system, a robotic system, and an intelligent instrument, in accordance with at least one aspect of the present disclosure.
FIG. 37 is a partial perspective view of a surgical hub enclosure, and of a combo generator module slidably receivable in a drawer of the surgical hub enclosure, in accordance with at least one aspect of the present disclosure.
FIG. 38 is a perspective view of a combo generator module with bipolar, ultrasonic, and monopolar contacts and a smoke evacuation component, in accordance with at least one aspect of the present disclosure.
FIG. 39 illustrates individual power bus attachments for a plurality of lateral docking ports of a lateral modular housing configured to receive a plurality of modules, in accordance with at least one aspect of the present disclosure.
FIG. 40 illustrates a vertical modular housing configured to receive a plurality of modules, in accordance with at least one aspect of the present disclosure.
FIG. 41 illustrates a surgical data network comprising a modular communication hub configured to connect modular devices located in one or more operating theaters of a healthcare facility, or any room in a healthcare facility specially equipped for surgical operations, to the cloud, in accordance with at least one aspect of the present disclosure.
FIG. 42 illustrates a computer-implemented interactive surgical system, in accordance with at least one aspect of the present disclosure.
FIG. 43 illustrates a surgical hub comprising a plurality of modules coupled to the modular control tower, in accordance with at least one aspect of the present disclosure.
FIG. 44 illustrates one aspect of a Universal Serial Bus (USB) network hub device, in accordance with at least one aspect of the present disclosure.
FIG. 45 illustrates a logic diagram of a control system of a surgical instrument or tool, in accordance with at least one aspect of the present disclosure.
FIG. 46 illustrates a control circuit configured to control aspects of the surgical instrument or tool, in accordance with at least one aspect of the present disclosure.
FIG. 47 illustrates a combinational logic circuit configured to control aspects of the surgical instrument or tool, in accordance with at least one aspect of the present disclosure.
FIG. 48 illustrates a sequential logic circuit configured to control aspects of the surgical instrument or tool, in accordance with at least one aspect of the present disclosure.
FIG. 49 illustrates a surgical instrument or tool comprising a plurality of motors which can be activated to perform various functions, in accordance with at least one aspect of the present disclosure.
FIG. 50 is a schematic diagram of a robotic surgical instrument configured to operate a surgical tool described herein, in accordance with at least one aspect of the present disclosure.
FIG. 51 illustrates a block diagram of a surgical instrument programmed to control the distal translation of a displacement member, in accordance with at least one aspect of the present disclosure.
FIG. 52 is a schematic diagram of a surgical instrument configured to control various functions, in accordance with at least one aspect of the present disclosure.
FIG. 53 is a simplified block diagram of a generator configured to provide inductorless tuning, among other benefits, in accordance with at least one aspect of the present disclosure.
FIG. 54 illustrates an example of a generator, which is one form of the generator of FIG. 53, in accordance with at least one aspect of the present disclosure.
FIG. 55 is a timeline depicting situational awareness of a surgical hub, in accordance with one aspect of the present disclosure.

### DETAILED DESCRIPTION

Applicant of the present application owns the following U.S. Patent Applications, filed on December 4, 2018, the disclosure of each of which is herein incorporated by reference in its entirety:
- Attorney Docket No. END8495USNP/170727M, titled METHOD OF HUB COMMUNICATION, PROCESSING, STORAGE AND DISPLAY;
- Attorney Docket No. END8495USNP1/170727-1M, titled METHOD OF HUB COMMUNICATION;
- Attorney Docket No. END8496USNP/170728M, titled METHOD OF CLOUD BASED DATA ANALYTICS FOR USE WITH THE HUB;
- Attorney Docket No. END8497USNP/170729M, titled METHOD OF ROBOTIC HUB COMMUNICATION, DETECTION, AND CONTROL;
- Attorney Docket No. END8505USNP/170772M, titled METHOD OF HUB COMMUNICATION, PROCESSING, DISPLAY, AND CLOUD ANALYTICS;
- Attorney Docket No. END8538USNP/170751M, titled METHOD OF COMPRESSING TISSUE WITHIN A STAPLING DEVICE AND SIMULTANEOUSLY DISPLAYING THE LOCATION OF THE TISSUE WITHIN THE JAWS;
- Attorney Docket No. END8539USNP/170752M, titled METHOD OF USING REINFORCED FLEXIBLE CIRCUITS WITH MULTIPLE SENSORS TO OPTIMIZE PERFORMANCE OF RADIO FREQUENCY DEVICES;
- Attorney Docket No. END8540USNP/170753M, titled METHOD OF SENSING PARTICULATE FROM SMOKE EVACUATED FROM A PATIENT, ADJUSTING THE PUMP SPEED BASED ON THE SENSED INFORMATION, AND COMMUNICATING THE FUNCTIONAL PARAMETERS OF THE SYSTEM TO THE HUB;
- Attorney Docket No. END8558USNP1/180138-1M, titled METHOD FOR CONTROLLING SMART ENERGY DEVICES;
- Attorney Docket No. END8559USNP1/180141-1M, titled METHOD FOR SMART ENERGY DEVICE INFRASTRUCTURE;
- Attorney Docket No. END9011USNP1/180510-1M, titled METHOD FOR ADAPTIVE CONTROL SCHEMES FOR SURGICAL NETWORK CONTROL AND INTERACTION;
- Attorney Docket No. END9015USNP1/180514-1M, titled METHOD FOR SITUATIONAL AWARENESS FOR SURGICAL NETWORK OR SURGICAL NETWORK CONNECTED DEVICE CAPABLE OF ADJUSTING FUNCTION BASED ON A SENSED SITUATION OR USAGE;
- Attorney Docket No. END9017USNP1/180516-1M, titled METHOD FOR FACILITY DATA COLLECTION AND INTERPRETATION; and
- Attorney Docket No. END9033USNP1/180520-1M, titled METHOD FOR CIRCULAR STAPLER CONTROL ALGORITHM ADJUSTMENT BASED ON SITUATIONAL AWARENESS.

Applicant of the present application owns the following U.S. Patent Applications, filed on November 6, 2018, the disclosure of each of which is herein incorporated by reference in its entirety:
- U.S. Patent Application No. 16/182,224, titled SURGICAL NETWORK, INSTRUMENT, AND CLOUD RESPONSES BASED ON VALIDATION OF RECEIVED DATASET AND AUTHENTICATION OF ITS SOURCE AND INTEGRITY;
- U.S. Patent Application No. 16/182,230, titled SURGICAL SYSTEM FOR PRESENTING INFORMATION INTERPRETED FROM EXTERNAL DATA;
- U.S. Patent Application No. 16/182,233, titled SURGICAL SYSTEMS WITH AUTONOMOUSLY ADJUSTABLE CONTROL PROGRAMS;
- U.S. Patent Application No. 16/182,239, titled ADJUSTMENT OF DEVICE CONTROL PROGRAMS BASED ON STRATIFIED CONTEXTUAL DATA IN ADDITION TO THE DATA;
- U.S. Patent Application No. 16/182,243, titled SURGICAL HUB AND MODULAR DEVICE RESPONSE ADJUSTMENT BASED ON SITUATIONAL AWARENESS;
- U.S. Patent Application No. 16/182,248, titled DETECTION AND ESCALATION OF SECURITY RESPONSES OF SURGICAL INSTRUMENTS TO INCREASING SEVERITY THREATS;
- U.S. Patent Application No. 16/182,251, titled INTERACTIVE SURGICAL SYSTEM;
- U.S. Patent Application No. 16/182,260, titled AUTOMATED DATA SCALING, ALIGNMENT, AND ORGANIZING BASED ON PREDEFINED PARAMETERS WITHIN SURGICAL NETWORKS;
- U.S. Patent Application No. 16/182,267, titled SENSING THE PATIENT POSITION AND CONTACT UTILIZING THE MONO-POLAR RETURN PAD ELECTRODE TO PROVIDE SITUATIONAL AWARENESS TO THE HUB;
- U.S. Patent Application No. 16/182,249, titled POWERED SURGICAL TOOL WITH PREDEFINED ADJUSTABLE CONTROL ALGORITHM FOR CONTROLLING END EFFECTOR PARAMETER;
- U.S. Patent Application No. 16/182,246, titled ADJUSTMENTS BASED ON AIRBORNE PARTICLE PROPERTIES;
- U.S. Patent Application No. 16/182,256, titled ADJUSTMENT OF A SURGICAL DEVICE FUNCTION BASED ON SITUATIONAL AWARENESS;
- U.S. Patent Application No. 16/182,242, titled REAL-TIME ANALYSIS OF COMPREHENSIVE COST OF ALL INSTRUMENTATION USED IN SURGERY UTILIZING DATA FLUIDITY TO TRACK INSTRUMENTS THROUGH STOCKING AND IN-HOUSE PROCESSES;
- U.S. Patent Application No. 16/182,255, titled USAGE AND TECHNIQUE ANALYSIS OF SURGEON / STAFF PERFORMANCE AGAINST A BASELINE TO OPTIMIZE DEVICE UTILIZATION AND PERFORMANCE FOR BOTH CURRENT AND FUTURE PROCEDURES;
- U.S. Patent Application No. 16/182,269, titled IMAGE CAPTURING OF THE AREAS OUTSIDE THE ABDOMEN TO IMPROVE PLACEMENT AND CONTROL OF A SURGICAL DEVICE IN USE;
- U.S. Patent Application No. 16/182,278, titled COMMUNICATION OF DATA WHERE A SURGICAL NETWORK IS USING CONTEXT OF THE DATA AND REQUIREMENTS OF A RECEIVING SYSTEM / USER TO INFLUENCE INCLUSION OR LINKAGE OF DATA AND METADATA TO ESTABLISH CONTINUITY;
- U.S. Patent Application No. 16/182,290, titled SURGICAL NETWORK RECOMMENDATIONS FROM REAL TIME ANALYSIS OF PROCEDURE VARIABLES AGAINST A BASELINE HIGHLIGHTING DIFFERENCES FROM THE OPTIMAL SOLUTION;
- U.S. Patent Application No. 16/182,232, titled CONTROL OF A SURGICAL SYSTEM THROUGH A SURGICAL BARRIER;
- U.S. Patent Application No. 16/182,227, titled SURGICAL NETWORK DETERMINATION OF PRIORITIZATION OF COMMUNICATION, INTERACTION, OR PROCESSING BASED ON SYSTEM OR DEVICE NEEDS;
- U.S. Patent Application No. 16/182,231, titled WIRELESS PAIRING OF A SURGICAL DEVICE WITH ANOTHER DEVICE WITHIN A STERILE SURGICAL FIELD BASED ON THE USAGE AND SITUATIONAL AWARENESS OF DEVICES;
- U.S. Patent Application No. 16/182,229, titled ADJUSTMENT OF STAPLE HEIGHT OF AT LEAST ONE ROW OF STAPLES BASED ON THE SENSED TISSUE THICKNESS OR FORCE IN CLOSING;
- U.S. Patent Application No. 16/182,234, titled STAPLING DEVICE WITH BOTH COMPULSORY AND DISCRETIONARY LOCKOUTS BASED ON SENSED PARAMETERS;
- U.S. Patent Application No. 16/182,240, titled POWERED STAPLING DEVICE CONFIGURED TO ADJUST FORCE, ADVANCEMENT SPEED, AND OVERALL STROKE OF CUTTING MEMBER BASED ON SENSED PARAMETER OF FIRING OR CLAMPING;
- U.S. Patent Application No. 16/182,235, titled VARIATION OF RADIO FREQUENCY AND ULTRASONIC POWER LEVEL IN COOPERATION WITH VARYING CLAMP ARM PRESSURE TO ACHIEVE PREDEFINED HEAT FLUX OR POWER APPLIED TO TISSUE; and
- U.S. Patent Application No. 16/182,238, titled ULTRASONIC ENERGY DEVICE WHICH VARIES PRESSURE APPLIED BY CLAMP ARM TO PROVIDE THRESHOLD CONTROL PRESSURE AT A CUT PROGRESSION LOCATION.

Applicant of the present application owns the following U.S. Patent Applications that were filed on October 26, 2018, the disclosure of each of which is herein incorporated by reference in its entirety:
- U.S. Patent Application No. 16/172,303, titled METHOD FOR OPERATING A POWERED ARTICULATING MULTI-CLIP APPLIER;
- U.S. Patent Application No. 16/172,130, titled CLIP APPLIER COMPRISING INTERCHANGEABLE CLIP RELOADS;
- U.S. Patent Application No. 16/172,066, titled CLIP APPLIER COMPRISING A MOVABLE CLIP MAGAZINE;
- U.S. Patent Application No. 16/172,078, titled CLIP APPLIER COMPRISING A ROTATABLE CLIP MAGAZINE;
- U.S. Patent Application No. 16/172,087, titled CLIP APPLIER COMPRISING CLIP ADVANCING SYSTEMS;
- U.S. Patent Application No. 16/172,094, titled CLIP APPLIER COMPRISING A CLIP CRIMPING SYSTEM;
- U.S. Patent Application No. 16/172,128, titled CLIP APPLIER COMPRISING A RECIPROCATING CLIP ADVANCING MEMBER;
- U.S. Patent Application No. 16/172,168, titled CLIP APPLIER COMPRISING A MOTOR CONTROLLER;
- U.S. Patent Application No. 16/172,164, titled SURGICAL SYSTEM COMPRISING A SURGICAL TOOL AND A SURGICAL HUB;
- U.S. Patent Application No. 16/172,328, titled METHOD OF HUB COMMUNICATION WITH SURGICAL INSTRUMENT SYSTEMS;
- U.S. Patent Application No. 16/172,280, titled METHOD FOR PRODUCING A SURGICAL INSTRUMENT COMPRISING A SMART ELECTRICAL SYSTEM;
- U.S. Patent Application No. 16/172,219, titled METHOD OF HUB COMMUNICATION WITH SURGICAL INSTRUMENT SYSTEMS;
- U.S. Patent Application No. 16/172,248, titled METHOD OF HUB COMMUNICATION WITH SURGICAL INSTRUMENT SYSTEMS;
- U.S. Patent Application No. 16/172,198, titled METHOD OF HUB COMMUNICATION WITH SURGICAL INSTRUMENT SYSTEMS; and
- U.S. Patent Application No. 16/172,155, titled METHOD OF HUB COMMUNICATION WITH SURGICAL INSTRUMENT SYSTEMS.

Applicant of the present application owns the following U.S. Patent Applications, filed on August 28, 2018, the disclosure of each of which is herein incorporated by reference in its entirety:
- U.S. Patent Application No. 16/115,214, titled ESTIMATING STATE OF ULTRASONIC END EFFECTOR AND CONTROL SYSTEM THEREFOR;
- U.S. Patent Application No. 16/115,205, titled TEMPERATURE CONTROL OF ULTRASONIC END EFFECTOR AND CONTROL SYSTEM THEREFOR;
- U.S. Patent Application No. 16/115,233, titled RADIO FREQUENCY ENERGY DEVICE FOR DELIVERING COMBINED ELECTRICAL SIGNALS;
- U.S. Patent Application No. 16/115,208, titled CONTROLLING AN ULTRASONIC SURGICAL INSTRUMENT ACCORDING TO TISSUE LOCATION;
- U.S. Patent Application No. 16/115,220, titled CONTROLLING ACTIVATION OF AN ULTRASONIC SURGICAL INSTRUMENT ACCORDING TO THE PRESENCE OF TISSUE;
- U.S. Patent Application No. 16/115,232, titled DETERMINING TISSUE COMPOSITION VIA AN ULTRASONIC SYSTEM;
- U.S. Patent Application No. 16/115,239, titled DETERMINING THE STATE OF AN ULTRASONIC ELECTROMECHANICAL SYSTEM ACCORDING TO FREQUENCY SHIFT;
- U.S. Patent Application No. 16/115,247, titled DETERMINING THE STATE OF AN ULTRASONIC END EFFECTOR;
- U.S. Patent Application No. 16/115,211, titled SITUATIONAL AWARENESS OF ELECTROSURGICAL SYSTEMS;
- U.S. Patent Application No. 16/115,226, titled MECHANISMS FOR CONTROLLING DIFFERENT ELECTROMECHANICAL SYSTEMS OF AN ELECTROSURGICAL INSTRUMENT;
- U.S. Patent Application No. 16/115,240, titled DETECTION OF END EFFECTOR EMERSION IN LIQUID;
- U.S. Patent Application No. 16/115,249, titled INTERRUPTION OF ENERGY DUE TO INADVERTENT CAPACITIVE COUPLING;
- U.S. Patent Application No. 16/115,256, titled INCREASING RADIO FREQUENCY TO CREATE PAD-LESS MONOPOLAR LOOP;
- U.S. Patent Application No. 16/115,223, titled BIPOLAR COMBINATION DEVICE THAT AUTOMATICALLY ADJUSTS PRESSURE BASED ON ENERGY MODALITY; and
- U.S. Patent Application No. 16/115,238, titled ACTIVATION OF ENERGY DEVICES.

Applicant of the present application owns the following U.S. Patent Applications, filed on August 24, 2018, the disclosure of each of which is herein incorporated by reference in its entirety:
- U.S. Patent Application No. 16/112,129, titled SURGICAL SUTURING INSTRUMENT CONFIGURED TO MANIPULATE TISSUE USING MECHANICAL AND ELECTRICAL POWER;
- U.S. Patent Application No. 16/112,155, titled SURGICAL SUTURING INSTRUMENT COMPRISING A CAPTURE WIDTH WHICH IS LARGER THAN TROCAR DIAMETER;
- U.S. Patent Application No. 16/112,168, titled SURGICAL SUTURING INSTRUMENT COMPRISING A NON-CIRCULAR NEEDLE;
- U.S. Patent Application No. 16/112,180, titled ELECTRICAL POWER OUTPUT CONTROL BASED ON MECHANICAL FORCES;
- U.S. Patent Application No. 16/112,193, titled REACTIVE ALGORITHM FOR SURGICAL SYSTEM;
- U.S. Patent Application No. 16/112,099, titled SURGICAL INSTRUMENT COMPRISING AN ADAPTIVE ELECTRICAL SYSTEM;
- U.S. Patent Application No. 16/112,112, titled CONTROL SYSTEM ARRANGEMENTS FOR A MODULAR SURGICAL INSTRUMENT;
- U.S. Patent Application No. 16/112,119, titled ADAPTIVE CONTROL PROGRAMS FOR A SURGICAL SYSTEM COMPRISING MORE THAN ONE TYPE OF CARTRIDGE;
- U.S. Patent Application No. 16/112,097, titled SURGICAL INSTRUMENT SYSTEMS COMPRISING BATTERY ARRANGEMENTS;
- U.S. Patent Application No. 16/112,109, titled SURGICAL INSTRUMENT SYSTEMS COMPRISING HANDLE ARRANGEMENTS;
- U.S. Patent Application No. 16/112,114, titled SURGICAL INSTRUMENT SYSTEMS COMPRISING FEEDBACK MECHANISMS;
- U.S. Patent Application No. 16/112,117, titled SURGICAL INSTRUMENT SYSTEMS COMPRISING LOCKOUT MECHANISMS;
- U.S. Patent Application No. 16/112,095, titled SURGICAL INSTRUMENTS COMPRISING A LOCKABLE END EFFECTOR SOCKET;
- U.S. Patent Application No. 16/112,121, titled SURGICAL INSTRUMENTS COMPRISING A SHIFTING MECHANISM;
- U.S. Patent Application No. 16/112,151, titled SURGICAL INSTRUMENTS COMPRISING A SYSTEM FOR ARTICULATION AND ROTATION COMPENSATION;
- U.S. Patent Application No. 16/112,154, titled SURGICAL INSTRUMENTS COMPRISING A BIASED SHIFTING MECHANISM;
- U.S. Patent Application No. 16/112,226, titled SURGICAL INSTRUMENTS COMPRISING AN ARTICULATION DRIVE THAT PROVIDES FOR HIGH ARTICULATION ANGLES;
- U.S. Patent Application No. 16/112,062, titled SURGICAL DISSECTORS AND MANUFACTURING TECHNIQUES;
- U.S. Patent Application No. 16/112,098, titled SURGICAL DISSECTORS CONFIGURED TO APPLY MECHANICAL AND ELECTRICAL ENERGY;
- U.S. Patent Application No. 16/112,237, titled SURGICAL CLIP APPLIER CONFIGURED TO STORE CLIPS IN A STORED STATE;
- U.S. Patent Application No. 16/112,245, titled SURGICAL CLIP APPLIER COMPRISING AN EMPTY CLIP CARTRIDGE LOCKOUT;
- U.S. Patent Application No. 16/112,249, titled SURGICAL CLIP APPLIER COMPRISING AN AUTOMATIC CLIP FEEDING SYSTEM;
- U.S. Patent Application No. 16/112,253, titled SURGICAL CLIP APPLIER COMPRISING ADAPTIVE FIRING CONTROL; and
- U.S. Patent Application No. 16/112,257, titled SURGICAL CLIP APPLIER COMPRISING ADAPTIVE CONTROL IN RESPONSE TO A STRAIN GAUGE CIRCUIT.

Applicant of the present application owns the following U.S. Patent Applications, filed on June 29, 2018, the disclosure of each of which is herein incorporated by reference in its entirety:
- U.S. Patent Application No. 16/024,090, titled CAPACITIVE COUPLED RETURN PATH PAD WITH SEPARABLE ARRAY ELEMENTS;
- U.S. Patent Application No. 16/024,057, titled CONTROLLING A SURGICAL INSTRUMENT ACCORDING TO SENSED CLOSURE PARAMETERS;
- U.S. Patent Application No. 16/024,067, titled SYSTEMS FOR ADJUSTING END EFFECTOR PARAMETERS BASED ON PERIOPERATIVE INFORMATION;
- U.S. Patent Application No. 16/024,075, titled SAFETY SYSTEMS FOR SMART POWERED SURGICAL STAPLING;
- U.S. Patent Application No. 16/024,083, titled SAFETY SYSTEMS FOR SMART POWERED SURGICAL STAPLING;
- U.S. Patent Application No. 16/024,094, titled SURGICAL SYSTEMS FOR DETECTING END EFFECTOR TISSUE DISTRIBUTION IRREGULARITIES;
- U. S. Patent Application No. 16/024,13 8, titled SYSTEMS FOR DETECTING PROXIMITY OF SURGICAL END EFFECTOR TO CANCEROUS TISSUE;
- U.S. Patent Application No. 16/024,150, titled SURGICAL INSTRUMENT CARTRIDGE SENSOR ASSEMBLIES;
- U.S. Patent Application No. 16/024,160, titled VARIABLE OUTPUT CARTRIDGE SENSOR ASSEMBLY;
- U.S. Patent Application No. 16/024,124, titled SURGICAL INSTRUMENT HAVING A FLEXIBLE ELECTRODE;
- U.S. Patent Application No. 16/024,132, titled SURGICAL INSTRUMENT HAVING A FLEXIBLE CIRCUIT;
- U.S. Patent Application No. 16/024,141, titled SURGICAL INSTRUMENT WITH A TISSUE MARKING ASSEMBLY;
- U.S. Patent Application No. 16/024,162, titled SURGICAL SYSTEMS WITH PRIORITIZED DATA TRANSMISSION CAPABILITIES;
- U.S. Patent Application No. 16/024,066, titled SURGICAL EVACUATION SENSING AND MOTOR CONTROL;
- U.S. Patent Application No. 16/024,096, titled SURGICAL EVACUATION SENSOR ARRANGEMENTS;
- U.S. Patent Application No. 16/024,116, titled SURGICAL EVACUATION FLOW PATHS;
- U.S. Patent Application No. 16/024,149, titled SURGICAL EVACUATION SENSING AND GENERATOR CONTROL;
- U.S. Patent Application No. 16/024,180, titled SURGICAL EVACUATION SENSING AND DISPLAY;
- U.S. Patent Application No. 16/024,245, titled COMMUNICATION OF SMOKE EVACUATION SYSTEM PARAMETERS TO HUB OR CLOUD IN SMOKE EVACUATION MODULE FOR INTERACTIVE SURGICAL PLATFORM;
- U.S. Patent Application No. 16/024,258, titled SMOKE EVACUATION SYSTEM INCLUDING A SEGMENTED CONTROL CIRCUIT FOR INTERACTIVE SURGICAL PLATFORM;
- U.S. Patent Application No. 16/024,265, titled SURGICAL EVACUATION SYSTEM WITH A COMMUNICATION CIRCUIT FOR COMMUNICATION BETWEEN A FILTER AND A SMOKE EVACUATION DEVICE; and
- U.S. Patent Application No. 16/024,273, titled DUAL IN-SERIES LARGE AND SMALL DROPLET FILTERS.

Applicant of the present application owns the following U.S. Patent Applications, filed on March 29, 2018, the disclosure of each of which is herein incorporated by reference in its entirety:
- U.S. Patent Application No. 15/940,641, titled INTERACTIVE SURGICAL SYSTEMS WITH ENCRYPTED COMMUNICATION CAPABILITIES;
- U.S. Patent Application No. 15/940,648, titled INTERACTIVE SURGICAL SYSTEMS WITH CONDITION HANDLING OF DEVICES AND DATA CAPABILITIES;
- U.S. Patent Application No. 15/940,656, titled SURGICAL HUB COORDINATION OF CONTROL AND COMMUNICATION OF OPERATING ROOM DEVICES;
- U.S. Patent Application No. 15/940,666, titled SPATIAL AWARENESS OF SURGICAL HUBS IN OPERATING ROOMS;
- U.S. Patent Application No. 15/940,670, titled COOPERATIVE UTILIZATION OF DATA DERIVED FROM SECONDARY SOURCES BY INTELLIGENT SURGICAL HUBS;
- U.S. Patent Application No. 15/940,677, titled SURGICAL HUB CONTROL ARRANGEMENTS;
- U.S. Patent Application No. 15/940,632, titled DATA STRIPPING METHOD TO INTERROGATE PATIENT RECORDS AND CREATE ANONYMIZED RECORD;
- U.S. Patent Application No. 15/940,640, titled COMMUNICATION HUB AND STORAGE DEVICE FOR STORING PARAMETERS AND STATUS OF A SURGICAL DEVICE TO BE SHARED WITH CLOUD BASED ANALYTICS SYSTEMS;
- U.S. Patent Application No. 15/940,645, titled SELF DESCRIBING DATA PACKETS GENERATED AT AN ISSUING INSTRUMENT;
- U.S. Patent Application No. 15/940,649, titled DATA PAIRING TO INTERCONNECT A DEVICE MEASURED PARAMETER WITH AN OUTCOME;
- U.S. Patent Application No. 15/940,654, titled SURGICAL HUB SITUATIONAL AWARENESS;
- U.S. Patent Application No. 15/940,663, titled SURGICAL SYSTEM DISTRIBUTED PROCESSING;
- U.S. Patent Application No. 15/940,668, titled AGGREGATION AND REPORTING OF SURGICAL HUB DATA;
- U.S. Patent Application No. 15/940,671, titled SURGICAL HUB SPATIAL AWARENESS TO DETERMINE DEVICES IN OPERATING THEATER;
- U.S. Patent Application No. 15/940,686, titled DISPLAY OF ALIGNMENT OF STAPLE CARTRIDGE TO PRIOR LINEAR STAPLE LINE;
- U.S. Patent Application No. 15/940,700, titled STERILE FIELD INTERACTIVE CONTROL DISPLAYS;
- U.S. Patent Application No. 15/940,629, titled COMPUTER IMPLEMENTED INTERACTIVE SURGICAL SYSTEMS;
- U.S. Patent Application No. 15/940,704, titled USE OF LASER LIGHT AND RED-GREEN-BLUE COLORATION TO DETERMINE PROPERTIES OF BACK SCATTERED LIGHT;
- U.S. Patent Application No. 15/940,722, titled CHARACTERIZATION OF TISSUE IRREGULARITIES THROUGH THE USE OF MONO-CHROMATIC LIGHT REFRACTIVITY;
- U.S. Patent Application No. 15/940,742, titled DUAL CMOS ARRAY IMAGING;
- U.S. Patent Application No. 15/940,636, titled ADAPTIVE CONTROL PROGRAM UPDATES FOR SURGICAL DEVICES;
- U.S. Patent Application No. 15/940,653, titled ADAPTIVE CONTROL PROGRAM UPDATES FOR SURGICAL HUBS;
- U.S. Patent Application No. 15/940,660, titled CLOUD-BASED MEDICAL ANALYTICS FOR CUSTOMIZATION AND RECOMMENDATIONS TO A USER;
- U.S. Patent Application No. 15/940,679, titled CLOUD-BASED MEDICAL ANALYTICS FOR LINKING OF LOCAL USAGE TRENDS WITH THE RESOURCE ACQUISITION BEHAVIORS OF LARGER DATA SET;
- U.S. Patent Application No. 15/940,694, titled CLOUD-BASED MEDICAL ANALYTICS FOR MEDICAL FACILITY SEGMENTED INDIVIDUALIZATION OF INSTRUMENT FUNCTION;
- U.S. Patent Application No. 15/940,634, titled CLOUD-BASED MEDICAL ANALYTICS FOR SECURITY AND AUTHENTICATION TRENDS AND REACTIVE MEASURES;
- U.S. Patent Application No. 15/940,706, titled DATA HANDLING AND PRIORITIZATION IN A CLOUD ANALYTICS NETWORK;
- U.S. Patent Application No. 15/940,675, titled CLOUD INTERFACE FOR COUPLED SURGICAL DEVICES;
- U.S. Patent Application No. 15/940,627, titled DRIVE ARRANGEMENTS FOR ROBOT-ASSISTED SURGICAL PLATFORMS;
- U.S. Patent Application No. 15/940,637, titled COMMUNICATION ARRANGEMENTS FOR ROBOT-ASSISTED SURGICAL PLATFORMS;
- U.S. Patent Application No. 15/940,642, titled CONTROLS FOR ROBOT-ASSISTED SURGICAL PLATFORMS;
- U.S. Patent Application No. 15/940,676, titled AUTOMATIC TOOL ADJUSTMENTS FOR ROBOT-ASSISTED SURGICAL PLATFORMS;
- U.S. Patent Application No. 15/940,680, titled CONTROLLERS FOR ROBOT-ASSISTED SURGICAL PLATFORMS;
- U.S. Patent Application No. 15/940,683, titled COOPERATIVE SURGICAL ACTIONS FOR ROBOT-ASSISTED SURGICAL PLATFORMS;
- U.S. Patent Application No. 15/940,690, titled DISPLAY ARRANGEMENTS FOR ROBOT-ASSISTED SURGICAL PLATFORMS; and
- U.S. Patent Application No. 15/940,711, titled SENSING ARRANGEMENTS FOR ROBOT-ASSISTED SURGICAL PLATFORMS.

Applicant of the present application owns the following U.S. Provisional Patent Applications, filed on March 8, 2018, the disclosure of each of which is herein incorporated by reference in its entirety:
- U.S. Provisional Patent Application No. 62/640,417, titled TEMPERATURE CONTROL IN ULTRASONIC DEVICE AND CONTROL SYSTEM THEREFOR; and
- U.S. Provisional Patent Application No. 62/640,415, titled ESTIMATING STATE OF ULTRASONIC END EFFECTOR AND CONTROL SYSTEM THEREFOR.

Before explaining various aspects of surgical devices and generators in detail, it should be noted that the illustrative examples are not limited in application or use to the details of construction and arrangement of parts illustrated in the accompanying drawings and description. The illustrative examples may be implemented or incorporated in other aspects, variations and modifications, and may be practiced or carried out in various ways. Further, unless otherwise indicated, the terms and expressions employed herein have been chosen for the purpose of describing the illustrative examples for the convenience of the reader and are not for the purpose of limitation thereof. Also, it will be appreciated that one or more of the following-described aspects, expressions of aspects, and/or examples, can be combined with any one or more of the other following-described aspects, expressions of aspects and/or examples.

### Energy Devices and Smoke Evacuation

The present disclosure relates to energy devices and intelligent surgical evacuation systems for evacuating smoke and/or other fluids and/or particulates from a surgical site. Smoke is often generated during a surgical procedure that utilizes one or more energy devices. Energy devices use energy to affect tissue. In an energy device, the energy is supplied by a generator. Energy devices include devices with tissue-contacting electrodes, such as an electrosurgical device having one or more radio frequency (RF) electrodes, and devices with vibrating surfaces, such as an ultrasonic device having an ultrasonic blade. For an electrosurgical device, a generator is configured to generate oscillating electric currents to energize the electrodes. For an ultrasonic device, a generator is configured to generate ultrasonic vibrations to energize the ultrasonic blade. Generators are further described herein.

Ultrasonic energy can be utilized for coagulation and cutting tissue. Ultrasonic energy coagulates and cuts tissue by vibrating an energy-delivery surface (e.g. an ultrasonic blade) in contact with tissue. The ultrasonic blade can be coupled to a waveguide that transmits the vibrational energy from an ultrasonic transducer, which generates mechanical vibrations and is powered by a generator. Vibrating at high frequencies (e.g., 55,500 times per second), the ultrasonic blade generates friction and heat between the blade and the tissue, i.e. at the blade-tissue interface, which denatures the proteins in the tissue to form a sticky coagulum. Pressure exerted on the tissue by the blade surface collapses blood vessels and allows the coagulum to form a hemostatic seal. The precision of cutting and coagulation can be controlled by the clinician's technique and by adjusting the power level, blade edge, tissue traction, and blade pressure, for example.

Ultrasonic surgical instruments are finding increasingly widespread applications in surgical procedures by virtue of the unique performance characteristics of such instruments. Depending upon specific instrument configurations and operational parameters, ultrasonic surgical instruments can provide substantially simultaneous cutting of tissue and hemostasis by coagulation, which can desirably minimize patient trauma. The cutting action is typically realized by an end effector, or blade tip, at the distal end of the ultrasonic instrument. The ultrasonic end effector transmits the ultrasonic energy to tissue brought into contact with the end effector. Ultrasonic instruments of this nature can be configured for open surgical use, laparoscopic surgical procedures, or endoscopic surgical procedures, including robotic-assisted procedures, for example.

Electrical energy can also be utilized for coagulation and/or cutting. An electrosurgical device typically includes a handpiece and an instrument having a distally-mounted end effector (e.g., one or more electrodes). The end effector can be positioned against and/or adjacent to the tissue such that electrical current is introduced into the tissue. Electrosurgery is widely-used and offers many advantages including the use of a single surgical instrument for both coagulation and cutting.

The electrode or tip of the electrosurgical device is small at the point of contact with the patient to produce an RF current with a high current density in order to produce a surgical effect of coagulating and/or cutting tissue through cauterization. The return electrode carries the same RF signal back to the electrosurgical generator after it passes through the patient, thus providing a return path for the RF signal.

Electrosurgical devices can be configured for bipolar or monopolar operation. During bipolar operation, current is introduced into and returned from the tissue by active and return electrodes, respectively, of the end effector. During monopolar operation, current is introduced into the tissue by an active electrode of the end effector and returned through a return electrode (e.g., a grounding pad) separately located on or against a patient's body. Heat generated by the current flowing through the tissue may form hemostatic seals within the tissue and/or between tissues and, thus, may be particularly useful for sealing blood vessels, for example. The end effector of an electrosurgical device also may include a cutting member that is movable relative to the tissue and the electrodes to transect the tissue.

In application, an electrosurgical device can transmit low frequency RF current through tissue, which causes ionic agitation, or friction (in effect resistive heating), thereby increasing the temperature of the tissue. Because a boundary is created between the affected tissue and the surrounding tissue, clinicians can operate with a high level of precision and control, without sacrificing un-targeted adjacent tissue. The low operating temperature of RF energy is useful for removing, shrinking, or sculpting soft tissue while simultaneously sealing blood vessels. RF energy can work particularly well on connective tissue, which is primarily comprised of collagen and shrinks when contacted by heat. Other electrosurgical instruments include, without limitation, irreversible and/or reversible electroporation, and/or microwave technologies, among others. The techniques disclosed herein are applicable to ultrasonic, bipolar and/or monopolar RF (electrosurgical), irreversible and/or reversible electroporation, and/or microwave based surgical instruments, among others.

Electrical energy applied by an electrosurgical device can be transmitted to the instrument from a generator. The generator is configured to convert electricity to high frequency waveforms comprised of oscillating electric currents, which are transmitted to the electrodes to affect tissue. The current passes through tissue to fulgurate (a form of coagulation in which a current arc over the tissue creates tissue charring), desiccate (a direct energy application that drives water of the cells), and/or cut (an indirect energy application that vaporizes cellular fluid causing cellular explosions) tissue. The tissue's response to the current is a function of the resistance of the tissue, the current density passing through the tissue, the power output, and the duration of current application. In certain instances, as further described herein, the current waveform can be adjusted to affect a different surgical function and/or accommodate tissue of different properties. For example, different types of tissue-vascular tissue, nerve tissue, muscles, skin, fat and/or bone-can respond differently to the same waveform.

The electrical energy may be in the form of RF energy that may be in a frequency range described in EN 60601-2-2:2009+A11:2011, Definition 201.3.218-HIGH FREQUENCY. For example, the frequencies in monopolar RF applications are typically restricted to less than 5 MHz to minimize the problems associated with high frequency leakage current. Frequencies above 200 kHz can be typically used for monopolar applications in order to avoid the unwanted stimulation of nerves and muscles that would result from the use of low frequency current.

In bipolar RF applications, the frequency can be almost anything. Lower frequencies may be used for bipolar techniques in certain instances, such as if a risk analysis shows that the possibility of neuromuscular stimulation has been mitigated to an acceptable level. It is generally recognized that 10 mA is the lower threshold of thermal effects on tissue. Higher frequencies may also be used in the case of bipolar techniques.

In certain instances, a generator can be configured to generate an output waveform digitally and provide it to a surgical device such that the surgical device may utilize the waveform for various tissue effects. The generator can be a monopolar generator, a bipolar generator, and/or an ultrasonic generator. For example, a single generator can supply energy to a monopolar device, a bipolar device, an ultrasonic device, or a combination electrosurgery/ultrasonic device. The generator can promote tissue-specific effects via wave-shaping, and/or can drive RF and ultrasonic energy simultaneously and/or sequentially to a single surgical instrument or multiple surgical instruments.

In one instance, a surgical system can include a generator and various surgical instruments usable therewith, including an ultrasonic surgical instrument, an RF electrosurgical instrument, and a combination ultrasonic/RF electrosurgical instrument. The generator can be configurable for use with the various surgical instruments as further described in U.S. Patent Application No. 15/265,279, titled TECHNIQUES FOR OPERATING GENERATOR FOR DIGITALLY GENERATING ELECTRICAL SIGNAL WAVEFORMS AND SURGICAL INSTRUMENTS, filed September 14, 2016, now U.S. Patent Application Publication No. 2017/0086914, which is herein incorporated by reference in its entirety.

As described herein, medical procedures of cutting tissue and/or cauterizing blood vessels are often performed by utilizing RF electrical energy, which is produced by a generator and transmitted to a patient's tissue through an electrode that is operated by a clinician. The electrode delivers an electrical discharge to cellular matter of the patient's body adjacent to the electrode. The discharge causes the cellular matter to heat up in order to cut tissue and/or cauterize blood vessels.

The high temperatures involved in electrosurgery can cause thermal necrosis of the tissue adjacent to the electrode. The longer time at which tissue is exposed to the high temperatures involved with electrosurgery, the more likely it is that the tissue will suffer thermal necrosis. In certain instances, thermal necrosis of the tissue can decrease the speed of cutting the tissue and increase post-operative complications, eschar production, and healing time, as well as increasing incidences of heat damage to the tissue positioned away from the cutting site.

The concentration of the RF energy discharge affects both the efficiency with which the electrode is able to cut tissue and the likelihood of tissue damage away from the cutting site. With a standard electrode geometry, the RF energy tends to be uniformly distributed over a relatively large area adjacent to the intended incision site. A generally uniform distribution of the RF energy discharge increases the likelihood of extraneous charge loss into the surrounding tissue, which may increase the likelihood of unwanted tissue damage in the surrounding tissue.

Typical electrosurgical generators generate various operating frequencies of RF electrical energy and output power levels. The specific operating frequency and power output of a generator varies based upon the particular electrosurgical generator used and the needs of the physician during the electrosurgical procedure. The specific operating frequency and power output levels can be manually adjusted on the generator by a clinician or other operating room personnel. Properly adjusting these various settings requires great knowledge, skill, and attention from the clinician or other personnel. Once the clinician has made the desired adjustments to the various settings on the generator, the generator can maintain those output parameters during electrosurgery. Generally, wave generators used for electrosurgery are adapted to produce RF waves with an output power in the range of 1-300 W in a cut mode and 1-120 W in coagulation mode, and a frequency in the range of 300-600 kHz. Typical wave generators are adapted to maintain the selected settings during the electrosurgery. For example, if the clinician were to set the output power level of the generator to 50 W and then touch the electrode to the patient to perform electrosurgery, the power level of the generator would quickly rise to and be maintained at 50 W. While setting the power level to a specific setting, such as 50 W, will allow the clinician to cut through the patient's tissue, maintaining such a high power level increases the likelihood of thermal necrosis of the patient's tissue.

In some forms, a generator is configured to provide sufficient power to effectively perform electrosurgery in connection with an electrode that increases the concentration of the RF energy discharge, while at the same time limiting unwanted tissue damage, reducing post-operative complications, and facilitating quicker healing. For example, the waveform from the generator can be optimized by a control circuit throughout the surgical procedure. The subject matter claimed herein, however, is not limited to aspects that solve any disadvantages or that operate only in environments such as those described above. Rather, this background is only provided to illustrate one example of a technology area where some aspects described herein may be practiced.

As provided herein, energy devices delivery mechanical and/or electrical energy to target tissue in order to treat the tissue (e.g. to cut the tissue, cauterize blood vessels and/or coagulate the tissue within and/or near the targeted tissue). The cutting, cauterization, and/or coagulation of tissue can result in fluids and/or particulates being released into the air. Such fluids and/or particulates emitted during a surgical procedure can constitute smoke, for example, which can comprise carbon particles and/or other particles suspended in air. In other words, a fluid can comprise smoke and/or other fluidic matter. Approximately 90% of endoscopic and open surgical procedures generate some level of smoke. The smoke can be unpleasant to the olfactory senses of the clinician(s), the assistant(s), and/or the patient(s), may obstruct the clinician(s)'s view of the surgical site, and may be unhealthy to inhale in certain instances. For example, smoke generated during an electrosurgical procedure can contain toxic chemicals including acrolein, acetonitrile, acrylonitrile, acetylene, alkyl benzenes, benzene, butadiene, butene, carbon monoxide, creosols, ethane, ethylene, formaldehyde, free radicals, hydrogen cyanide, isobutene, methane, phenol, polycyclic aromatic hydrocarbons, propene, propylene, pyridene, pyrrole, styrene, toluene, and xylene, as well as dead and live cellular material (including blood fragments), and viruses. Certain material that has been identified in surgical smoke has been identified as known carcinogens. It is estimated that one gram of tissue cauterized during an electrosurgical procedure can be equivalent to the toxins and carcinogens of six unfiltered cigarettes. Additionally, exposure to the smoke released during an electrosurgical procedure has been reported to cause eye and lung irritation to health care workers.

In addition to the toxicity and odors associated with the material in surgical smoke, the size of particulate matter in surgical smoke can be harmful to the respiratory system of the clinician(s), the assistant(s), and/or the patient(s). In certain instances, the particulates can be extremely small. Repeated inhalation of extremely small particulate matter can lead to acute and chronic respiratory conditions in certain instances.

Many electrosurgical systems employ a surgical evacuation system that captures the resultant smoke from a surgical procedure, and directs the captured smoke through a filter and an exhaust port away from the clinician(s) and/or from the patient(s). For example, an evacuation system can be configured to evacuate smoke that is generated during an electrosurgical procedure. The reader will appreciate that such an evacuation system can be referred to as a "smoke evacuation system" though such evacuation systems can be configured to evacuate more than just smoke from a surgical site. Throughout the present disclosure, the "smoke" evacuated by an evacuation system is not limited to just smoke. Rather, the smoke evacuation systems disclosed herein can be used to evacuate a variety of fluids, including liquids, gases, vapors, smoke, steam, or combinations thereon. The fluids can be biologic in origin and/or can be introduced to the surgical site from an external source during a procedure. The fluids can include water, saline, lymph, blood, exudate, and/or pyogenic discharge, for example. Moreover, the fluids can include particulates or other matter (e.g. cellular matter or debris) that is evacuated by the evacuation system. For example, such particulates can be suspended in the fluid.

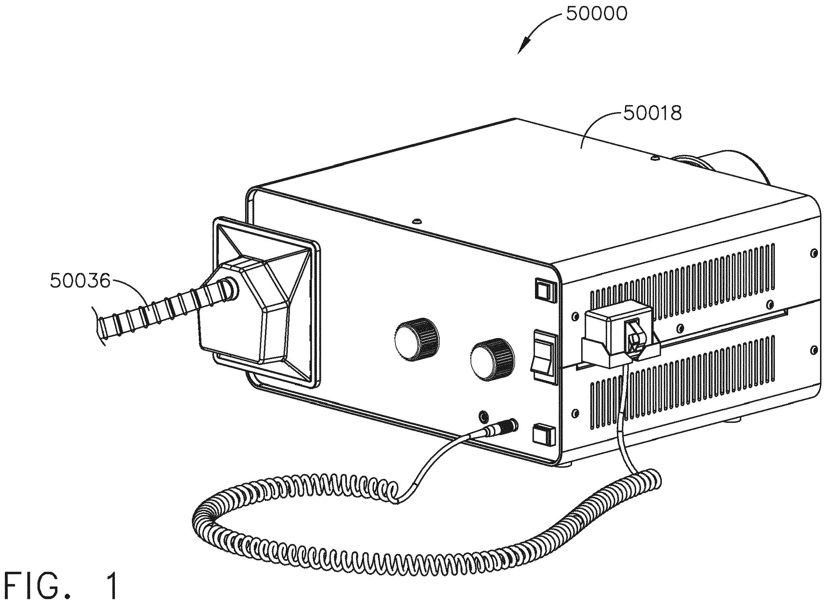

Evacuation systems often include a pump and a filter. The pump creates suction that draws the smoke into the filter. For example, suction can be configured to draw smoke from the surgical site into a conduit opening, through an evacuation conduit, and into an evacuator housing of the evacuation system. An evacuator housing 50018 for a surgical evacuation system 50000 is shown in FIG. 1. In one aspect of the present disclosure, a pump and a filter are positioned within the evacuator housing 50018. Smoke drawn into the evacuator housing 50018 travels to the filter via a suction conduit 50036, and harmful toxins and offensive smells are filtered out of the smoke as it moves through the filter. The suction conduit can also be referred to as vacuum and/or evacuation conduit and/or tube, for example. Filtered air may then exit the surgical evacuation system as exhaust. In certain instances, various evacuation systems disclosed herein can also be configured to deliver fluids to a desired location, such as a surgical site.

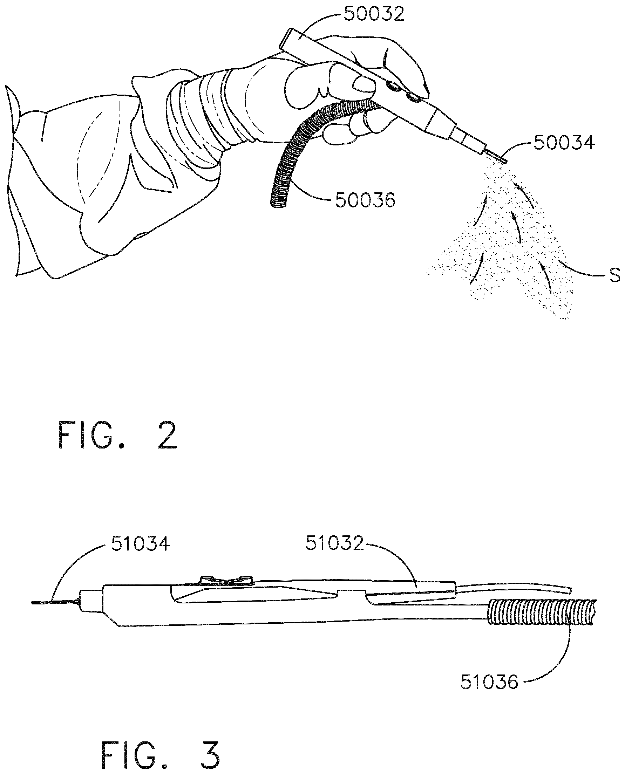

Referring now to FIG. 2, the suction conduit 50036 from the evacuator housing 50018 (FIG. 1) may terminate at a hand piece, such as the handpiece 50032. The handpiece 50032 comprises an electrosurgical instrument that includes an electrode tip 50034 and an evacuation conduit opening near and/or adjacent to the electrode tip 50034. The evacuation conduit opening is configured to capture the fluid and/or particulates that are released during a surgical procedure. In such an instance, the evacuation system 50000 is integrated into the electrosurgical instrument 50032. Referring still to FIG. 2, smoke S is being pulled into the suction conduit 50036.

In certain instances, the evacuation system 50000 can include a separate surgical tool that comprises a conduit opening and is configured to suck the smoke out into the system. In still other instances, a tool comprising the evacuation conduit and opening can be snap fit onto an electrosurgical tool as depicted in FIG. 3. For example, a portion of a suction conduit 51036 can be positioned around (or adjacent to) an electrode tip 51034. In one instance, the suction conduit 51036 can be releasably secured to a handpiece 51032 of an electrosurgical tool comprising the electrode tip 51034 with clips or other fasteners.

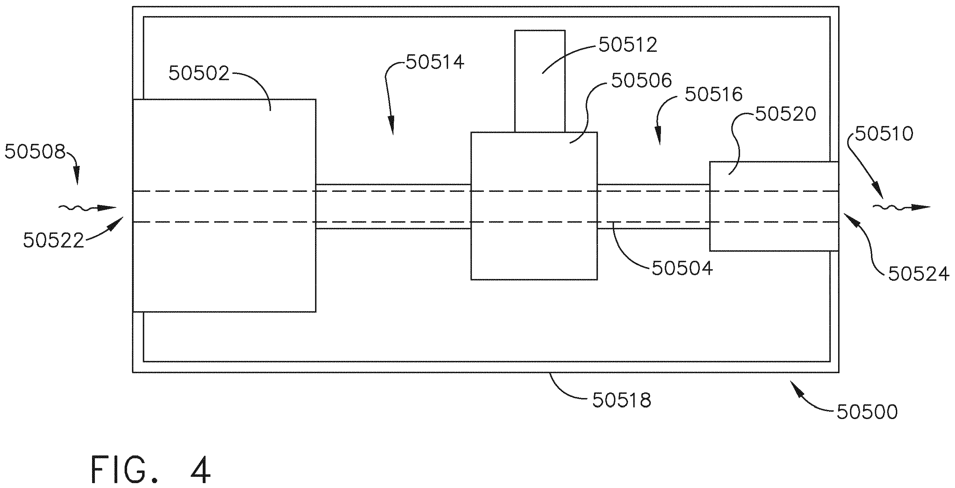

Various internal components of an evacuator housing 50518 are shown in FIG. 4. In various instances, the internal components in FIG. 4 can also be incorporated into the evacuator housing 50018 of FIG. 1. Referring primarily to FIG. 4, an evacuation system 50500 includes the evacuator housing 50518, a filter 50502, an exhaust mechanism 50520, and a pump 50506. The evacuation system 50500 defines a flow path 50504 through the evacuator housing 50518 having an inlet port 50522 and an outlet port 50524. The filter 50502, the exhaust mechanism 50520, and the pump 50506 are sequentially arranged in-line with the flow path 50504 through the evacuator housing 50518 between the inlet port 50522 and the outlet port 50524. The inlet port 50522 can be fluidically coupled to a suction conduit, such as the suction conduit 50036 in FIG. 1, for example, which can comprise a distal conduit opening positionable at the surgical site.

The pump 50506 is configured to produce a pressure differential in the flow path 50504 by a mechanical action. The pressure differential is configured to draw smoke 50508 from the surgical site into the inlet port 50522 and along the flow path 50504. After the smoke 50508 has moved through the filter 50502, the smoke 50508 can be considered to be filtered smoke, or air, 50510, which can continue through the flow path 50504 and is expelled through the outlet port 50524. The flow path 50504 includes a first zone 50514 and a second zone 50516. The first zone 50514 is upstream from the pump 50506; the second zone 50516 is downstream from the pump 50506. The pump 50506 is configured to pressurize the fluid in the flow path 50504 so that the fluid in the second zone 50516 has a higher pressure than the fluid in the first zone 50514. A motor 50512 drives the pump 50506. Various suitable motors are further described herein. The exhaust mechanism 50520 is a mechanism that can control the velocity, the direction, and/or other properties of the filtered smoke 50510 exiting the evacuation system 50500 at the outlet port 50524.

The flow path 50504 through the evacuation system 50500 can be comprised of a tube or other conduit that substantially contains and/or isolates the fluid moving through the flow path 50504 from the fluid outside the flow path 50504. For example, the first zone 50514 ofthe flow path 50504 can comprise a tube through which the flow path 50504 extends between the filter 50502 and the pump 50506. The second zone 50516 of the flow path 50504 can also comprise a tube through which the flow path 50504 extends between the pump 50506 and the exhaust mechanism 50520. The flow path 50504 also extends through the filter 50502, the pump 50506, and the exhaust mechanism 50520 so that the flow path 50504 extends continuously from the inlet port 50522 to the outlet port 50524.

In operation, the smoke 50508 can flow into the filter 50502 at the inlet port 50522 and can be pumped through the flow path 50504 by the pump 50506 such that the smoke 50508 is drawn into the filter 50502. The filtered smoke 50510 can then be pumped through the exhaust mechanism 50520 and out the outlet port 50524 of the evacuation system 50500. The filtered smoke 50510 exiting the evacuation system 50500 at the outlet port 50524 is the exhaust, and can consist of filtered gases that have passed through the evacuation system 50500.

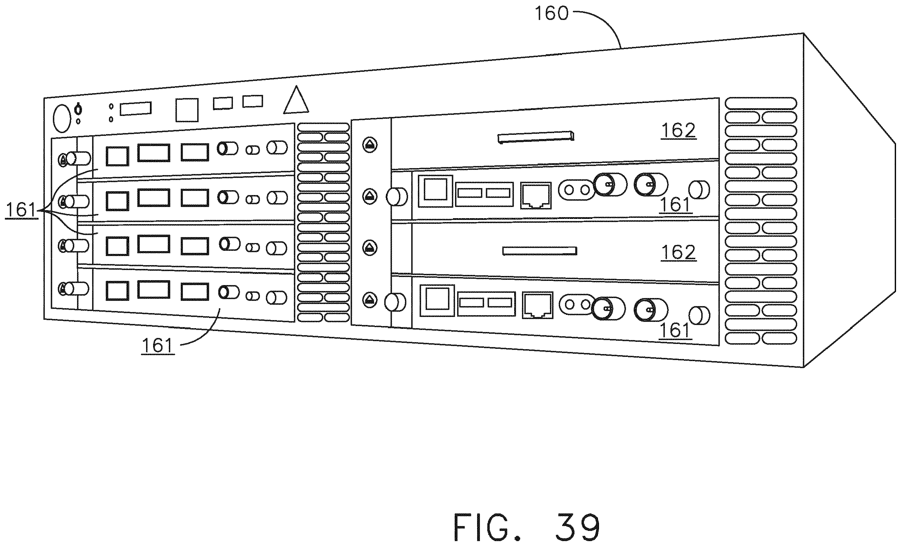

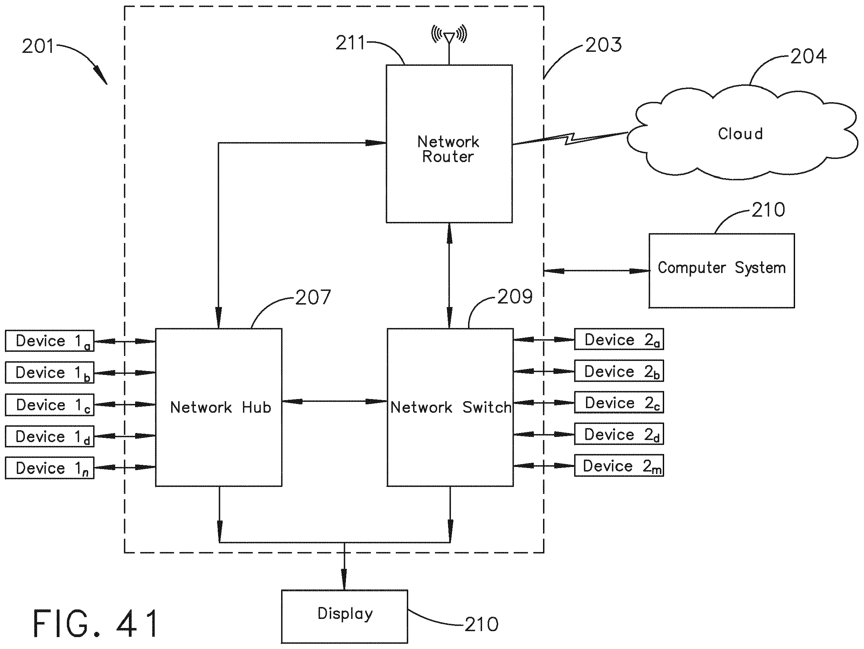

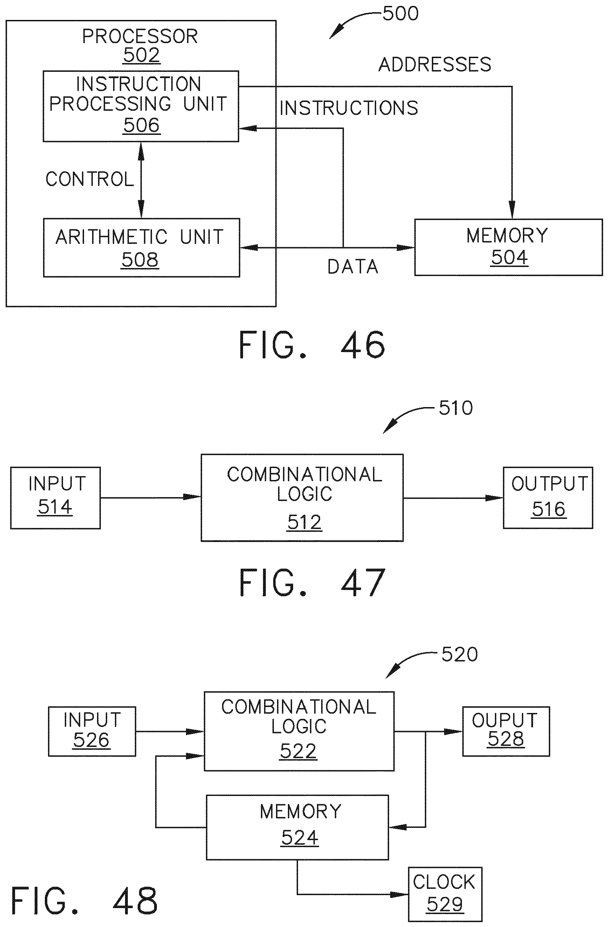

In various instances, the evacuation systems disclosed herein (e.g. the evacuation system 50000 and the evacuation system 50500) can be incorporated into a computer-implemented interactive surgical system, such as the system 100 (FIG. 39) or the system 200 (FIG. 47), for example. In one aspect of the present disclosure, for example, the computer-implemented surgical system 100 can include at least one hub 106 and a cloud 104. Referring primarily to FIG. 41, the hub 106 includes a smoke evacuation module 126. Operation of the smoke evacuation module 126 can be controlled by the hub 106 based on its situational awareness and/or feedback from the components thereof and/or based on information from the cloud 104. The computer-implemented surgical systems 100 and 200, as well as situational awareness therefor, are further described herein.

Situational awareness encompasses the ability of some aspects of a surgical system to determine or infer information related to a surgical procedure from data received from databases and/or instruments. The information can include the type of procedure being undertaken, the type of tissue being operated on, or the body cavity that is the subject of the procedure. With the contextual information related to the surgical procedure, the surgical system can, for example, improve the manner in which it controls the modular devices (e.g. a smoke evacuation system) that are connected to it and provide contextualized information or suggestions to the clinician during the course of the surgical procedure. Situational awareness is further described herein and in U.S. Provisional Patent Application Serial No. 62/611,341, entitled INTERACTIVE SURGICAL PLATFORM, filed December 28, 2017, which is incorporated by reference herein in its entirety.

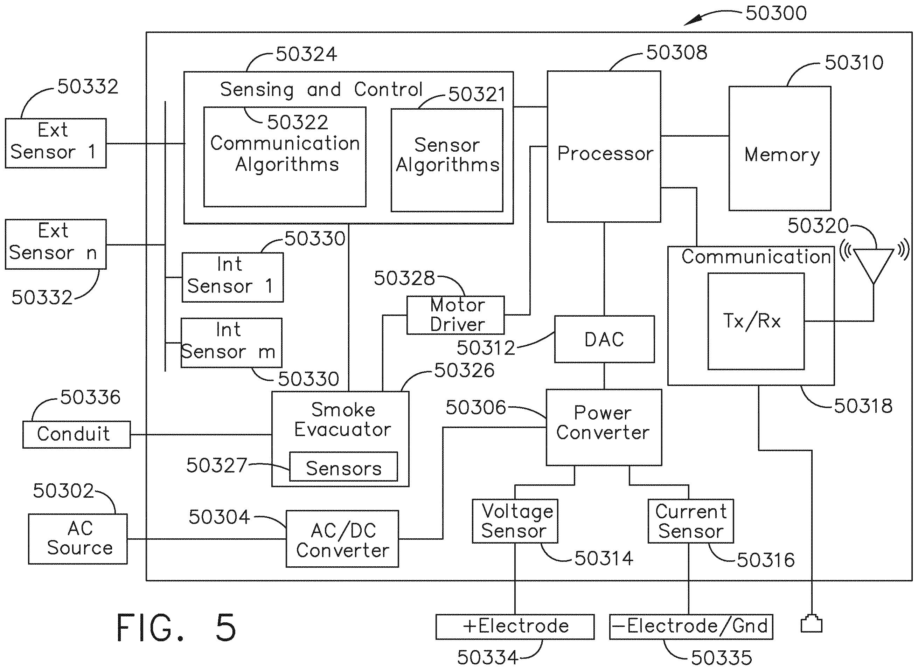

In various instances, the surgical systems and/or evacuation systems disclosed herein can include a processor. The processor can be programmed to control one or more operational parameters of the surgical system and/or the evacuation system based on sensed and/or aggregated data and/or one or more user inputs, for example. FIG. 5 is a schematic representation of an electrosurgical system 50300 including a processor 50308. The electrosurgical system 50300 is powered by an AC source 50302, which provides either 120 V or 240 V alternating current. The voltage supplied by the AC source 50302 is directed to an AC/DC converter 50304, which converts the 120 V or 240 V of alternating current to 360 V of direct current. The 360 V of direct current is then directed to a power converter 50306 (e.g., a buck converter). The power converter 50306 is a step-down DC to DC converter. The power converter 50306 is adapted to step-down the incoming 360 V to a desired level within a range between 0-150 V.

The processor 50308 can be programmed to regulate various aspects, functions, and parameters of the electrosurgical system 50300. For instance, the processor 50308 can determine the desired output power level at an electrode tip 50334, which can be similar in many respects to the electrode tip 50034 in FIG. 2 and/or the electrode tip 51034 in FIG. 3, for example, and direct the power converter 50306 to step-down the voltage to a specified level so as to provide the desired output power. The processor 50308 is coupled to a memory 50310 configured to store machine executable instructions to operate the electrosurgical system 50300 and/or subsystems thereof.

Connected between the processor 50308 and the power converter 50306 is a digital-to-analog converter ("DAC") 50312. The DAC 50312 is adapted to convert a digital code created by the processor 50308 to an analog signal (current, voltage, or electric charge) which governs the voltage step-down performed by the power converter 50306. Once the power converter 50306 steps-down the 360 V to a level that the processor 50308 has determined will provide the desired output power level, the stepped-down voltage is directed to the electrode tip 50334 to effectuate electrosurgical treatment of a patient's tissue and is then directed to a return or ground electrode 50335. A voltage sensor 50314 and a current sensor 50316 are adapted to detect the voltage and current present in the electrosurgical circuit and communicate the detected parameters to the processor 50308 so that the processor 50308 can determine whether to adjust the output power level. As noted herein, typical wave generators are adapted to maintain the selected settings throughout an electrosurgical procedure. In other instances, the operational parameters of a generator can be optimized during a surgical procedure based on one or more inputs to the processor 5308, such as inputs from a surgical hub, cloud, and/or situational awareness module, for example, as further described herein.

The processor 50308 is coupled to a communication device 50318 to communicate over a network. The communication device includes a transceiver 50320 configured to communicate over physical wires or wirelessly. The communication device 50318 may further include one or more additional transceivers. The transceivers may include, but are not limited to cellular modems, wireless mesh network transceivers, Wi-Fi® transceivers, low power wide area (LPWA) transceivers, and/or near field communications transceivers (NFC). The communication device 50318 may include or may be configured to communicate with a mobile telephone, a sensor system (e.g., environmental, position, motion, etc.) and/or a sensor network (wired and/or wireless), a computing system (e.g., a server, a workstation computer, a desktop computer, a laptop computer, a tablet computer (e.g., iPad®, GalaxyTab® and the like), an ultraportable computer, an ultramobile computer, a netbook computer and/or a subnotebook computer; etc. In at least one aspect of the present disclosure, one of the devices may be a coordinator node.

The transceivers 50320 may be configured to receive serial transmit data via respective UARTs from the processor 50308, to modulate the serial transmit data onto an RF carrier to produce a transmit RF signal and to transmit the transmit RF signal via respective antennas. The transceiver(s) are further configured to receive a receive RF signal via respective antennas that includes an RF carrier modulated with serial receive data, to demodulate the receive RF signal to extract the serial receive data and to provide the serial receive data to respective UARTs for provision to the processor. Each RF signal has an associated carrier frequency and an associated channel bandwidth. The channel bandwidth is associated with the carrier frequency, the transmit data and/or the receive data. Each RF carrier frequency and channel bandwidth are related to the operating frequency range(s) of the transceiver(s) 50320. Each channel bandwidth is further related to the wireless communication standard and/or protocol with which the transceiver(s) 50320 may comply. In other words, each transceiver 50320 may correspond to an implementation of a selected wireless communication standard and/or protocol, e.g., IEEE 802.11 a/b/g/n for Wi-Fi® and/or IEEE 802.15.4 for wireless mesh networks using Zigbee routing.

The processor 50308 is coupled to a sensing and intelligent controls device 50324 that is coupled to a smoke evacuator 50326. The smoke evacuator 50326 can include one or more sensors 50327, and can also include a pump and a pump motor controlled by a motor driver 50328. The motor driver 50328 is communicatively coupled to the processor 50308 and a pump motor in the smoke evacuator 50326. The sensing and intelligent controls device 50324 includes sensor algorithms 50321 and communication algorithms 50322 that facilitate communication between the smoke evacuator 50326 and other devices to adapt their control programs. The sensing and intelligent controls device 50324 is configured to evaluate extracted fluids, particulates, and gases via an evacuation conduit 50336 to improve smoke extraction efficiency and/or reduce device smoke output, for example, as further described herein. In certain instances, the sensing and intelligent controls device 50324 is communicatively coupled to one or more sensors 50327 in the smoke evacuator 50326, one or more internal sensors 50330 and/or one or more external sensors 50332 of the electrosurgical system 50300.

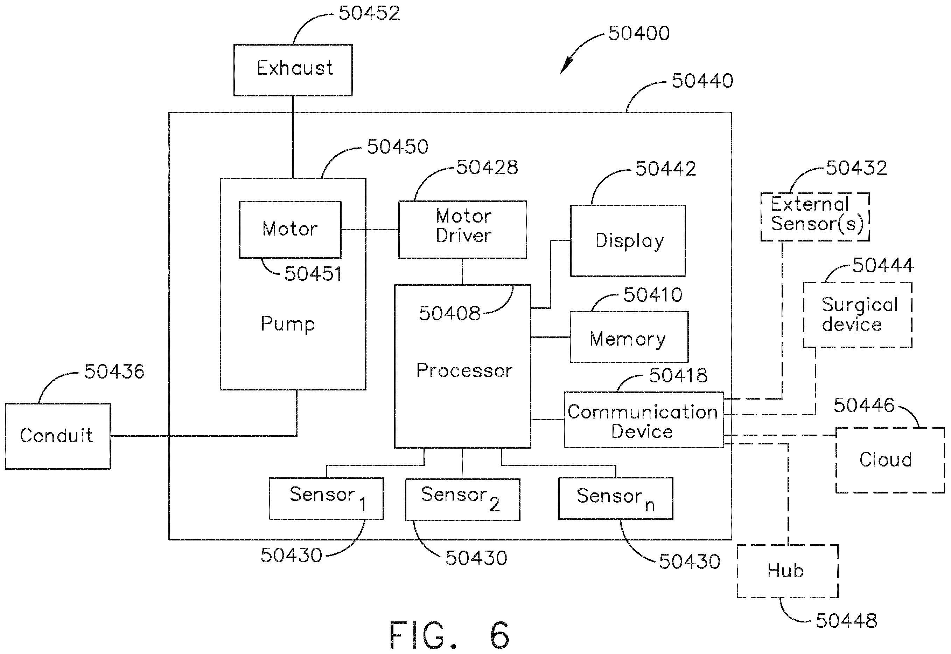

In certain instances, a processor can be located within an evacuator housing of a surgical evacuation system. For example, referring to FIG. 6, a processor 50408 and a memory 50410 therefor are positioned within an evacuator housing 50440 of a surgical evacuation system 50400. The processor 50408 is in signal communication with a motor driver 50428, various internal sensors 50430, a display 50442, the memory 50410, and a communication device 50418. The communication device 50418 is similar in many respects to the communication device 50318 described above with respect to FIG. 5. The communication device 50418 can allow the processor 50408 in the surgical evacuation system 50400 to communicate with other devices within a surgical system. For example, the communication device 50418 can allow wired and/or wireless communication to one or more external sensors 50432, one or more surgical devices 50444, one or more hubs 50448, one or more clouds 50446, and/or one or more additional surgical systems and/or tools. The reader will readily appreciate that the surgical evacuation system 50400 of FIG. 6 can be incorporated into the electrosurgical system 50300 of FIG. 5 in certain instances. The surgical evacuation system 50400 also includes a pump 50450, including a pump motor 50451 thereof, an evacuation conduit 50436, and an exhaust 50452. Various pumps, evacuation conduits and exhausts are further described herein. The surgical evacuation system 50400 can also include a sensing and intelligent controls device, which can be similar in many respects to the sensing and intelligent controls device 50324, for example. For example, such a sensing and intelligent controls device can be in signal communication with the processor 50408 and/or one or more of the sensors 50430 and/or external sensors 50432.

The electrosurgical system 50300 (FIG. 5) and/or the surgical evacuation system 50400 (FIG. 6) can be programmed to monitor one or more parameters of a surgical system and can affect a surgical function based on one or more algorithms stored in a memory in signal communication with the processor 50308 and/or 50408. Various exemplary aspects disclosed herein can be implemented by such algorithms, for example.

In one aspect of the present disclosure, a processor and sensor system, such as the processors 50308 and 50408 and respective sensor systems in communication therewith (FIGS. 5 and 6), are configured to sense the airflow through a vacuum source in order to adjust parameters of the smoke evacuation system and/or external devices and/or systems that are used in tandem with the smoke evacuation system, such as an electrosurgical system, energy device, and/or generator, for example. In one aspect of the present disclosure, the sensor system may include multiple sensors positioned along the airflow path of the surgical evacuation system. The sensors can measure a pressure differential within the evacuation system, in order to detect a state or status of the system between the sensors. For example, the system between two sensors can be a filter, and the pressure differential can be used to increase the speed of the pump motor as flow through the filter is reduced, in order to maintain a flow rate through the system. As another example, the system can be a fluid trap of the evacuation system, and the pressure differential can be used to determine an airflow path through the evacuation system. In still another example, the system can be the inlet and outlet (or exhaust) of the evacuation system, and the pressure differential can be used to determine the maximum suction load in the evacuation system in order to maintain the maximum suction load below a threshold value.

In one aspect of the present disclosure, a processor and sensor system, such as the processors 50308 and 50408 and respective sensor systems in communication therewith (FIGS. 5 and 6), are configured to detect the ratio of an aerosol or carbonized particulate, i.e. smoke, in the fluid extracted from a surgical site. For example, the sensing system may include a sensor that detects the size and/or the composition of particles, which is used to select an airflow path through the evacuation system. In such instances, the evacuation system can include a first filtering path, or first filtering state, and a second filtering path, or second filtering state, which can have different properties. In one instance, the first path includes only a particulate filter, and the second path includes both a fluid filter and the particulate filter. In certain instances, the first path includes a particulate filter, and the second path includes the particulate filter and a finer particulate filter arranged in series. Additional and/or alternative filtering paths are also envisioned.

In one aspect of the present disclosure, a processor and sensor system, such as the processors 50308 and 50408 and respective sensor systems in communication therewith (FIGS. 5 and 6), are configured to perform a chemical analysis on the particles evacuated from within the abdomen cavity of a patient. For example, the sensing and intelligent controls device 50324 may sense the particle count and type in order to adjust the power level of the ultrasonic generator in order to induce the ultrasonic blade to produce less smoke. In another example, the sensor systems may include sensors for detecting the particle count, the temperature, the fluid content, and/or the contamination percentage of the evacuated fluid, and can communicate the detected property or properties to a generator in order to adjust its output. For example, the smoke evacuator 50326 and/or the sensing and intelligent controls device 50324 therefor can be configured to adjust the evacuation flow rate and/or the pump's motor speed and, at a predefined particulate level, may operably affect the output power or waveform of the generator to lower the smoke generated by the end effector.

In one aspect of the present disclosure, a processor and sensor system, such as the processors 50308 and 50408 and respective sensor systems therewith (FIGS. 5 and 6), are configured to evaluate particle count and contamination in the operating room by evaluating one or more properties in the ambient air and/or the exhaust from the evacuator housing. The particle count and/or the air quality can be displayed on the smoke evacuation system, such as on the evacuator housing, for example, in order to communicate the information to a clinician and/or to establish the effectiveness of the smoke evacuation system and filter(s) thereof.

In one aspect of the present disclosure, a processor, such as the processor 50308 or the processor 50408 (FIGS. 5 and 6), for example, is configured to compare a sample rate image obtained from an endoscope to the evacuator particle count from the sensing system (e.g. the sensing and intelligent controls device 50324) in order to determine a correlation and/or to adjust the rate of the pump's revolutions-per-minute (RPM). In one instance, the activation of the generator can be communicated to the smoke evacuator such that an anticipated, required rate of smoke evacuation can be implemented. The generator activation can be communicated to the surgical evacuation system through a surgical hub, cloud communication system, and/or direct connection, for example.

In one aspect of the present disclosure, sensor systems and algorithms for a smoke evacuation system (see, e.g. FIGS. 5 and 6) can be configured to control the smoke evacuator, and can adapt motor parameters thereof to adjust the filtering efficiency of the smoke evacuator based on the needs of the surgical field at a given time. In one instance, an adaptive airflow pump speed algorithm is provided to automatically change the motor pump speed based on the sensed particulate into the inlet of the smoke evacuator and/or out of the outlet or exhaust of the smoke evacuator. For example, the sensing and intelligent controls device 50324 (FIG. 5) can include a user-selectable speed and an auto-mode speed, for example. In the auto-mode speed, the airflow through the evacuation system can be scalable based on the smoke into the evacuation system and/or a lack of filtered particles out of the smoke evacuation system. The auto-mode speed can provide automatic sensing and compensation for laparoscopic mode in certain instances.

In one aspect of the present disclosure, the evacuation system can include an electrical and communication architecture (see, e.g. FIGS. 5 and 6) that provides data collection and communication features, in order to improve interactivity with a surgical hub and a cloud. In one example, a surgical evacuation system and/or processor therefor, such as the processor 50308 (FIG. 5) and the processor 50408 (FIG. 6), for example, can include a segmented control circuit that is energized in a staged method to check for errors, shorts, and/or safety checks of the system. The segmented control circuit may also be configured to have a portion energized and a portion not energized until the energized portion performs a first function. The segmented control circuit can include circuit elements to identify and display status updates to the user of attached components. The segmented control circuit also includes circuit elements for running the motor in a first state, in which the motor is activated by the user, and in a second state, in which the motor has not been activated by the user but runs the pump in a quieter manner and at a slower rate. A segmented control circuit can allow the smoke evacuator to be energized in stages, for example.

The electrical and communication architecture for the evacuation system (see, e.g. FIGS. 5 and 6) can also provide interconnectivity of the smoke evacuator with other components within the surgical hub for interactions, as well as communication of data with a cloud. Communication of surgical evacuation system parameters to a surgical hub and/or cloud can be provided to affect the output or operation of other attached devices. The parameters can be operational or sensed. Operational parameters include airflow, pressure differentials, and air quality. Sensed parameters include particulate concentration, aerosol percentage, and chemical analysis.

In one aspect of the present disclosure, the evacuation system, such as the surgical evacuation system 50400, for example, can also include an enclosure and replaceable components, controls, and a display. Circuit elements are provided for communicating the security identification (ID) between such replaceable components. For example, communication between a filter and the smoke evacuation electronics can be provided to verify authenticity, remaining life of the component, to update parameters in the component, to log errors, and/or to limit the number and/or the type of components that can be identified by the system. In various instances, the communication circuit can authenticate features for enabling and/or disabling of configuration parameters. The communication circuit can employ encryption and/or error handling schemes to manage security and proprietary relationships between the component and the smoke evacuation electronics. Disposable/re-useable components are included in certain instances.

In one aspect of the present disclosure, the evacuation systems can provide fluid management and extraction filters and airflow configurations. For example, a surgical evacuation system including a fluid capture mechanism is provided where the fluid capture mechanism has a first and a second set of extraction or airflow control features, which are in series with each other to extract large and small fluid droplets, respectively. In certain instances, the airflow path can contain a recirculation channel or secondary fluid channel back to the primary reservoir from downstream of the exhaust port of the main fluid management chamber.

In one aspect of the present disclosure, an advanced pad can be coupled to the electrosurgical system. For example, the ground electrode 50335 of the electrosurgical system 50300 (FIG. 5) can include an advanced pad having localized sensing that is integrated into the pad while maintaining the capacitive coupling. For example, the capacitive coupling return path pad can have small separable array elements, which can be used to sense nerve control signals and/or movement of select anatomic locations, in order to detect the proximity of the monopolar tip to a nerve bundle.

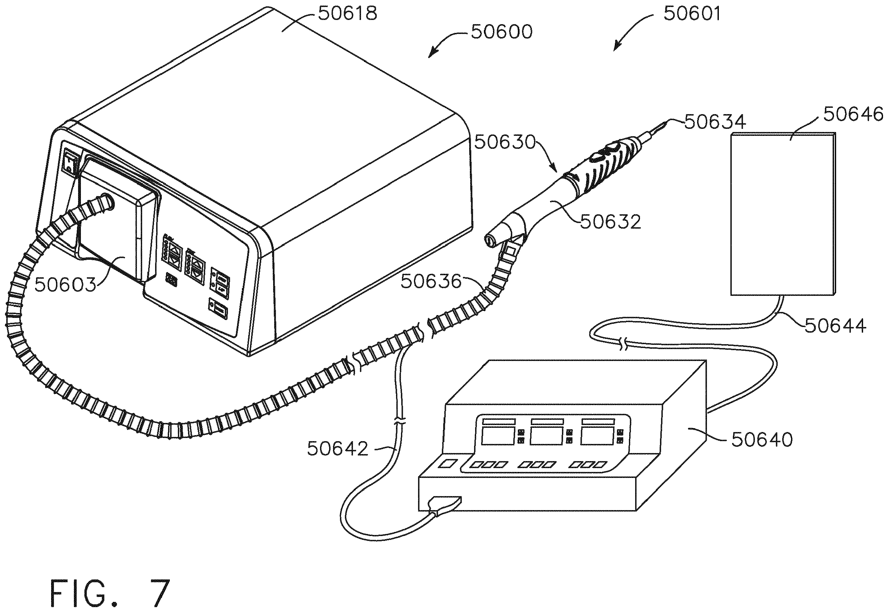

An electrosurgical system can includes a signal generator, an electrosurgical instrument, a return electrode, and a surgical evacuation system. The generator may be an RF wave generator that produces RF electrical energy. Connected to the electrosurgical instrument is a utility conduit. The utility conduit includes a cable that communicates electrical energy from the signal generator to the electrosurgical instrument. The utility conduit also includes a vacuum hose that conveys captured/collected smoke and/or fluid away from a surgical site. Such an exemplary electrosurgical system 50601 is shown in FIG. 7. More specifically, the electrosurgical system 50601 includes a generator 50640, an electrosurgical instrument 50630, a return electrode 50646, and an evacuation system 50600. The electrosurgical instrument 50630 includes a handle 50632 and a distal conduit opening 50634 that is fluidically coupled to a suction hose 50636 of the evacuation system 50600. The electrosurgical instrument 50630 also includes an electrode that is powered by the generator 50640. A first electrical connection 50642, e.g., a wire, extends from the electrosurgical instrument 50630 to the generator 50640. A second electrical connection 50644, e.g., a wire, extends from the electrosurgical instrument 50630 to electrode, i.e., the return electrode 50646. In other instances, the electrosurgical instrument 50630 can be a bipolar electrosurgical instrument. The distal conduit opening 50634 on the electrosurgical instrument 50630 is fluidically coupled to the suction hose 50636 that extends to a filter end cap 50603 of a filter that is installed in an evacuator housing 50618 of the evacuation system 50600.

In other instances, the distal conduit opening 50634 for the evacuation system 50600 can be on a handpiece or tool that is separate from the electrosurgical instrument 50630. For example, the evacuation system 50600 can include a surgical tool that is not coupled to the generator 50640 and/or does not include tissue-energizing surfaces. In certain instances, the distal conduit opening 50634 for the evacuation system 50600 can be releasably attached to an electrosurgical tool. For example, the evacuation system 50600 can include a clip-on or snap-on conduit terminating at a distal conduit opening, which can be releasably attached to a surgical tool (see, e.g., FIG. 3).

The electrosurgical instrument 50630 is configured to deliver electrical energy to target tissue of a patient to cut the tissue and/or cauterize blood vessels within and/or near the target tissue, as described herein. Specifically, an electrical discharge is provided by the electrode tip to the patient in order to cause heating of cellular matter of the patient that is in close contact with or adjacent to electrode tip. The tissue heating takes place at an appropriately high temperature to allow the electrosurgical instrument 50630 to be used to perform electrosurgery. The return electrode 50646 is either applied to or placed in close proximity to the patient (depending on the type of return electrode), in order to complete the circuit and provide a return electrical path to the generator 50640 for energy that passes into the patient's body.

The heating of cellular matter of the patient by the electrode tip, or cauterization of blood vessels to prevent bleeding, often results in smoke being released where the cauterization takes place, as further described herein. In such instances, because the evacuation conduit opening 50634 is near the electrode tip, the evacuation system 50600 is configured to capture the smoke that is released during a surgical procedure. Vacuum suction may draw the smoke into the conduit opening 50634, through the electrosurgical instrument 50630, and into the suction hose 50636 toward the evacuator housing 50618 of the evacuation system 50600.

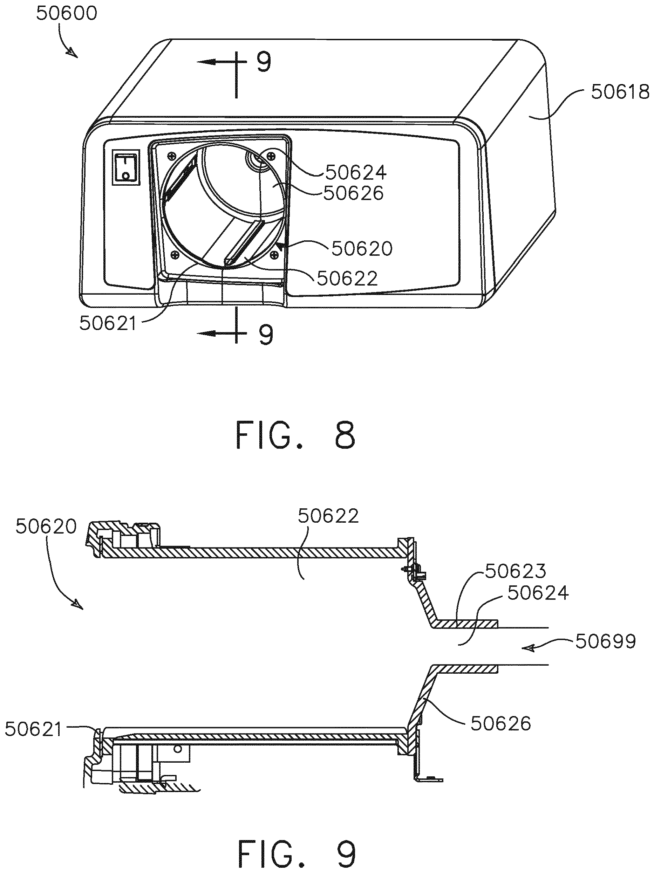

Referring now to FIG. 8, the evacuator housing 50618 of the evacuation system 50600 (FIG. 7) is depicted. The evacuator housing 50618 includes a socket 50620 that is dimensioned and structured to receive a filter. The evacuator housing 50618 can completely or partially encompass the internal components of the evacuator housing 50618. The socket 50620 includes a first receptacle 50622 and a second receptacle 50624. A transition surface 50626 extends between the first receptacle 50622 and the second receptacle 50624.

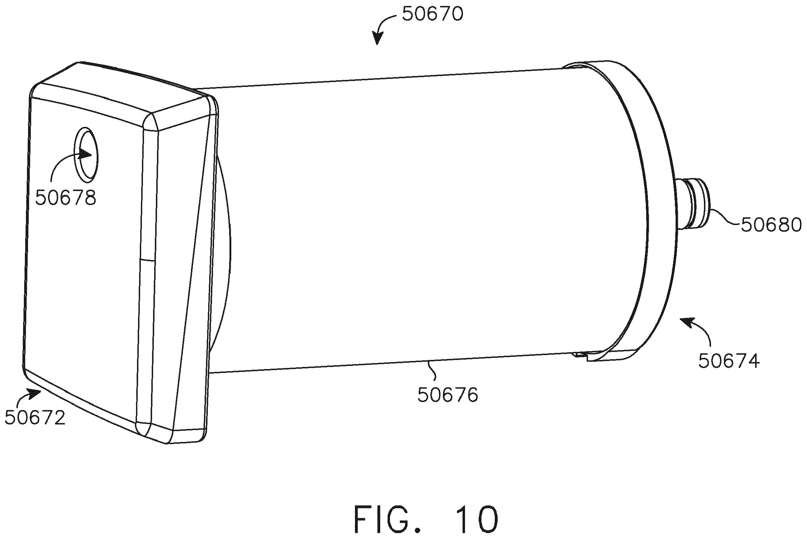

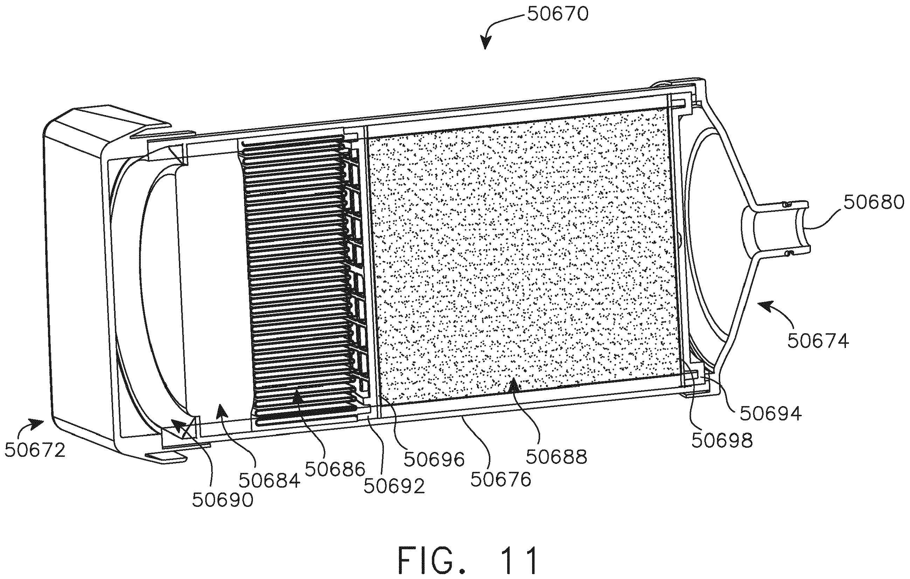

Referring primarily now to FIG. 9, the socket 50620 is depicted along a cross sectional plane indicated in FIG. 8. The socket 50620 includes a first end 50621 that is open to receive a filter and a second end 50623 in communication with a flow path 50699 through the evacuator housing 50618. A filter 50670 (FIGS. 10 and 11) may be removably positioned with the socket 50620. For example, the filter 50670 can be inserted and removed from the first end 50621 of the socket 50620. The second receptacle 50624 is configured to receive a connection nipple of the filter 50670.

Surgical evacuation systems often use filters to remove unwanted pollutants from the smoke before the smoke is released as exhaust. In certain instances, the filters can be replaceable. The reader will appreciate that the filter 50670 depicted in FIGS. 10 and 11 can be employed in various evacuation systems disclosed herein. The filter 50670 can be a replaceable and/or disposable filter.

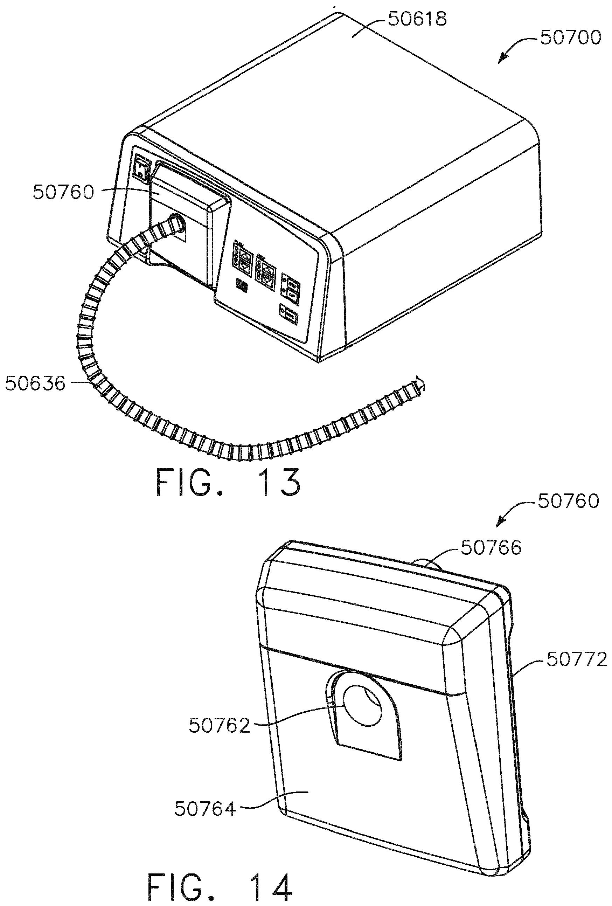

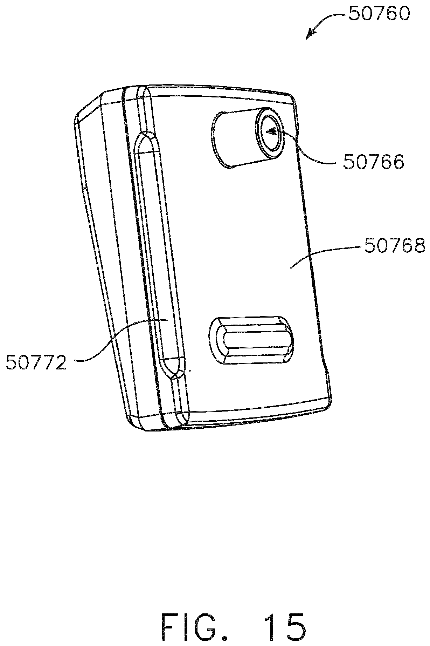

The filter 50670 includes a front cap 50672, a back cap 50674, and a filter body 50676 disposed therebetween. The front cap 50672 includes a filter inlet 50678, which, in certain instances, is configured to receive smoke directly from the suction hose 50636 (FIG. 7) or other smoke source. In some aspects of the present disclosure, the front cap 50672 can be replaced by a fluid trap (e.g. the fluid trap 50760 depicted in FIGS. 14-17) that directs the smoke directly from the smoke source, and after removing at least a portion of the fluid therefrom, passes the partially processed smoke into the filter body 50676 for further processing. For example, the filter inlet 50678 can be configured to receive smoke via a fluid trap exhaust port, such as a port 50766 in a fluid trap 50760 (FIGS. 14-17) to communicate partially processed smoke into the filter 50670.

Once the smoke enters the filter 50670, the smoke can be filtered by components housed within the filter body 50676. The filtered smoke can then exit the filter 50670 through a filter exhaust 50680 defined in the back cap 50674 of the filter 50670. When the filter 50670 is associated with an evacuation system, suction generated in the evacuator housing 50618 of the evacuation system 50600 can be communicated to the filter 50670 through the filter exhaust 50680 to pull the smoke through the internal filtering components of the filter 50670. A filter often includes a particulate filter and a charcoal filter. The particulate filter can be a high-efficiency particulate air (HEPA) filter or an ultra-low penetration air (ULPA) filter, for example. ULPA filtration utilizes a depth filter that is similar to a maze. The particulate can be filtered using at least one of the following methods: direct interception (in which particles over 1.0 micron are captured because they are too large to pass through the fibers of the media filter), inertial impaction (in which particles between 0.5 and 1.0 micron collide with the fibers and remain there, and diffusional interception (in which particles less than 0.5 micron are captured by the effect of Brownian random thermal motion as the particles "search out" fibers and adhere to them).

The charcoal filter is configured to remove toxic gases and/or odor generated by the surgical smoke. In various instances, the charcoal can be "activated" meaning it has been treated with a heating process to expose the active absorption sites. The charcoal can be from activated virgin coconut shells, for example.

Referring now to FIG. 11, the filter 50670 includes a coarse media filter layer 50684 followed by a fine particulate filter layer 50686. In other instances, the filter 50670 may consist of a single type of filter. In still other instances, the filter 50670 can include more than two filter layers and/or more than two different types of filter layers. After the particulate matter is removed by the filter layers 50684 and 50686, the smoke is drawn through a carbon reservoir 50688 in the filter 50670 to remove gaseous contaminants within the smoke, such as volatile organic compounds, for example. In various instances, the carbon reservoir 50688 can comprise a charcoal filter. The filtered smoke, which is now substantially free of particulate matter and gaseous contaminants, is drawn through the filter exhaust 50680 and into the evacuation system 50600 for further processing and/or elimination.

The filter 50670 includes a plurality of dams between components of the filter body 50676. For example, a first dam 50690 is positioned intermediate the filter inlet 50678 (FIG. 10) and a first particulate filter, such as the coarse media filter 50684, for example. A second dam 50692 is positioned intermediate a second particulate filter, such as the fine particulate filter 50686, for example, and the carbon reservoir 50688. Additionally, a third dam 50694 is positioned intermediate the carbon reservoir 50688 and the filter exhaust 50680. The dams 50690, 50692, and 50694 can comprise a gasket or O-ring, which is configured to prevent movement of the components within the filter body 50676. In various instances, the size and shape of the dams 50690, 50692, and 50694 can be selected to prevent distention of the filter components in the direction of the applied suction.

The coarse media filter 50684 can include a low-air-resistant filter material, such as fiberglass, polyester, and/or pleated filters that are configured to remove a majority of particulate matter larger than 10 µm, for example. In some aspects of the present disclosure, this includes filters that remove at least 85% of particulate matter larger than 10 µm, greater than 90% of particulate matter larger than 10 µm, greater than 95% of particular matter larger than 10 µm, greater than 99% of particular matter larger than 10 µm, greater than 99.9% particulate matter larger than 10 µm, or greater than 99.99% particulate matter larger than 10 µm.

Additionally or alternatively, the coarse media filter 50684 can include a low-air-resistant filter that removes the majority of particulate matter greater than 1 µm. In some aspects of the present disclosure, this includes filters that remove at least 85% particulate matter larger than 1 µm, greater than 90% of particulate matter larger than 1 µm, greater than 95% of particular matter larger than 1 µm, greater than 99% of particular matter larger than 1 µm, greater than 99.9% particulate matter larger than 1 µm, or greater than 99.99% particulate matter larger than 1 µm.

The fine particulate filter 50686 can include any filter of higher efficiency than the coarse media filter 50684. This includes, for example, filters that are capable of filtering a higher percentage of the same sized particles as the coarse media filter 50684 and/or capable of filtering smaller sized particles than the coarse media filter 50684. In some aspects of the present disclosure, the fine particulate filter 50686 can include a HEPA filter or an ULPA filter. Additionally or alternatively, the fine particulate filter 50686 can be pleated to increase the surface area thereof. In some aspects of the present disclosure, the coarse media filter 50684 includes a pleated HEPA filter and the fine particulate filter 50686 includes a pleated ULPA filter.

Subsequent to particulate filtration, smoke enters a downstream section of the filter 50670 that includes the carbon reservoir 50688. The carbon reservoir 50688 is bounded by porous dividers 50696 and 50698 disposed between the intermediate and terminal dams 50692 and 50694, respectively. In some aspects of the present disclosure, the porous dividers 50696 and 50698 are rigid and/or inflexible and define a constant spatial volume for the carbon reservoir 50688.

The carbon reservoir 50688 can include additional sorbents that act cumulatively with or independently from the carbon particles to remove gaseous pollutants. The additional sorbents can include, for example, sorbents such as magnesium oxide and/or copper oxide, for example, which can act to adsorb gaseous pollutants such as carbon monoxide, ethylene oxide, and/or ozone, for example. In some aspects of the present disclosure, additional sorbents are dispersed throughout the reservoir 50688 and/or are positioned in distinct layers above, below, or within the reservoir 50688.

Referring again to FIG. 4, the evacuation system 50500 includes the pump 50506 within the evacuator housing 50518. Similarly, the evacuation system 50600 depicted in FIG. 7 can include a pump located in the evacuator housing 50618, which can generate suction to pull smoke from the surgical site, through the suction hose 50636 and through the filter 50670 (FIGS. 10 and 11). In operation, the pump can create a pressure differential within the evacuator housing 50618 that causes the smoke to travel into the filter 50670 and out an exhaust mechanism (e.g. exhaust mechanism 50520 in FIG. 4) at the outlet of the flow path. The filter 50670 is configured to extract harmful, foul, or otherwise unwanted particulates from the smoke.

The pump can be disposed in-line with the flow path through the evacuator housing 50618 such that the gas flowing through the evacuator housing 50618 enters the pump at one end and exits the pump at the other end. The pump can provide a sealed positive displacement flow path. In various instances, the pump can produce the sealed positive displacement flow path by trapping (sealing) a first volume of gas and decreasing that volume to a second smaller volume as the gas moves through the pump. Decreasing the volume of the trapped gas increases the pressure of the gas. The second pressurized volume of gas can be released from the pump at a pump outlet. For example, the pump can be a compressor. More specifically, the pump can comprise a hybrid regenerative blower, a claw pump, a lobe compressor, and/or a scroll compressor. Positive displacement compressors can provide improved compression ratios and operating pressures while limiting vibration and noise generated by the evacuation system 50600. Additionally or alternatively, the evacuation system 50600 can include a fan for moving fluid therethrough.

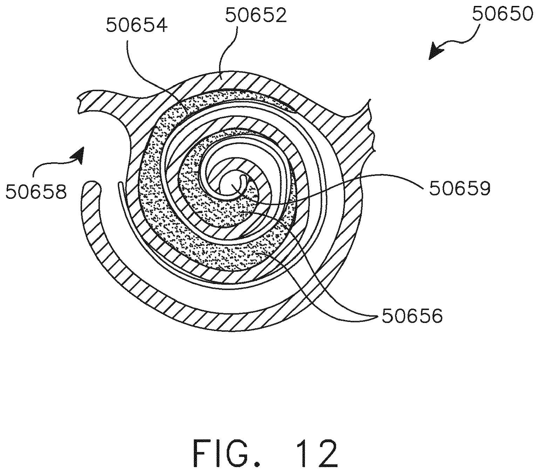

An example of a positive displacement compressor, e.g. a scroll compressor pump 50650, is depicted in FIG. 12. The scroll compressor pump 50650 includes a stator scroll 50652 and a moving scroll 50654. The stator scroll 50652 can be fixed in position while the moving scroll 50654 orbits eccentrically. For example, the moving scroll 50654 can orbit eccentrically such that it rotates about the central longitudinal axis of the stator scroll 50652. As depicted in FIG. 12, the central longitudinal axes of the stator scroll 50652 and the moving scroll 50654 extend perpendicular to the viewing plane of the scrolls 50652, 50654. The stator scroll 50652 and the moving scroll 50654 are interleaved with each other to form discrete sealed compression chambers 50656.

In use, a gas can enter the scroll compressor pump 50650 at an inlet 50658. As the moving scroll 50654 orbits relative to the stator scroll 50652, the inlet gas is first trapped in the compression chamber 50656. The compression chamber 50656 is configured to move a discrete volume of gas along the spiral contour of the scrolls 50652 and 50654 toward the center of the scroll compressor pump 50650. The compression chamber 50656 defines a sealed space in which the gas resides. Moreover, as the moving scroll 50654 moves the captured gas toward the center of the stator scroll 50652, the compression chamber 50656 decreases in volume. This decrease in volume increases the pressure of the gas inside the compression chamber 50656. The gas inside the sealed compression chamber 50656 is trapped while the volume decreases, thus pressurizing the gas. Once the pressurized gas reaches the center of the scroll compressor pump 50650, the pressurized gas is released through an outlet 50659.

Referring now to FIG. 13, a portion of an evacuation system 50700 is depicted. The evacuation system 50700 can be similar in many respects to the evacuation system 50600 (FIG. 7). For example, the evacuation system 50700 includes the evacuator housing 50618 and the suction hose 50636. Referring again to FIG. 7, the evacuation system 50600 is configured to produce suction and thereby draw smoke from the distal end of the suction hose 50636 into the evacuator housing 50618 for processing. Notably, the suction hose 50636 is not connected to the evacuator housing 50618 through the filter end cap 50603 in FIG. 13. Rather, the suction hose 50636 is connected to the evacuator housing 50618 through the fluid trap 50760. A filter, similar to the filter 50670 can be positioned within the socket of the evacuator housing 50618 behind the fluid trap 50760.

The fluid trap 50760 is a first processing point that extracts and retains at least a portion of the fluid (e.g. liquid) from the smoke before relaying the partially-processed smoke to the evacuation system 50700 for further processing and filtration. The evacuation system 50700 is configured to process, filter, and otherwise clean the smoke to reduce or eliminate unpleasant odors or other problems associated with smoke generation in the surgical theater (or other operating environment), as described herein. By extracting liquid droplets and/or aerosol from the smoke before it is further processed by the evacuation system 50700, the fluid trap 50760 can, among other things, increase the efficiency of the evacuation system 50700 and/or increase the life of filters associated therewith, in certain instances.

Referring primarily to FIGS. 14-17, the fluid trap 50760 is depicted detached from the evacuator housing 50618 (FIG. 13). The fluid trap 50760 includes an inlet port 50762 defined in a front cover or surface 50764 of the fluid trap 50760. The inlet port 50762 can be configured to releasably receive the suction hose 50636 (FIG. 13). For example, an end of the suction hose 50636 can be inserted at least partially within the inlet port 50762 and can be secured with an interference fit therebetween. In various instances, the interference fit can be a fluid tight and/or airtight fit so that substantially all of the smoke passing through the suction hose 50636 is transferred into the fluid trap 50760. In some instances, other mechanisms for coupling or joining the suction hose 50636 to the inlet port 50762 can be employed such as a latch-based compression fitting, an O-ring, threadably coupling the suction hose 50636 with the inlet port 50762, for example, and/or other coupling mechanisms.

In various instances, a fluid tight and/or airtight fit between the suction hose 50636 and the fluid trap 50760 is configured to prevent fluids and/or other materials in the evacuated smoke from leaking at or near the junction of these components. In some instances, the suction hose 50636 can be associated with the inlet port 50762 through an intermediate coupling device, such as an O-ring and/or adaptor, for example, to further ensure an airtight and/or fluid tight connection between the suction hose 50636 and the fluid trap 50760.

As discussed above, the fluid trap 50760 includes the exhaust port 50766. The exhaust port extends away from a rear cover or surface 50768 of the fluid trap 50760. The exhaust port 50766 defines an open channel between an interior chamber 50770 of the fluid trap 50760 and the exterior environment. In some instances, the exhaust port 50766 is sized and shaped to tightly associate with a surgical evacuation system or components thereof. For example, the exhaust port 50766 can be sized and shaped to associate with and communicate at least partially processed smoke from the fluid trap 50760 to a filter housed within an evacuator housing 50618 (FIG. 13). In certain instances, the exhaust port 50766 can extend away from the front plate, a top surface, or a side surface of the fluid trap 50760.

In certain instances, the exhaust port 50766 includes a membrane, which spaces the exhaust port 50766 apart from the evacuator housing 50618. Such a membrane can act to prevent water or other liquid collected in the fluid trap 50760 from being passed through the exhaust port 50766 and into the evacuator housing 50618 while permitting air, water and/or vapor to freely pass into the evacuator housing 50618. For example, a high flow rate microporous polytetrafluoroethylene (PTFE) can be positioned downstream of the exhaust port 50766 and upstream of a pump to protect the pump or other components of the evacuation system 50700 from damage and/or contamination.

The fluid trap 50760 also includes a gripping region 50772, which is positioned and dimensioned to assist a user in handling the fluid trap 50760 and/or connecting the fluid trap 50760 with the suction hose 50636 and/or the evacuator housing 50618. The gripping region 50772 is depicted as being an elongate recess; however, the reader will readily appreciate that the gripping region 50772 may include at least one recess, groove, protrusion, tassel, and/or ring, for example, which can be sized and shaped to accommodate a user's digits or to otherwise provide a gripping surface.

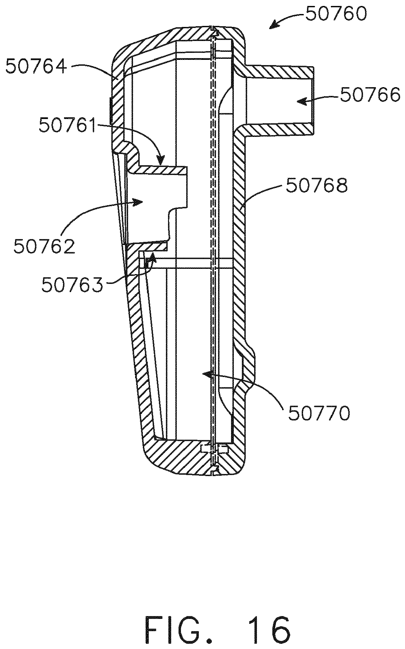

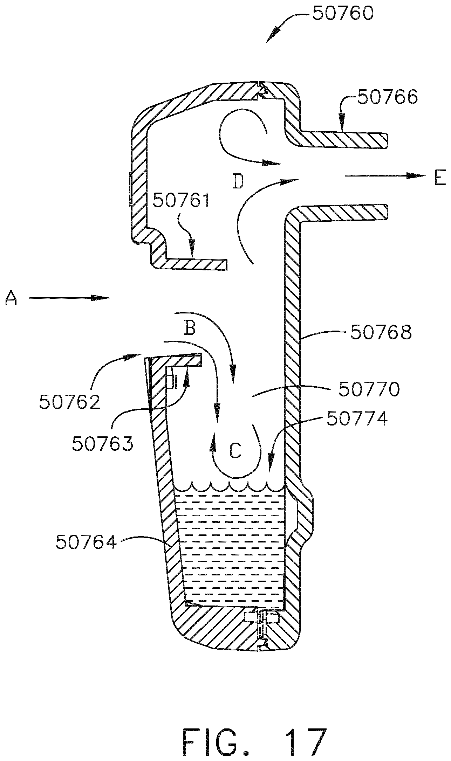

Referring primarily now to FIGS. 16 and 17, the interior chamber 50770 of the fluid trap 50760 is depicted. The relative positioning of the inlet port 50762 and the exhaust port 50766 is configured to promote the extraction and the retention of fluid from the smoke as it passes into the fluid trap 50760. In certain instances, the inlet port 50762 can comprise a notched cylindrical shape, which can direct the smoke and the accompanying fluid towards a fluid reservoir 50774 of the fluid trap 50760 or otherwise directionally away from the exhaust port 50766. An example of such a fluid flow is depicted with arrows A, B, C, D, and E in FIG. 17.

As shown, smoke enters the fluid trap 50760 through the inlet port 50762 (illustrated by the arrow A) and exits the fluid trap 50760 through the exhaust port 50766 (illustrated by the arrow E). At least partially due to the geometry of the inlet port (*e.g.,* a longer, upper sidewall 50761 and a shorter, lower sidewall 50763), the smoke entering the inlet port 50762 is initially directed primarily downward into the fluid reservoir 50774 of the fluid trap 50760 (illustrated by the arrows B). As smoke continues to be pulled downward into the fluid trap 50760 along the arrows A and B, the smoke that was initially directed downward, tumbles downward, and is directed laterally away from its source to travel in a substantially opposite but parallel path towards the upper portion of the fluid trap 50760 and out of the exhaust port 50766 (illustrated by the arrows D and E).

The directional flow of smoke through the fluid trap 50760 can ensure that liquids within the smoke are extracted and retained within the lower portion (e.g. the fluid reservoir 50774) of the fluid trap 50760. Furthermore, the relative positioning of the exhaust port 50766 vertically above the inlet port 50762 when the fluid trap 50760 is in an upright position is configured to discourage liquid from inadvertently being carried through the exhaust port 50766 by the flow of smoke while not substantially hindering fluid flow into and out of the fluid trap 50760. Additionally, in certain instances, the configuration of the inlet port 50762 and the outlet port 50766 and/or the size and shape of the fluid trap 50760 itself, can enable the fluid trap 50760 to be spill resistant.

In various instances, an evacuation system can include a plurality of sensors and intelligent controls, as further described herein with respect to FIGS. 5 and 6, for example. In one aspect of the present disclosure, an evacuation system can include one or more temperatures sensors, one or more fluid detection sensors, one or more pressure sensors, one or more particle sensors, and/or one or more chemical sensors. A temperature sensor can be positioned to detect the temperature of a fluid at the surgical site, moving through a surgical evacuation system, and/or being exhaust into a surgical theater from a surgical evacuation system. A pressure sensor can be positioned to detect a pressure within the evacuation system, such as within the evacuator housing. For example, a pressure sensor can be positioned upstream of the filter, between the filter and the pump, and/or downstream of the pump. In certain instances, a pressure sensor can be positioned to detect a pressure in the ambient environment outside of the evacuation system. Similarly, a particle sensor can be positioned to detect particles within the evacuation system, such as within the evacuator housing. A particle sensor can be upstream of the filter, between the filter and the pump, and/or downstream of the pump, for example. In various instances, a particle sensor can be positioned to detect particles in the ambient environment in order to determine the air quality in the surgical theater, for example.

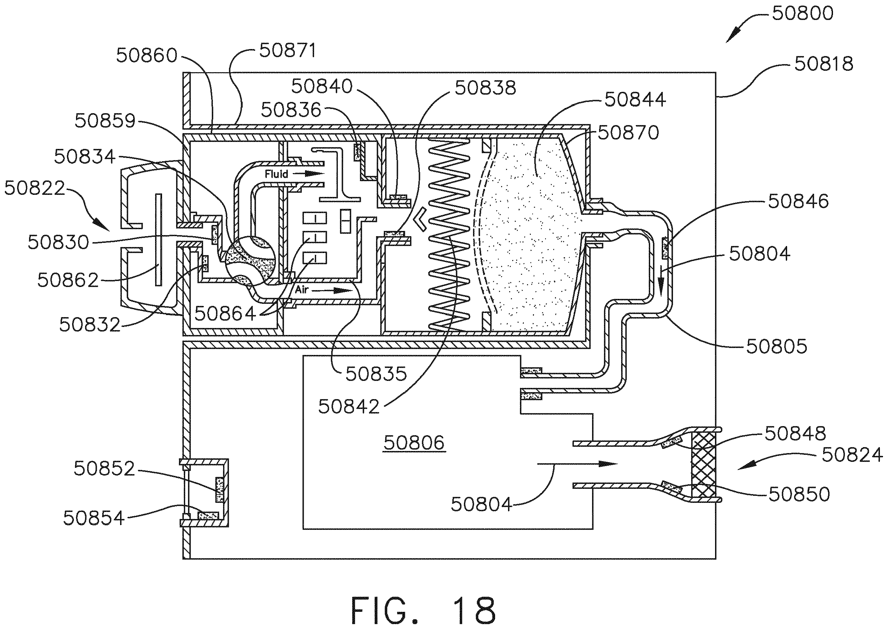

An evacuator housing 50818 for an evacuation system 50800 is schematically depicted in FIG. 18. The evacuator housing 50818 can be similar in many respects to the evacuator housings 50018 and/or 50618, for example, and/or can be incorporated into various evacuation systems disclosed herein. The evacuator housing 50818 includes numerous sensors, which are further described herein. The reader will appreciate that certain evacuator housings may not include each sensor depicted in FIG. 18 and/or may include additional sensor(s). Similar to the evacuator housings 50018 and 50618 disclosed herein, the evacuator housing 50818 of FIG. 18 includes an inlet 50822 and an outlet 50824. A fluid trap 50860, a filter 50870, and a pump 50806 are sequentially aligned along a flow path 50804 through the evacuator housing 50818 between the inlet 50822 and the outlet 50824.

An evacuator housing can include modular and/or replaceable components, as further described herein. For example, an evacuator housing can include a socket or a receptacle 50871 dimensioned to receive a modular fluid trap and/or a replaceable filter. In certain instances, a fluid trap and a filter can be incorporated into a single interchangeable module 50859, as depicted in FIG. 18. More specifically, the fluid trap 50860 and the filter 50870 form the interchangeable module 50859, which can be modular and/or replaceable, and can be removably installed in the receptacle 50871 in the evacuator housing 50818. In other instances, the fluid trap 50860 and the filter 50870 can be separate and distinct modular components, which can be assembled together and/or separately installed in the evacuator housing 50818.

Referring still to the evacuator housing 50818, the evacuator housing 50818 includes a plurality of sensors for detecting various parameters therein and/or parameters of the ambient environment. Additionally or alternatively, one or more modular components installed in the evacuator housing 50818 can include one or more sensors. For example, referring still to FIG. 18, the interchangeable module 50859 includes a plurality of sensors for detecting various parameters therein.

In various instances, the evacuator housing 50818 and/or a modular component(s) compatible with the evacuator housing 50818 can include a processor, such as the processor 50308 and 50408 (FIGS. 5 and 6, respectively), which is configured to receive inputs from one or more sensors and/or to communicate outputs to one more systems and/or drivers. Various processors for use with the evacuator housing 50818 are further described herein.

In operation, smoke from a surgical site can be drawn into the inlet 50822 to the evacuator housing 50818 via the fluid trap 50860. The flow path 50804 through the evacuator housing 50818 in FIG. 18 can comprise a sealed conduit or tube 50805 extending between the various in-line components. In various instances, the smoke can flow past a fluid detection sensor 50830 and a chemical sensor 50832 to a diverter valve 50834, which is further described herein. A fluid detection sensor, such as the sensor 50830, can detect fluid particles in the smoke. In one instance, the fluid detection sensor 50830 can be a continuity sensor. For example, the fluid detection sensor 50830 can include two spaced-apart electrodes and a sensor for detecting the degree of continuity therebetween. When no fluid is present, the continuity can be zero, or substantially zero, for example. The chemical sensor 50832 can detect the chemical properties of the smoke.

At the diverter valve 50834, fluid can be directed into a condenser 50835 of the fluid trap 50860 and the smoke can continue toward the filter 50870. Baffles 50864 are positioned within the condenser 50835 to facilitate the condensation of fluid droplets from the smoke into a reservoir in the fluid trap 50860. A fluid detection sensor 50836 can ensure any fluid in the evacuator housing is entirely, or at least substantially, captured within the fluid trap 50860.

Referring still to FIG. 18, the smoke can then be directed to flow into the filter 50870 of the interchangeable module 50859. At the inlet to the filter 50870, the smoke can flow past a particle sensor 50838 and a pressure sensor 50840. In one form, the particle sensor 50838 can comprise a laser particle counter, as further described herein. The smoke can be filtered via a pleated ultra-low penetration air (ULPA) filter 50842 and a charcoal filter 50844, as depicted in FIG. 18.

Upon exiting the filter, the filtered smoke can flow past a pressure sensor 50846 and can then continue along the flow path 50804 within the evacuator housing 50818 toward the pump 50806. Upon moving through the pump 50806, the filtered smoke can flow past a particle sensor 50848 and a pressure sensor 50850 at the outlet to the evacuator housing 50818. In one form, the particle sensor 50848 can comprise a laser particle counter, as further described herein. The evacuator housing 50818 in FIG. 18 also includes an air quality particle sensor 50852 and an ambient pressure sensor 50854 to detect various properties of the ambient environment, such as the environment within the surgical theater. The air quality particle sensor, or external/ambient air particle sensor, 50852 can comprise a laser particle counter in at least one form. The various sensors depicted in FIG. 18 are further described herein. Moreover, in various instances, alternative sensing means can be utilized in the smoke evacuation systems disclosed herein. For example, alternative sensors for counting particles and/or determining particulate concentration in a fluid are further disclosed herein.

In various instances, the fluid trap 50860 depicted in FIG. 18 can be configured to prevent spillage and/or leakage of the captured fluid. For example, the geometry of the fluid trap 50860 can be selected to prevent the captured fluid from spilling and/or leaking. In certain instances, the fluid trap 50860 can include baffles and/or splatter screens, such as the screen 50862, for preventing the captured fluid from splashing out of the fluid trap 50860. In one or more instances, the fluid trap 50860 can include sensors for detecting the volume of fluid within the fluid trap and/or determining if the fluid trap 50860 is filled to capacity. The fluid trap 50860 may include a valve for empty the fluid therefrom. The reader will readily appreciate that various alternative fluid trap arrangements and geometries can be employed to capture fluid drawn into the evacuator housing 50818.

In certain instances, the filter 50870 can include additional and/or fewer filtering levels. For example, the filter 50870 can include one or more filtering layers selected from the following group of filters: a course media filter, a fine media filter, and a sorbent-based filter. The course media filter can be a low-air-resistant filter, which can be comprised of fiberglass, polyester, and/or pleated filters, for example. The fine media filter can be a high efficiency particulate air (HEPA) filter and/or ULPA filter. The sorbent-based filter can be an activated-carbon filter, for example. The reader will readily appreciate that various alternative filter arrangements and geometries can be employed to filter smoke drawn along the flow path through the evacuator housing 50818.

In one or more instances, the pump 50806 depicted in FIG. 18 can be replaced by and/or used in combination with another compressor and/or pump, such as a hybrid regenerative blower, a claw pump, and/or a lobe compressor, for example. The reader will readily appreciate that various alternative pumping arrangements and geometries can be employed to generate suction within the flow path 50804 to draw smoke into the evacuator housing 50818.

The various sensors in an evacuation system, such as the sensors depicted in FIG. 18, can communicate with a processor. The processor can be incorporated into the evacuation system and/or can be a component of another surgical instrument and/or a surgical hub. Various processors are further described herein. An on-board processor can be configured to adjust one or more operational parameters of the evacuator system (e.g. a motor for the pump 50806) based on input from the sensor(s). Additionally or alternatively, an on-board processor can be configured to adjust one or more operational parameters of another device, such as an electrosurgical tool and/or imaging device based on input from the sensor(s).

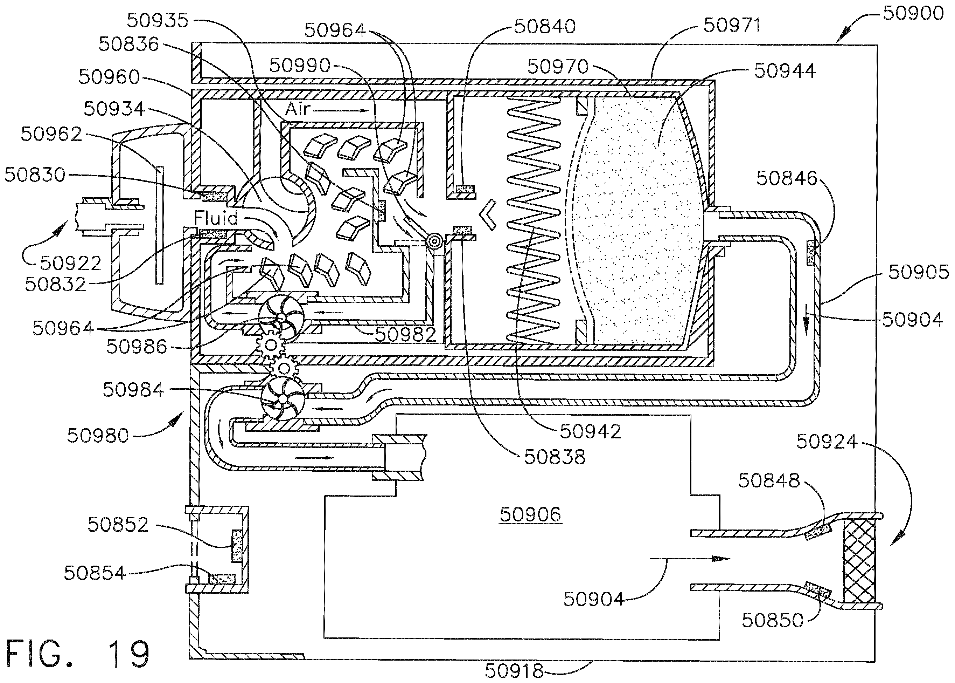

Referring now to FIG. 19, another evacuator housing 50918 for an evacuation system 50900 is depicted. The evacuator housing 50918 in FIG. 19 can be similar in many respects to the evacuator housing 50818 in FIG. 18. For example, the evacuator housing 50918 defines a flow path 50904 between an inlet 50922 to the evacuator housing 50918 and an outlet 50924 to the evacuator housing 50918. Intermediate the inlet 50922 and the outlet 50924, a fluid trap 50960, a filter 50970, and a pump 50906 are sequentially arranged. The evacuator housing 50918 can include a socket or a receptacle 50971 dimensioned to receive a modular fluid trap and/or a replaceable filter, similar to the receptacle 50871, for example. At a diverter valve 50934, fluid can be directed into a condenser 50935 of the fluid trap 50960 and the smoke can continue toward the filter 50970. In certain instances, the fluid trap 50960 can include baffles, such as the baffles 50964, and/or splatter screens, such as the screen 50962, for example, for preventing the captured fluid from splashing out of the fluid trap 50960. The filter 50970 includes a pleated ultra-low penetration air (ULPA) filter 50942 and a charcoal filter 50944. A sealed conduit or tube 50905 extends between the various in-line components. The evacuator housing 50918 also includes the sensors 50830, 50832, 50836, 50838, 50840, 50846, 50848, 50850, 50852, and 50854 which are further described herein and shown in FIG. 18 and FIG. 19.

Referring still to FIG. 19, the evacuator housing 50918 also includes a centrifugal blower arrangement 50980 and a recirculating valve 50990. The recirculating valve 50990 can selectively open and close to recirculate fluid through the fluid trap 50960. For example, if the fluid detection sensor 50836 detects a fluid, the recirculating valve 50990 can be opened such that the fluid is directed back away from the filter 50970 and back into the fluid trap 50960. If the fluid detection sensor 50836 does not detect a fluid, the valve 50990 can be closed such that the smoke is directed into the filter 50970. When fluid is recirculated via the recirculating valve 50990, the fluid can be drawn through a recirculation conduit 50982. The centrifugal blower arrangement 50980 is engaged with the recirculation conduit 50982 to generate a recirculating suction force in the recirculation conduit 50982. More specifically, when the recirculating valve 50990 is open and the pump 50906 is activated, the suction force generated by the pump 50906 downstream of the filter 50970 can generate rotation of the first centrifugal blower, or squirrel cage, 50984, which can be transferred to the second centrifugal blower, or squirrel cage, 50986, which draws the recirculated fluid through the recirculating valve 50990 and into the fluid trap 50960.

In various aspects of the present disclosure, the control schematics of FIGS. 5 and 6 can be utilized with the various sensor systems and evacuator housings of FIGS. 18 and 19.

### Communication Of Smoke Evacuation System Parameters to Surgical Hub Or Cloud To Effect The Output Or Operation Of Other Attached Devices END8546USNP (M-1/164469)

During the course of surgical procedures in which electrosurgical instruments have been used, parameters such as the type of particles, the particle concentration, and particle size in surgical smoke have not previously been monitored. This lack of smoke monitoring during the surgical procedures implies that smoke evacuation devices present at surgical sites have failed to adjust operation to more carefully evacuate the smoke in order to compensate as parameters change over the course of the surgical procedure (e.g., higher particle concentration in smoke being evacuated, etc.).

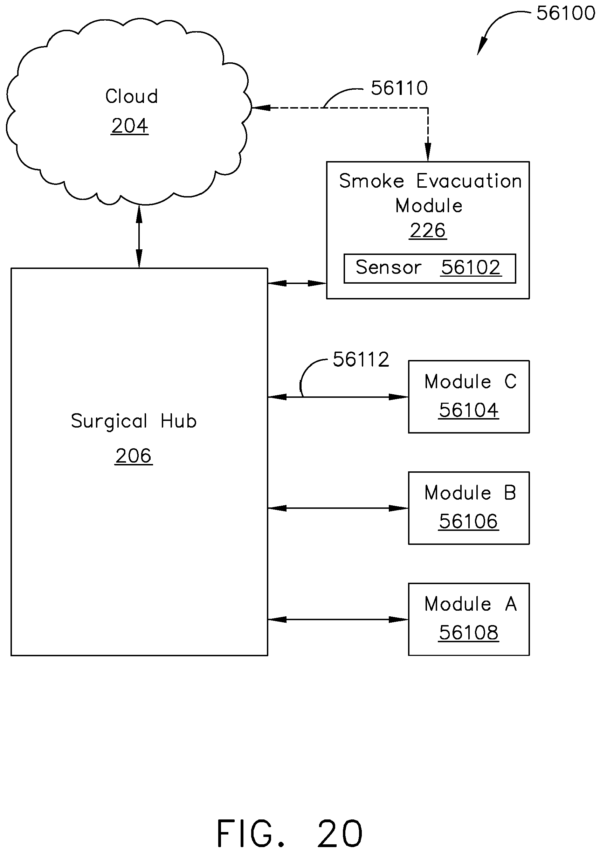

With reference to FIGS. 20-22 and FIGS. 25-35, an example smart smoke evacuation system 56100 as described herein is able to communicate detected or sensed parameters to a surgical hub 206 or a cloud analytics computing environment 204 (the cloud 204 hereafter), which in turn effects the output and operation of other devices in communication with the surgical hub 206 or the cloud 204. For example, if a varied particle concentration is detected, this varied parameter is communicated to the surgical hub 206 or the cloud 204, and the surgical hub 206 or the cloud 204 may adjust operation of another component in the smart smoke evacuation system 56100, such as the power level applied to the electrosurgical instrument, to compensate for the varied parameter.

By automatically adjusting operation of other devices due to parameters detected by the smart smoke evacuation system, smoke produced by operation of an electrosurgical instrument is more effectively removed, and the danger to physicians and other personnel present during surgical procedures is decreased.

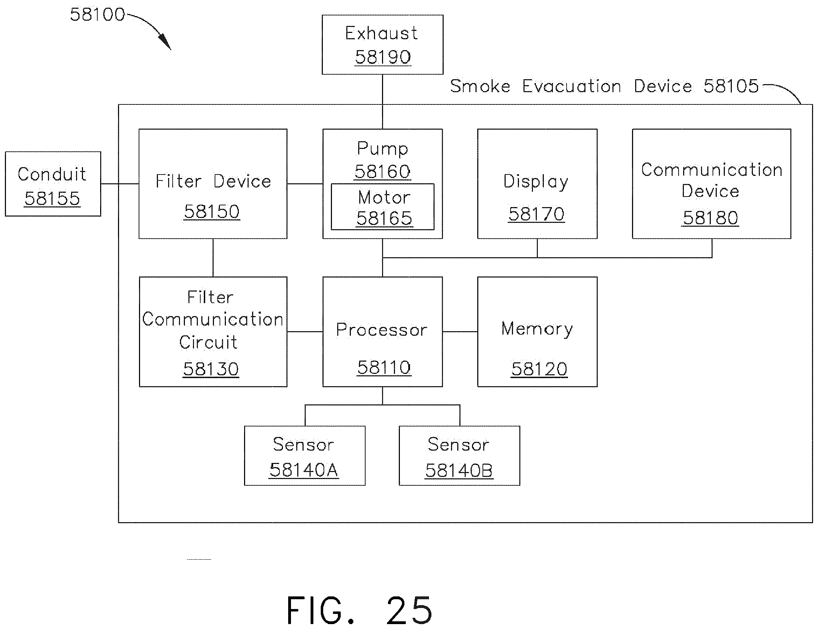

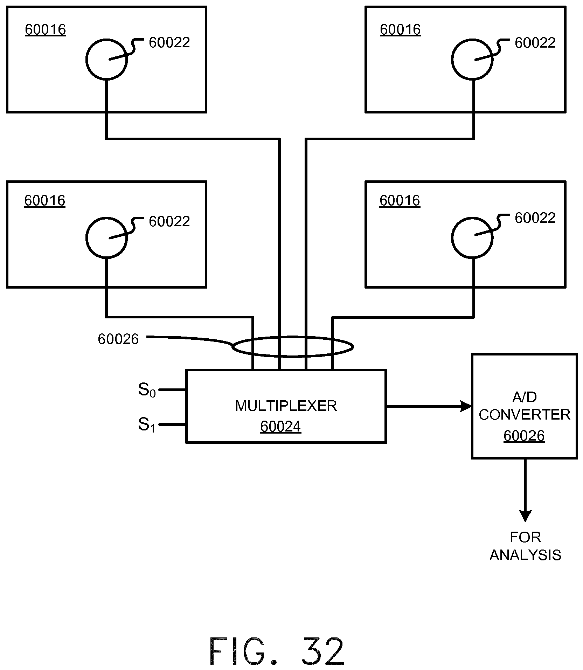

FIG. 20 illustrates a smoke evacuation system 56100, in which the various modules and devices are in communication with one another, in accordance with at least one aspect of the present disclosure. Communication between the modules and devices identified in smoke evacuation system 56100 may occur directly or indirectly via a connection through the surgical hub 204 and/or the cloud 204. The smoke evacuation system 56100 can be interactively coupled to the surgical hub 206 and/or the cloud 204 as described, for example, in FIGS. 25-35. In this manner, parameters sensed or detected by a smoke evacuation module 226 may impact the functionality of other modules, devices, or components in a computer implemented surgical system, such as those illustrated in FIGS. 32-24. FIG. 20, for example, depicts a computer implemented smoke evacuation system 56100 comprising a cloud 204, a surgical hub 206, a smoke evacuation module 226, and module A 56108, module B 56106, and module C 56104. Modules A 56108, B 56106, and C 56104 may be any of the modules or devices that are in communication with, or a part of, the surgical hub 206. For example, modules A 56108, B 56106, and C 56104 maybe components of, the visualization system 208, components of the robotic system 222, an intelligent instrument 235, an imaging module 238, a generator module 240, a suction/irrigation module 228, a communication module 230, a processor module 232, a storage array 234, an operating-room mapping module 242, etc. Examples and descriptions of these various types of modules and components may be found in, for example, discussion of FIGS. 25-35.

The smoke evacuation module 226 comprises at least one sensor. This sensor can be any type of sensor, including, for example, sensors detecting operational parameters such as ambient pressure sensors, air quality particle sensors, etc. Further the sensors can be internal sensors such as, for example, a pressure sensor, a fluid detection sensor, a chemical sensor, a laser particle counter, etc. Further, more than one pressure sensor may be included in the smoke evacuation module 226 in order to determine a pressure differential across various portions of the smoke evacuation module 226. A description of sensors used in the context of a smoke evacuation system 56100 may be found in connection with FIGS. 4-6, 18, and 19, for example.

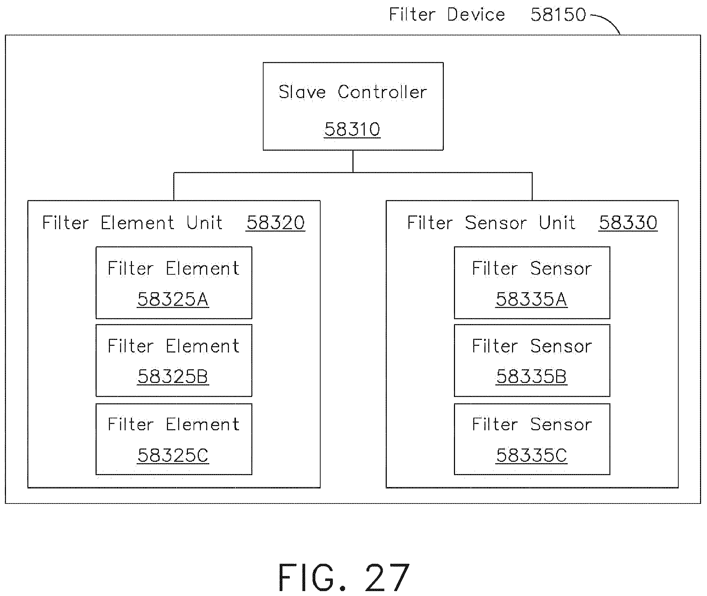

In various aspects, the smoke evacuation module 226 and modules A 56108, B 56106, and C 56104 maybe in direct or indirect communication with the surgical hub 206 and/or the cloud 204, for example. Turning back to FIG. 20, the smoke evacuation module 206 and modules A 56108, B 56106, and C 56104 are depicted as engaging in two way communication with the surgical hub 206. This two way communication is indicated by two directional arrows, such as arrow 56112. This two directional communication maybe accomplished, in an example, by connecting the modular housing ofthe smoke evacuation module 226 or modules A 56108, B 56106, and C 56104 to a modular backplane comprising an inner connector console. The modular backplane is configured to laterally or vertically connect to the modular housing of the smoke evacuation module 226 or modules A 56108, B 56106, and C 56104 with the surgical hub 206. An example of this is shown in FIGS. 27-31. In an alternate example, two way communication may be established by the housing of the smoke evacuation module 226 or modules A 56108, B 56106, and C 56104 being a stand alone housing that has a wired connection attachment on the back ofthe device that would allow it to interface with surgical hub 206. Alternatively, in another example, the smoke evacuation module 226 and modules A 56108, B 56106, and C 56104 may be connected directly to each other. In yet another example, the smoke evacuation module 226 and modules A 56108, B 56106, and C 56104 may comprise a wireless communication module to communicate with the surgical hub 206 and/or the cloud 204, either directly or indirectly. In the example illustrated in FIG. 20, the surgical hub 206 is depicted as being in two way communication with the cloud 204. Alternatively, in another example, the smoke evacuation module 226, as indicated by the broken line 56110, and also anyone of or all of the modules A 56108, B 56106, and C 56104, may be directly in communication with the cloud 204 in order to receive instructions or information directly from the cloud without proceeding through the surgical hub 206.

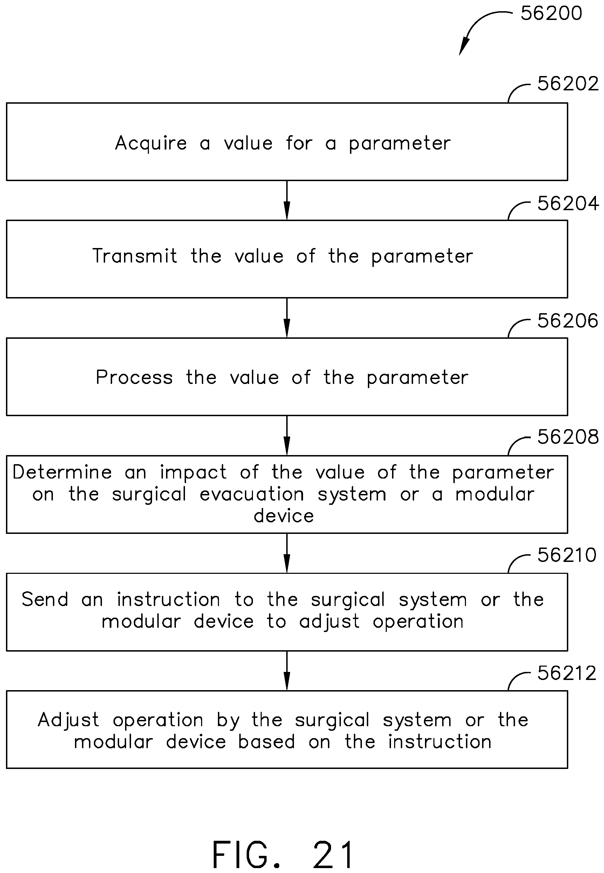

FIG. 21 depicts an example method flow diagram 56200 of interconnectivity between the surgical hub 206, the cloud 204, the smoke evacuation module 226, and/or various other modules A 56108, B 56106, and C 56104, in accordance with at least one aspect of the present disclosure. Although the example method 56200 is described with reference to the flowchart illustrated in Fig 21, it will be appreciated that many other methods of performing the acts associated with the method may be used. For example, the order of some of the blocks may be changed, certain blocks may be combined with other blocks, and some of the blocks described are optional.

With reference also to FIGS. 25-35, initially, a parameter is acquired 56202 by the smoke evacuation module 226. For example, a sensor component of the smoke evacuation module 226, such as the air quality particle sensor, will detect a parameter. This parameter may be any parameter related to the ambient environment of the surgical theater, such as, for example, the air quality of the surgical theater. Although in this example there is only one sensed parameter, this disclosure should not be limited in such a manner. In an alternate example, there may be many sensed parameters by any number of different sensors. How frequently the parameters are sensed, and how frequently the smoke evacuation module 226 is activated, is dependent upon the amount of smoke being evacuated. For example, if very little smoke is being evacuated, then the smoke evacuation module 226 may run more slowly and/or sense parameters less frequently. Alternatively, if the smoke evacuation module 226 is actively working to evacuate smoke that is rapidly being produced, it may be helpful for the smoke evacuation module 226 to run at a higher rate and/or sense parameters more frequently to ensure that the equipment is properly functioning. This may provide a safer environment for all surgeons, personnel, and medical equipment present in the surgical theater. Other parameters that may be sensed include those sensed by internal sensors, such as, for example, particulate concentration, aerosol percentage, or chemical analysis. Alternatively, operational parameters sensed by sensors on the smoke evacuation module 226 housing include, for example, air flow, pressure differentials, or air quality. A description of sensors used in the context of the smoke evacuation system 56100 maybe found at FIGS. 4-6, 18, and 19, for example.

Next, the smoke evacuation module 226 will transmit 56204 the value of this acquired parameter to the surgical hub 206 or the cloud 204. For example, if the value of the air quality of the surgical theater is acquired, it may be sent by the smoke evacuation module 226 to the surgical hub 206. The surgical hub 206 either may process the parameter, and/or it may communicate the received air quality parameter to the cloud 204. This may be accomplished via a network router 211, network hub 207, network switch 209, or any combination thereof. In an alternate example, the smoke evacuation module 226 may communicate the air quality parameter directly to the cloud 204 using wired or wireless communication techniques.

Next, the value of the parameter is processed 56206, and it is determined 56208 whether the value of the parameter has an impact on the way the surgical evacuation system 56100 or another modular device should operate. This processing may occur in the surgical hub 206, in the cloud 204, or a combination of the surgical hub 206 and the cloud 204. The processing performed on the sensed parameter includes using the value of the sensed parameter in various algorithms and learning software to determine if operation of the smoke evacuation module 226 or any of modules A 56108, B 56106, and C 56104 should be automatically adjusted to compensate for the value of the parameter that was detected. The value of the parameter is a smoke evacuation variable. The algorithms and learning software may be stored in the cloud 204 (storage 105) or surgical hub 206 (storage 248) prior to processing, and may be remotely updated as needed.

For example, if the value of the sensed air quality parameter indicates a large quantity of smoke particles in the air, the cloud 204 may contain software algorithms that can use the value of the air quality parameter to determine how to alter the operation of the smoke evacuation module or modules A 56108, B 56106, and C 56104 in order to improve the ambient air quality of the surgical theater. In the example, the cloud 204 may determine that the operation of an intelligent instrument 235 must be modified, and this operation is best adjusted by adjusting the output of the generator supplying waveforms to the intelligent instrument 235. A generator module 240 converts the electricity to high frequency waveforms comprised of oscillating electric currents, which are transmitted to the electrodes to effect tissue. In an alternate example, the cloud 204 may determine that none of modules A 56108, B 56106, and C 56104 must be adjusted, but that the operation of the smoke evacuation module 226 must be adjusted. In this alternate example, the cloud 204 may determine that the value of the air quality parameter indicates that the smoke evacuation module 226 may provide a safer environment if operation of the smoke evacuation module 226 is adjusted in order to evacuate smoke from the operating theater more quickly.

Next, an instruction is sent 56210 to the surgical evacuation system 56100 or the modular device to adjust operation. For example, the cloud 204 may determine that based off the value of the sensed air quality parameter, the operation of the intelligent instrument 235 should be adjusted. In the example, this was previously determined by the cloud 204 to be best effectuated by modifying the output of the generator module 240. Therefore, in this example step 56210, the cloud 204 sends an instruction to the generator module 240 (e.g., module A 56108). In the example, an instruction is sent to the generator module 240 that directs the output of the generator module 240 to be modified in order to change the operation of the intelligent instrument 235.

Finally, operation of the surgical evacuation system 56100 or the modular device is adjusted 56212 based on the instruction. For example, the generator module 240 will comply with the instruction it received from the cloud 204, and change the shape of the waveform output to the intelligent instrument 235. This adjustment causes the intelligent instrument 235 to effect tissue in a desired manner. In an example, the instructions to the various modules are pre-stored in the cloud 204 or the surgical hub 206, but may be updated remotely as needed. Alternatively, these instructions are dynamically generated by the cloud 204 or the surgical hub 206 as needed.

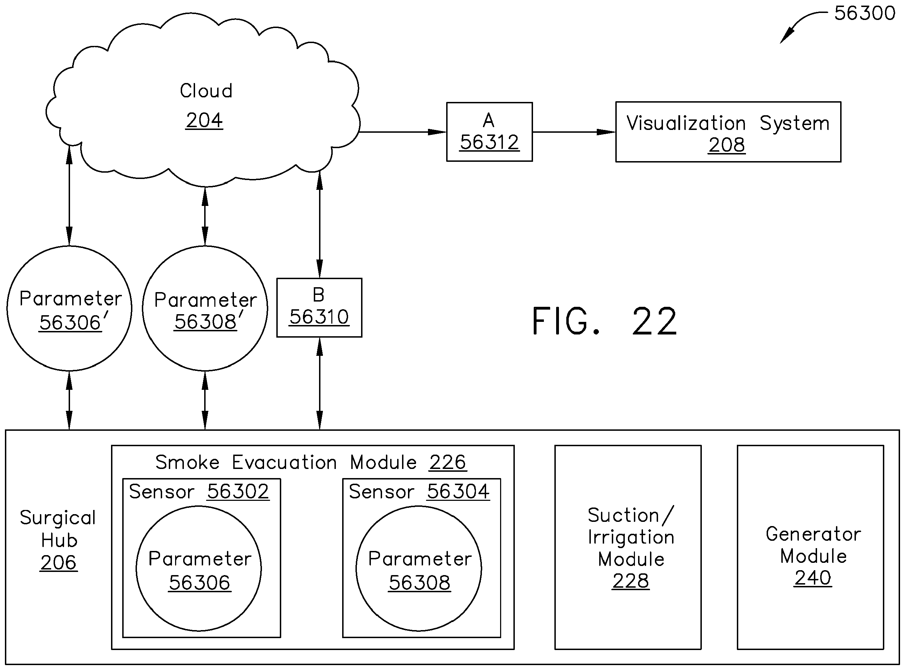

FIG. 22 illustrates a smoke evacuation system 56300 in communication with various other modules and devices, in accordance with at least one aspect of the present disclosure. The example illustrated in FIG. 22 discloses an alternative example of the computer implemented surgical system shown in FIG. 20. With continued reference to FIGS. 25-35 and to FIG. 22, a cloud 204 and a surgical hub 206 are depicted. Although the illustrated example depicts one cloud 204 and one surgical hub 206, in alternate examples there may be more than one cloud 204 and/or surgical hub 206 in the computer implemented surgical system.

In FIG. 22, the surgical hub 206 may include various modules, such as a suction/irrigation module 228, a smoke evacuation module 226, and a generator module 240. The smoke evacuation module 226 may contain a first sensor 56302 and a second sensor 56304 that detect different parameters. For example, the first sensor 56302 may be a chemical sensor performing a chemical analysis on the smoke being evacuated from a surgical theater, and the second sensor 56304 may be a laser particle counter determining particle concentration of the smoke being evacuated from the surgical theater. In an alternate example, there may be more or less than two sensors in the smoke evacuation module 226.

In the example, the first sensor 56302 senses a first parameter 56306, for example, the pH of the smoke being evacuated from the surgical theater. The second sensor 56304 senses a second parameter 56308, for example, particle count (parts per million) of the smoke being evacuated from the surgical theater. The smoke evacuation module 226 may be connected to surgical hub 206, or may be a part of the surgical hub 206, and therefore the first parameter 56306 and the second parameter 56308 may be provided to the surgical hub 206 automatically after being sensed. In an alternate example, the surgical hub 206 may request any sensed parameters periodically from the smoke evacuation module 226, or upon a user request for sensed parameter information, and the smoke evacuation module 226 will provide the values of sensed parameters upon request. Alternatively, the smoke evacuation module 226 may provide sensed values to the surgical hub 206 periodically without being requested.

In an example, the surgical hub 206 may process, modify, or manipulate the first parameter 56306 and the second parameter 56308 to become a first processed parameter 56306' and a second processed parameter 56308'. In an alternate example, the surgical hub 206 may not perform any processing of the first parameter 56306 and the second parameter 56308. The surgical hub 206 may send the first processed parameter 56306' and the second processed parameter 56308' to the cloud 204 for further processing in order to determine whether operation of the various modules should be adjusted based on the values of the sensed parameters. The cloud 204 may run a variety of algorithms to determine if the values of the first processed parameter 56306' and the second processed parameter 56308' indicate whether any of the other modules and devices, including the smoke evacuation module 226, should adjust operation in order to create a more ideal surgical environment in the surgical theater. The cloud 204 may, in this example, determine that the values of the first processed parameter 56306' and the second processed parameter 56308' are either above or below acceptable thresholds, and therefore operation of other modules should be adjusted to compensate for the sensed/detected values of the first processed parameter 56306' and the second processed parameter 56308'.

In the example shown in FIG. 22, the cloud 204 may determine that the display of the visualization system 208 should be adjusted to accurately depict the surgical site. In this case, the cloud 204 may send an instruction A 56312 to the visualization system 208 to adjust its display of the surgical site. The visualization system 208 is communicatively coupled to the surgical hub 206.

Further, in the example, the cloud 204 may determine after running a variety of algorithms that the operation of the suction/irrigation module 228 should also be adjusted based on either the value of the first processed parameter 56306' or the second processed parameter 56308'. In the example, an instruction B 56310 may be sent to the surgical hub 206 from the cloud 204. The surgical hub 206 may transmit the instruction B 56310 to the suction/irrigation module 228. In an alternative example, the surgical hub 206 may cause the processor 244 located in surgical hub 206 to adjust the operation of the suction/irrigation module 228. In another alternate example, the cloud 204 may send the instruction B 56310 directly to the suction/irrigation module 228 without first sending the instruction B 56310 through the surgical hub 206.

The cloud 204 may determine after running various algorithms that the generator module 240 may not benefit from an adjustment in operation, or that the sensed parameters may not be effectively influenced from an adjustment in operation of the generator module 240. Therefore, in the example illustrated in FIG. 22, the cloud 204 may not send an instruction to the generator module 240. Alternatively, the cloud 204 may send the generator module 240 an indication that no modification in operation is necessary.

### Smoke Evacuation System Containing a Segmented Control Circuit Where the Circuit is Energized in a Staged Method to Check for Errors, Shorts, and Safety Checks of the System

If faults in the smoke evacuation system are undetected, the smoke evacuation system may be unknowingly operated in a surgical context when the evacuation system is not functioning properly. This may cause an unsafe surgical environment where dangerous or carcinogenic smoke is not properly being filtered out of an operating theater. Alternatively, although some portions of a smoke evacuation system may be properly functioning, other portions may not be properly functioning. This may render the smoke evacuation system generally dangerous to energize and operate.

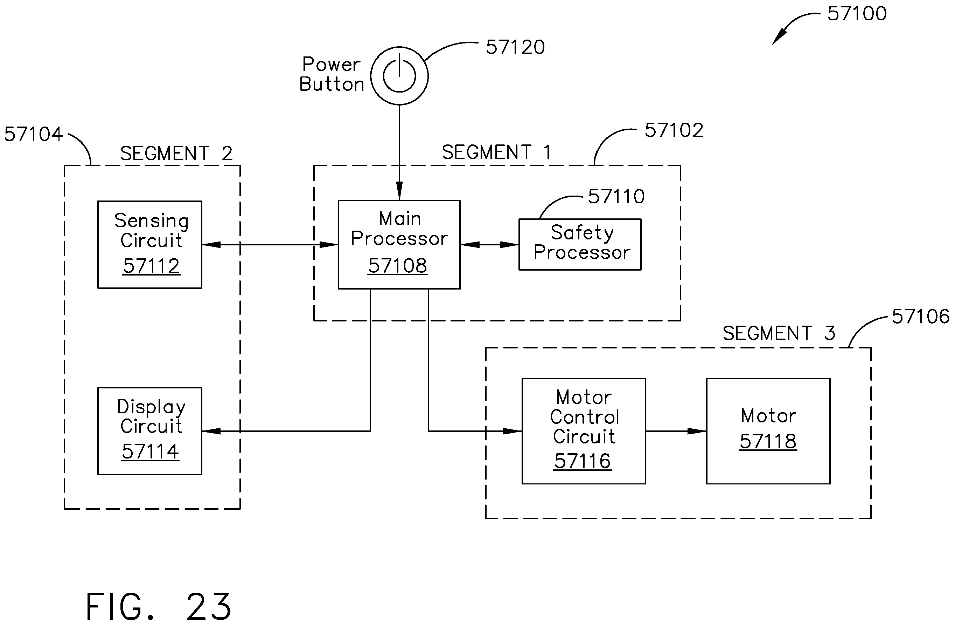

The smoke evacuation system 56100, 56300 comprising a smoke evacuation module 226 coupled to the surgical hub 206 and/or the cloud 204 as described in FIGS. 20-22, for example, may further comprise a segmented control circuit 57100 as described in FIG. 23. The segmented control circuit 57100 maybe energized in stages to check for faults, errors, shorts, and may run a variety of safety checks while starting up the smoke evacuation module 226 to help determine that the smoke evacuation module 226 is safe to operate.

By starting the smoke evacuation module 226 in a staged method, faults and errors may be identified before attempting to operate the smoke evacuation module 226. This may improve the overall safety of the surgical operation as the smoke evacuation system may be repaired, or if necessary, a replaced with a replacement smoke evacuation module.

In one aspect, the smoke evacuation module 226 described in FIGS. 20-22 comprises a segmented control circuit where the circuit may be energized in a staged method to check for errors, shorts, and perform safety checks of the system. FIG. 23 illustrates one aspect of a segmented control circuit 57100, in accordance with at least aspect of the present disclosure. It should be noted that although certain features, modules, processors, motors, etc. are grouped in various segments depicted in FIG. 23, the disclosure should not be limited to this example. In alternative examples, the segments may include more, less, or different components than those disclosed in FIG. 23. Further, in another alternate example, the number of segments in the example segmented circuit may be more or less than three segments.

In the example of FIG. 23, the segmented control circuit 57100 includes a first segment 57102, a second segment 57104, and a third segment 57106. In an example, the first segment 57102 includes a main processor 57108 and a safety processor 57110. The main processor 57108 maybe directly wired to a power button or switch 57120. Therefore, in this example, the first segment 57102, and specifically the main processor 57108, is the first segment 57102 and component 57108 to be activated.

The safety processor 57110 and/or the main processor 57108 may be configured to interact with one or more additional circuit segments and their components, such as the second segment 57104, which may include sensing circuit 57112 and display circuit 57114, and the third segment 57106, which may include a motor control circuit 57116 and a motor 57118. Each of the circuit segments and their components may be coupled to or in communication with the safety processor 57110 and/or the main processor 57108. The main processor 57108 and/or the safety processor 57110 may also be coupled to a memory, or include an internal memory. The main processor 57108 and/or the safety processor 57110 may also be coupled to or in wired or wireless communication with a communication device that will allow communication over a network such as the surgical hub 206 and/or the cloud 204.

The main processor 57108 may comprise a plurality of inputs coupled to, for example, one or more circuit segments, a battery, and/or a plurality of switches. The segmented control circuit 57100 may be implemented by any suitable circuit, such as, for example, a printed circuit board assembly (PCBA) within the smoke evacuation module 226. It should be understood that the term processor as used herein includes any microprocessor, processors, controller, controllers, or other basic computing device that incorporates the functions of a computer's central processing unit (CPU) on an integrated circuit or, at most, a few integrated circuits. The main processor 57108 and safety processor 57110 are multipurpose, programmable devices that accept digital data as input, processes it according to instructions stored in its memory, and provides results as output. It is an example of sequential digital logic, as it has internal memory. The safety processor 57110 may be configured specifically for safety critical applications, among others, to provide advanced integrated safety features while delivering scalable performance, connectivity, and memory options.

In the example shown in FIG. 23, the second segment 57104 may comprise a sensing circuit 57112 and a display circuit 57114 both coupled to or in signal communication with the main processor 57108. The sensing circuit 57112 of the smoke evacuation module 226 described generally herein includes various sensors, some being internal to the smoke evacuation module 226 and some being external and located on the housing of the smoke evacuation module 226. These sensors include various pressure sensors, fluid sensors, chemical sensors, laser particle counters or other laser sensors, and air quality sensors. A description of sensors used in the context of the smoke evacuation system 56100 may be found at FIGS. 4-6, 18, and 19, for example.

In an alternate aspect, the sensing circuit 57112 may include a communication device as some sensors located on the external housing of the smoke evacuation module 226 may involve communication with and/or connectivity to the sensing circuit 57112, the main processor 57108, and/or the safety processor 57102 via the communication device. The display circuit 57114 may include a screen or other display that may display text, images, graphs, charts, etc. obtained by the smoke evacuation module 226 or another device in communication with the smoke evacuation module 226 or the surgical hub 206. For example, the display circuit 57114 may include a monitor or screen (e.g., display 210, 217 or monitor 135) that may display and overlay images or data received from the imaging module 238, the device/instrument 235, and/or other visualization systems 208. This display or screen may present to the user the status of each segment, or status of each component in each segment, in the segmented control circuit 57100. The status of each segment, or the status of each component of each segment, may be discerned by the segmented control circuit 57100 by identifying each component or segment, receiving or determining a status of each component or segment, and displaying the status to the user for each component or segment. A component or segment may be identified using, for example, a unique data packet format, an authentication, an encryption, an ID number, a password, a signal communication, a flag, etc. A status of a component or segment may be determined using a flag, an indication, a value of a sensed parameter, a measurement, etc.

These functions (identification, determination, and display) maybe performed using, for example, Wi-Fi, a wired connection, signal communication, Thread, Bluetooth, RFID, or NFC. For example, components of segmented control circuit 57100 may include a communication device to communicate over a network, or may be coupled to an external communication device allowing for communication over a network. The communication devices may include a transceiver configured to communicate over physical wires or wirelessly. The device may further include one or more additional transceivers. The transceivers may include, but are not limited to cellular modems, wireless mesh network transceivers, Wi-Fi® transceivers, low power wide area (LPWA) transceivers, and/or near field communications transceivers (NFC). The communication device may include or may be configured to communicate with the main processor 57108, the safety processor 57110, the display circuit 57114, the surgical hub 206, or the cloud 204, which may verify the identities of, or may receive the identities of, the components and their associated status. The transceivers may be configured to receive serial transmit data via respective UARTs, from the processor, to modulate the serial transmit data onto an RF carrier to produce a transmit RF signal and to transmit the RF signal via respective antennas. Each transceiver may correspond to an implementation of a selected wireless communication standard and/or protocol, e.g., IEEE 802.11 a/b/g/n for Wi-Fi® and/or IEEE 802.15.4 for wireless mesh networks using Zigbee routing, as described herein. In alternative aspects, the display circuit may include a small speaker to audibly signal or a small light/display to indicate the status of the smoke detector, an on/off operation, filter life remaining, etc. Identification of each component and their status may be provided voluntarily by each component, sending or transmitting such information at regular or irregular time intervals. An example of an irregular time interval may be, for example, when a user directs information to be sent. In an alternate example, the identity and status information of each component or segment may be requested at regular or irregular time intervals. For example, an identity and status of the motor control circuit 57116 may be requested every 1 minute, 20 minutes, 40 minutes, one hour, 3 hours, 24 hours, etc.

In an alternate aspect, the segmented control circuit 57100 allows for the energizing of a status circuit that allows the smoke evacuation module 226 to query or poll all attached components. The status circuit may be located in any of the segments, but for this example may be located in the first segment 57102. This status circuit may be able to query/poll all components, including those components in the first segment 57102, the second segment 57104, the third segment 57106, as well as any other additional components such as those depicted in FIGS. 25-35. The polling/querying of the attached components may be done in order to verify compatibility, that the component or segment may be compatible with the smoke evacuation system or hub; authenticity, that the component or segment may be the correct component and can be trusted; used status, or the status of any segment or component that has a life span based on use, such as a filter; and correct insertion of the segment or component, such as correctly inserting a filter before energizing the motor and the pump connected to the motor. In an example, the intelligent instrument, such as intelligent instrument 235, may further be queried/polled in order to verify that the proper devices are being used and that the smoke evacuator system is able to function, or knows how to function, with the polled/queried intelligent instrument. For example, the intelligent instrument 235 polled or queried may be a Zip Pen®. Knowing which Zip Pen® is being used may help the smoke evacuation system determine how to function. If an unidentified intelligent instrument is being used, the smoke evacuation system may determine energizing the motor is inappropriate or unsafe and an error may be displayed by the display circuit 57114. This may be done for any component of the smart surgical environment, and not merely the intelligent instrument 235. Alternatively, if an unidentified intelligent instrument is being used, the smoke evacuation system may determine that the device may be used in a universally safe operating mode.

Energizing a status circuit, as noted above, that allows the smoke evacuation module 226 to query/poll other components may also provide for proprietary identification, authentication, and status text strings for the queried/polled components. This allows for a controlled identification of the smoke evacuation module 226 and sub-components of the smoke evacuation module 226 in order to keep mimicking devices from fooling the security device. For example, an example attempt at fooling the security device is by building a "skimmer" to make unique connections. In order to prevent the system from being fooled, an operation may be performed to authenticate each component. Each component attached to the smoke evacuation system may be authenticated based on stored data, security encryption, authentication, parameters, or some combination thereof. The data stored may include an identification number or another identifier, a product name, and a product type, a unique device identifier, a company trademark, a serial number and/or other manufacturing data, configuration parameters, usage information, features enabled or disabled, or an algorithm/instruction on how to use the attached component.

In the example shown in FIG. 23, the third segment 57106 may include a motor control circuit 57116 and a motor 57118. The motor control circuit 57116 and/or motor 57118 maybe coupled to or in signal communication with the main processor 57108 and/or the safety processor 57110. The main processor 57108 may direct the motor control circuit 57116 to reduce or increase the speed of the motor 57118. In an alternate example, the motor control circuit 57116 and the motor 57118 may be coupled to or in signal communication with the safety processor 57110. The motor 57118 maybe coupled to a pump in the smoke evacuation module 226 (see FIGS. 4 and 6-19), and operation of the motor 57118 may cause the pump to function. Specifically, in an example, the motor 57118 may operate at two different states. The first state may be when the motor 57118 is activated by a user. In this first state the motor 57118 may run at a higher rate than in the second state. In the second state, the motor 57118, the motor control circuit 57116, the main processor 57108, or the safety processor 57110 may sense a lack of user activity or may receive an instruction from the main processor 57108, the safety processor 57110, the surgical hub 204, the cloud 206, or another module located within the surgical hub 206 (e.g., processor module 232) to slow the rate of operation of the motor 57118.

The number of different states the motor 57118 may operate at is not limited to two. The motor 57118 may change or choose its state of operation based on various measured values of parameters. In another aspect, the motor 57118 may determine or set its operating state based on user activity levels falling within, above, or below specific thresholds. Further, these thresholds may relate to or interrelate with thresholds of other modules or components of the smoke evacuation module 226.

In an alternate example, the motor 57118 may optionally have a mode called sleep mode. In this example, sleep mode may be activated, or the motor 57118 may be shut down into a sleep mode (quite mode, or low power mode), when not used for a predetermined amount of time. The threshold amount of time of inactivity prior to entering into sleep mode may vary depending on a number of factors, including the type of surgical procedure, the specific surgeon performing the procedure, or other factors. Therefore, the predetermined amount of time may be preset, but may be easily adjusted. For example, the threshold from switching from the example active state to the sleep mode state maybe ten minutes. In this example, if an intelligent instrument 235 is not used by a surgeon in more than ten minutes, the motor control circuit 57116 or motor 57118 may receive an instruction to enter into sleep mode. In an alternate example, if the intelligent instrument 235 is not used by the surgeon in more than ten minutes, the intelligent instrument 235 may automatically activate a sleep mode by slowing the motor 57118 to the sleep mode state. Further, when the smoke evacuation module 226 or intelligent instrument 235 is used again or senses user interaction, the smoke evacuation module 226 may undergo a wakeup sequence to re-energize the segmented circuit 57100. This wakeup sequence may be the same as the start-up sequence used to energize the segmented circuit 57100 while checking for errors or faults. Alternatively, the wake up sequence may be an abbreviated version of the start-up sequence to quickly re-energize the segmented circuit 57100 when user interaction is sensed.

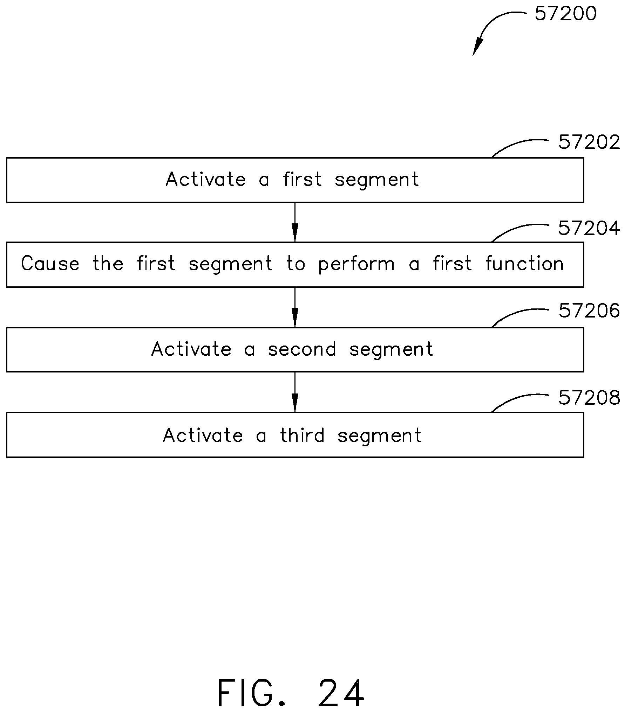

The example shown in FIG. 24 discloses a method 57200 of operating a control circuit portion of the smoke evacuation module 226 so as to energize the segmented control circuit 57100 in stages, in accordance with at least one aspect of the present disclosure. This way various portions of the control circuit will be able to perform safety checks prior to energizing subsequent segments, providing a safe electrical startup circuit method. Although the example method 57200 is described with reference to the flowchart illustrated in Fig 24, it will be appreciated that many other methods of performing the acts associated with the method may be used. For example, the order of some of the blocks may be changed, certain blocks may be combined with other blocks, and some of the blocks described are optional.

According to the method 57200, the control circuit 57100 first activates 57202 a first segment 57102. For example, as depicted previously in FIG. 23, a first segment 57102 may include a main processor 57108 and a safety processor 57110. In an alternate example, other components also maybe included in the first segment 57102, or the first segment 57102 may not include a main processor 57108 and/or a safety processor 57110.

Next, the first segment 57102 performs a first function 57204. This first function, for example, may be related to verifying the safety of the control circuit 57100 and/or smoke evacuation module 226. For example, the main processor 57108 or the safety processor 57110 may check each segment or the components of the segments for short circuits and other errors prior to engaging the high power portions of the segmented control circuit 57100. The main processor 57108 and/or the safety processor 57110 may perform safety checks on each component or each segment to determine they are properly functioning. Further, in another example, the main processor 57108 or the safety processor 57110 may perform a verification function to ensure components in the smoke evacuation module 226 are the correct components. For example, the main processor 57108 or the safety processor 57110 may include a Trusted Platform Module (TPM), or the TPM may be a separate component in the segmented control circuit 57100, for ensuring components of the smoke evacuator system 56100, 56300 are the correct components. This verification may be done in a variety of ways. For example, one example method is to have stored in the TPM a key for each component or segment. If the connected components are compared and the key for each component or segment is not found within the TPM, the smoke evacuation module 226 may not enable or power those modules. In a further example, the key discussed may incorporate encryption or, alternatively, may require authentication by a component of the smoke evacuation module 226, such as the main processor 57108 or the safety processor 57110.

Further, for example, the main processor 57108 and/or the safety processor 57110 may include switching logic stored in memory that prevents the segmented control circuit 57100 from activating portions of the circuit if specific safety conditions are not met. For example, there may exist a variety of conditions stored within the safety processor 57118 affiliated with a logic switch or a plurality of logic switches. For example, if a protection housing is not fully closed, if a filter module is not fully or properly inserted, if a fluid reservoir is not fully or properly inserted, if a hosing is not properly or fully inserted, and if a fluid reservoir is not adequately full, a logic switch may be triggered and an affiliated portion of the segmented control circuit 57100 or smoke evacuation module 226 may not be activated.

In an alternate example, there may exist a separate enclosure circuit to ensure the housing is fully and properly closed before allowing the smoke evacuation module 226, or one of the various segments, to be activated or operated. For example, if the housing is not fully closed, the circuit may not be complete. Therefore, the remaining segments or components of the segmented control circuit 57100 may not be activated. This enclosure circuit concept may be used in a tamper circuit to prevent unauthorized servicing. For example, a tamper circuit may include a confirmation circuit. If a housing is opened that should not be opened, the confirmation circuit may be permanently broken either electrically or mechanically. This breakable confirmation circuit has an inner connection strip which may bridge two rigidly fixed electrical connection blocks, one block may be located on a shell of the enclosure and the other block may be located on the base frame of the enclosure. The inner connection my have a rigid insertable electrical connector that may lock into the respective connection blocks with a latching feature on each of the shell and the base frame. When broken, it may be evident that the housing has been opened or tampered with. Once broken, the electrical connectors may be easily released and replaced once the housing is opened in order for replacement. In an alternate aspect, the blocks and latching features may be located at different positions, and each segment may include separate blocks and latching features. For example, the first segment 57102, the second segment 57104, and the third segment 57106 may each have a separate confirmation circuit. If the first segment 57102 is tampered with or opened, the confirmation circuit may be broken as the rigid inner connection strip may be broken, and therefore can be identified as being tampered with and/or the confirmation circuit may be replaced. By having separate confirmation circuits, it may be easily detectable if any of the various segments or components of the segmented control circuit 57100 has been tampered with.

Alternatively, an enclosure circuit may be used as a segmented security device. For example, if a portion of the system requires servicing, an enclosure circuit may enable a portion of the system to be opened and serviced, but prevent use of the segmented control circuit 57100 or smoke evacuation module 226 after an unauthorized, separate portion of the segmented control circuit 57100 may have been opened, dismantled, or modified. For example, if the smoke evacuation system's 226 segmented control circuit 57100 should be serviced, but only the display circuit 57114 needs servicing, there may be no reason for a service technician or other personnel to service the third segment 57106 or the first segment 57102. Therefore, the third segment 57106 and the first segment 57102 may include separate enclosure circuits containing single use fuses or other mechanical/electromechanical devices, or digital versions of the like. Once the third segment 57106 and the first segment 57102 are opened, modified, or dismantled, the fuses may blow, allowing no power to reach the third segment 57106 and the first segment 57102.

If an error, short circuit, fault, or other issue is detected, the segmented control circuit 57100 may be capable of running at least one segment of the segmented control circuit 57100, while not running other segments. In order to ensure safety of the segmented control circuit 57100, portions of the segmented control circuit 57100, and their connections therebetween, may be physically switched, fused, or breakered allowing the switches, fuses, and breakers to trip and prevent energizing if an unsafe condition exists. Any suitable mechanical, electromechanical, or solid state switches may be employed to implement the plurality of switches. For example, there may exist a single-use fuse, reset-able fuse, or a solid state switching device existing between the main processor 57108 and the motor control circuit 57116. Generally, a fuse is an electrical safety device that operates to provide overcurrent protection of an electrical circuit. It may include a metal wire or strip that melts when too much current flows through it, thereby interrupting the current. Generally, a resettable fuse is a polymeric positive temperature coefficient (PPTC) device that is a passive electronic component used to protect against overcurrent faults in electronic circuits. The device may be also known as a polyfuse or polyswitch. Generally, a solid-state switch operates under the influence of a magnetic field such as Hall-effect devices, magneto-resistive (MR) devices, giant magneto-resistive (GMR) devices, and magnetometers, among others. In other implementations, the switches may be solid-state switches that operate under the influence of light, such as optical sensors, infrared sensors, and ultraviolet sensors, among others. Still, the switches may be solid-state devices such as transistors (e.g., FET, Junction-FET, metal-oxide semiconductor-FET (MOSFET), bipolar, and the like). Other switches may include wireless switches, ultrasonic switches, accelerometers, and inertial sensors, among others.

Next, a second segment may be activated 57206. For example, the second segment 57104, including the sensing circuit 57112 and the display circuit 57114, may be energized. Next, a third segment maybe activated 57208. For example, the third segment 57106, including the motor control circuit 57116 and the motor 57118, may be energized. Although this example aspect discloses activating the first segment 57102 before the second, and the second segment 57104 before the third segment 57106, the order of segment activation may be modified if deemed appropriate. Further, in an alternate example, a variety of error checks, verifications, etc. may be performed between energizing the second segment 57104 and the third segment 57106.

The reader will readily appreciate that various surgical evacuation systems and components described herein can be incorporated into a computer-implemented interactive surgical system, a surgical hub, and/or a robotic system. For example, a surgical evacuation system can communicate data to a surgical hub, a robotic system, and/or a computer-implanted interactive surgical system and/or can receive data from a surgical hub, robotic system, and/or a computer-implemented interactive surgical system. Various examples of computer-implemented interactive surgical systems, robotic systems, and surgical hubs are further described below.

### Surgical Evacuation System With A Communication Circuit For Communication Between A Filter And A Smoke Evacuation Device

Generally, providing network services to medical devices may expose vulnerabilities of the medical devices to malicious attacks. Although there may be network-wide firewall services provided in the network system, these services may be vulnerable to security attacks from components inside the medical devices. That is, the firewall services may not have information with respect to the types, products, configurations, or authenticity of the components of the medical devices and, thus, may not be able to protect the medical device system from malicious attacks coming from unauthentic/unauthorized components of the medical devices. For example, an unauthentic/unauthorized component (e.g., filter device) may include ransomware, which may deny access of the medical device user to the medical device or the data in the medical device until a ransom payment is paid. Moreover, the unauthentic/unauthorized component may not be compatible with other authentic/authorized components of the medical device, which may result in reduction in the lifetime and/or performance of the overall medical components. This may also lead to an unexpected interruption in operation of the medical devices.

Aspects of the present disclosure may address the above-noted deficiencies. In some examples, a surgical evacuation system may include a communication circuit that may facilitate communication between a smoke evacuation device and a replaceable filter device having a plurality of filter components. The communication circuit may authenticate the filter device (including the plurality of filter components), verify a remaining life of the filter device, update parameters output from the filter device, and record errors output from the filter device. The communication circuit may limit a number or a type of a filter component that can be identified by the surgical evacuation system, and may enable/disable the plurality of filter components based on a result of the authentication. In some examples, the communication circuit may authenticate the filter device/components by using filter component information, which may include a product type, a product name, a unique device identifier, a product trademark, a serial number, or a configuration parameter of the filter device/components. In some examples, the filter device and/or the communication circuit may encrypt or decrypt data/parameters communicated between the filter device and the communication circuit.

In this way, the surgical evacuation system according to an example embodiment of the present disclosure may be able to detect an unauthentic/unauthorized component of a medical device, protect data/parameters of the medical device by encryption, and check possible issues in the filter device in advance by checking the remaining life and errors of the filter components. This may advantageously allow the surgical system to prevent possible malicious attacks and degradation in performance that may be caused by the unauthentic/unauthorized component and to continue to operate the surgical evacuation system without an unexpected interruption in the medical device operation.

FIG. 25 depicts a high-level component diagram of an example smoke evacuation system 58100 in accordance with one or more aspects of the present disclosure. The smoke evacuation system 58100 may include a smoke evacuation device 58105 having a pump 58160 and a motor 58165 operably coupled to the pump 58160, a display device 58170, a communication device 58180, a processor 58110, a memory 58120, and one or more sensors 58140A-B. In some examples, the smoke evacuation device 58105 may include a filter device 58150 and a filter communication circuit 58130. The filter device 58150 maybe in communication with the smoke evacuation device 58105 (e.g., processor 58110) through the filter communication circuit 58130.

The smoke evacuation system 58100 maybe similar to the smoke evacuation system depicted in FIG. 6. For example, the processor 58110 may be in signal communication with a motor driver or the motor 58165, various sensors 58140A-B, the display device 58170, the memory 58120, and the communication device 58180. The communication device 58180 may be similar to the communication device described above with respect to FIGS. 5 and 6. That is, the communication device 58180 may allow the processor 58110 in the smoke evacuation system 58100 to communicate with other devices within the surgical system. For example, the communication device 58180 may allow wired and/or wireless communication to external sensors, surgical devices, hubs, clouds, and/or various additional surgical systems and/or tools. The reader will readily appreciate that the smoke evacuation system of FIG. 25 can be incorporated into the surgical system of FIG. 5 in certain instances.

In some examples, the filter device 58150 maybe coupled to a sucking conduit 58155. An exhaust mechanism 58190 maybe coupled to the pump 58160. The exhaust mechanism 58190 may be similar to the exhaust mechanism 50520. In some examples, the suction conduit 58155, the filter device 58150, the pump 58160, and the exhaust mechanism 58190 may be sequentially arranged in-line with a flow path (e.g., the flow path 50504) between an inlet port (e.g., inlet port 50522) and an outlet port (e.g., outlet port 50524). The inlet may be fluidically coupled to the suction conduit 58155 comprising a distal conduit opening at the surgical site. Although the exhaust mechanism 58190 is depicted as located outside of the smoke evacuation device 58105, in some examples, the exhaust mechanism 58190 may be located in the smoke evacuation device 58105.

In some examples, the processor 58110 maybe in signal communication with the filter communication circuit 58130 for communication between the filter device 58150 and the smoke evacuation device 58105. In some examples, the filter communication circuit 58130 may be located in the smoke evacuation device 58105 or the filter device 58150. In other examples, the communication circuit 58130 may be located outside of the smoke evacuation device 58105. In some examples, the communication circuit 58130 may be part of the sensing and intelligent controls device depicted in FIG. 5.

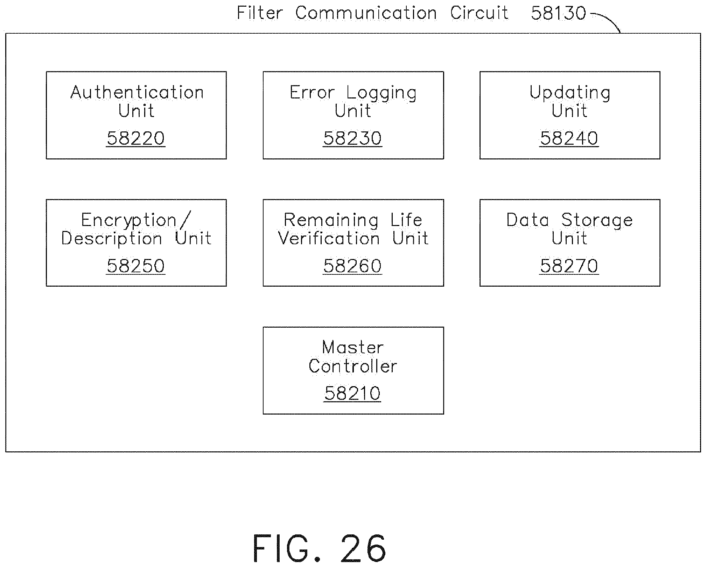

FIG. 26 illustrates a filter communication circuit 58130 according to an example embodiment of the present disclosure. The filter communication circuit 58220 may include a master controller 58210, an authentication unit 58220, an error logging unit 58230, an updating unit 58240, an encryption/description unit 58250, a remaining life verification unit 58260, and a data storage unit 58270. In some examples, the master controller 58210 maybe in signal communication with the processor 58110 and control other units 58220-58270 in the filter communication circuit 58130. In other examples, the processor 58110 may act as the master controller 58210.

FIG. 27 illustrates a filter device 58150 according to an example embodiment of the present disclosure. The filter device 58150 may include a plurality of filter components. The filter components may include a controller 58310, a filter element unit 58320, and a filter sensor unit 58330. The filter element unit 58320 may include one or more filter elements 58325A-C. The filter sensor unit 58330 may include one or more filter sensors 58335A-C. The controller 58310 may control and be in communication with the filter element unit 58320 and the filter sensor unit 58330. The filter device 58150 may be similar to the examples (e.g., filter 50670) depicted in FIGS. 10, 11, 18, and 19. In some examples, the one or more filter elements 58325A-C may be the fluid filter, coarse media filter 50684, fine particulate filter 50686, particulate filter, carbon reservoir 50688, or charcoal filter depicted in FIGS. 10, 11, 18, and 19 or any other filters in the filter device 58150. The filter elements 58325A-C may also include the diverter valve, the baffles, the squirrel cage, or any other elements (e.g., dams 50690, 50692, 50694, back cap 50674, etc.) in the filter device other than the sensors. In some examples, the one or more filter sensors 58335A-C maybe similar to the examples (e.g., fluid detect sensors 50830, chemical sensor 50832, fluid sensor 50836, pressure sensor 50840, laser particle counter 50838, etc.) depicted in FIGS. 18 and 19.

In some examples, the controller 58310 in the filter device 58150 may be in signal communication with the master controller 58210 of the filter communication circuit 58130. In some examples, the filter device 58150 may encrypt the parameters output from the plurality of filter components before sending the parameters to the communication circuit 58130. Upon receiving the encrypted parameters, the communication circuit (e.g., encryption/description unit 58250) may decrypt the encrypted parameters as discussed below.

Referring to FIG. 26 again, the authentication unit 58220 may authenticate/verify the filter device 58150 or the plurality of filter components. In some examples, the authentication unit 58220 may identify the number of filter components that is attached in the filter device 58150. The authentication unit 58220 may also limit the number or type of a filter component that can be identified by the communication circuit. For example, if the filter component is not the type of a component authorized to be used in the filter device 58150, or the number of filter components used in the filter device 58150 is equal to or greater than a predetermined value (e.g., 10, 20, 50, etc.), the authentication unit 58220 may disable the filter component or the filter device 58150. The authentication unit 58220 may also enable or disable the filter device 58150 or the plurality of filter components based on a result of the authentication.

The error logging unit 58230 may record errors or error messages from the plurality of filter components or the filter device 58150. In some examples, the error logging unit 58230 may record the errors and error messages in the data storage unit 58270. The filter communication circuit 58130 may read the error messages and use the error messages to figure out what happened in the filter device 58150. Examples of the errors and error messages may include errors that occur due to sensor/filter failures, strange/dangerous chemicals detected by sensors/filters, moisture detected in particulate filters; clogged filters, pressure differential (e.g., between the pressure sensors 50840 and 50846) over a predetermined value, and unauthentic/unauthorized filter device/component.

The updating unit 58240 may update parameters output from the plurality of filter components. The parameters updated may be operational or sensed. Operational parameters may include airflow, pressure differentials, air quality, or any other parameters related to the operation of the filter device 58150. Sensed parameters may include particulate concentration, aerosol percentage, chemical analysis, or any other values sensed by the sensors (e.g., pressure, fluid, chemical, particle) in the filter device 58150. These parameters may be stored in the data storage unit 58270, and automatically or manually updated by the updating unit 58240. For example, the updating unit 58240 may update the pressure differential values stored in the data storage unit 58270 when a change in the pressure differentials is detected by the pressure sensors (e.g., 50840, 50846). The filter communication circuit 58130 may receive these parameters directly from each of the filter components (e.g., filter elements 58325A-C/filter sensors 58335A-C) or through the slave controller 58310.

The encryption/description unit 58250 may encrypt or decrypt the parameters output from the plurality of filter components. The encryption/description unit 58250 may encrypt or decrypt any data or packet received from the filter device 58150. In some examples, the filter device 58150 may also include an encryption/description unit similar to the encryption/description unit 58250. The encryption/description unit of the filter device 58150 may encrypt the parameters output from the plurality of filter components before sending the parameters to the filter communication circuit 58130 and decrypt the data from the filter communication circuit 58130. The encrypted data/parameters communicated between the filter device 58150 and the filter communication circuit 58130 may not be visible/readable to filter components, filter device 58150, and the smoke evacuation device 58105 unless the encrypted data/parameters are decrypted.

In some examples, the encryption/description unit 58250 and the filter device 58150 may encrypt or decrypt the data/parameters by symmetrical encryption, which uses the same (secret) key to encrypt and decrypt the data. In other examples, the encryption/description unit 58250 and the filter device 58150 may encrypt or decrypt the data/parameters by asymmetrical encryption, which uses public and private keys to encrypt and decrypt data. In the asymmetrical encryption, one of the private/public key may be used to encrypt the data, and the other key may be used to decrypt the data.

The remaining life verification unit 58260 may verify/predict the remaining life of the plurality of filter components. In some examples, the remaining life verification unit 58260 may use usage information about the plurality of filter components to verify the remaining life of the filter components. The filter component usage information may include time of use data, the number of times each filter component was used, the number or type of errors that each filter component generated, a standard lifetime of each filter component, and pressure differentials between the pressure sensors located upstream (e.g., 50840) and downstream (e.g., 50846) ofthe filter elements 58325A-C. In some examples, if the pressure differential value of a filter element 58325A (e.g., ULPA filter) exceeds a predetermined value, which may indicate that the filter element 58325A is clogged, the remaining life verification unit 58260 may determine that the remaining life of the filter element 58325A is zero or will become zero soon, for example, within a predetermined time period (e.g., 1-5 hours, 1-5 days, 1-5 weeks, or 1-5 months) and it should be replaced. If the filter component usage information indicates that a significant amount of moisture was entered into a particulate or charcoal filter, the remaining life verification unit 58260 may determine that the remaining life of the particulate or charcoal filter is zero or will become zero soon, for example, within a predetermined time period (e.g., 1-5 hours, 1-5 days, 1-5 weeks, or 1-5 months) and it should be replaced. If the filter component usage information indicates that there is an error in a filter sensor 58335A or filter element 58325A (e.g., not operate properly), the remaining life verification unit 58260 may determine that the filter sensor 58335A or filter element 58325A is zero or will become zero soon, for example, within a predetermined time period (e.g., 1-5 hours, 1-5 days, 1-5 weeks, or 1-5 months) and it should be replaced. In some examples, the filter component usage information may be stored in the data storage unit 58270.

The data storage unit 58270 may store information about the filter components. The filter component information may include a product type, a product name, a unique device identifier, a product trademark, a serial number, and a configuration parameter of the plurality of filter components. In some examples, the information about the filter components may be generated, for example, from the filter component when the authentication unit 58220 authenticates/verifies the filter components. In some examples, the data storage unit 58270 may also include information about authentic/authorized filter components. The authentic filter component information may include a list of product types, product names, unique device identifiers, product trademarks, serial numbers, and configuration parameters of authentic/authorized filter components. In some examples, the filter component information and/or the authentic filter component information may be stored in plain text. In other examples, the filter component information and/or the authentic filter component information may be stored in an encrypted form. In some examples, the data storage unit 58270 may also store information about features disabled and enabled and algorithm or instructions for how the smoke evacuation device 58105 may use the filter components.

In some examples, the data/parameters from the filter device 58150 may be delivered to the smoke evacuation device 58105 (e.g., data storage unit 58270) nonsequentially, for example, as a data packet. As used herein, a data packet may refer to the unit of data that is communicated between two devices (e.g., filter device 58150 and smoke evacuation device 58105). The smoke evacuation device 58105 (e.g., processor 58110, master controller 58210) may know how to combine the received data packets into the original data/parameters.

In some examples, the authentication unit 58220 may authenticate/verify the plurality of filter components in the filter device 58150 by using the filter component information and/or the authentic filter component information. For example, the authentication unit 58220 may compare the filter component information of the filter components with the authentic filter component information. That is, the authentication unit 58220 may check whether the filter component information (e.g., unique device identifier/trademark/serial number of a filter in the filter device 58150) matches the prestored authentic filter component information (e.g., in the list of unique device identifiers/trademarks/serial numbers of authentic filter components). If it is determined that the filter component information of a filter component does not match the authentic filter component information, the authentication unit 58220 may determine that the filter component is not authentic/authorized. If it is determined that a filter component is not authentic, the authentication unit 58220 may disable part or the whole filter device functions (e.g., smoke filtering, smoke sensing, data processing, etc.) or the filter device/components. In some examples, the authentication unit 58220 may disable the filter device/components or filter device functions by stopping the pump 58160/motor 58165 or closing an input port of one of the filters.

In some examples, the serial number may be located in a chip, such as Erasable Programmable Read-Only Memory (EPROM) or Electrically Erasable Programmable Read-Only Memory (EEPROM) of the filter device/components (e.g., slave controller 58310). For example, in some cases, only certain family of chips may be used for authentic filter devices/components and the serial numbers on those chips may indicate that the filter devices/components having the chips are authentic. In some examples, when the filter device 58150 is connected to the smoke evacuation device 58105, the authentication unit 58220 may read the serial number of the chip (e.g., EPROM/EEPROM) in the filter components and check whether it is authentic. In some examples, the authentication unit 58220 may be programmed to accept a set serial number range.

In some examples, the filter communication circuit 58130 (e.g., master controller 58210) may act as a master device and the filter device 58150 (e.g., plurality of filter components, including the slave controller 58310) may act as a slave device. In some examples, the communication between the master device and the slave device may be unidirectional from the master device to the slave device when performing the authentication step. That is, it may be only the master device that is able to authenticate/verify the slave device, and the slave device cannot authenticate/verify the master device. In this case, the slave device may only provide information (e.g., filter component information, including a product type, a product name, a unique device identifier, a product trademark, a serial number, and a configuration parameter of the filter components) requested from the master device. In some examples, the communication between the master device and the slave device may be bidirectional.

In some examples, the plurality of filter components may have a tiered structure. For example, one (e.g., slave controller 58310) of the filter components may act as a master component and the rest of the filter components may act as a slave component. In this case, the rest of the filter components may report data/parameters directly to the master component, which in turn may report the received data/parameters to the master device (master controller 58210). In other examples, each of the filter components may report data/parameters directly to the master device.

In some examples, the smoke evacuation device 58105 and the filter device 58150 may communicate with each other, for example, through the filter communication circuit 58130, using a (bidirectional or unidirectional) wireless connection. Examples of the wireless connection may include RFID (read only or read/write), Bluetooth, Zigbee, IR, or any other suitable wireless protocols. In other examples, the smoke evacuation device 58105 and the filter device 58150 may communicate with each other using a wire connection. In this case, an electrical connector is provided between the smoke evacuation device 58105 and the filter device 58150. For example, referring back to FIGS. 13-14, the electrical connector may be located on the socket 2120 that is configured to receive the filter device 58150. In some examples, the first receptacle 2122 and/or the second receptacle 2124 may act as the electrical connector electrically connecting the smoke evacuation device 58105 (e.g., processor 58110, master controller 58210) and the filter device 58150 (e.g., slave controller 58310 or other filter components 58325A-C, 58335A-C). In some examples, the electrical connector may be a pogo pin or a plug type connector.

Referring back to FIG. 7, in some examples, there may be a cable connector, e.g., a wire, extends from the smoke evacuation device 50600, 58100 to the generator 50640. The cable connector may deliver activation signals and information about the energy delivery, and the smoke evacuation device 50600, 58100 may control the components in the smoke evacuation device 50600, 58100 based on the activation signals and the energy delivery information. For example, the smoke evacuation device 50600 may lower the suction force/rate or stop the suction, for example, by lowering the pump power/motor speed or by stopping the pump 58160/motor 58165 in response to receiving energy delivery information/signal indicating that the generator is not activated or not activated fully. The smoke evacuation device 50600 may also increase the suction force/rate in response to receiving energy delivery information/signal indicating that the generator is activated or activated fully. In this way, the smoke evacuation device 50600 may be able to change the level of the suction force/rate as the generator 50640 is activated or deactivated.

In some examples, the filter communication circuit 58130 may include a Trusted Platform Module (TPM), which may be used to protect unencrypted keys and authentication information from malicious software attacks. In some examples, the TPM may be a specialized microprocessor or chip that provides a protected space for key operations and other security related tasks. In some examples, the TPM may use a monotonic counter for anti-replay protection, for example, to limit the number of failed accesses. For example, the TPM, using the monotonic counter, may prevent attempts to transmit data that is maliciously or fraudulently repeated by an unauthorized component of the filter device 58150. The TPM may provide a decentralized and enhanced security to the system 58100.

In some examples, the display device 58170 may act as an interactive data point, receiving inputs and displaying outputs for the smoke evacuation system 58100. In some examples, the display device 58170 may include a touch screen. In some examples, the display device 58170 may display a smoke evacuation console having keys/buttons to control (e.g., activate/deactivate) or check the status of the components in the smoke evacuation system 58100. For example, using the keys/buttons, a user may check the activation status or the data/parameters (e.g., magnitude of the fan/motor speed) output from the components in the smoke evacuation system 58100. In other examples, the evacuation system may include a mechanical console having keys/buttons to control or check the status of the components in the smoke evacuation system 58100. In some examples, the smoke evacuation console on the display device 58170 may look similar to the mechanical console, for example, by a default setting. In this case, the display device 58170 may display a (small) icon that, for example, on a corner of the display device 58170 that may allow the user to access a menu structure, which when activated, showing more adjustment options.

In some examples, the display device 58170 may operate interactively with other display devices (e.g., hub display 135) in the surgical system 100. For example, the display device 58170 may act as a primary display device when the smoke evacuation device 58105 is not connected to the hub 106. When the smoke evacuation device 58105 is connected to the hub 106, the display device 58170 may act as a secondary display device while the hub display 135 acts as a primary display device. In this case, the display device 58170 may also include control buttons to control not only the smoke evacuation device 58105, but also the hub 106. In some examples, the hub display 135 and/or the display device 58170 may include an icon that may allow one of the hub display 135 and the display device 58170 to become an input device for the other.

In some examples, one or more components in the surgical system may be disposable/re-useable, including the filter device 58150, filter components in the filter device 58150, fluid trap 50760 (e.g., including fluid reservoir 50774), air hose 50636, electrosurgical instrument 50630 (e.g., Zip Pen®), blade in the surgical instrument, or any other components in the smoke evacuation system 58100.

### Dual In-Series Large And Small Droplet Filters

The fluid extracted from a surgical site by a smoke evacuation system may contain liquid (e.g., large and small droplets) and various particulates in addition to smoke, which may be generated during a surgical procedure. The combination of different types and/or states of matter in the evacuated fluid may make it difficult to filter the fluid output from the surgical site. Moreover, certain types of matter in the fluid may be detrimental to certain filters in the smoke evacuation system. For example, the presence of liquid droplets in the fluid may damage certain filters, such as particulate/charcoal filters, which may be very expensive. Also, these filters may be easily damaged/blocked by not only large droplets, but also relatively small droplets.

Aspects of the present disclosure may address the above-noted deficiencies. In certain instances, a surgical evacuation system may include a pump, a motor operably coupled to the pump, and a flow path fluidically coupled to the pump. The flow path may include a first fluid filter configured to extract a large droplet in a fluid moving through the flow path, and a second fluid filter configured to extract a small droplet in the fluid. The first fluid filter may be coupled in series with the second fluid filter and positioned upstream of the second fluid filter. An outlet port of the second fluid filter may be coupled to an inlet port of a non-fluid filter, which can be damaged when a moisture/droplet is entered therein. In certain instances, the surgical evacuation system may also include one or more recirculation channels configured to recirculate the fluid output from the first fluid filter or the second fluid filter.

In this way, the present disclosure may allow the smoke evacuation system to extract not only large droplets, but also small droplets before the fluid enters a non-fluid filter, which may be damaged by the large and small droplets. Also, the second fluid filter may use a filter element that may be more sophisticated and more expensive than the components used in the first fluid filter, and this filter element in the second fluid filter may tend to be easily and quickly clogged by large droplets. In the present disclosure, by providing the first fluid filter configured to extract large droplets upstream of the second fluid filter, the evacuation system may effectively protect the second fluid filter from damages and/or blockages, saving pump power and money. Finally, by providing one or more recirculation channels, the present disclosure allows the evacuation system to ensure that no droplet that may possibly damage the non-fluid filters enters the non-fluid filter.

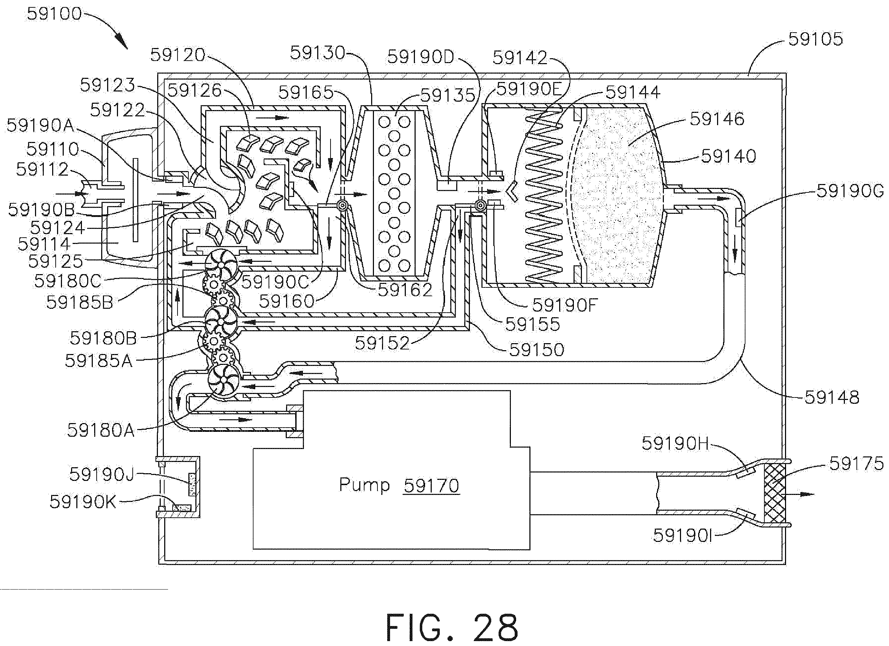

FIG. 28 depicts a schematic of a housing of a smoke evacuation system 59100, in accordance with at least one aspect of the present disclosure. The smoke evacuation system 59100 may include an evacuation housing 59105 and a fluid trap 59110 coupled to the evacuation housing 59105. The evacuation system 59100 may also include a first fluid filter device 59120, a second fluid filter device 59130, a non-fluid filter device 59140, and a pump 59170. The pump 59170 may be operably coupled to a motor. The smoke evacuation system 59100 may further include a plurality of sensors 59190A-K and intelligent controls. The fluid trap 59110, the non-fluid filter device 59140, the pump 59170 may be similar to the examples (e.g., fluid trap, ULPA filter, charcoal filter, scroll pump) depicted in FIGS. 18-19. The fluid trap 59110, the filter devices 59120, 59130, 59140, and the pump 59170 may be sequentially aligned along a flow path through the evacuator housing 59105 between the inlet 59112 and the outlet 59175. As used herein, a non-fluid filter device 59140 may refer to a filter device or a filter (e.g., particulate/charcoal filters) that may be vulnerable to droplets and can be damaged when droplets are entered therein.

In various instances, the plurality of sensors may include one or more fluid detection sensors, one or more pressure sensors, one or more particle sensors, and/or one or more chemical sensors. The plurality of sensors 59190A-K maybe similar to the sensors (e.g., sensors 50830, 50832, 50836, 50840, 50838, 50846, 50848, 50850, 50854, 50852) depicted in FIGS. 18 and 19. For example, a pressure sensor may be positioned to detect pressure within the evacuation system 59100, such as within the evacuator housing 59105. For example, a pressure sensor may be positioned upstream of one of the filter devices 59120, 59130, 59140 (e.g., sensor 59190E), between the filter devices 59120, 59130, 59140 and the pump 59170 (e.g., sensor 59190G), and/or downstream of the pump 59170 (e.g., sensor 591901). In certain instances, a pressure sensor 59190K may be positioned outside the evacuation system 59100 to detect pressure in the ambient environment.

Similarly, a particle sensor 59190F, 59190H may be positioned to detect particles within the evacuation system 59100, such as within the evacuator housing 59105. A particle sensor maybe positioned upstream of one of the filter devices 59120, 59130, 59140 (e.g., sensor 59190F), between the filter devices 59120, 59130, 59140 and the pump 59170, and/or downstream of the pump 59170 (e.g., sensor 59190H), for example. In various instances, a particle sensor 59190J may be positioned to detect particles in the ambient environment to determine the air quality in the surgical theater, for example.

In various instances, a fluid detection sensor may be positioned upstream of one of the filter devices 59120, 59130, 59140 (e.g., sensor 59190A, 59190C, 59190D), between the filter devices 59120, 59130, 59140 and the pump 59170, downstream of the pump 59170, or outside of the evacuation housing 59106. Similarly, a chemical sensor maybe positioned upstream of one of the filter devices 59120, 59130, 59140 (e.g., sensor 59190B), between the filter devices 59120, 59130, 59140 and the pump 59170, downstream of the pump 59170, or outside of the evacuation housing 59106.

Those skilled in the art will appreciate that certain evacuation systems may not include each sensor depicted in FIG. 28 and/or may include additional sensor(s). The components in the evacuation system 59100 may be modular and/or replaceable. For example, the fluid trap 59110, filter devices 59120, 59130, 59140, the pump 59170, the plurality of sensors 59190A-K may be modular and/or replaceable.

The plurality of sensors 59190A-K may detect various parameters of the fluid moving through the fluid path in the evacuation housing 59105 and/or of the ambient environment. In various instances, the evacuation housing 59105 and/or a modular component compatible with the housing 59105 may include a processor, which may be configured to receive inputs from one or more sensors (e.g., 59190A-K) and/or to communicate outputs to one more drivers.

As used herein, fluid may refer to any material coming into the inlet 59112, for example, from a suction conduit, including liquids, gases, vapors, smoke, or combinations thereon. The fluids may be biologic in origin and/or can be introduced to the surgical site from an external source during a procedure. The fluids may also include water, saline, lymph, blood, exudate, and/or pyogenic discharge. Moreover, the fluids may also include particulates or other matter (e.g. cellular matter or debris) that is evacuated by the evacuation system. In an example, such particulates maybe suspended in the fluid.

In operation, fluid from a surgical site can be drawn into the inlet 59112 of the evacuator housing 59105 via the fluid trap 59110. The flow path through the housing 59105 in FIG. 28 may be a sealed conduit or tube extending between the various in-line components. In various instances, the fluid may flow past a fluid detection sensor 59190A and chemical sensor 59190B to the first fluid filter device 59120. A fluid detection sensor 59190A may detect fluid particles in the fluid/smoke and the chemical sensor 59190B may detect chemical properties of the fluid. The fluid detection sensor 59190A may also detect the concentration (e.g., liquid-to-gas ratio) and/or size of droplets in the fluid near the fluid detection sensor 59190A. The first fluid filter device 59120 may extract large droplets in the fluid. Then, the fluid may be directed to flow into the second fluid filter 59130. In the second fluid filter 59130, small droplets in the fluid output from the first fluid filter device 59120 may be extracted. The fluid may then flow past the second fluid filter device 59130 and may be directed to flow into the non-fluid filter device 59140.

At the inlet of the non-fluid filter device 59140, the fluid may flow past a laser particle counter 59190F and a pressure sensor 59190E. The fluid may be filtered via one or more non-fluid type filters 59144, 59146. In certain instances, the non-fluid filter device 59140 depicted in FIG. 28 may include additional and/or fewer filtering levels. For example, the non-fluid filter device 59140 may include one or more filtering layers selected from the following group of filters: a course media filter, a fine media filter, and a sorbent-based filter. The course media filter may be a low air resistant filter, which may be comprised of fiberglass, polyester, and/or pleated filters, for example. The fine media filter may be a high efficiency particulate air (HEPA) filter and/or ULPA filter. The sorbent-based filter may be an activated-carbon filter (e.g., charcoal filter), for example. In certain instances, the non-fluid filter device 59140 may also include one or more baffles 59142 or similar structures, upon which the fluid input into the non-fluid filter device 59140 may condensate. In certain instances, the baffle 59142 may be located near the inlet port of the non-fluid filter device 59140. In certain other instances, the baffle 59142 may be positioned in any other suitable location in the non-fluid filter device 59140.

Upon exiting the non-fluid filter device 59140, the fluid may flow past a pressure sensor 59190G and then continue along the flow path 59148 within the evacuator housing 59105 toward the pump 59170. Upon moving through the pump 59170, the filtered fluid may flow past a laser particle sensor 59190H and a pressure sensor 591901 at the outlet 59175 of the evacuator housing 59105. The evacuator housing 59105 may also include an air quality particle sensor 59190J and an ambient pressure sensor 59190K to detect various properties of the ambient environment such as the environment within the surgical theater.

In various instances, the fluid trap 59110 or the first fluid filter device 59120 may be configured to prevent spillage and/or leakage of the captured fluid. For example, the geometry of the fluid trap 59110 or the first fluid filter device 59120 may be selected to prevent the captured fluid from spilling and/or leaking. In certain instances, the fluid trap 59110 or first fluid filter device 59120 may include one or more baffles 59126 and/or splatter screens for preventing the captured fluid from splashing out of the fluid trap 59110 or first fluid filter device 59120. In one or more instances, the fluid trap 59110/first fluid filter device 59120 may include sensors for detecting the volume of fluid within the fluid trap 59110/first fluid filter device 59120 and/or if the fluid trap 59110/first fluid filter device 59120 is filled to capacity. The fluid trap 59110/first fluid filter device 59120 may include a valve for empty the fluid in the fluid reservoir 59114 of the fluid trap 59110 or in the fluid reservoir 59125 of the first fluid filter device 59120.

The various sensors in the evacuation system 59100 may communicate with a controller, which may be incorporated into the evacuation system 59100 and/or may be a component of another surgical instrument and/or a surgical hub. The controller may adjust one or more operational parameters of the evacuator system (e.g. a motor for the evacuator pump) based on input from the sensor(s) or operational parameters of another device, such as an electrosurgical tool and/or imaging device based on input from the sensor(s).

Referring to FIG. 28 again, in certain instances, the first fluid filter device 59120 may be configured to extract a large droplet in the fluid moving through the flow path, and the second fluid filter device 59130 may be configured to extract a small droplet in the fluid. As illustrated in FIG. 28, the first fluid filter device 59120 maybe coupled in series with the second fluid filter device 59130. The first fluid filter device 59120 may be positioned upstream of the second fluid filter device 59130. In certain instances, an outlet port of the second fluid filter device 59130 may be coupled to an inlet port of a non-fluid filter device 59140.

As used herein, a droplet larger than 10-20 µm may be considered as a large droplet. Also, a droplet smaller than 10-20 µm may be considered as a small droplet. In certain instances, the first fluid filter device 59120 may remove a majority of droplets larger than 20 µm. In certain instances, the first fluid filter device 59120 may remove at least 85% of droplets larger than 20 µm, greater than 90% of droplets larger than 20 µm, greater than 95% of droplets larger than 20 µm, greater than 99% of droplets larger than 20 µm, greater than 99.9% of droplets larger than 20 µm, or greater than 99.99% droplets larger than 20 µm.

Additionally or alternatively, the first fluid filter device 59120 may remove the majority of droplets greater than 10 µm. In certain instances, the first fluid filter device 59120 may remove at least 85% droplets larger than 10 µm, greater than 90% of droplets larger than 10 µm, greater than 95% of droplets larger than 10 µm, greater than 99% of droplets larger than 10 µm, greater than 99.9% droplets larger than 10 µm, or greater than 99.99% droplets larger than 10 µm.

The second fluid filter device 59130 may remove a majority of droplets larger than 1 µm, for example. In certain instances, the second fluid filter device 59130 may remove at least 85% of droplets larger than 1 µm, greater than 90% of droplets larger than 1 µm, greater than 95% of droplets larger than 1 µm, greater than 99% of droplets larger than 1 µm, greater than 99.9% of droplets larger than 1 µm, or greater than 99.99% droplets larger than 1 µm.

Additionally or alternatively, the second fluid filter device 59130 may remove a majority of droplets larger than 0.1 µm, for example. In certain instances, the second fluid filter device 59130 may remove at least 85% of droplets larger than 0.1 µm, greater than 90% of droplets larger than 0.1 µm, greater than 95% of droplets larger than 0.1 µm, greater than 99% of droplets larger than 0.1 µm, greater than 99.9% of droplets larger than 0.1 µm, or greater than 99.99% droplets larger than 0.1 µm.

In certain instances, the first fluid filter device 59120 may include a diverter valve 59122. The diverter valve 59122 maybe similar to the diverter valve 50834, 50934 depicted in FIGS. 18-19. For example, when the diverter valve 59122 is in a first position, the fluid intake through the diverter valve 59122 may be directed along a first path 59123. When the diverter valve 59122 is in a second position, as illustrated in FIG. 28, fluid intake through the diverter valve 59122 may be directed along a second path 59124. In certain instances, the first path 59123 may correspond to a flow path when almost no liquid/droplet has been detected within the fluid or when the detected liquid-to-gas ratio is below a threshold value. In certain other instances, the first path 59123 may correspond to a flow path when the size of the majority of the detected droplets (e.g., 80%, 90%, 95%, or 99%) is smaller than a predetermined threshold value (e.g., 10-20 µm).

In certain instances, the second path 59124 may correspond to a flow path when liquid/droplet has been detected within the fluid, e.g. aerosol, or when the detected liquid-to-gas ratio is equal to or above the threshold value. In certain other instances, the second path 59124 may correspond to a flow path when the size of the majority of the detected droplets is equal to or greater than a predetermined threshold value (e.g., 10-20 µm). The fluid detection sensor 59190A may be configured to detect the presence of liquid droplets or aerosols in the fluid, the liquid-to-gas ratio, and/or the size of the droplet/aerosol. For example, the fluid detection sensor 59190A may be positioned at and/or near the output port of the fluid trap 59110 and/or the inlet port of the first fluid filter device 59120. A liquid-to-gas ratio equal to or above the threshold value (e.g., 1:2; 1:1; 2:1; 5:1; 10:1) may be considered an aerosol. The first path 59123 may bypass the first fluid filter device 59120, and the second path 59124 may direct the fluid through the first fluid filter device 59120 to capture large droplets from the fluid before the fluid is directed into the second fluid filter device 59130. By selecting a fluid path based on the liquid-to-gas ratio or the size of the droplets in the fluid, the efficiency of the surgical evacuation system 59100 may be improved.

As discussed above, if the fluid detection sensor 59190A detects a liquid-to-gas ratio equal to or above a threshold value, a droplet larger than a threshold size, or a combination of both, the fluid intake may be diverted into the second path 59124 before entering the second fluid filter device 59130. The second path 59124 may be configured to condense liquid droplets in the flow path. For example, the second path 59124 may include a plurality of baffles 591266 or other similar structures, upon which the fluid may be configured to condensate. As fluid flows past the second path 59124, the liquid may condensate on the baffles 59126 therein, and maybe directed to drip downward into the fluid reservoir 59125.

Conversely, if the fluid detection sensor 59190A detects a liquid-to-gas ratio below the threshold value, a droplet smaller than a threshold size, or a combination of both, the fluid intake may be directed directly to the second fluid filter device 59130. The diverter valve 59122 may be positioned to bypass the second path 59124 and the first fluid filter device 59120 such that the fluid flows directly to the second fluid filter device 59130. By bypassing the first fluid filter device 59120, the surgical evacuation system 59100 may require less power from the motor that drives the pump 59170. For example, the motor may require more power to draw an aerosol through the surgical evacuation system than to draw a non-aerosol smoke through the surgical evacuation system.

In certain instances, the second fluid filter device 59130 may include a filter 59135 that is configured to capture small droplets (e.g., smaller than 10-20 µm). In certain instances, the filter 59135 may be configured to extract droplets larger than a threshold size (e.g., 0.1-1 µm). In certain instances, the filter 59135 may be at least one of a membrane filter, a honeycomb filter, and/or a porous structure filter (e.g., thin porous pad), or any other suitable filter that is capable of extracting small droplets or droplets larger than 0.1-1 µm. The fluid output from the second fluid filter device 59130 may flow into the non-fluid filter device 59140. In certain instances, the second fluid filter device 59130 may also include one or more baffles or similar structures, upon which the fluid input into the second fluid filter device may condensate. In certain instances, the baffles may be located near the inlet port of the second fluid filter device 59130. In certain other instances, the baffles may be positioned in any other suitable location in the second fluid filter device 50130.

Referring still to FIG. 28, the evacuation system 59100 may also include a first recirculation channel 59150. The inlet port 59152 of the first recirculation channel 59150 may be positioned between the second fluid filter device 59130 and the non-fluid filter device 59140. The first recirculation channel 59150 may be configured to recirculate the fluid output from the second fluid filter device 59130.

The fluid directed into the first recirculation channel 59150 may be injected into the fluid path upstream of the second fluid filter device 59130. For example, the fluid directed into the first recirculation channel 59150 may be injected into the first fluid filter device 59120 (e.g., fluid reservoir 59125), as illustrated in FIG. 28. In certain other instances, the fluid directed into the first recirculation channel 59150 may be injected into an upstream portion of the second fluid filter device 59130 (e.g., inlet port of the second fluid filter device 59130) or a flow path between the first fluid filter device 59120 and the second fluid filter device 59130.

In certain instances, the first recirculation channel 59150 (e.g., a portion of the first recirculation channel 59150 near the inlet port 59152) may extend downward from the inlet port 59152 of the first recirculation channel 59150. This may allow the large droplet or the small droplet in the fluid output from the second fluid filter device 59130 to be directed to the first recirculation channel 59150 via gravity.

In certain instances, the evacuation system 59100 may also include a first recirculation valve 59155. The first recirculation valve 59155 may be configured to close and/or open the first recirculation channel 59150. When the first recirculation valve 59155 is opened, the fluid output from the second fluid filter device 59130 maybe directed into the first recirculation channel 59150. In certain instances, the evacuation system 59100 may further include a fluid detect sensor 59190D. The fluid detection sensor 59190D may be positioned near the first recirculation valve 59155. The fluid detection sensor 59190D may be similar to the fluid detection sensor 59190A. The fluid detection sensor 59190D may be configured to detect a parameter of the fluid (e.g., the size of the droplets in the fluid, liquid-to-gas ratio). The first recirculation valve 59155 may open the first recirculation channel 59150 when the parameter detected by the fluid detection sensor 59190D is equal to or greater than a predetermined threshold value. For example, if the fluid detection sensor 59190D detects a liquid-to-gas ratio equal to or above a threshold value (e.g., 1:2; 1:1; 2:1; 5:1; 10:1) and/or a droplet size larger than a threshold value (e.g., 0.1-1 µm), the fluid output from the second fluid filter device 59130 may be diverted into the first recirculation channel. In this way, the evacuation system 59100 may prevent droplets/moisture that may damage the filters 59144, 59146 from entering the non-fluid filter device 59140. If the fluid detection sensor 59190D detects a liquid-to-gas ratio below a threshold value and/or a droplet size smaller than a threshold value (e.g., 0.1-1 µm), the first recirculation valve 59155 may be closed such that the fluid output from the second fluid filter device 59130 is directed into the non-fluid filter device 59140.

In certain instances, the recirculated fluid through the first recirculation channel 59150 may go through the first fluid filter device 59120 and/or through second fluid filter device 59130 again, and the recirculation steps may be repeated until the parameter detected by the fluid detection sensor 59190D becomes below a predetermined threshold value. In certain instances, if the number of the repetition is equal to or greater than a predetermined threshold value (e.g., 5 times, 10 times, or any other suitable value greater than 0), which may indicate that some components in the first/second fluid filter devices 59120/59130 are not working properly (e.g., due to sensor failure, damages or blockages to filters/baffles), the first/second fluid filter devices 59120/59130 or the evacuation system 59100 may be disabled, for example, by stopping the pump 59170 or the motor. In this case, the processor of the evacuation system 59100 may notify the evacuation system 59100 or the user that there is an error in the first/second fluid filter devices 59120/59130.

In certain instances, the evacuation system 59100 may also include a second recirculation channel 59160. An inlet port 59162 of the second recirculation channel 59160 may be positioned between the first fluid filter device 59120 and the second fluid filter device 59130. The second recirculation channel 59160 may be configured to recirculate the fluid output from the first fluid filter 59120. In certain instances, the fluid directed into the second recirculation channel 59160 may be injected into the fluid path upstream of the first fluid filter device 59120 (e.g., the reservoir 59114 or the fluid trap 59110) or an upstream portion of the first fluid filter device 59120 (e.g., inlet port of the first fluid filter or the reservoir 59125 of the first fluid filter device 59120). In certain instances, the second recirculation channel 59160 (e.g., a portion of the second recirculation channel 59160 near the inlet port 59162) may extend downward from the inlet port 59162 of the second recirculation channel 59160. This may allow the large droplet or the small droplet in the fluid output from the first fluid filter device 59120 to be directed to the second recirculation channel 59160 via gravity.

In certain instances, the evacuation system 59100 may further include a second recirculation valve 59165. The second recirculation valve 59165 maybe configured to close and/or open the second recirculation channel 59160. When the second recirculation valve is opened, the fluid output from the first fluid filter device 59120 may be recirculated through the second recirculation channel 59160.

In certain instances, the evacuation system 59100 may also use a fluid detection sensor 59190C to control the second recirculation valve 59165. The fluid detection sensor 59190C may be similar to the fluid detection sensor 59190A, D. The fluid sensor 59190C may be positioned near the second recirculation valve 59165. The fluid sensor 59190C maybe configured to detect a parameter of the fluid (e.g., the size of the droplets in the fluid, liquid-to-gas ratio). The second recirculation valve 59165 may open the second recirculation channel 59160 when the parameter detected by the fluid detection sensor 59190C is equal to or greater than a predetermined threshold value. For example, if the fluid detection sensor 59190C detects a liquid-to-gas ratio equal to or above a threshold value (e.g., 1:2; 1:1; 2:1; 5:1; 10:1) and/or a droplet size larger than a threshold value (e.g., 10-20 µm), the fluid output from the first fluid filter device 59120 maybe diverted into the second recirculation channel 59160. In this way, the evacuation system 59100 may prevent large droplets/moistures that may easily and/or quickly clog the filter 59135 from entering the second fluid filter device 59140. If the fluid sensor 59190C detects a liquid-to-gas ratio below a threshold value and/or a droplet size smaller than a threshold value (e.g., 10-20 µm), the second recirculation valve 59165 may be closed such that the fluid output from the first fluid filter device 59120 is directed into the second fluid filter device 59130.

In certain instances, the recirculated fluid through the second recirculation channel 59160 may go through the first fluid filter device 59120 again, and the recirculation steps may be repeated until the parameter detected by the fluid detection sensor 59190C becomes below a predetermined threshold value. In certain instances, if the number of the repetition is equal to or greater than a predetermined threshold value (e.g., 5 times, 10 times, or any other suitable value greater than 0), which may indicate that some components in the first fluid filter device 59120 is not working properly (e.g., due to sensor failure, damages or blockages to baffles), the first fluid filter device 59120 or the evacuation system 59100 may be disabled, for example, by stopping the pump 59170 or the motor. In this case, the processor of the evacuation system 59100 may notify the evacuation system 59100 or the user that there is an error in the first fluid filter device 59120.

In certain instances, the first recirculation valve 59155 may be configured to open and/or close the flow path between the second fluid filter device 59130 and the non-fluid filter device 59140. For example, when the parameter detected by the fluid detection sensor 59190D is equal to or greater than a predetermined threshold value, the first recirculation valve 59155 opens the first recirculation channel 59150 and closes the flow path between the second fluid filter device 59130 and the non-fluid filter device 59140 at the same time. In this way, the present disclosure may advantageously allows the evacuation system 59100 to divert almost all fluid output from the second fluid filter device 59130, which may include droplets that may damage the filters 59144, 59146 of the non-fluid filter device 59140, into the first recirculation channel 59150. Also, in certain instances, the closing of the first recirculation channel 59150 and the opening of the flow path between the second fluid filter device 59130 and the non-fluid filter device 59140 can be done with a single step/operation as opposed to multiple steps/operations. For example, as illustrated in FIG. 28, when the first recirculation valve 59155 is opened 90 degrees, the first recirculation valve 59155 closes the flow path between the second fluid filter device 59130 and the non-fluid filter device 59140.

Similarly, in certain instances, the second recirculation valve 59165 may be configured to open and/or close the flow path between the first fluid filter device 59120 and the second fluid filter device 59130. In certain instances, when the parameter detected by the fluid detection sensor 59190C is equal to or greater than a predetermined threshold value, the second recirculation valve 59165 may open the second recirculation channel 59160 and close the flow path between the first fluid filter device 59120 and the second fluid filter device 59130 at the same time. In this way, the present disclosure may advantageously allows the evacuation system 59100 to divert almost all fluid output from the first fluid filter device 59120, which may include large droplets that may easily/quickly clog the second fluid filter device 59130 and/or the filter 59135 of the second fluid filter device 59130, into the second recirculation channel 59160. Also, in certain instances, the closing of the second recirculation channel 59160 and the opening of the flow path between the first fluid filter device 59120 and the second fluid filter device 59130 can be done with a single step/operation as opposed to multiple steps/operations. For example, as illustrated in FIG. 28, when the second recirculation valve 59165 is opened 90 degrees, the second recirculation valve 59165 closes the flow path between the first fluid filter device 59120 and the second fluid filter device 59130. This may advantageously reduce the number of signals/commands between the processor and the components in the evacuation system 59100, preventing possible signal delay and malfunction of the components due to the signal delay.

In certain instances, the evacuation system 59100 may include one or more centrifugal blower arrangements. For example, a first centrifugal blower 59180A (e.g., squirrel cage) may be provided to the flow path 59148 between the non-fluid filter device 59140 and the pump 59170, and a second centrifugal blower 59180B may be provided to the first recirculation channel 59150. The first centrifugal blower 59180A may be operably coupled to the second centrifugal blower 59180B, for example, via one or more gears 59185A. For example, when the first recirculation valve 59155 is open and the pump 59170 is activated, the suction force generated by the pump 59170 may generate rotation of the first centrifugal blower 59180A, which may be transferred to the second centrifugal blower 59180B, via the gear 59185A, which draws the recirculated fluid through the first recirculation channel 59150.

Similarly, a third centrifugal blower 59180C may be provided to the second recirculation channel 59160. In certain instances, the third centrifugal blower 59180C may be operably coupled to the first centrifugal blower 59180A, for example, via one or more gears 59185A-B and the second centrifugal blower 59180B, as illustrated in FIG. 28. In this case, when the second recirculating valve 59165 is open and the pump 59170 is activated, the suction force generated by the pump 59170 may generate rotation of the first centrifugal blower 59180A, which may be transferred to the second centrifugal blower 59180B and, in turn, to the third centrifugal blower 59180C, which draws the recirculated fluid through the second recirculation channel 59160. In certain other instances, the third centrifugal blower 59180C may be operably coupled to the first centrifugal blower 59180A, for example, via the gears 59185B without having the second centrifugal blower 59180B therebetween. In this case, when the second recirculating valve 59165 is open and the pump 59170 is activated, the suction force generated by the pump 59170 may generate rotation of the first centrifugal blower 59180A, which may be transferred to the third centrifugal blower 59180C, via one or more gears 59185B, which draws the recirculated fluid through the second recirculation channel 59160. In this way, the present disclosure may advantageously use less power from the motor/pump by reusing the motor/pump power in generating a suction force for the first and/or second recirculation channel 59150, 59160. In certain other instances, the first recirculation channel 59150 and/or the second recirculation channel 59160 maybe provided with a separate pump to generate the suction force.

The reader will readily appreciate that various surgical evacuation systems and components described herein can be incorporated into a computer-implemented interactive surgical system, a surgical hub, and/or a robotic system. For example, a surgical evacuation system can communicate data to a surgical hub, a robotic system, and/or a computer-implanted interactive surgical system and/or can receive data from a surgical hub, robotic system, and/or a computer-implemented interactive surgical system. Various examples of computer-implemented interactive surgical systems, robotic systems, and surgical hubs are further described below.

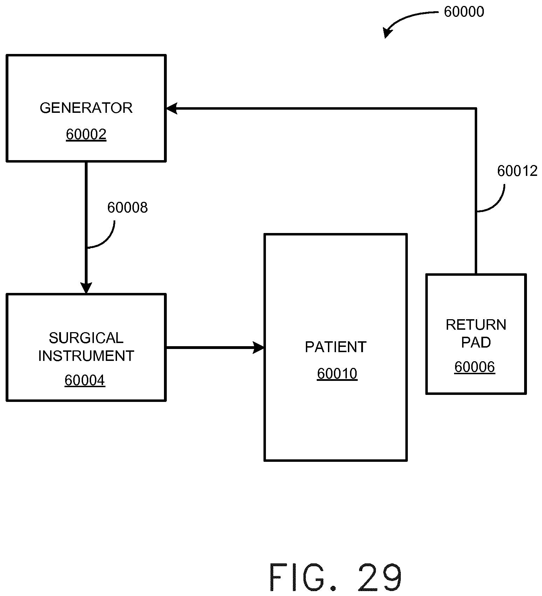

FIG. 29 illustrates an electrosurgical system 60000, in accordance with at least one aspect of the present disclosure. The electrosurgical system 60000 includes a generator 60002, an electrosurgical instrument 60004 and a return pad 60006. The generator 60002 supplies alternating current at radiofrequency levels to the electrosurgical instrument 60004 via a first conductor/cable 60008. The electrosurgical instrument 60004 includes an electrode tip (i.e., an active electrode) which can be positioned at a target tissue of a patient. The electrosurgical instrument 60004 receives the alternating current from the generator 60002 and delivers the alternating current to the target tissue of a patient 60010 via the electrode tip. The alternating current is received at the target tissue, and the resistance from the tissue creates heat which provides the desired effect (e.g., sealing and/or cutting) at the surgical site. The alternating current is conducted through the patient's body and ultimately is received by the return pad 60006. The alternating current received by the return pad 60006 is carried back to the generator 60002 via a second conductor/cable 60012 to complete the closed path followed by the alternating current.

According to various aspects, the generator 60002 is similar to the generator 900 described above, and may include, for example, a processor and a waveform generator similar to the processor 902 and the waveform generator 904 described above.

The electrosurgical instrument 60004 may be configured for monopolar operation where electrosurgical energy supplied by the generator 60002 is introduced into the patient's tissue by an active electrode of the surgical instrument 60004 and is return to the generator 60002 via the return pad 6006. According to various aspects, the electrosurgical instrument 60004 includes a handpiece or pencil and an electrode tip. The electrode tip serves as the active electrode of the electrosurgical system 60000 and introduces electrosurgical energy to the target tissue of a patient 60010. Specifically, an electrical discharge is delivered from electrode tip to the patient 60010 in order to cause heating of cellular matter of the patient 60010 that is in close contact with or adjacent to electrode tip. The tissue heating takes place at an appropriately high temperature to allow the electrosurgical instrument 60004 to be used to perform electrosurgery.

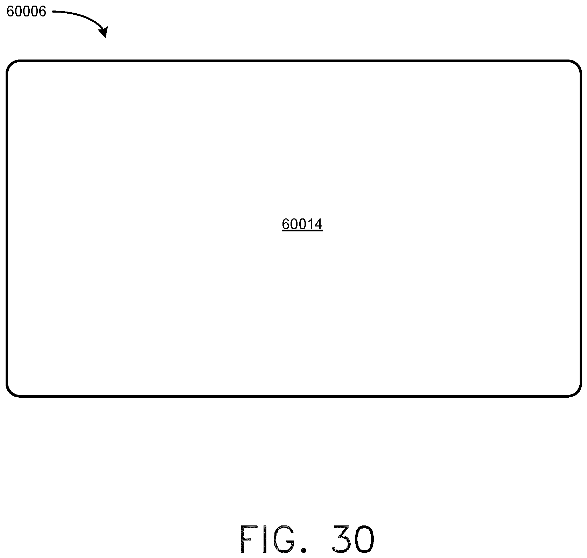

FIG. 30 illustrates the return pad 60006 of the electrosurgical system 60000 of FIG. 29, in accordance with at least one aspect of the present disclosure. The return pad 60006 can be similar to the MEGA SOFT® patient return electrode commercially available from Megadyne Medical Products, Inc. in that the return pad 60006 can include a sleeve or covering 60014, can be separated a small distance from the patient's body, can be capacitively coupled with the patient's body and is configured to carry the amount of current introduced into the patient's body by the electrosurgical instrument 60004, but is different.

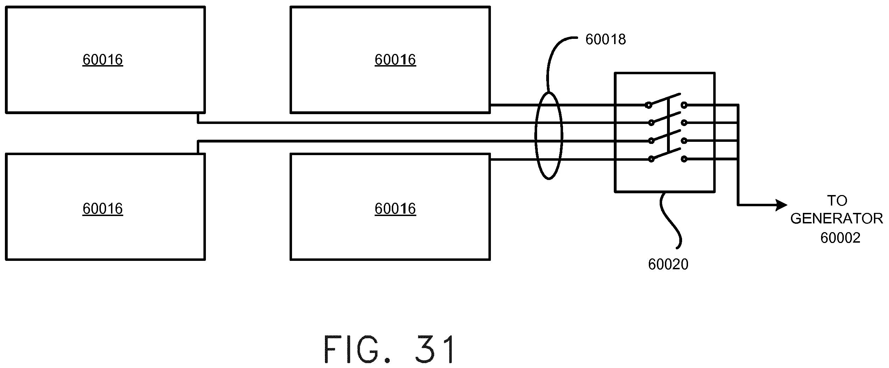

The return pad 60006 is different from the MEGA SOFT® patient return electrode commercially available from Megadyne Medical Products, Inc. in that in lieu of one return electrode, the return pad includes a plurality of electrodes 60016 (See FIG. 31) which can be capacitively coupled with the patient's body and collectively are configured to carry the amount of current introduced into the patient's body by the electrosurgical instrument 60004. For this capacitive coupling, the patient's body effectively acts as one plate of a capacitor and collectively the plurality of electrodes of the return pad effectively act together as the other plate of the capacitor. A more detailed description of capacitive coupling can be found, for example, in U.S. Patent No. 6,214,000, titled CAPACITIVE REUSABLE ELECTROSURGICAL RETURN ELECTRODE, issued April 10, 2001 and in U.S. Patent No. 6,582,424, titled CAPACITIVE REUSABLE ELECTROSURGICAL RETURN ELECTRODE, issued June 24, 2003, the entire contents of which are each incorporated by reference. Although the return pad 60006 is shown in FIG. 30 as being substantially rectangular, it will be appreciated that the return pad 60006 can be of any suitable shape.

FIG. 31 illustrates a plurality of electrodes 60016 of the return pad 60006 of FIG. 30, in accordance with at least one aspect of the present disclosure. For purposes of clarity, the sleeve or covering 60014 of the return pad 60006 is not shown in FIG. 31. Although four electrodes 60016 are shown in FIG. 31, it will be appreciated that the return pad 60006 may include any number of electrodes 60016. For example, according to various aspects, the return pad 60006 includes sixteen electrodes 60016. Also, although the individual electrodes 60016 are shown in FIG. 31 as being substantially rectangular, it will be appreciated that the individual electrodes can be of any suitable shape.

The electrodes 60016 of the return pad 60006 can be considered the return electrodes of the electrosurgical system 60000 of FIG. 29, and can also be considered to be segmented electrodes as the electrodes 60016 can be selectively decoupled from the patient's body and/or the generator 60002. The electrodes 60016 of the return pad 60006 can also be coupled together to effectively act as one large electrode. For example, according to various aspects, each of the electrodes 60016 of the return pad 60006 can be connected by respective conductive members 60018 to inputs of a switching device 60020 as shown in FIG. 24. When the switching device 60020 is in an open position as shown in FIG. 31, the respective electrodes 60016 of the return pad 60006 are decoupled from one another as well as from the patient's body and/or the generator 60002. In contrast, when the switching device 60020 is in a closed position, the respective electrodes 60016 of the return pad 60006 are coupled together to effectively act as one large electrode.

The switching device 60020 can be controlled by a processing circuit (e.g., a processing circuit of the generator 60002 of the electrosurgical system, a processing circuit of the surgical hub 206, a processing circuit of the surgical hub 106, etc.). For purposes of simplicity, the processing circuit is not shown in FIG. 31. According to various aspects, the switching device 60020 shown in FIG. 31 can be incorporated into the return pad 60006. According to other aspects, the switching device 60006 shown in FIG. 31 can be incorporated into the second conductor/cable 60012 of the electrosurgical system 60000 of FIG. 29. The return pad 60006 can also include a plurality of sensing devices 60022 (See FIG. 32).

By being able to couple the plurality of electrodes 60016 together for use during an electrosurgical procedure, the total effective size of the electrodes 60016 of the return pad 60006 is large enough and/or has sufficient surface area to keep the current density sufficiently low to mitigate the likelihood of any unwanted burning of the patient 60010.

FIG. 32 illustrates an array of sensing devices 60022 of the return pad 60006 of FIG. 30, in accordance with at least one aspect of the present disclosure. For purposes of clarity, the sleeve or covering 60014 of the return pad 60006 is not shown in FIG. 32. According to various aspects, the number of sensing devices 60022 corresponds to the number of electrodes 60016 such that there is one sensing device 60022 for each electrode 60016, and each sensing device 60022 is mounted to or integrated with a corresponding electrode 60016. However, although the number of sensing devices 60022 shown in FIG. 32 corresponds to the number of electrodes 60016, it will be appreciated that the return pad 60006 may include any number of sensing devices 60022. For example, for aspects of the return pad 60006 which include sixteen electrodes 60016, the return pad 60006 may only include four or eight sensing devices 60022. Although the sensing devices 60022 are shown in FIG. 32 as being centered on the corresponding electrodes 60016 , it will be appreciated that the sensing devices 60022 can be positioned on any portion of the corresponding electrodes 60016 and can be positioned differently on different electrodes 60016.

The sensing devices 60022 are configured to detect a monopolar nerve control signal applied to the patient and/or a movement of an anatomical feature of the patient (e.g., a muscle twitch) resulting from application of the nerve control signal. The monopolar nerve control signal may be applied by the electrosurgical instrument 60004 of the electrosurgical system 60000 of FIG. 29, or may be applied by a different surgical instrument which is coupled to a different generator. Each sensing device 60022 may include, for example, a pressure sensor, an accelerometer, combinations thereof, etc. and is configured to output a signal indicative of the detected nerve control signal and/or the detected movement of an anatomical feature of the patient. Such pressure sensors may include for example, piezoresistive strain gauges, capacitive pressure sensors, electromagnetic pressure sensors and/or piezoelectric pressure sensors. Such accelerometers may include, for example, mechanical accelerometers, capacitive accelerometers, piezoelectric accelerometers, electromagnetic accelerometers and/or microelectromechanical system (MEMS) accelerometers. The respective output signals of the respective sensing devices 60022 may be in the form of analog signals and/or digital signals.

Using Coulomb's law and the respective locations of the active electrode of the electrosurgical instrument 60004, the patient's body and the respective sensing devices 60022, the respective output signals of the respected sensing devices 60022, which are indicative of a detected nerve control signal and/or movement of an anatomical feature of the patient, can be analyzed to determine the location of a nerve within the patient's body. Coulomb's law states that E=K(Q/r²), where E is the threshold current required at a nerve to stimulate the nerve, K is a constant, Q is the minimal current from the nerve stimulation electrode and r is the distance from the nerve. The further the nerve stimulation electrode is from the nerve, the current needed to stimulate the nerve is proportionately greater. A constant current stimulus can thus be utilized to estimate the distance from the nerve stimulation electrode to the nerve. In general, the respective strengths of the output signals of the respective sensing devices 60022 are indicative of how close or far the respective sensing devices 60022 are from the stimulated nerve of the patient 60010.

According to various aspects, the analysis of the respective output signals of the respective sensing devices 60022 can be performed by a processing circuit of the generator 60002 of the electrosurgical system 60000 of FIG. 29, by a processing circuit of a nerve monitoring system which is separate from the generator 60002 of the electrosurgical system 60000 of FIG. 29, by a processing circuit of the surgical hub 206, by a processing circuit of the surgical hub 106, etc. The analysis can be performed in real time or in near-real time. According to various aspects, the respective output signals serve as inputs to a monopolar nerve stimulation algorithm which is executed by the processing circuit.

As shown in FIG. 32, according to various aspects, the output signals of the respective sensing devices 60022 can be input into a multiple input - single output switching device 60024 (e.g., a multiplexer) via respective conductive members 60026. By controlling the selection signals S₀, S₁ to the multiple input - single output switching device 60024, the multiple input - single output switching device 60024 can be controlled to output only one of the output signals of the respective sensing devices 60024 at a time for the above-described analysis. For example, with reference to FIG. 32, by setting the selection signals S₀, S₁ to 0,0, the output signal from the sensing device 60022 associated with the electrode 60016 in the upper left hand corner of FIG. 32 can be output by the multiple input - single output switching device 60024 for analysis by the applicable processing circuit. By setting the selection signals S₀, S₁ to 0,1, the output signal from the sensing device 60022 associated with the electrode 60016 in the upper right hand corner of FIG. 32 can be output by the multiple input - single output switching device 60024 for analysis by the applicable processing circuit. By setting the selection signals S₀, S₁ to 1,0, the output signal from the sensing device 60022 associated with the electrode 60016 in the lower left hand corner of FIG. 32 can be output by the multiple input - single output switching device 60024 for analysis by the applicable processing circuit. By setting the selection signals S₀, S₁ to 1,1, the output signal from the sensing device 60022 associated with the electrode 60016 in the lower right hand corner of FIG. 32 can be output by the multiple input - single output switching device 60024 for analysis by the applicable processing circuit.

The selection signals S₀, S₁ can be provided to the multiple input - single output switching device 60024 by a processing circuit such as, for example, a processing circuit of the generator 60002 of the electrosurgical system 60000 of FIG. 29, a processing circuit of a nerve monitoring system which is separate from the generator 60002 of the electrosurgical system 60000 of FIG. 29, by a processing circuit of the surgical hub 206, by a processing circuit of the surgical hub 106, etc. For purposes of simplicity, the processing circuit is not shown in FIG. 32. By providing the various selection signals at a fast enough rate, the output signals of the respective sensing devices can effectively be scanned at a rate which allows for the timely analysis of all of the output signals of the respective sensing devices to determine the position of the stimulated nerve.

According to various aspects, the multiple input - single output switching device 60024 shown in FIG. 32 can be incorporated into the return pad 60006. According to other aspects, the multiple input - single output switching device 60024 shown in FIG. 32 can be incorporated into the second conductor/cable 60012 of the electrosurgical system 60000 of FIG. 29.

The control of the multiple input - single output switching device 60024 was described above in the context of a four input-one output switching device, corresponding to the four sensing devices 60022 shown in FIG. 32. It will be appreciated that for aspects where there are more than four sensing devices 60022 (e.g., sixteen sensing devices), the output signals of the more than more than four sensing devices 60022 will be input to the multiple input - single output switching device 60024, and more than two selection signals (e.g., S₀, S₁, S₂ and S₃) are required to control the output of the multiple input - single output switching device 60024.

For aspects where the output signals of the sensing devices 60024 are analog signals, the output of the multiple input - single output switching device 60024 can be converted into a corresponding digital signal by an analog-to-digital converter 60026 (shown as dashed lines in FIG. 32) prior to the performance of the analysis of the output signals by the applicable processing circuit.

By incorporating an array of sensing devices 60022 into a plurality of electrodes 60016 of the return pad 60006, the position of a nerve of the patient relative to the electrode tip of the electrosurgical instrument 60004 can be determined. The determined nerve location can be presented to the surgeon, thereby mitigating the likelihood of the surgeon inadvertently injuring or severing the nerve while utilizing the electrode tip to cut target tissue of the patient 60010.

According to various aspects, the detection of the nerve control signal and/or the movement of an anatomical feature of the patient by the sensing devices 60022 can be performed while the electrodes 60016 of the return pad 60006 are coupled to one another or while the electrodes 60016 are uncoupled from one another. For example, with regard to performing the detection when the respective electrodes 60016 of the return pad 60006 are uncoupled from one another, after positioning the patient 60010 on the operating table but before starting a surgical procedure, the return pad 60006 can be placed in a "sensing mode" by controlling the switching device 60020 shown in FIG. 31 to uncouple the respective electrodes 60016 of the return pad 60006 from one another. While the respective electrodes 60016 are uncoupled from one another, a nerve and/or a nerve bundle can be stimulated with an electrosurgical instrument 60004 as described above, and the respective output signals of the sensing devices 60022 of the return pad 60006 can be analyzed as described above to identify where the nerve, nerve bundle and/or nerve nexuses associated therewith are located. The locations may be input into a monopolar nerve stimulation algorithm profile which may reside, for example, in a memory circuit of the generator 60002, in a memory circuit of a different generator which is coupled to surgical instrument other than the electrosurgical instrument 60004, etc. Once the locations are input into the monopolar nerve stimulation algorithm profile, the locations are effectively isolated from the capacitive operation of the electrodes 60016 of the return pad 60006 and are used as sensing nodes of the monopolar nerve stimulation algorithm profile to inform the surgeon as the surgeon approaches a nerve and/or a nerve bundle while performing a tissue cutting procedure. According to various aspects, the surgeon may be informed of the nearby location of the nerve and/or nerve bundle via an audible warning, a visual warning, a vibratory warning, etc.

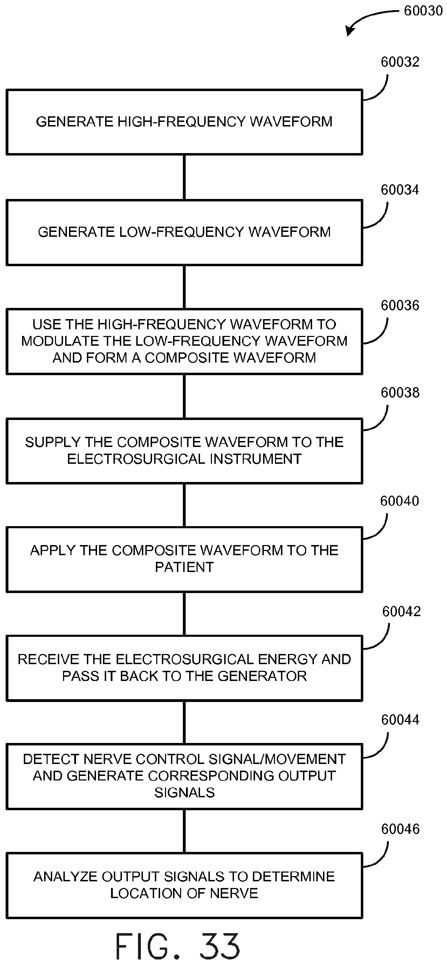

With regard to performing the detection when the respective electrodes 60016 of the return pad 60006 are coupled with one another, according to various aspects, as described in more detail below with reference to FIG. 33, the generator 60002 of the electrosurgical system 60000 of FIG. 29 can generate a high frequency waveform (the alternating current at radio frequency) which is modulated on a carrier wave, where the carrier wave is of a low enough frequency to stimulate a nerve of the patient. This allows for the sensing of the nerve control signal and/or the movement of an anatomical feature to be concurrent with the capacitive coupling of the respective electrodes 60016 of the return pad 60006 with the patient's body. By applying a specific waveform to the patient 60010 and sensing a specific response, there is a high level of confidence that the movement of the anatomical feature is a result of the applied waveform and not just some general movement. The modulation can be adjusted over time to stimulate different nerve sizes. According to various aspects, the modulation can be varied in amplitude over time in order to allow the applicable processing circuit to determine the distance the nerve and/or nerve bundle is from the signal without having to constantly stimulate the nerve and/or nerve bundle.

FIG. 33 illustrates a method 60030 for concurrently applying a nerve stimulation signal and electrosurgical energy to a patient, in accordance with at least one aspect of the present disclosure. In this illustrative procedure, the generator 60002 generates 60032 an alternating current at a radiofrequency level as a high frequency waveform. According to various aspects, a waveform generator of the generator 60002 performs this function.

The generator 60002 generates 60034 a low frequency waveform which is configured to stimulate a nerve of the patient. According to various aspects, the low frequency waveform is a monopolar nerve control signal and a waveform generator of the generator 60002 performs this function.

The generator 60002 utilizes 60036 the high frequency waveform generated at step 60032 to modulate the low frequency waveform generated at step 60034 to form a composite waveform. According to various aspects, a waveform generator of the generator 60002 performs this step. According to other embodiments, a processing circuit of the generator 60002 performs this function.

The generator 60002 supplies 60038 the composite waveform generated at step 60036 to the electrosurgical instrument 60004.

The electrode (i.e., the active electrode) of electrosurgical instrument 60004 applies 60040 the composite waveform to the patient 60010. The high frequency waveform operates to heat the target tissue of the patient 60010 and exits the patient's body before being received by the electrodes 60016 of the return pad 60006. The low frequency waveform operates to stimulate a nerve of the patient and/or cause a movement of an anatomical feature of the patient (e.g., a muscle twitch) resulting from application of the nerve control signal.

The plurality of electrodes 60016 which are coupled together via the switching device 60020, receive 60042 the electrosurgical energy exiting the patient's body and pass the electrosurgical energy back to the generator 60002 byway of the respective conductive members 60018, the switching device 60020 and the second conductor/cable 60012.

The sensing devices 60022 detect 60044 the monopolar nerve control signal applied to the patient 60010 and/or a movement of an anatomical feature of the patient 60010 (e.g., a muscle twitch) resulting from application of the nerve control signal, and generate respective output signals corresponding thereto. The outputs of the respective sensing devices 60022 are sampled as described above and forwarded to a processing circuit for analysis. The processing circuit can be, for example, a processing circuit of the generator 60002 of the electrosurgical system 60000 of FIG. 29, a processing circuit of a nerve monitoring system which is separate from the generator 60002 of the electrosurgical system 60000 of FIG. 29, by a processing circuit of the surgical hub 206, by a processing circuit of the surgical hub 106, etc.

The applicable processing circuit analyzes 60046 the output signals and determines where the nerve, nerve bundle and/or nerve nexuses associated with the output signals are located. As described above, according to various aspects, the respective output signals can serve as inputs to a monopolar nerve stimulation algorithm which is executed by the processing circuit, and the monopolar nerve stimulation algorithm operates to help prevent the surgeon from cutting or damaging the nerve, nerve bundle and/or nerve nexuses.

### Computer-Implemented Interactive Surgical System

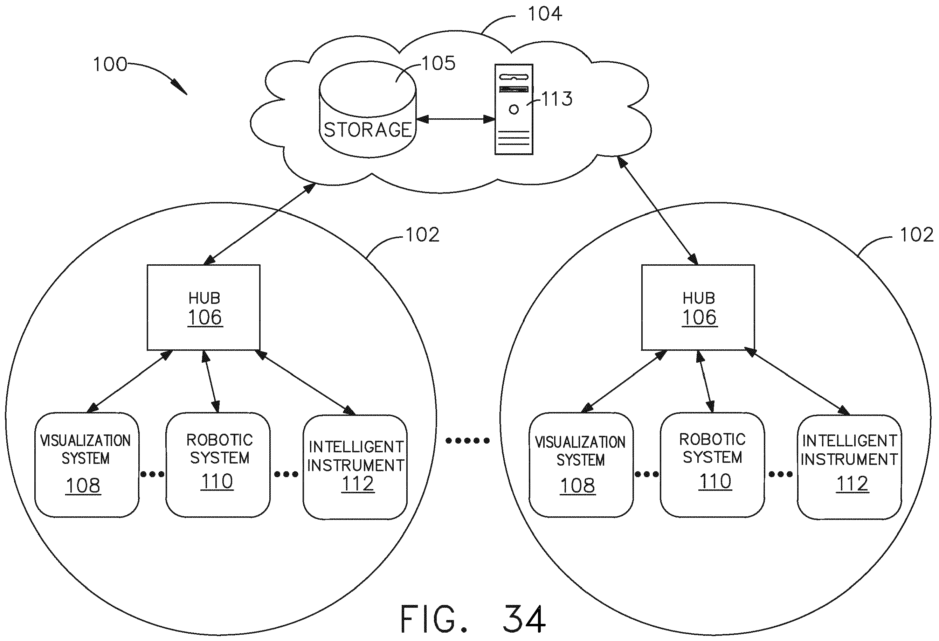

Referring to FIG. 34, a computer-implemented interactive surgical system 100 includes one or more surgical systems 102 and a cloud-based system (e.g., the cloud 104 that may include a remote server 113 coupled to a storage device 105). Each surgical system 102 includes at least one surgical hub 106 in communication with the cloud 104 that may include a remote server 113. In one example, as illustrated in FIG. 34, the surgical system 102 includes a visualization system 108, a robotic system 110, and a handheld intelligent surgical instrument 112, which are configured to communicate with one another and/or the hub 106. In some aspects, a surgical system 102 may include an M number of hubs 106, an N number of visualization systems 108, an O number of robotic systems 110, and a P number of handheld intelligent surgical instruments 112, where M, N, O, and P are integers greater than or equal to one.

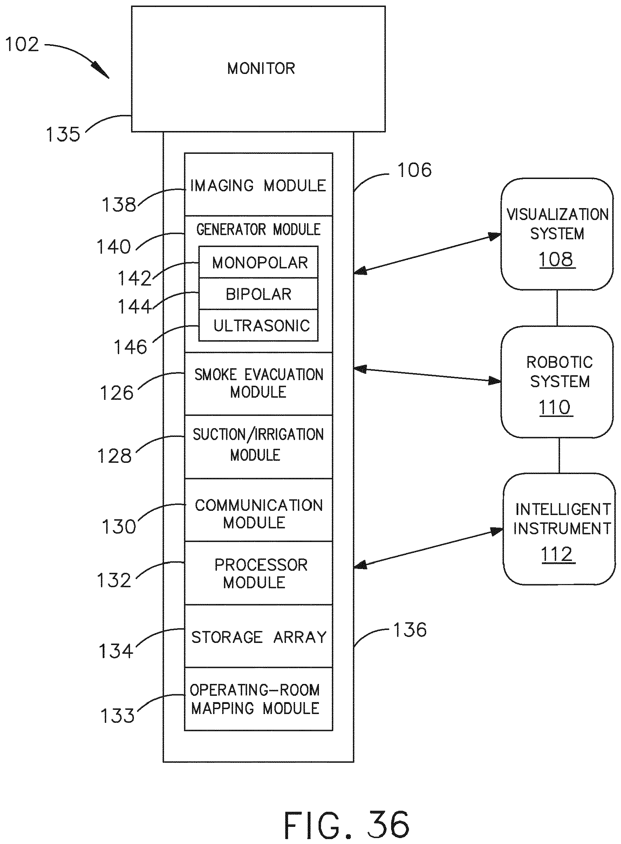

FIG. 36 depicts an example of a surgical system 102 being used to perform a surgical procedure on a patient who is lying down on an operating table 114 in a surgical operating room 116. A robotic system 110 is used in the surgical procedure as a part of the surgical system 102. The robotic system 110 includes a surgeon's console 118, a patient side cart 120 (surgical robot), and a surgical robotic hub 122. The patient side cart 120 can manipulate at least one removably coupled surgical tool 117 through a minimally invasive incision in the body of the patient while the surgeon views the surgical site through the surgeon's console 118. An image of the surgical site can be obtained by a medical imaging device 124, which can be manipulated by the patient side cart 120 to orient the imaging device 124. The robotic hub 122 can be used to process the images of the surgical site for subsequent display to the surgeon through the surgeon's console 118.

Other types of robotic systems can be readily adapted for use with the surgical system 102. Various examples of robotic systems and surgical tools that are suitable for use with the present disclosure are described in U.S. Provisional Patent Application Serial No. 62/611,339, titled ROBOT ASSISTED SURGICAL PLATFORM, filed December 28, 2017, the disclosure of which is herein incorporated by reference in its entirety.

Various examples of cloud-based analytics that are performed by the cloud 104, and are suitable for use with the present disclosure, are described in U.S. Provisional Patent Application Serial No. 62/611,340, titled CLOUD-BASED MEDICAL ANALYTICS, filed December 28, 2017, the disclosure of which is herein incorporated by reference in its entirety.

In various aspects, the imaging device 124 includes at least one image sensor and one or more optical components. Suitable image sensors include, but are not limited to, Charge-Coupled Device (CCD) sensors and Complementary Metal-Oxide Semiconductor (CMOS) sensors.

The optical components of the imaging device 124 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The one or more illumination sources may be configured to radiate electromagnetic energy in the visible spectrum as well as the invisible spectrum. The visible spectrum, sometimes referred to as the optical spectrum or luminous spectrum, is that portion of the electromagnetic spectrum that is visible to (i.e., can be detected by) the human eye and may be referred to as visible light or simply light. A typical human eye will respond to wavelengths in air that are from about 380 nm to about 750 nm.

The invisible spectrum (i.e., the non-luminous spectrum) is that portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 124 is configured for use in a minimally invasive procedure. Examples of imaging devices suitable for use with the present disclosure include, but not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

In one aspect, the imaging device employs multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information the human eye fails to capture with its receptors for red, green, and blue. The use of multi-spectral imaging is described in greater detail under the heading "Advanced Imaging Acquisition Module" in U.S. Provisional Patent Application Serial No. 62/611,341, titled INTERACTIVE SURGICAL PLATFORM, filed December 28, 2017, the disclosure of which is herein incorporated by reference in its entirety. Multi-spectrum monitoring can be a useful tool in relocating a surgical field after a surgical task is completed to perform one or more of the previously described tests on the treated tissue.

It is axiomatic that strict sterilization of the operating room and surgical equipment is required during any surgery. The strict hygiene and sterilization conditions required in a "surgical theater," i.e., an operating or treatment room, necessitate the highest possible sterility of all medical devices and equipment. Part of that sterilization process is the need to sterilize anything that comes in contact with the patient or penetrates the sterile field, including the imaging device 124 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

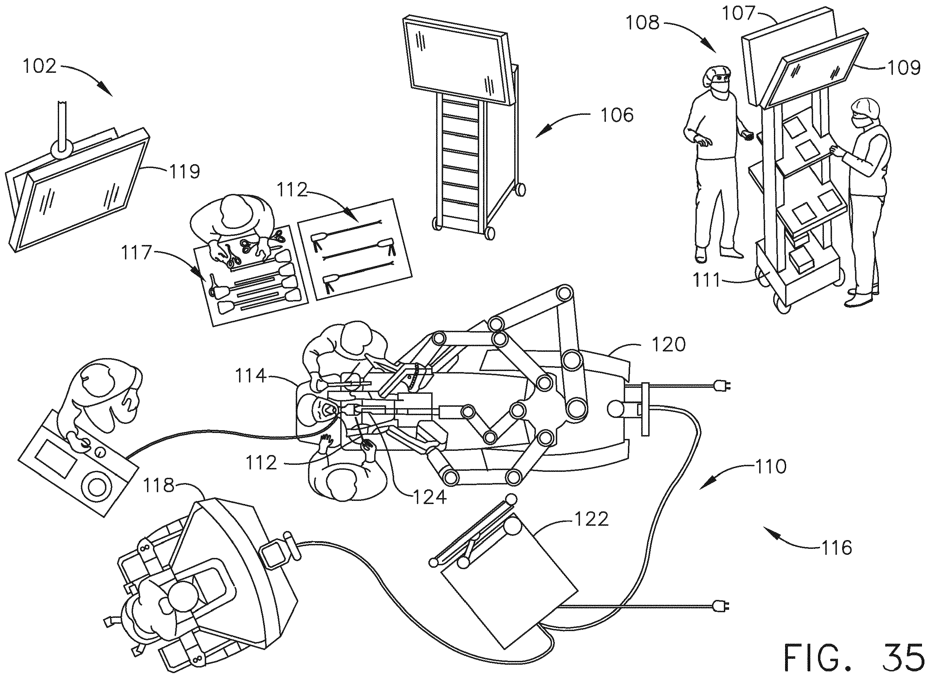

In various aspects, the visualization system 108 includes one or more imaging sensors, one or more image processing units, one or more storage arrays, and one or more displays that are strategically arranged with respect to the sterile field, as illustrated in FIG. 35. In one aspect, the visualization system 108 includes an interface for HL7, PACS, and EMR. Various components of the visualization system 108 are described under the heading "Advanced Imaging Acquisition Module" in U.S. Provisional Patent Application Serial No. 62/611,341, titled INTERACTIVE SURGICAL PLATFORM, filed December 28, 2017, the disclosure of which is herein incorporated by reference in its entirety.

As illustrated in FIG. 35, a primary display 119 is positioned in the sterile field to be visible to an operator at the operating table 114. In addition, a visualization tower 111 is positioned outside the sterile field. The visualization tower 111 includes a first non-sterile display 107 and a second non-sterile display 109, which face away from each other. The visualization system 108, guided by the hub 106, is configured to utilize the displays 107, 109, and 119 to coordinate information flow to operators inside and outside the sterile field. For example, the hub 106 may cause the visualization system 108 to display a snap-shot of a surgical site, as recorded by an imaging device 124, on a non-sterile display 107 or 109, while maintaining a live feed of the surgical site on the primary display 119. The snap-shot on the non-sterile display 107 or 109 can permit a non-sterile operator to perform a diagnostic step relevant to the surgical procedure, for example.

In one aspect, the hub 106 is also configured to route a diagnostic input or feedback entered by a non-sterile operator at the visualization tower 111 to the primary display 119 within the sterile field, where it can be viewed by a sterile operator at the operating table. In one example, the input can be in the form of a modification to the snap-shot displayed on the non-sterile display 107 or 109, which can be routed to the primary display 119 by the hub 106.

Referring to FIG. 35, a surgical instrument 112 is being used in the surgical procedure as part of the surgical system 102. The hub 106 is also configured to coordinate information flow to a display of the surgical instrument 112. For example, in U.S. Provisional Patent Application Serial No. 62/611,341, titled INTERACTIVE SURGICAL PLATFORM, filed December 28, 2017, the disclosure of which is herein incorporated by reference in its entirety. A diagnostic input or feedback entered by a non-sterile operator at the visualization tower 111 can be routed by the hub 106 to the surgical instrument display 115 within the sterile field, where it can be viewed by the operator of the surgical instrument 112. Example surgical instruments that are suitable for use with the surgical system 102 are described under the heading "Surgical Instrument Hardware" and in U.S. Provisional Patent Application Serial No. 62/611,341, titled INTERACTIVE SURGICAL PLATFORM, filed December 28, 2017, the disclosure of which is herein incorporated by reference in its entirety, for example.

Referring now to FIG. 36, a hub 106 is depicted in communication with a visualization system 108, a robotic system 110, and a handheld intelligent surgical instrument 112. The hub 106 includes a hub display 135, an imaging module 138, a generator module 140, a communication module 130, a processor module 132, and a storage array 134. In certain aspects, as illustrated in FIG. 36, the hub 106 further includes a smoke evacuation module 126 and/or a suction/irrigation module 128.

During a surgical procedure, energy application to tissue, for sealing and/or cutting, is generally associated with smoke evacuation, suction of excess fluid, and/or irrigation of the tissue. Fluid, power, and/or data lines from different sources are often entangled during the surgical procedure. Valuable time can be lost addressing this issue during a surgical procedure. Detangling the lines may necessitate disconnecting the lines from their respective modules, which may require resetting the modules. The hub modular enclosure 136 offers a unified environment for managing the power, data, and fluid lines, which reduces the frequency of entanglement between such lines.

Aspects of the present disclosure present a surgical hub for use in a surgical procedure that involves energy application to tissue at a surgical site. The surgical hub includes a hub enclosure and a combo generator module slidably receivable in a docking station of the hub enclosure. The docking station includes data and power contacts. The combo generator module includes two or more of an ultrasonic energy generator component, a bipolar RF energy generator component, and a monopolar RF energy generator component that are housed in a single unit. In one aspect, the combo generator module also includes a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component.

In one aspect, the fluid line is a first fluid line and a second fluid line extends from the remote surgical site to a suction and irrigation module slidably received in the hub enclosure. In one aspect, the hub enclosure comprises a fluid interface.

Certain surgical procedures may require the application of more than one energy type to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 136 is configured to accommodate different generators, and facilitate an interactive communication therebetween. One of the advantages of the hub modular enclosure 136 is enabling the quick removal and/or replacement of various modules.

Aspects of the present disclosure present a modular surgical enclosure for use in a surgical procedure that involves energy application to tissue. The modular surgical enclosure includes a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts.

Further to the above, the modular surgical enclosure also includes a second energy-generator module configured to generate a second energy, different than the first energy, for application to the tissue, and a second docking station comprising a second docking port that includes second data and power contacts, wherein the second energy-generator module is slidably movable into an electrical engagement with the power and data contacts, and wherein the second energy-generator module is slidably movable out of the electrical engagement with the second power and data contacts.

In addition, the modular surgical enclosure also includes a communication bus between the first docking port and the second docking port, configured to facilitate communication between the first energy-generator module and the second energy-generator module.

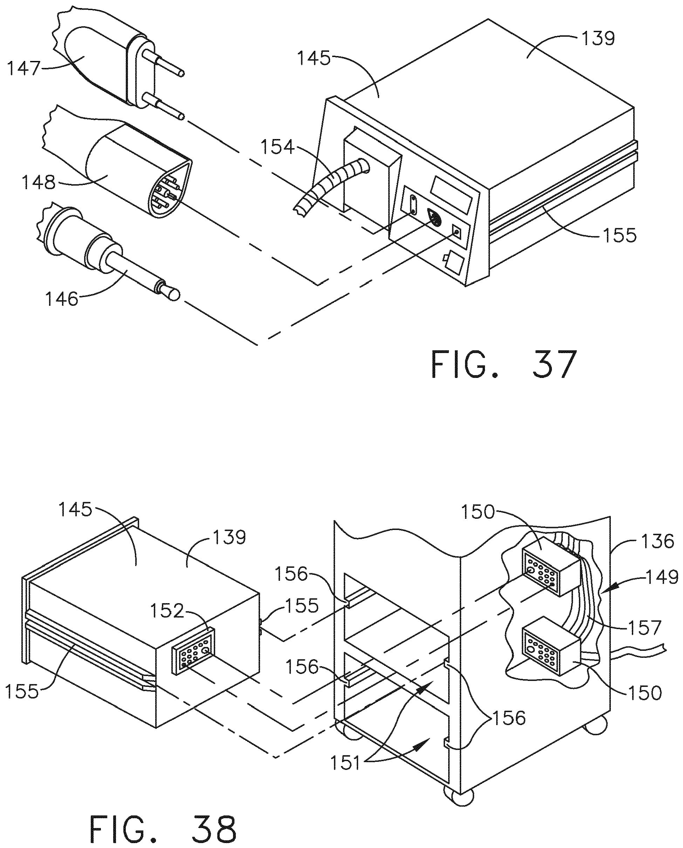

Referring to FIGS. 3-7, aspects of the present disclosure are presented for a hub modular enclosure 136 that allows the modular integration of a generator module 140, a smoke evacuation module 126, and a suction/irrigation module 128. The hub modular enclosure 136 further facilitates interactive communication between the modules 140, 126, 128. As illustrated in FIG. 38, the generator module 140 can be a generator module with integrated monopolar, bipolar, and ultrasonic components supported in a single housing unit 139 slidably insertable into the hub modular enclosure 136. As illustrated in FIG. 38, the generator module 140 can be configured to connect to a monopolar device 146, a bipolar device 147, and an ultrasonic device 148. Alternatively, the generator module 140 may comprise a series of monopolar, bipolar, and/or ultrasonic generator modules that interact through the hub modular enclosure 136. The hub modular enclosure 136 can be configured to facilitate the insertion of multiple generators and interactive communication between the generators docked into the hub modular enclosure 136 so that the generators would act as a single generator.

In one aspect, the hub modular enclosure 136 comprises a modular power and communication backplane 149 with external and wireless communication headers to enable the removable attachment of the modules 140, 126, 128 and interactive communication therebetween.

In one aspect, the hub modular enclosure 136 includes docking stations, or drawers, 151, herein also referred to as drawers, which are configured to slidably receive the modules 140, 126, 128. FIG. 37 illustrates a partial perspective view of a surgical hub enclosure 136, and a combo generator module 145 slidably receivable in a docking station 151 of the surgical hub enclosure 136. A docking port 152 with power and data contacts on a rear side of the combo generator module 145 is configured to engage a corresponding docking port 150 with power and data contacts of a corresponding docking station 151 of the hub modular enclosure 136 as the combo generator module 145 is slid into position within the corresponding docking station 151 of the hub module enclosure 136. In one aspect, the combo generator module 145 includes a bipolar, ultrasonic, and monopolar module and a smoke evacuation module integrated together into a single housing unit 139, as illustrated in FIG. 38.

In various aspects, the smoke evacuation module 126 includes a fluid line 154 that conveys captured/collected smoke and/or fluid away from a surgical site and to, for example, the smoke evacuation module 126. Vacuum suction originating from the smoke evacuation module 126 can draw the smoke into an opening of a utility conduit at the surgical site. The utility conduit, coupled to the fluid line, can be in the form of a flexible tube terminating at the smoke evacuation module 126. The utility conduit and the fluid line define a fluid path extending toward the smoke evacuation module 126 that is received in the hub enclosure 136.

In various aspects, the suction/irrigation module 128 is coupled to a surgical tool comprising an aspiration fluid line and a suction fluid line. In one example, the aspiration and suction fluid lines are in the form of flexible tubes extending from the surgical site toward the suction/irrigation module 128. One or more drive systems can be configured to cause irrigation and aspiration of fluids to and from the surgical site.

In one aspect, the surgical tool includes a shaft having an end effector at a distal end thereof and at least one energy treatment associated with the end effector, an aspiration tube, and an irrigation tube. The aspiration tube can have an inlet port at a distal end thereof and the aspiration tube extends through the shaft. Similarly, an irrigation tube can extend through the shaft and can have an inlet port in proximity to the energy deliver implement. The energy deliver implement is configured to deliver ultrasonic and/or RF energy to the surgical site and is coupled to the generator module 140 by a cable extending initially through the shaft.

The irrigation tube can be in fluid communication with a fluid source, and the aspiration tube can be in fluid communication with a vacuum source. The fluid source and/or the vacuum source can be housed in the suction/irrigation module 128. In one example, the fluid source and/or the vacuum source can be housed in the hub enclosure 136 separately from the suction/irrigation module 128. In such example, a fluid interface can be configured to connect the suction/irrigation module 128 to the fluid source and/or the vacuum source.

In one aspect, the modules 140, 126, 128 and/or their corresponding docking stations on the hub modular enclosure 136 may include alignment features that are configured to align the docking ports of the modules into engagement with their counterparts in the docking stations of the hub modular enclosure 136. For example, as illustrated in FIG. 37, the combo generator module 145 includes side brackets 155 that are configured to slidably engage with corresponding brackets 156 of the corresponding docking station 151 of the hub modular enclosure 136. The brackets cooperate to guide the docking port contacts of the combo generator module 145 into an electrical engagement with the docking port contacts of the hub modular enclosure 136.

In some aspects, the drawers 151 of the hub modular enclosure 136 are the same, or substantially the same size, and the modules are adjusted in size to be received in the drawers 151. For example, the side brackets 155 and/or 156 can be larger or smaller depending on the size of the module. In other aspects, the drawers 151 are different in size and are each designed to accommodate a particular module.

Furthermore, the contacts of a particular module can be keyed for engagement with the contacts of a particular drawer to avoid inserting a module into a drawer with mismatching contacts.

As illustrated in FIG. 37, the docking port 150 of one drawer 151 can be coupled to the docking port 150 of another drawer 151 through a communications link 157 to facilitate an interactive communication between the modules housed in the hub modular enclosure 136. The docking ports 150 of the hub modular enclosure 136 may alternatively, or additionally, facilitate a wireless interactive communication between the modules housed in the hub modular enclosure 136. Any suitable wireless communication can be employed, such as for example Air Titan-Bluetooth.

FIG. 39 illustrates individual power bus attachments for a plurality of lateral docking ports of a lateral modular housing 160 configured to receive a plurality of modules of a surgical hub 206. The lateral modular housing 160 is configured to laterally receive and interconnect the modules 161. The modules 161 are slidably inserted into docking stations 162 of lateral modular housing 160, which includes a backplane for interconnecting the modules 161. As illustrated in FIG. 39, the modules 161 are arranged laterally in the lateral modular housing 160. Alternatively, the modules 161 may be arranged vertically in a lateral modular housing.

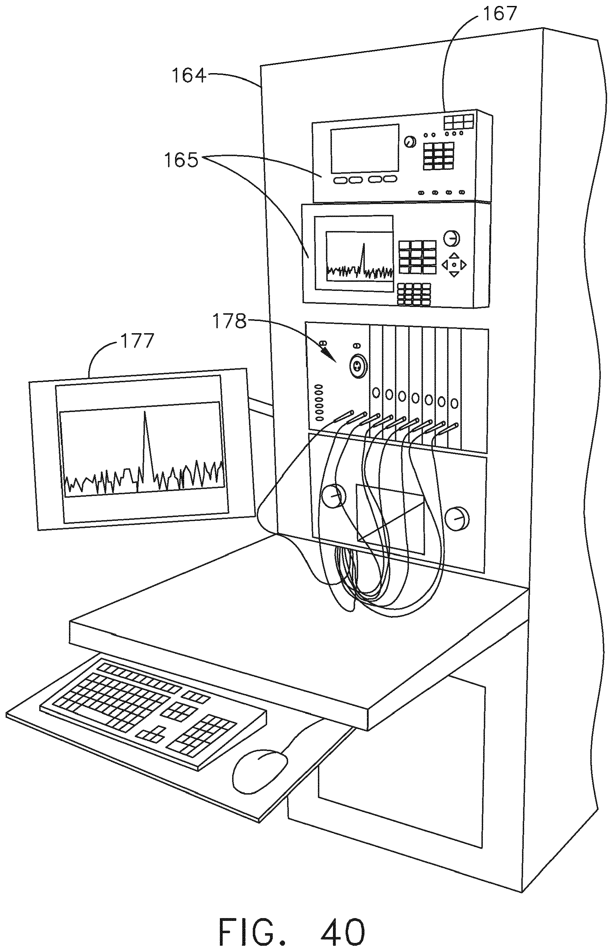

FIG. 40 illustrates a vertical modular housing 164 configured to receive a plurality of modules 165 of the surgical hub 106. The modules 165 are slidably inserted into docking stations, or drawers, 167 of vertical modular housing 164, which includes a backplane for interconnecting the modules 165. Although the drawers 167 of the vertical modular housing 164 are arranged vertically, in certain instances, a vertical modular housing 164 may include drawers that are arranged laterally. Furthermore, the modules 165 may interact with one another through the docking ports of the vertical modular housing 164. In the example of FIG. 31, a display 177 is provided for displaying data relevant to the operation of the modules 165. In addition, the vertical modular housing 164 includes a master module 178 housing a plurality of sub-modules that are slidably received in the master module 178.

In various aspects, the imaging module 138 comprises an integrated video processor and a modular light source and is adapted for use with various imaging devices. In one aspect, the imaging device is comprised of a modular housing that can be assembled with a light source module and a camera module. The housing can be a disposable housing. In at least one example, the disposable housing is removably coupled to a reusable controller, a light source module, and a camera module. The light source module and/or the camera module can be selectively chosen depending on the type of surgical procedure. In one aspect, the camera module comprises a CCD sensor. In another aspect, the camera module comprises a CMOS sensor. In another aspect, the camera module is configured for scanned beam imaging. Likewise, the light source module can be configured to deliver a white light or a different light, depending on the surgical procedure.

During a surgical procedure, removing a surgical device from the surgical field and replacing it with another surgical device that includes a different camera or a different light source can be inefficient. Temporarily losing sight of the surgical field may lead to undesirable consequences. The module imaging device of the present disclosure is configured to permit the replacement of a light source module or a camera module midstream during a surgical procedure, without having to remove the imaging device from the surgical field.

In one aspect, the imaging device comprises a tubular housing that includes a plurality of channels. A first channel is configured to slidably receive the camera module, which can be configured for a snap-fit engagement with the first channel. A second channel is configured to slidably receive the light source module, which can be configured for a snap-fit engagement with the second channel. In another example, the camera module and/or the light source module can be rotated into a final position within their respective channels. A threaded engagement can be employed in lieu of the snap-fit engagement.

In various examples, multiple imaging devices are placed at different positions in the surgical field to provide multiple views. The imaging module 138 can be configured to switch between the imaging devices to provide an optimal view. In various aspects, the imaging module 138 can be configured to integrate the images from the different imaging device.

Various image processors and imaging devices suitable for use with the present disclosure are described in U.S. Patent No. 7,995,045, titled COMBINED SBI AND CONVENTIONAL IMAGE PROCESSOR, which issued on August 9, 2011, which is herein incorporated by reference in its entirety. In addition, U.S. Patent No. 7,982,776, titled SBI MOTION ARTIFACT REMOVAL APPARATUS AND METHOD, which issued on July 19, 2011, which is herein incorporated by reference in its entirety, describes various systems for removing motion artifacts from image data. Such systems can be integrated with the imaging module 138. Furthermore, U.S. Patent Application Publication No. 2011/0306840, titled CONTROLLABLE MAGNETIC SOURCE TO FIXTURE INTRACORPOREAL APPARATUS, which published on December 15, 2011, and U.S. Patent Application Publication No. 2014/0243597, titled SYSTEM FOR PERFORMING A MINIMALLY INVASIVE SURGICAL PROCEDURE, which published on August 28, 2014, each of which is herein incorporated by reference in its entirety.

FIG. 41 illustrates a surgical data network 201 comprising a modular communication hub 203 configured to connect modular devices located in one or more operating theaters of a healthcare facility, or any room in a healthcare facility specially equipped for surgical operations, to a cloud-based system (e.g., the cloud 204 that may include a remote server 213 coupled to a storage device 205). In one aspect, the modular communication hub 203 comprises a network hub 207 and/or a network switch 209 in communication with a network router. The modular communication hub 203 also can be coupled to a local computer system 210 to provide local computer processing and data manipulation. The surgical data network 201 may be configured as passive, intelligent, or switching. A passive surgical data network serves as a conduit for the data, enabling it to go from one device (or segment) to another and to the cloud computing resources. An intelligent surgical data network includes additional features to enable the traffic passing through the surgical data network to be monitored and to configure each port in the network hub 207 or network switch 209. An intelligent surgical data network may be referred to as a manageable hub or switch. A switching hub reads the destination address of each packet and then forwards the packet to the correct port.

Modular devices 1a-1n located in the operating theater may be coupled to the modular communication hub 203. The network hub 207 and/or the network switch 209 may be coupled to a network router 211 to connect the devices 1a-1n to the cloud 204 or the local computer system 210. Data associated with the devices 1a-1n maybe transferred to cloud-based computers via the router for remote data processing and manipulation. Data associated with the devices 1a-1n may also be transferred to the local computer system 210 for local data processing and manipulation. Modular devices 2a-2m located in the same operating theater also maybe coupled to a network switch 209. The network switch 209 may be coupled to the network hub 207 and/or the network router 211 to connect to the devices 2a-2m to the cloud 204. Data associated with the devices 2a-2n may be transferred to the cloud 204 via the network router 211 for data processing and manipulation. Data associated with the devices 2a-2m may also be transferred to the local computer system 210 for local data processing and manipulation.

It will be appreciated that the surgical data network 201 may be expanded by interconnecting multiple network hubs 207 and/or multiple network switches 209 with multiple network routers 211. The modular communication hub 203 maybe contained in a modular control tower configured to receive multiple devices 1a-1n/2a-2m. The local computer system 210 also may be contained in a modular control tower. The modular communication hub 203 is connected to a display 212 to display images obtained by some of the devices 1a-1n/2a-2m, for example during surgical procedures. In various aspects, the devices 1a-1n/2a-2m may include, for example, various modules such as an imaging module 138 coupled to an endoscope, a generator module 140 coupled to an energy-based surgical device, a smoke evacuation module 126, a suction/irrigation module 128, a communication module 130, a processor module 132, a storage array 134, a surgical device coupled to a display, and/or a non-contact sensor module, among other modular devices that may be connected to the modular communication hub 203 of the surgical data network 201.

In one aspect, the surgical data network 201 may comprise a combination of network hub(s), network switch(es), and network router(s) connecting the devices 1a-1n/2a-2m to the cloud. Any one of or all of the devices 1a-1n/2a-2m coupled to the network hub or network switch may collect data in real time and transfer the data to cloud computers for data processing and manipulation. It will be appreciated that cloud computing relies on sharing computing resources rather than having local servers or personal devices to handle software applications. The word "cloud" may be used as a metaphor for "the Internet," although the term is not limited as such. Accordingly, the term "cloud computing" may be used herein to refer to "a type of Internet-based computing," where different services-such as servers, storage, and applications-are delivered to the modular communication hub 203 and/or computer system 210 located in the surgical theater (e.g., a fixed, mobile, temporary, or field operating room or space) and to devices connected to the modular communication hub 203 and/or computer system 210 through the Internet. The cloud infrastructure may be maintained by a cloud service provider. In this context, the cloud service provider may be the entity that coordinates the usage and control of the devices 1a-1n/2a-2m located in one or more operating theaters. The cloud computing services can perform a large number of calculations based on the data gathered by smart surgical instruments, robots, and other computerized devices located in the operating theater. The hub hardware enables multiple devices or connections to be connected to a computer that communicates with the cloud computing resources and storage.

Applying cloud computer data processing techniques on the data collected by the devices 1a-1n/2a-2m, the surgical data network provides improved surgical outcomes, reduced costs, and improved patient satisfaction. At least some of the devices 1a-1n/2a-2m may be employed to view tissue states to assess leaks or perfusion of sealed tissue after a tissue sealing and cutting procedure. At least some of the devices 1a-1n/2a-2m may be employed to identify pathology, such as the effects of diseases, using the cloud-based computing to examine data including images of samples of body tissue for diagnostic purposes. This includes localization and margin confirmation of tissue and phenotypes. At least some of the devices 1a-1n/2a-2m maybe employed to identify anatomical structures of the body using a variety of sensors integrated with imaging devices and techniques such as overlaying images captured by multiple imaging devices. The data gathered by the devices 1a-1n/2a-2m, including image data, may be transferred to the cloud 204 or the local computer system 210 or both for data processing and manipulation including image processing and manipulation. The data may be analyzed to improve surgical procedure outcomes by determining if further treatment, such as the application of endoscopic intervention, emerging technologies, a targeted radiation, targeted intervention, and precise robotics to tissue-specific sites and conditions, may be pursued. Such data analysis may further employ outcome analytics processing, and using standardized approaches may provide beneficial feedback to either confirm surgical treatments and the behavior of the surgeon or suggest modifications to surgical treatments and the behavior of the surgeon.

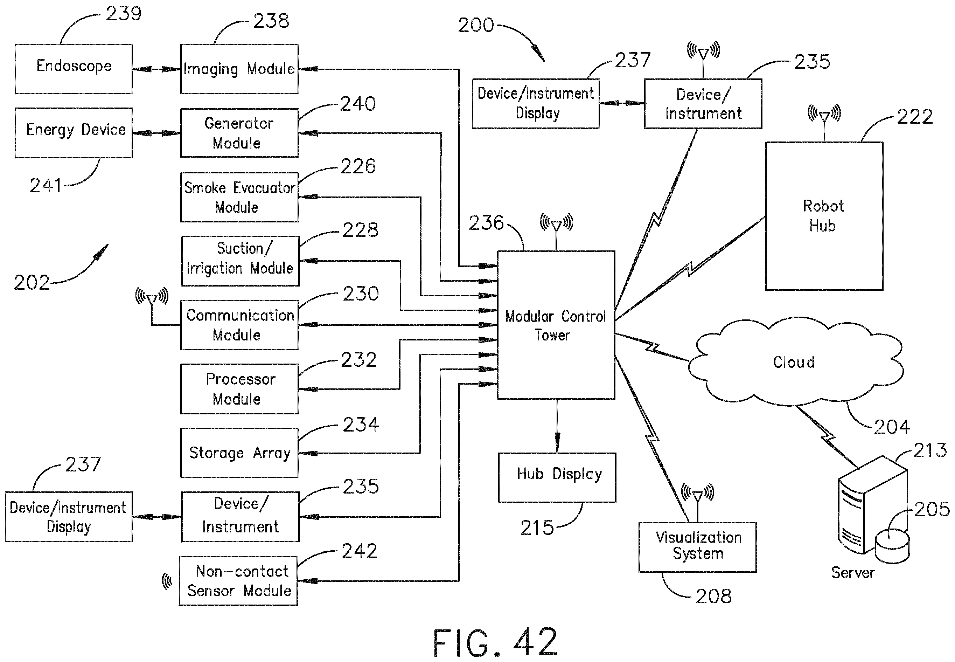

In one implementation, the operating theater devices 1a-1n may be connected to the modular communication hub 203 over a wired channel or a wireless channel depending on the configuration of the devices 1a-1n to a network hub. The network hub 207 may be implemented, in one aspect, as a local network broadcast device that works on the physical layer of the Open System Interconnection (OSI) model. The network hub provides connectivity to the devices 1a-1n located in the same operating theater network. The network hub 207 collects data in the form of packets and sends them to the router in half duplex mode. The network hub 207 does not store any media access control/internet protocol (MAC/IP) to transfer the device data. Only one of the devices 1a-1n can send data at a time through the network hub 207. The network hub 207 has no routing tables or intelligence regarding where to send information and broadcasts all network data across each connection and to a remote server 213 (FIG. 42) over the cloud 204. The network hub 207 can detect basic network errors such as collisions, but having all information broadcast to multiple ports can be a security risk and cause bottlenecks.

In another implementation, the operating theater devices 2a-2m may be connected to a network switch 209 over a wired channel or a wireless channel. The network switch 209 works in the data link layer of the OSI model. The network switch 209 is a multicast device for connecting the devices 2a-2m located in the same operating theater to the network. The network switch 209 sends data in the form of frames to the network router 211 and works in full duplex mode. Multiple devices 2a-2m can send data at the same time through the network switch 209. The network switch 209 stores and uses MAC addresses of the devices 2a-2m to transfer data.

The network hub 207 and/or the network switch 209 are coupled to the network router 211 for connection to the cloud 204. The network router 211 works in the network layer of the OSI model. The network router 211 creates a route for transmitting data packets received from the network hub 207 and/or network switch 211 to cloud-based computer resources for further processing and manipulation of the data collected by any one of or all the devices 1a-1n/2a-2m. The network router 211 may be employed to connect two or more different networks located in different locations, such as, for example, different operating theaters of the same healthcare facility or different networks located in different operating theaters of different healthcare facilities. The network router 211 sends data in the form of packets to the cloud 204 and works in full duplex mode. Multiple devices can send data at the same time. The network router 211 uses IP addresses to transfer data.

In one example, the network hub 207 may be implemented as a USB hub, which allows multiple USB devices to be connected to a host computer. The USB hub may expand a single USB port into several tiers so that there are more ports available to connect devices to the host system computer. The network hub 207 may include wired or wireless capabilities to receive information over a wired channel or a wireless channel. In one aspect, a wireless USB short-range, high-bandwidth wireless radio communication protocol may be employed for communication between the devices 1a-1n and devices 2a-2m located in the operating theater.

In other examples, the operating theater devices 1a-1n/2a-2m may communicate to the modular communication hub 203 via Bluetooth wireless technology standard for exchanging data over short distances (using short-wavelength UHF radio waves in the ISM band from 2.4 to 2.485 GHz) from fixed and mobile devices and building personal area networks (PANs). In other aspects, the operating theater devices 1a-1n/2a-2m may communicate to the modular communication hub 203 via a number of wireless or wired communication standards or protocols, including but not limited to Wi-Fi (IEEE 802.11 family), WiMAX (IEEE 802.16 family), IEEE 802.20, long-term evolution (LTE), and Ev-DO, HSPA+, HSDPA+, HSUPA+, EDGE, GSM, GPRS, CDMA, TDMA, DECT, and Ethernet derivatives thereof, as well as any other wireless and wired protocols that are designated as 3G, 4G, 5G, and beyond. The computing module may include a plurality of communication modules. For instance, a first communication module may be dedicated to shorter-range wireless communications such as Wi-Fi and Bluetooth, and a second communication module may be dedicated to longer-range wireless communications such as GPS, EDGE, GPRS, CDMA, WiMAX, LTE, Ev-DO, and others.

The modular communication hub 203 may serve as a central connection for one or all of the operating theater devices 1a-1n/2a-2m and handles a data type known as frames. Frames carry the data generated by the devices 1a-1n/2a-2m. When a frame is received by the modular communication hub 203, it is amplified and transmitted to the network router 211, which transfers the data to the cloud computing resources by using a number of wireless or wired communication standards or protocols, as described herein.

The modular communication hub 203 can be used as a standalone device or be connected to compatible network hubs and network switches to form a larger network. The modular communication hub 203 is generally easy to install, configure, and maintain, making it a good option for networking the operating theater devices 1a-1n/2a-2m.

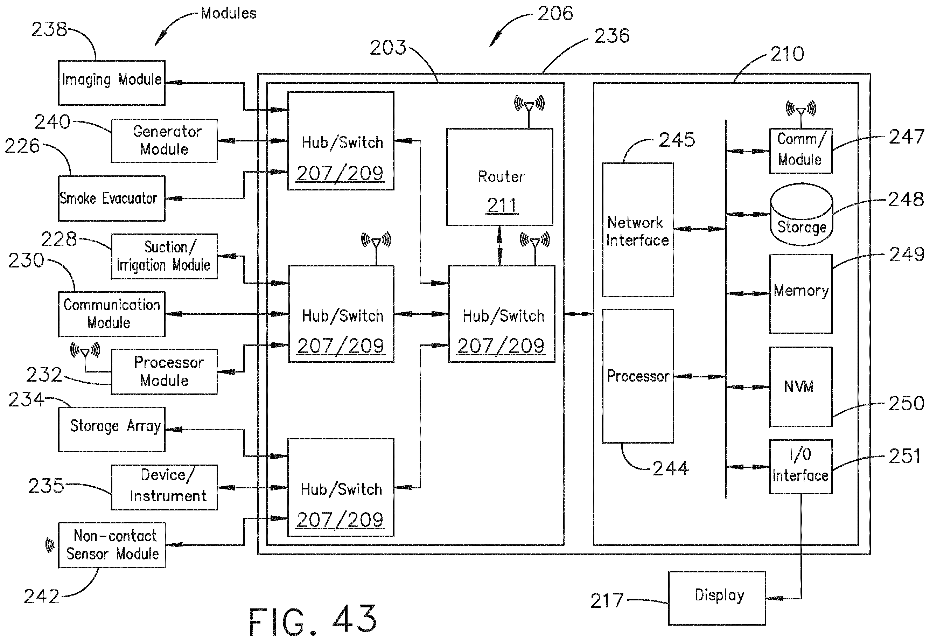

FIG. 42 illustrates a computer-implemented interactive surgical system 200. The computer-implemented interactive surgical system 200 is similar in many respects to the computer-implemented interactive surgical system 100. For example, the computer-implemented interactive surgical system 200 includes one or more surgical systems 202, which are similar in many respects to the surgical systems 102. Each surgical system 202 includes at least one surgical hub 206 in communication with a cloud 204 that may include a remote server 213. In one aspect, the computer-implemented interactive surgical system 200 comprises a modular control tower 236 connected to multiple operating theater devices such as, for example, intelligent surgical instruments, robots, and other computerized devices located in the operating theater. As shown in FIG. 43, the modular control tower 236 comprises a modular communication hub 203 coupled to a computer system 210. As illustrated in the example of FIG. 42, the modular control tower 236 is coupled to an imaging module 238 that is coupled to an endoscope 239, a generator module 240 that is coupled to an energy device 241, a smoke evacuator module 226, a suction/irrigation module 228, a communication module 230, a processor module 232, a storage array 234, a smart device/instrument 235 optionally coupled to a display 237, and a non-contact sensor module 242. The operating theater devices are coupled to cloud computing resources and data storage via the modular control tower 236. A robot hub 222 also may be connected to the modular control tower 236 and to the cloud computing resources. The devices/instruments 235, visualization systems 208, among others, may be coupled to the modular control tower 236 via wired or wireless communication standards or protocols, as described herein. The modular control tower 236 may be coupled to a hub display 215 (e.g., monitor, screen) to display and overlay images received from the imaging module, device/instrument display, and/or other visualization systems 208. The hub display also may display data received from devices connected to the modular control tower in conjunction with images and overlaid images.

FIG. 43 illustrates a surgical hub 206 comprising a plurality of modules coupled to the modular control tower 236. The modular control tower 236 comprises a modular communication hub 203, e.g., a network connectivity device, and a computer system 210 to provide local processing, visualization, and imaging, for example. As shown in FIG. 43, the modular communication hub 203 may be connected in a tiered configuration to expand the number of modules (e.g., devices) that may be connected to the modular communication hub 203 and transfer data associated with the modules to the computer system 210, cloud computing resources, or both. As shown in FIG. 43, each of the network hubs/switches in the modular communication hub 203 includes three downstream ports and one upstream port. The upstream network hub/switch is connected to a processor to provide a communication connection to the cloud computing resources and a local display 217. Communication to the cloud 204 may be made either through a wired or a wireless communication channel.

The surgical hub 206 employs a non-contact sensor module 242 to measure the dimensions of the operating theater and generate a map of the surgical theater using either ultrasonic or laser-type non-contact measurement devices. An ultrasound-based non-contact sensor module scans the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, the disclosure of which is herein incorporated by reference in its entirety, in which the sensor module is configured to determine the size of the operating theater and to adjust Bluetooth-pairing distance limits. A laser-based non-contact sensor module scans the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

The computer system 210 comprises a processor 244 and a network interface 245. The processor 244 is coupled to a communication module 247, storage 248, memory 249, non-volatile memory 250, and input/output interface 251 via a system bus. The system bus can be any of several types of bus structure(s) including the memory bus or memory controller, a peripheral bus or external bus, and/or a local bus using any variety of available bus architectures including, but not limited to, 9-bit bus, Industrial Standard Architecture (ISA), Micro-Channel Architecture (MSA), Extended ISA (EISA), Intelligent Drive Electronics (IDE), VESA Local Bus (VLB), Peripheral Component Interconnect (PCI), USB, Advanced Graphics Port (AGP), Personal Computer Memory Card International Association bus (PCMCIA), Small Computer Systems Interface (SCSI), or any other proprietary bus.

The processor 244 may be any single-core or multicore processor such as those known under the trade name ARM Cortex by Texas Instruments. In one aspect, the processor may be an LM4F230H5QR ARM Cortex-M4F Processor Core, available from Texas Instruments, for example, comprising an on-chip memory of 256 KB single-cycle flash memory, or other non-volatile memory, up to 40 MHz, a prefetch buffer to improve performance above 40 MHz, a 32 KB single-cycle serial random access memory (SRAM), an internal read-only memory (ROM) loaded with StellarisWare® software, a 2 KB electrically erasable programmable read-only memory (EEPROM), and/or one or more pulse width modulation (PWM) modules, one or more quadrature encoder inputs (QEI) analogs, one or more 12-bit analog-to-digital converters (ADCs) with 12 analog input channels, details of which are available for the product datasheet.

In one aspect, the processor 244 may comprise a safety controller comprising two controller-based families such as TMS570 and RM4x, known under the trade name Hercules ARM Cortex R4, also by Texas Instruments. The safety controller may be configured specifically for IEC 61508 and ISO 26262 safety critical applications, among others, to provide advanced integrated safety features while delivering scalable performance, connectivity, and memory options.

The system memory includes volatile memory and non-volatile memory. The basic input/output system (BIOS), containing the basic routines to transfer information between elements within the computer system, such as during start-up, is stored in non-volatile memory. For example, the non-volatile memory can include ROM, programmable ROM (PROM), electrically programmable ROM (EPROM), EEPROM, or flash memory. Volatile memory includes random-access memory (RAM), which acts as external cache memory. Moreover, RAM is available in many forms such as SRAM, dynamic RAM (DRAM), synchronous DRAM (SDRAM), double data rate SDRAM (DDR SDRAM), enhanced SDRAM (ESDRAM), Synchlink DRAM (SLDRAM), and direct Rambus RAM (DRRAM).

The computer system 210 also includes removable/non-removable, volatile/non-volatile computer storage media, such as for example disk storage. The disk storage includes, but is not limited to, devices like a magnetic disk drive, floppy disk drive, tape drive, Jaz drive, Zip drive, LS-60 drive, flash memory card, or memory stick. In addition, the disk storage can include storage media separately or in combination with other storage media including, but not limited to, an optical disc drive such as a compact disc ROM device (CD-ROM), compact disc recordable drive (CD-R Drive), compact disc rewritable drive (CD-RW Drive), or a digital versatile disc ROM drive (DVD-ROM). To facilitate the connection of the disk storage devices to the system bus, a removable or non-removable interface may be employed.

It is to be appreciated that the computer system 210 includes software that acts as an intermediary between users and the basic computer resources described in a suitable operating environment. Such software includes an operating system. The operating system, which can be stored on the disk storage, acts to control and allocate resources of the computer system. System applications take advantage of the management of resources by the operating system through program modules and program data stored either in the system memory or on the disk storage. It is to be appreciated that various components described herein can be implemented with various operating systems or combinations of operating systems.

A user enters commands or information into the computer system 210 through input device(s) coupled to the I/O interface 251. The input devices include, but are not limited to, a pointing device such as a mouse, trackball, stylus, touch pad, keyboard, microphone, joystick, game pad, satellite dish, scanner, TV tuner card, digital camera, digital video camera, web camera, and the like. These and other input devices connect to the processor through the system bus via interface port(s). The interface port(s) include, for example, a serial port, a parallel port, a game port, and a USB. The output device(s) use some of the same types of ports as input device(s). Thus, for example, a USB port may be used to provide input to the computer system and to output information from the computer system to an output device. An output adapter is provided to illustrate that there are some output devices like monitors, displays, speakers, and printers, among other output devices that require special adapters. The output adapters include, by way of illustration and not limitation, video and sound cards that provide a means of connection between the output device and the system bus. It should be noted that other devices and/or systems of devices, such as remote computer(s), provide both input and output capabilities.

The computer system 210 can operate in a networked environment using logical connections to one or more remote computers, such as cloud computer(s), or local computers. The remote cloud computer(s) can be a personal computer, server, router, network PC, workstation, microprocessor-based appliance, peer device, or other common network node, and the like, and typically includes many or all of the elements described relative to the computer system. For purposes of brevity, only a memory storage device is illustrated with the remote computer(s). The remote computer(s) is logically connected to the computer system through a network interface and then physically connected via a communication connection. The network interface encompasses communication networks such as local area networks (LANs) and wide area networks (WANs). LAN technologies include Fiber Distributed Data Interface (FDDI), Copper Distributed Data Interface (CDDI), Ethernet/IEEE 802.3, Token Ring/IEEE 802.5 and the like. WAN technologies include, but are not limited to, point-to-point links, circuit-switching networks like Integrated Services Digital Networks (ISDN) and variations thereon, packet-switching networks, and Digital Subscriber Lines (DSL).

In various aspects, the computer system 210 of FIG. 43, the imaging module 238 and/or visualization system 208, and/or the processor module 232 of FIGS. 9-10, may comprise an image processor, image processing engine, media processor, or any specialized digital signal processor (DSP) used for the processing of digital images. The image processor may employ parallel computing with single instruction, multiple data (SIMD) or multiple instruction, multiple data (MIMD) technologies to increase speed and efficiency. The digital image processing engine can perform a range of tasks. The image processor may be a system on a chip with multicore processor architecture.

The communication connection(s) refers to the hardware/software employed to connect the network interface to the bus. While the communication connection is shown for illustrative clarity inside the computer system, it can also be external to the computer system 210. The hardware/software necessary for connection to the network interface includes, for illustrative purposes only, internal and external technologies such as modems, including regular telephone-grade modems, cable modems, and DSL modems, ISDN adapters, and Ethernet cards.

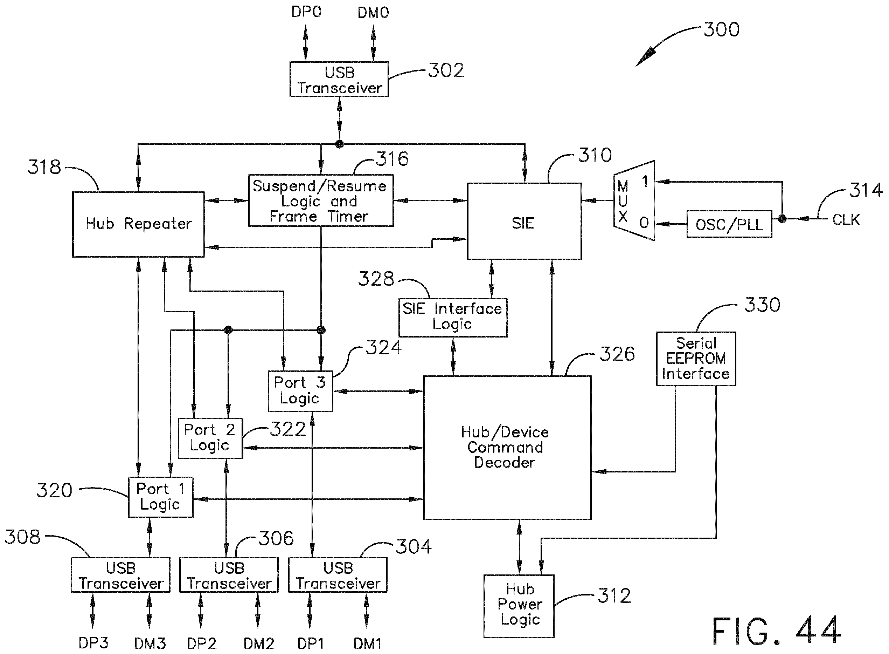

FIG. 44 illustrates a functional block diagram of one aspect of a USB network hub 300 device, according to one aspect of the present disclosure. In the illustrated aspect, the USB network hub device 300 employs a TUSB2036 integrated circuit hub by Texas Instruments. The USB network hub 300 is a CMOS device that provides an upstream USB transceiver port 302 and up to three downstream USB transceiver ports 304, 306, 308 in compliance with the USB 2.0 specification. The upstream USB transceiver port 302 is a differential root data port comprising a differential data minus (DM0) input paired with a differential data plus (DP0) input. The three downstream USB transceiver ports 304, 306, 308 are differential data ports where each port includes differential data plus (DP1-DP3) outputs paired with differential data minus (DM1-DM3) outputs.

The USB network hub 300 device is implemented with a digital state machine instead of a microcontroller, and no firmware programming is required. Fully compliant USB transceivers are integrated into the circuit for the upstream USB transceiver port 302 and all downstream USB transceiver ports 304, 306, 308. The downstream USB transceiver ports 304, 306, 308 support both full-speed and low-speed devices by automatically setting the slew rate according to the speed of the device attached to the ports. The USB network hub 300 device may be configured either in bus-powered or self-powered mode and includes a hub power logic 312 to manage power.

The USB network hub 300 device includes a serial interface engine 310 (SIE). The SIE 310 is the front end of the USB network hub 300 hardware and handles most of the protocol described in chapter 8 of the USB specification. The SIE 310 typically comprehends signaling up to the transaction level. The functions that it handles could include: packet recognition, transaction sequencing, SOP, EOP, RESET, and RESUME signal detection/generation, clock/data separation, non-return-to-zero invert (NRZI) data encoding/decoding and bit-stuffing, CRC generation and checking (token and data), packet ID (PID) generation and checking/decoding, and/or serial-parallel/parallel-serial conversion. The 310 receives a clock input 314 and is coupled to a suspend/resume logic and frame timer 316 circuit and a hub repeater circuit 318 to control communication between the upstream USB transceiver port 302 and the downstream USB transceiver ports 304, 306, 308 through port logic circuits 320, 322, 324. The SIE 310 is coupled to a command decoder 326 via interface logic to control commands from a serial EEPROM via a serial EEPROM interface 330.

In various aspects, the USB network hub 300 can connect 127 functions configured in up to six logical layers (tiers) to a single computer. Further, the USB network hub 300 can connect to all peripherals using a standardized four-wire cable that provides both communication and power distribution. The power configurations are bus-powered and self-powered modes. The USB network hub 300 may be configured to support four modes of power management: a bus-powered hub, with either individual-port power management or ganged-port power management, and the self-powered hub, with either individual-port power management or ganged-port power management. In one aspect, using a USB cable, the USB network hub 300, the upstream USB transceiver port 302 is plugged into a USB host controller, and the downstream USB transceiver ports 304, 306, 308 are exposed for connecting USB compatible devices, and so forth.

### Surgical Instrument Hardware

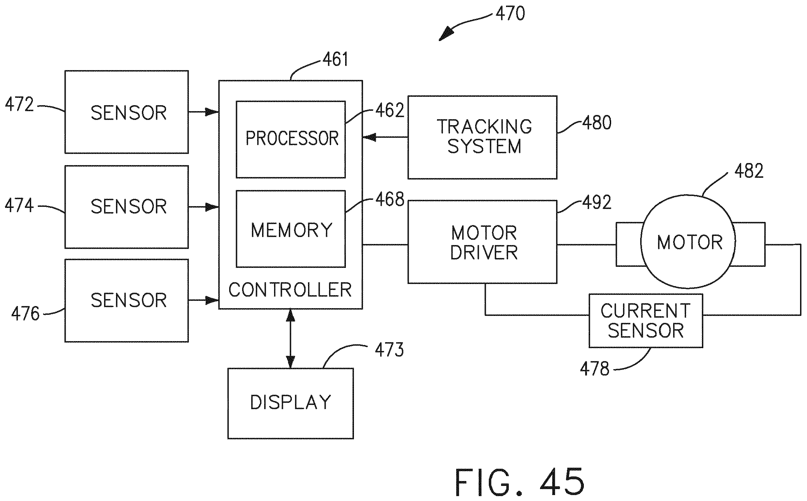

FIG. 45 illustrates a logic diagram of a control system 470 of a surgical instrument or tool in accordance with one or more aspects of the present disclosure. The system 470 comprises a control circuit. The control circuit includes a microcontroller 461 comprising a processor 462 and a memory 468. One or more of sensors 472, 474, 476, for example, provide real-time feedback to the processor 462. A motor 482, driven by a motor driver 492, operably couples a longitudinally movable displacement member to drive the I-beam knife element. A tracking system 480 is configured to determine the position of the longitudinally movable displacement member. The position information is provided to the processor 462, which can be programmed or configured to determine the position of the longitudinally movable drive member as well as the position of a firing member, firing bar, and I-beam knife element. Additional motors may be provided at the tool driver interface to control I-beam firing, closure tube travel, shaft rotation, and articulation. A display 473 displays a variety of operating conditions of the instruments and may include touch screen functionality for data input. Information displayed on the display 473 may be overlaid with images acquired via endoscopic imaging modules.

In one aspect, the microcontroller 461 may be any single-core or multicore processor such as those known under the trade name ARM Cortex by Texas Instruments. In one aspect, the main microcontroller 461 may be an LM4F230H5QR ARM Cortex-M4F Processor Core, available from Texas Instruments, for example, comprising an on-chip memory of 256 KB single-cycle flash memory, or other non-volatile memory, up to 40 MHz, a prefetch buffer to improve performance above 40 MHz, a 32 KB single-cycle SRAM, and internal ROM loaded with StellarisWare® software, a 2 KB EEPROM, one or more PWM modules, one or more QEI analogs, and/or one or more 12-bit ADCs with 12 analog input channels, details of which are available for the product datasheet.

In one aspect, the microcontroller 461 may comprise a safety controller comprising two controller-based families such as TMS570 and RM4x, known under the trade name Hercules ARM Cortex R4, also by Texas Instruments. The safety controller may be configured specifically for IEC 61508 and ISO 26262 safety critical applications, among others, to provide advanced integrated safety features while delivering scalable performance, connectivity, and memory options.

The microcontroller 461 may be programmed to perform various functions such as precise control over the speed and position of the knife and articulation systems. In one aspect, the microcontroller 461 includes a processor 462 and a memory 468. The electric motor 482 may be a brushed direct current (DC) motor with a gearbox and mechanical links to an articulation or knife system. In one aspect, a motor driver 492 may be an A3941 available from Allegro Microsystems, Inc. Other motor drivers may be readily substituted for use in the tracking system 480 comprising an absolute positioning system. A detailed description of an absolute positioning system is described in U.S. Patent Application Publication No. 2017/0296213, titled SYSTEMS AND METHODS FOR CONTROLLING A SURGICAL STAPLING AND CUTTING INSTRUMENT, which published on October 19, 2017, which is herein incorporated by reference in its entirety.

The microcontroller 461 may be programmed to provide precise control over the speed and position of displacement members and articulation systems. The microcontroller 461 may be configured to compute a response in the software of the microcontroller 461. The computed response is compared to a measured response of the actual system to obtain an "observed" response, which is used for actual feedback decisions. The observed response is a favorable, tuned value that balances the smooth, continuous nature of the simulated response with the measured response, which can detect outside influences on the system.

In one aspect, the motor 482 may be controlled by the motor driver 492 and can be employed by the firing system of the surgical instrument or tool. In various forms, the motor 482 may be a brushed DC driving motor having a maximum rotational speed of approximately 25,000 RPM. In other arrangements, the motor 482 may include a brushless motor, a cordless motor, a synchronous motor, a stepper motor, or any other suitable electric motor. The motor driver 492 may comprise an H-bridge driver comprising field-effect transistors (FETs), for example. The motor 482 can be powered by a power assembly releasably mounted to the handle assembly or tool housing for supplying control power to the surgical instrument or tool. The power assembly may comprise a battery which may include a number of battery cells connected in series that can be used as the power source to power the surgical instrument or tool. In certain circumstances, the battery cells of the power assembly may be replaceable and/or rechargeable. In at least one example, the battery cells can be lithium-ion batteries which can be couplable to and separable from the power assembly.

The motor driver 492 may be an A3941 available from Allegro Microsystems, Inc. The A3941 492 is a full-bridge controller for use with external N-channel power metal-oxide semiconductor field-effect transistors (MOSFETs) specifically designed for inductive loads, such as brush DC motors. The driver 492 comprises a unique charge pump regulator that provides full (>10 V) gate drive for battery voltages down to 7 V and allows the A3941 to operate with a reduced gate drive, down to 5.5 V. A bootstrap capacitor may be employed to provide the above battery supply voltage required for N-channel MOSFETs. An internal charge pump for the high-side drive allows DC (100% duty cycle) operation. The full bridge can be driven in fast or slow decay modes using diode or synchronous rectification. In the slow decay mode, current recirculation can be through the high-side or the lowside FETs. The power FETs are protected from shoot-through by resistor-adjustable dead time. Integrated diagnostics provide indications of undervoltage, overtemperature, and power bridge faults and can be configured to protect the power MOSFETs under most short circuit conditions. Other motor drivers may be readily substituted for use in the tracking system 480 comprising an absolute positioning system.

The tracking system 480 comprises a controlled motor drive circuit arrangement comprising a position sensor 472 according to one aspect of this disclosure. The position sensor 472 for an absolute positioning system provides a unique position signal corresponding to the location of a displacement member. In one aspect, the displacement member represents a longitudinally movable drive member comprising a rack of drive teeth for meshing engagement with a corresponding drive gear of a gear reducer assembly. In other aspects, the displacement member represents the firing member, which could be adapted and configured to include a rack of drive teeth. In yet another aspect, the displacement member represents a firing bar or the I-beam, each of which can be adapted and configured to include a rack of drive teeth. Accordingly, as used herein, the term displacement member is used generically to refer to any movable member of the surgical instrument or tool such as the drive member, the firing member, the firing bar, the I-beam, or any element that can be displaced. In one aspect, the longitudinally movable drive member is coupled to the firing member, the firing bar, and the I-beam. Accordingly, the absolute positioning system can, in effect, track the linear displacement of the I-beam by tracking the linear displacement of the longitudinally movable drive member. In various other aspects, the displacement member may be coupled to any position sensor 472 suitable for measuring linear displacement. Thus, the longitudinally movable drive member, the firing member, the firing bar, or the I-beam, or combinations thereof, may be coupled to any suitable linear displacement sensor. Linear displacement sensors may include contact or non-contact displacement sensors. Linear displacement sensors may comprise linear variable differential transformers (LVDT), differential variable reluctance transducers (DVRT), a slide potentiometer, a magnetic sensing system comprising a movable magnet and a series of linearly arranged Hall effect sensors, a magnetic sensing system comprising a fixed magnet and a series of movable, linearly arranged Hall effect sensors, an optical sensing system comprising a movable light source and a series of linearly arranged photo diodes or photo detectors, an optical sensing system comprising a fixed light source and a series of movable linearly, arranged photo diodes or photo detectors, or any combination thereof.

The electric motor 482 can include a rotatable shaft that operably interfaces with a gear assembly that is mounted in meshing engagement with a set, or rack, of drive teeth on the displacement member. A sensor element may be operably coupled to a gear assembly such that a single revolution of the position sensor 472 element corresponds to some linear longitudinal translation of the displacement member. An arrangement of gearing and sensors can be connected to the linear actuator, via a rack and pinion arrangement, or a rotary actuator, via a spur gear or other connection. A power source supplies power to the absolute positioning system and an output indicator may display the output of the absolute positioning system. The displacement member represents the longitudinally movable drive member comprising a rack of drive teeth formed thereon for meshing engagement with a corresponding drive gear of the gear reducer assembly. The displacement member represents the longitudinally movable firing member, firing bar, I-beam, or combinations thereof.

A single revolution of the sensor element associated with the position sensor 472 is equivalent to a longitudinal linear displacement d1 of the of the displacement member, where d1 is the longitudinal linear distance that the displacement member moves from point "a" to point "b" after a single revolution of the sensor element coupled to the displacement member. The sensor arrangement may be connected via a gear reduction that results in the position sensor 472 completing one or more revolutions for the full stroke of the displacement member. The position sensor 472 may complete multiple revolutions for the full stroke of the displacement member.

A series of switches, where n is an integer greater than one, may be employed alone or in combination with a gear reduction to provide a unique position signal for more than one revolution of the position sensor 472. The state of the switches are fed back to the microcontroller 461 that applies logic to determine a unique position signal corresponding to the longitudinal linear displacement d1 + d2 + ... dn of the displacement member. The output of the position sensor 472 is provided to the microcontroller 461. The position sensor 472 of the sensor arrangement may comprise a magnetic sensor, an analog rotary sensor like a potentiometer, or an array of analog Hall-effect elements, which output a unique combination of position signals or values.

The position sensor 472 may comprise any number of magnetic sensing elements, such as, for example, magnetic sensors classified according to whether they measure the total magnetic field or the vector components of the magnetic field. The techniques used to produce both types of magnetic sensors encompass many aspects of physics and electronics. The technologies used for magnetic field sensing include search coil, fluxgate, optically pumped, nuclear precession, SQUID, Hall-effect, anisotropic magnetoresistance, giant magnetoresistance, magnetic tunnel junctions, giant magnetoimpedance, magnetostrictive/piezoelectric composites, magnetodiode, magnetotransistor, fiber-optic, magneto-optic, and microelectromechanical systems-based magnetic sensors, among others.

In one aspect, the position sensor 472 for the tracking system 480 comprising an absolute positioning system comprises a magnetic rotary absolute positioning system. The position sensor 472 may be implemented as an AS5055EQFT single-chip magnetic rotary position sensor available from Austria Microsystems, AG. The position sensor 472 is interfaced with the microcontroller 461 to provide an absolute positioning system. The position sensor 472 is a low-voltage and low-power component and includes four Hall-effect elements in an area of the position sensor 472 that is located above a magnet. A high-resolution ADC and a smart power management controller are also provided on the chip. A coordinate rotation digital computer (CORDIC) processor, also known as the digit-by-digit method and Volder's algorithm, is provided to implement a simple and efficient algorithm to calculate hyperbolic and trigonometric functions that require only addition, subtraction, bitshift, and table lookup operations. The angle position, alarm bits, and magnetic field information are transmitted over a standard serial communication interface, such as a serial peripheral interface (SPI) interface, to the microcontroller 461. The position sensor 472 provides 12 or 14 bits of resolution. The position sensor 472 maybe an AS5055 chip provided in a small QFN 16-pin 4x4x0.85mm package.

The tracking system 480 comprising an absolute positioning system may comprise and/or be programmed to implement a feedback controller, such as a PID, state feedback, and adaptive controller. A power source converts the signal from the feedback controller into a physical input to the system: in this case the voltage. Other examples include a PWM of the voltage, current, and force. Other sensor(s) may be provided to measure physical parameters of the physical system in addition to the position measured by the position sensor 472. In some aspects, the other sensor(s) can include sensor arrangements such as those described in U.S. Patent No. 9,345,481, titled STAPLE CARTRIDGE TISSUE THICKNESS SENSOR SYSTEM, which issued on May 24, 2016, which is herein incorporated by reference in its entirety; U.S. Patent Application Publication No. 2014/0263552, titled STAPLE CARTRIDGE TISSUE THICKNESS SENSOR SYSTEM, which published on September 18, 2014, which is herein incorporated by reference in its entirety; and U.S. Patent Application Serial No. 15/628,175, titled TECHNIQUES FOR ADAPTIVE CONTROL OF MOTOR VELOCITY OF A SURGICAL STAPLING AND CUTTING INSTRUMENT, filed June 20, 2017, which is herein incorporated by reference in its entirety. In a digital signal processing system, an absolute positioning system is coupled to a digital data acquisition system where the output of the absolute positioning system will have a finite resolution and sampling frequency. The absolute positioning system may comprise a compare-and-combine circuit to combine a computed response with a measured response using algorithms, such as a weighted average and a theoretical control loop, that drive the computed response towards the measured response. The computed response of the physical system takes into account properties like mass, inertial, viscous friction, inductance resistance, etc., to predict what the states and outputs of the physical system will be by knowing the input.

The absolute positioning system provides an absolute position of the displacement member upon power-up of the instrument, without retracting or advancing the displacement member to a reset (zero or home) position as may be required with conventional rotary encoders that merely count the number of steps forwards or backwards that the motor 482 has taken to infer the position of a device actuator, drive bar, knife, or the like.

A sensor 474, such as, for example, a strain gauge or a micro-strain gauge, is configured to measure one or more parameters of the end effector, such as, for example, the amplitude of the strain exerted on the anvil during a clamping operation, which can be indicative of the closure forces applied to the anvil. The measured strain is converted to a digital signal and provided to the processor 462. Alternatively, or in addition to the sensor 474, a sensor 476, such as, for example, a load sensor, can measure the closure force applied by the closure drive system to the anvil. The sensor 476, such as, for example, a load sensor, can measure the firing force applied to an I-beam in a firing stroke of the surgical instrument or tool. The I-beam is configured to engage a wedge sled, which is configured to upwardly cam staple drivers to force out staples into deforming contact with an anvil. The I-beam also includes a sharpened cutting edge that can be used to sever tissue as the I-beam is advanced distally by the firing bar. Alternatively, a current sensor 478 can be employed to measure the current drawn by the motor 482. The force required to advance the firing member can correspond to the current drawn by the motor 482, for example. The measured force is converted to a digital signal and provided to the processor 462.

In one form, the strain gauge sensor 474 can be used to measure the force applied to the tissue by the end effector. A strain gauge can be coupled to the end effector to measure the force on the tissue being treated by the end effector. A system for measuring forces applied to the tissue grasped by the end effector comprises a strain gauge sensor 474, such as, for example, a micro-strain gauge, that is configured to measure one or more parameters of the end effector, for example. In one aspect, the strain gauge sensor 474 can measure the amplitude or magnitude of the strain exerted on a jaw member of an end effector during a clamping operation, which can be indicative of the tissue compression. The measured strain is converted to a digital signal and provided to a processor 462 of the microcontroller 461. A load sensor 476 can measure the force used to operate the knife element, for example, to cut the tissue captured between the anvil and the staple cartridge. A magnetic field sensor can be employed to measure the thickness of the captured tissue. The measurement of the magnetic field sensor also may be converted to a digital signal and provided to the processor 462.

The measurements of the tissue compression, the tissue thickness, and/or the force required to close the end effector on the tissue, as respectively measured by the sensors 474, 476, can be used by the microcontroller 461 to characterize the selected position of the firing member and/or the corresponding value of the speed of the firing member. In one instance, a memory 468 may store a technique, an equation, and/or a lookup table which can be employed by the microcontroller 461 in the assessment.

The control system 470 of the surgical instrument or tool also may comprise wired or wireless communication circuits to communicate with the modular communication hub as shown in FIGS. 8-11.

FIG. 46 illustrates a control circuit 500 configured to control aspects of the surgical instrument or tool according to one aspect of this disclosure. The control circuit 500 can be configured to implement various processes described herein. The control circuit 500 may comprise a microcontroller comprising one or more processors 502 (e.g., microprocessor, microcontroller) coupled to at least one memory circuit 504. The memory circuit 504 stores machine-executable instructions that, when executed by the processor 502, cause the processor 502 to execute machine instructions to implement various processes described herein. The processor 502 may be any one of a number of single-core or multicore processors known in the art. The memory circuit 504 may comprise volatile and non-volatile storage media. The processor 502 may include an instruction processing unit 506 and an arithmetic unit 508. The instruction processing unit may be configured to receive instructions from the memory circuit 504 of this disclosure.

FIG. 47 illustrates a combinational logic circuit 510 configured to control aspects of the surgical instrument or tool according to one aspect of this disclosure. The combinational logic circuit 510 can be configured to implement various processes described herein. The combinational logic circuit 510 may comprise a finite state machine comprising a combinational logic 512 configured to receive data associated with the surgical instrument or tool at an input 514, process the data by the combinational logic 512, and provide an output 516.

FIG. 48 illustrates a sequential logic circuit 520 configured to control aspects of the surgical instrument or tool according to one aspect of this disclosure. The sequential logic circuit 520 or the combinational logic 522 can be configured to implement various processes described herein. The sequential logic circuit 520 may comprise a finite state machine. The sequential logic circuit 520 may comprise a combinational logic 522, at least one memory circuit 524, and a clock 529, for example. The at least one memory circuit 524 can store a current state of the finite state machine. In certain instances, the sequential logic circuit 520 may be synchronous or asynchronous. The combinational logic 522 is configured to receive data associated with the surgical instrument or tool from an input 526, process the data by the combinational logic 522, and provide an output 528. In other aspects, the circuit may comprise a combination of a processor (e.g., processor 502, FIG. 46) and a finite state machine to implement various processes herein. In other aspects, the finite state machine may comprise a combination of a combinational logic circuit (e.g., combinational logic circuit 510, FIG. 47) and the sequential logic circuit 520.

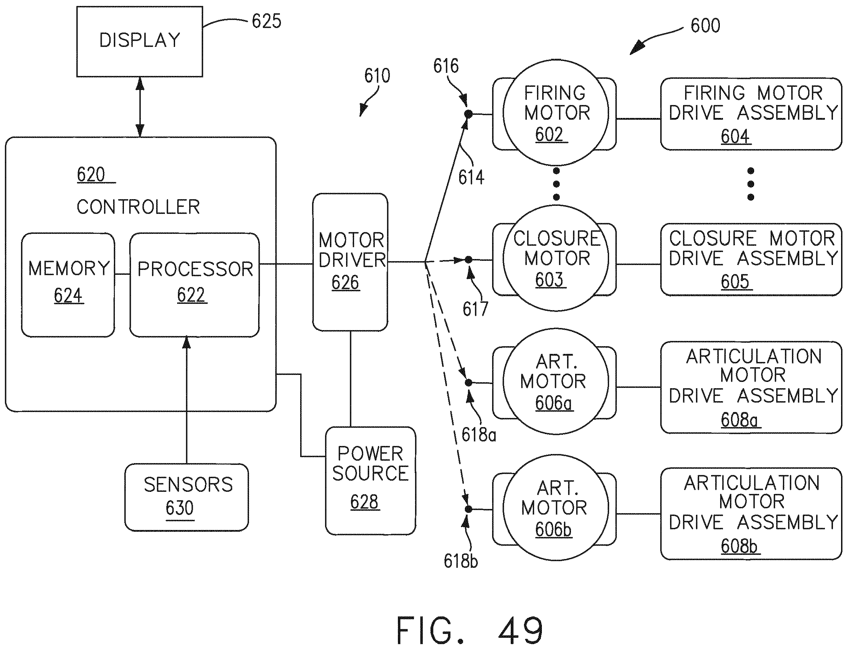

FIG. 49 illustrates a surgical instrument or tool comprising a plurality of motors which can be activated to perform various functions. In certain instances, a first motor can be activated to perform a first function, a second motor can be activated to perform a second function, a third motor can be activated to perform a third function, a fourth motor can be activated to perform a fourth function, and so on. In certain instances, the plurality of motors of robotic surgical instrument 600 can be individually activated to cause firing, closure, and/or articulation motions in the end effector. The firing, closure, and/or articulation motions can be transmitted to the end effector through a shaft assembly, for example.

In certain instances, the surgical instrument system or tool may include a firing motor 602. The firing motor 602 may be operably coupled to a firing motor drive assembly 604 which can be configured to transmit firing motions, generated by the motor 602 to the end effector, in particular to displace the I-beam element. In certain instances, the firing motions generated by the motor 602 may cause the staples to be deployed from the staple cartridge into tissue captured by the end effector and/or the cutting edge of the I-beam element to be advanced to cut the captured tissue, for example. The I-beam element may be retracted by reversing the direction of the motor 602.

In certain instances, the surgical instrument or tool may include a closure motor 603. The closure motor 603 may be operably coupled to a closure motor drive assembly 605 which can be configured to transmit closure motions, generated by the motor 603 to the end effector, in particular to displace a closure tube to close the anvil and compress tissue between the anvil and the staple cartridge. The closure motions may cause the end effector to transition from an open configuration to an approximated configuration to capture tissue, for example. The end effector may be transitioned to an open position by reversing the direction of the motor 603.

In certain instances, the surgical instrument or tool may include one or more articulation motors 606a, 606b, for example. The motors 606a, 606b may be operably coupled to respective articulation motor drive assemblies 608a, 608b, which can be configured to transmit articulation motions generated by the motors 606a, 606b to the end effector. In certain instances, the articulation motions may cause the end effector to articulate relative to the shaft, for example.

As described above, the surgical instrument or tool may include a plurality of motors which may be configured to perform various independent functions. In certain instances, the plurality of motors of the surgical instrument or tool can be individually or separately activated to perform one or more functions while the other motors remain inactive. For example, the articulation motors 606a, 606b can be activated to cause the end effector to be articulated while the firing motor 602 remains inactive. Alternatively, the firing motor 602 can be activated to fire the plurality of staples, and/or to advance the cutting edge, while the articulation motor 606 remains inactive. Furthermore the closure motor 603 may be activated simultaneously with the firing motor 602 to cause the closure tube and the I-beam element to advance distally as described in more detail hereinbelow.

In certain instances, the surgical instrument or tool may include a common control module 610 which can be employed with a plurality of motors of the surgical instrument or tool. In certain instances, the common control module 610 may accommodate one of the plurality of motors at a time. For example, the common control module 610 can be couplable to and separable from the plurality of motors of the robotic surgical instrument individually. In certain instances, a plurality of the motors of the surgical instrument or tool may share one or more common control modules such as the common control module 610. In certain instances, a plurality of motors of the surgical instrument or tool can be individually and selectively engaged with the common control module 610. In certain instances, the common control module 610 can be selectively switched from interfacing with one of a plurality of motors of the surgical instrument or tool to interfacing with another one of the plurality of motors of the surgical instrument or tool.

In at least one example, the common control module 610 can be selectively switched between operable engagement with the articulation motors 606a, 606b and operable engagement with either the firing motor 602 or the closure motor 603. In at least one example, as illustrated in FIG. 49, a switch 614 can be moved or transitioned between a plurality of positions and/or states. In a first position 616, the switch 614 may electrically couple the common control module 610 to the firing motor 602; in a second position 617, the switch 614 may electrically couple the common control module 610 to the closure motor 603; in a third position 618a, the switch 614 may electrically couple the common control module 610 to the first articulation motor 606a; and in a fourth position 618b, the switch 614 may electrically couple the common control module 610 to the second articulation motor 606b, for example. In certain instances, separate common control modules 610 can be electrically coupled to the firing motor 602, the closure motor 603, and the articulations motor 606a, 606b at the same time. In certain instances, the switch 614 may be a mechanical switch, an electromechanical switch, a solid-state switch, or any suitable switching mechanism.

Each of the motors 602, 603, 606a, 606b may comprise a torque sensor to measure the output torque on the shaft of the motor. The force on an end effector may be sensed in any conventional manner, such as by force sensors on the outer sides of the jaws or by a torque sensor for the motor actuating the jaws.

In various instances, as illustrated in FIG. 49, the common control module 610 may comprise a motor driver 626 which may comprise one or more H-Bridge FETs. The motor driver 626 may modulate the power transmitted from a power source 628 to a motor coupled to the common control module 610 based on input from a microcontroller 620 (the "controller"), for example. In certain instances, the microcontroller 620 can be employed to determine the current drawn by the motor, for example, while the motor is coupled to the common control module 610, as described above.

In certain instances, the microcontroller 620 may include a microprocessor 622 (the "processor") and one or more non-transitory computer-readable mediums or memory units 624 (the "memory"). In certain instances, the memory 624 may store various program instructions, which when executed may cause the processor 622 to perform a plurality of functions and/or calculations described herein. In certain instances, one or more of the memory units 624 may be coupled to the processor 622, for example.

In certain instances, the power source 628 can be employed to supply power to the microcontroller 620, for example. In certain instances, the power source 628 may comprise a battery (or "battery pack" or "power pack"), such as a lithium-ion battery, for example. In certain instances, the battery pack may be configured to be releasably mounted to a handle for supplying power to the surgical instrument 600. A number of battery cells connected in series may be used as the power source 628. In certain instances, the power source 628 may be replaceable and/or rechargeable, for example.

In various instances, the processor 622 may control the motor driver 626 to control the position, direction of rotation, and/or velocity of a motor that is coupled to the common control module 610. In certain instances, the processor 622 can signal the motor driver 626 to stop and/or disable a motor that is coupled to the common control module 610. It should be understood that the term "processor" as used herein includes any suitable microprocessor, microcontroller, or other basic computing device that incorporates the functions of a computer's central processing unit (CPU) on an integrated circuit or, at most, a few integrated circuits. The processor is a multipurpose, programmable device that accepts digital data as input, processes it according to instructions stored in its memory, and provides results as output. It is an example of sequential digital logic, as it has internal memory. Processors operate on numbers and symbols represented in the binary numeral system.

In one instance, the processor 622 may be any single-core or multicore processor such as those known under the trade name ARM Cortex by Texas Instruments. In certain instances, the microcontroller 620 may be an LM 4F230H5QR, available from Texas Instruments, for example. In at least one example, the Texas Instruments LM4F230H5QR is an ARM Cortex-M4F Processor Core comprising an on-chip memory of 256 KB single-cycle flash memory, or other non-volatile memory, up to 40 MHz, a prefetch buffer to improve performance above 40 MHz, a 32 KB single-cycle SRAM, an internal ROM loaded with StellarisWare® software, a 2 KB EEPROM, one or more PWM modules, one or more QEI analogs, one or more 12-bit ADCs with 12 analog input channels, among other features that are readily available for the product datasheet. Other microcontrollers may be readily substituted for use with the module 4410. Accordingly, the present disclosure should not be limited in this context.

In certain instances, the memory 624 may include program instructions for controlling each of the motors of the surgical instrument 600 that are couplable to the common control module 610. For example, the memory 624 may include program instructions for controlling the firing motor 602, the closure motor 603, and the articulation motors 606a, 606b. Such program instructions may cause the processor 622 to control the firing, closure, and articulation functions in accordance with inputs from algorithms or control programs of the surgical instrument or tool.

In certain instances, one or more mechanisms and/or sensors such as, for example, sensors 630 can be employed to alert the processor 622 to the program instructions that should be used in a particular setting. For example, the sensors 630 may alert the processor 622 to use the program instructions associated with firing, closing, and articulating the end effector. In certain instances, the sensors 630 may comprise position sensors which can be employed to sense the position of the switch 614, for example. Accordingly, the processor 622 may use the program instructions associated with firing the I-beam of the end effector upon detecting, through the sensors 630 for example, that the switch 614 is in the first position 616; the processor 622 may use the program instructions associated with closing the anvil upon detecting, through the sensors 630 for example, that the switch 614 is in the second position 617; and the processor 622 may use the program instructions associated with articulating the end effector upon detecting, through the sensors 630 for example, that the switch 614 is in the third or fourth position 618a, 618b.

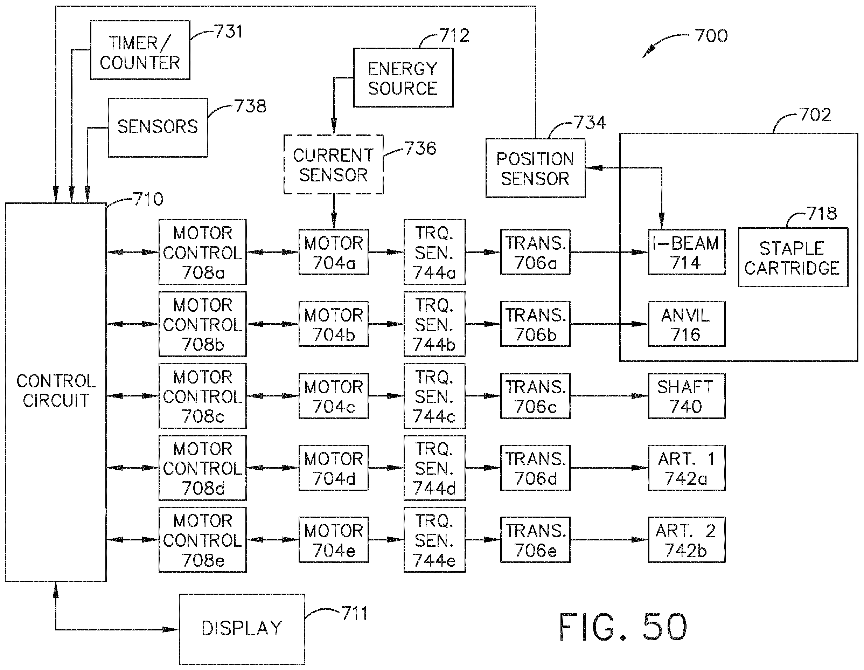

FIG. 50 is a schematic diagram of a robotic surgical instrument 700 configured to operate a surgical tool described herein according to one aspect of this disclosure. The robotic surgical instrument 700 may be programmed or configured to control distal/proximal translation of a displacement member, distal/proximal displacement of a closure tube, shaft rotation, and articulation, either with single or multiple articulation drive links. In one aspect, the surgical instrument 700 may be programmed or configured to individually control a firing member, a closure member, a shaft member, and/or one or more articulation members. The surgical instrument 700 comprises a control circuit 710 configured to control motor-driven firing members, closure members, shaft members, and/or one or more articulation members.

In one aspect, the robotic surgical instrument 700 comprises a control circuit 710 configured to control an anvil 716 and an I-beam 714 (including a sharp cutting edge) portion of an end effector 702, a removable staple cartridge 718, a shaft 740, and one or more articulation members 742a, 742b via a plurality of motors 704a-704e. A position sensor 734 maybe configured to provide position feedback of the I-beam 714 to the control circuit 710. Other sensors 738 may be configured to provide feedback to the control circuit 710. A timer/counter 731 provides timing and counting information to the control circuit 710. An energy source 712 may be provided to operate the motors 704a-704e, and a current sensor 736 provides motor current feedback to the control circuit 710. The motors 704a-704e can be operated individually by the control circuit 710 in an open-loop or closed-loop feedback control.

In one aspect, the control circuit 710 may comprise one or more microcontrollers, microprocessors, or other suitable processors for executing instructions that cause the processor or processors to perform one or more tasks. In one aspect, a timer/counter 731 provides an output signal, such as the elapsed time or a digital count, to the control circuit 710 to correlate the position of the I-beam 714 as determined by the position sensor 734 with the output of the timer/counter 731 such that the control circuit 710 can determine the position of the I-beam 714 at a specific time (t) relative to a starting position or the time (t) when the I-beam 714 is at a specific position relative to a starting position. The timer/counter 731 may be configured to measure elapsed time, count external events, or time external events.

In one aspect, the control circuit 710 may be programmed to control functions of the end effector 702 based on one or more tissue conditions. The control circuit 710 maybe programmed to sense tissue conditions, such as thickness, either directly or indirectly, as described herein. The control circuit 710 may be programmed to select a firing control program or closure control program based on tissue conditions. A firing control program may describe the distal motion of the displacement member. Different firing control programs may be selected to better treat different tissue conditions. For example, when thicker tissue is present, the control circuit 710 maybe programmed to translate the displacement member at a lower velocity and/or with lower power. When thinner tissue is present, the control circuit 710 maybe programmed to translate the displacement member at a higher velocity and/or with higher power. A closure control program may control the closure force applied to the tissue by the anvil 716. Other control programs control the rotation of the shaft 740 and the articulation members 742a, 742b.

In one aspect, the control circuit 710 may generate motor set point signals. The motor set point signals may be provided to various motor controllers 708a-708e. The motor controllers 708a-708e may comprise one or more circuits configured to provide motor drive signals to the motors 704a-704e to drive the motors 704a-704e as described herein. In some examples, the motors 704a-704e may be brushed DC electric motors. For example, the velocity of the motors 704a-704e maybe proportional to the respective motor drive signals. In some examples, the motors 704a-704e maybe brushless DC electric motors, and the respective motor drive signals may comprise a PWM signal provided to one or more stator windings of the motors 704a-704e. Also, in some examples, the motor controllers 708a-708e may be omitted and the control circuit 710 may generate the motor drive signals directly.

In one aspect, the control circuit 710 may initially operate each of the motors 704a-704e in an open-loop configuration for a first open-loop portion of a stroke of the displacement member. Based on the response of the robotic surgical instrument 700 during the open-loop portion of the stroke, the control circuit 710 may select a firing control program in a closed-loop configuration. The response of the instrument may include a translation distance of the displacement member during the open-loop portion, a time elapsed during the open-loop portion, the energy provided to one of the motors 704a-704e during the open-loop portion, a sum of pulse widths of a motor drive signal, etc. After the open-loop portion, the control circuit 710 may implement the selected firing control program for a second portion of the displacement member stroke. For example, during a closed-loop portion of the stroke, the control circuit 710 may modulate one of the motors 704a-704e based on translation data describing a position of the displacement member in a closed-loop manner to translate the displacement member at a constant velocity.

In one aspect, the motors 704a-704e may receive power from an energy source 712. The energy source 712 may be a DC power supply driven by a main alternating current power source, a battery, a super capacitor, or any other suitable energy source. The motors 704a-704e may be mechanically coupled to individual movable mechanical elements such as the I-beam 714, anvil 716, shaft 740, articulation 742a, and articulation 742b via respective transmissions 706a-706e. The transmissions 706a-706e may include one or more gears or other linkage components to couple the motors 704a-704e to movable mechanical elements. A position sensor 734 may sense a position of the I-beam 714. The position sensor 734 may be or include any type of sensor that is capable of generating position data that indicate a position of the I-beam 714. In some examples, the position sensor 734 may include an encoder configured to provide a series of pulses to the control circuit 710 as the I-beam 714 translates distally and proximally. The control circuit 710 may track the pulses to determine the position of the I-beam 714. Other suitable position sensors maybe used, including, for example, a proximity sensor. Other types of position sensors may provide other signals indicating motion of the I-beam 714. Also, in some examples, the position sensor 734 may be omitted. Where any of the motors 704a-704e is a stepper motor, the control circuit 710 may track the position of the I-beam 714 by aggregating the number and direction of steps that the motor 704 has been instructed to execute. The position sensor 734 may be located in the end effector 702 or at any other portion of the instrument. The outputs of each of the motors 704a-704e include a torque sensor 744a-744e to sense force and have an encoder to sense rotation of the drive shaft.

In one aspect, the control circuit 710 is configured to drive a firing member such as the I-beam 714 portion of the end effector 702. The control circuit 710 provides a motor set point to a motor control 708a, which provides a drive signal to the motor 704a. The output shaft of the motor 704a is coupled to a torque sensor 744a. The torque sensor 744a is coupled to a transmission 706a which is coupled to the I-beam 714. The transmission 706a comprises movable mechanical elements such as rotating elements and a firing member to control the movement of the I-beam 714 distally and proximally along a longitudinal axis of the end effector 702. In one aspect, the motor 704a may be coupled to the knife gear assembly, which includes a knife gear reduction set that includes a first knife drive gear and a second knife drive gear. A torque sensor 744a provides a firing force feedback signal to the control circuit 710. The firing force signal represents the force required to fire or displace the I-beam 714. A position sensor 734 may be configured to provide the position of the I-beam 714 along the firing stroke or the position of the firing member as a feedback signal to the control circuit 710. The end effector 702 may include additional sensors 738 configured to provide feedback signals to the control circuit 710. When ready to use, the control circuit 710 may provide a firing signal to the motor control 708a. In response to the firing signal, the motor 704a may drive the firing member distally along the longitudinal axis of the end effector 702 from a proximal stroke start position to a stroke end position distal to the stroke start position. As the firing member translates distally, an I-beam 714, with a cutting element positioned at a distal end, advances distally to cut tissue located between the staple cartridge 718 and the anvil 716.

In one aspect, the control circuit 710 is configured to drive a closure member such as the anvil 716 portion of the end effector 702. The control circuit 710 provides a motor set point to a motor control 708b, which provides a drive signal to the motor 704b. The output shaft of the motor 704b is coupled to a torque sensor 744b. The torque sensor 744b is coupled to a transmission 706b which is coupled to the anvil 716. The transmission 706b comprises movable mechanical elements such as rotating elements and a closure member to control the movement of the anvil 716 from the open and closed positions. In one aspect, the motor 704b is coupled to a closure gear assembly, which includes a closure reduction gear set that is supported in meshing engagement with the closure spur gear. The torque sensor 744b provides a closure force feedback signal to the control circuit 710. The closure force feedback signal represents the closure force applied to the anvil 716. The position sensor 734 maybe configured to provide the position of the closure member as a feedback signal to the control circuit 710. Additional sensors 738 in the end effector 702 may provide the closure force feedback signal to the control circuit 710. The pivotable anvil 716 is positioned opposite the staple cartridge 718. When ready to use, the control circuit 710 may provide a closure signal to the motor control 708b. In response to the closure signal, the motor 704b advances a closure member to grasp tissue between the anvil 716 and the staple cartridge 718.

In one aspect, the control circuit 710 is configured to rotate a shaft member such as the shaft 740 to rotate the end effector 702. The control circuit 710 provides a motor set point to a motor control 708c, which provides a drive signal to the motor 704c. The output shaft of the motor 704c is coupled to a torque sensor 744c. The torque sensor 744c is coupled to a transmission 706c which is coupled to the shaft 740. The transmission 706c comprises movable mechanical elements such as rotating elements to control the rotation of the shaft 740 clockwise or counterclockwise up to and over 360°. In one aspect, the motor 704c is coupled to the rotational transmission assembly, which includes a tube gear segment that is formed on (or attached to) the proximal end of the proximal closure tube for operable engagement by a rotational gear assembly that is operably supported on the tool mounting plate. The torque sensor 744c provides a rotation force feedback signal to the control circuit 710. The rotation force feedback signal represents the rotation force applied to the shaft 740. The position sensor 734 may be configured to provide the position of the closure member as a feedback signal to the control circuit 710. Additional sensors 738 such as a shaft encoder may provide the rotational position of the shaft 740 to the control circuit 710.

In one aspect, the control circuit 710 is configured to articulate the end effector 702. The control circuit 710 provides a motor set point to a motor control 708d, which provides a drive signal to the motor 704d. The output shaft of the motor 704d is coupled to a torque sensor 744d. The torque sensor 744d is coupled to a transmission 706d which is coupled to an articulation member 742a. The transmission 706d comprises movable mechanical elements such as articulation elements to control the articulation of the end effector 702±65°. In one aspect, the motor 704d is coupled to an articulation nut, which is rotatably journaled on the proximal end portion of the distal spine portion and is rotatably driven thereon by an articulation gear assembly. The torque sensor 744d provides an articulation force feedback signal to the control circuit 710. The articulation force feedback signal represents the articulation force applied to the end effector 702. Sensors 738, such as an articulation encoder, may provide the articulation position of the end effector 702 to the control circuit 710.

In another aspect, the articulation function of the robotic surgical system 700 may comprise two articulation members, or links, 742a, 742b. These articulation members 742a, 742b are driven by separate disks on the robot interface (the rack), which are driven by the two motors 708d, 708e. When the separate firing motor 704a is provided, each of articulation links 742a, 742b can be antagonistically driven with respect to the other link in order to provide a resistive holding motion and a load to the head when it is not moving and to provide an articulation motion as the head is articulated. The articulation members 742a, 742b attach to the head at a fixed radius as the head is rotated. Accordingly, the mechanical advantage of the push-and-pull link changes as the head is rotated. This change in the mechanical advantage may be more pronounced with other articulation link drive systems.

In one aspect, the one or more motors 704a-704e may comprise a brushed DC motor with a gearbox and mechanical links to a firing member, closure member, or articulation member. Another example includes electric motors 704a-704e that operate the movable mechanical elements such as the displacement member, articulation links, closure tube, and shaft. An outside influence is an unmeasured, unpredictable influence of things like tissue, surrounding bodies, and friction on the physical system. Such outside influence can be referred to as drag, which acts in opposition to one of electric motors 704a-704e. The outside influence, such as drag, may cause the operation of the physical system to deviate from a desired operation of the physical system.

In one aspect, the position sensor 734 may be implemented as an absolute positioning system. In one aspect, the position sensor 734 may comprise a magnetic rotary absolute positioning system implemented as an AS5055EQFT single-chip magnetic rotary position sensor available from Austria Microsystems, AG. The position sensor 734 may interface with the control circuit 710 to provide an absolute positioning system. The position may include multiple Hall-effect elements located above a magnet and coupled to a CORDIC processor, also known as the digit-by-digit method and Volder's algorithm, that is provided to implement a simple and efficient algorithm to calculate hyperbolic and trigonometric functions that require only addition, subtraction, bitshift, and table lookup operations.

In one aspect, the control circuit 710 may be in communication with one or more sensors 738. The sensors 738 may be positioned on the end effector 702 and adapted to operate with the robotic surgical instrument 700 to measure the various derived parameters such as the gap distance versus time, tissue compression versus time, and anvil strain versus time. The sensors 738 may comprise a magnetic sensor, a magnetic field sensor, a strain gauge, a load cell, a pressure sensor, a force sensor, a torque sensor, an inductive sensor such as an eddy current sensor, a resistive sensor, a capacitive sensor, an optical sensor, and/or any other suitable sensor for measuring one or more parameters of the end effector 702. The sensors 738 may include one or more sensors. The sensors 738 may be located on the staple cartridge 718 deck to determine tissue location using segmented electrodes. The torque sensors 744a-744e may be configured to sense force such as firing force, closure force, and/or articulation force, among others. Accordingly, the control circuit 710 can sense (1) the closure load experienced by the distal closure tube and its position, (2) the firing member at the rack and its position, (3) what portion of the staple cartridge 718 has tissue on it, and (4) the load and position on both articulation rods.

In one aspect, the one or more sensors 738 may comprise a strain gauge, such as a micro-strain gauge, configured to measure the magnitude of the strain in the anvil 716 during a clamped condition. The strain gauge provides an electrical signal whose amplitude varies with the magnitude of the strain. The sensors 738 may comprise a pressure sensor configured to detect a pressure generated by the presence of compressed tissue between the anvil 716 and the staple cartridge 718. The sensors 738 may be configured to detect impedance of a tissue section located between the anvil 716 and the staple cartridge 718 that is indicative of the thickness and/or fullness of tissue located therebetween.

In one aspect, the sensors 738 may be implemented as one or more limit switches, electromechanical devices, solid-state switches, Hall-effect devices, magneto-resistive (MR) devices, giant magneto-resistive (GMR) devices, magnetometers, among others. In other implementations, the sensors 738 may be implemented as solid-state switches that operate under the influence of light, such as optical sensors, IR sensors, ultraviolet sensors, among others. Still, the switches may be solid-state devices such as transistors (e.g., FET, junction FET, MOSFET, bipolar, and the like). In other implementations, the sensors 738 may include electrical conductorless switches, ultrasonic switches, accelerometers, and inertial sensors, among others.

In one aspect, the sensors 738 may be configured to measure forces exerted on the anvil 716 by the closure drive system. For example, one or more sensors 738 can be at an interaction point between the closure tube and the anvil 716 to detect the closure forces applied by the closure tube to the anvil 716. The forces exerted on the anvil 716 can be representative of the tissue compression experienced by the tissue section captured between the anvil 716 and the staple cartridge 718. The one or more sensors 738 can be positioned at various interaction points along the closure drive system to detect the closure forces applied to the anvil 716 by the closure drive system. The one or more sensors 738 may be sampled in real time during a clamping operation by the processor of the control circuit 710. The control circuit 710 receives real-time sample measurements to provide and analyze time-based information and assess, in real time, closure forces applied to the anvil 716.

In one aspect, a current sensor 736 can be employed to measure the current drawn by each of the motors 704a-704e. The force required to advance any of the movable mechanical elements such as the I-beam 714 corresponds to the current drawn by one of the motors 704a-704e. The force is converted to a digital signal and provided to the control circuit 710. The control circuit 710 can be configured to simulate the response of the actual system of the instrument in the software of the controller. A displacement member can be actuated to move an I-beam 714 in the end effector 702 at or near a target velocity. The robotic surgical instrument 700 can include a feedback controller, which can be one of any feedback controllers, including, but not limited to a PID, a state feedback, a linear-quadratic (LQR), and/or an adaptive controller, for example. The robotic surgical instrument 700 can include a power source to convert the signal from the feedback controller into a physical input such as case voltage, PWM voltage, frequency modulated voltage, current, torque, and/or force, for example. Additional details are disclosed in U.S. Patent Application Serial No. 15/636,829, titled CLOSED LOOP VELOCITY CONTROL TECHNIQUES FOR ROBOTIC SURGICAL INSTRUMENT, filed June 29, 2017, which is herein incorporated by reference in its entirety.

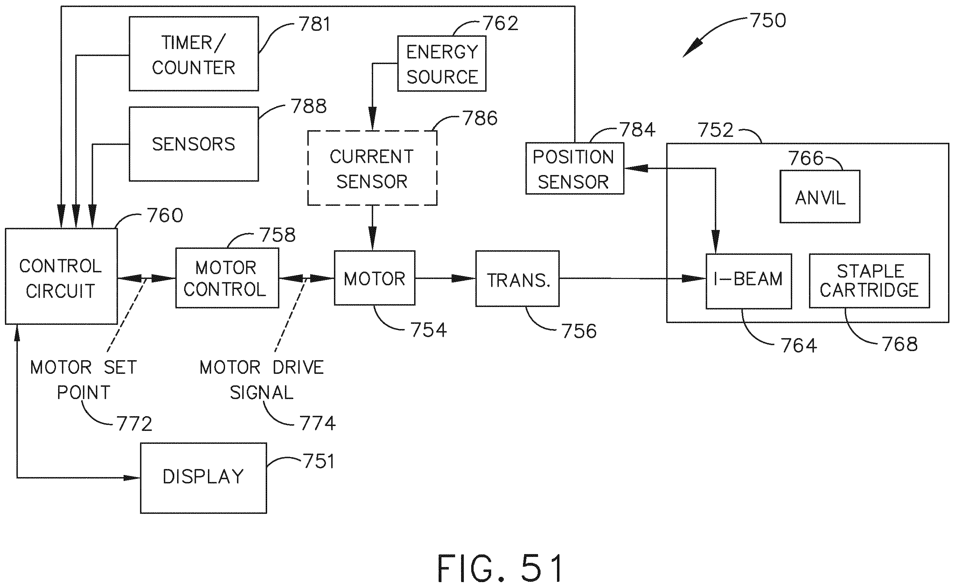

FIG. 51 illustrates a block diagram of a surgical instrument 750 programmed to control the distal translation of a displacement member according to one aspect of this disclosure. In one aspect, the surgical instrument 750 is programmed to control the distal translation of a displacement member such as the I-beam 764. The surgical instrument 750 comprises an end effector 752 that may comprise an anvil 766, an I-beam 764 (including a sharp cutting edge), and a removable staple cartridge 768.

The position, movement, displacement, and/or translation of a linear displacement member, such as the I-beam 764, can be measured by an absolute positioning system, sensor arrangement, and position sensor 784. Because the I-beam 764 is coupled to a longitudinally movable drive member, the position of the I-beam 764 can be determined by measuring the position of the longitudinally movable drive member employing the position sensor 784. Accordingly, in the following description, the position, displacement, and/or translation of the I-beam 764 can be achieved by the position sensor 784 as described herein. A control circuit 760 may be programmed to control the translation of the displacement member, such as the I-beam 764. The control circuit 760, in some examples, may comprise one or more microcontrollers, microprocessors, or other suitable processors for executing instructions that cause the processor or processors to control the displacement member, e.g., the I-beam 764, in the manner described. In one aspect, a timer/counter 781 provides an output signal, such as the elapsed time or a digital count, to the control circuit 760 to correlate the position of the I-beam 764 as determined by the position sensor 784 with the output of the timer/counter 781 such that the control circuit 760 can determine the position of the I-beam 764 at a specific time (t) relative to a starting position. The timer/counter 781 may be configured to measure elapsed time, count external events, or time external events.

The control circuit 760 may generate a motor set point signal 772. The motor set point signal 772 may be provided to a motor controller 758. The motor controller 758 may comprise one or more circuits configured to provide a motor drive signal 774 to the motor 754 to drive the motor 754 as described herein. In some examples, the motor 754 may be a brushed DC electric motor. For example, the velocity of the motor 754 may be proportional to the motor drive signal 774. In some examples, the motor 754 may be a brushless DC electric motor and the motor drive signal 774 may comprise a PWM signal provided to one or more stator windings of the motor 754. Also, in some examples, the motor controller 758 may be omitted, and the control circuit 760 may generate the motor drive signal 774 directly.

The motor 754 may receive power from an energy source 762. The energy source 762 may be or include a battery, a super capacitor, or any other suitable energy source. The motor 754 may be mechanically coupled to the I-beam 764 via a transmission 756. The transmission 756 may include one or more gears or other linkage components to couple the motor 754 to the I-beam 764. A position sensor 784 may sense a position of the I-beam 764. The position sensor 784 may be or include any type of sensor that is capable of generating position data that indicate a position of the I-beam 764. In some examples, the position sensor 784 may include an encoder configured to provide a series of pulses to the control circuit 760 as the I-beam 764 translates distally and proximally. The control circuit 760 may track the pulses to determine the position of the I-beam 764. Other suitable position sensors may be used, including, for example, a proximity sensor. Other types of position sensors may provide other signals indicating motion of the I-beam 764. Also, in some examples, the position sensor 784 may be omitted. Where the motor 754 is a stepper motor, the control circuit 760 may track the position of the I-beam 764 by aggregating the number and direction of steps that the motor 754 has been instructed to execute. The position sensor 784 may be located in the end effector 752 or at any other portion of the instrument.

The control circuit 760 may be in communication with one or more sensors 788. The sensors 788 may be positioned on the end effector 752 and adapted to operate with the surgical instrument 750 to measure the various derived parameters such as gap distance versus time, tissue compression versus time, and anvil strain versus time. The sensors 788 may comprise a magnetic sensor, a magnetic field sensor, a strain gauge, a pressure sensor, a force sensor, an inductive sensor such as an eddy current sensor, a resistive sensor, a capacitive sensor, an optical sensor, and/or any other suitable sensor for measuring one or more parameters of the end effector 752. The sensors 788 may include one or more sensors.

The one or more sensors 788 may comprise a strain gauge, such as a micro-strain gauge, configured to measure the magnitude of the strain in the anvil 766 during a clamped condition. The strain gauge provides an electrical signal whose amplitude varies with the magnitude of the strain. The sensors 788 may comprise a pressure sensor configured to detect a pressure generated by the presence of compressed tissue between the anvil 766 and the staple cartridge 768. The sensors 788 may be configured to detect impedance of a tissue section located between the anvil 766 and the staple cartridge 768 that is indicative of the thickness and/or fullness of tissue located therebetween.

The sensors 788 may be is configured to measure forces exerted on the anvil 766 by a closure drive system. For example, one or more sensors 788 can be at an interaction point between a closure tube and the anvil 766 to detect the closure forces applied by a closure tube to the anvil 766. The forces exerted on the anvil 766 can be representative of the tissue compression experienced by the tissue section captured between the anvil 766 and the staple cartridge 768. The one or more sensors 788 can be positioned at various interaction points along the closure drive system to detect the closure forces applied to the anvil 766 by the closure drive system. The one or more sensors 788 may be sampled in real time during a clamping operation by a processor of the control circuit 760. The control circuit 760 receives real-time sample measurements to provide and analyze time-based information and assess, in real time, closure forces applied to the anvil 766.

A current sensor 786 can be employed to measure the current drawn by the motor 754. The force required to advance the I-beam 764 corresponds to the current drawn by the motor 754. The force is converted to a digital signal and provided to the control circuit 760.

The control circuit 760 can be configured to simulate the response of the actual system of the instrument in the software of the controller. A displacement member can be actuated to move an I-beam 764 in the end effector 752 at or near a target velocity. The surgical instrument 750 can include a feedback controller, which can be one of any feedback controllers, including, but not limited to a PID, a state feedback, LQR, and/or an adaptive controller, for example. The surgical instrument 750 can include a power source to convert the signal from the feedback controller into a physical input such as case voltage, PWM voltage, frequency modulated voltage, current, torque, and/or force, for example.

The actual drive system of the surgical instrument 750 is configured to drive the displacement member, cutting member, or I-beam 764, by a brushed DC motor with gearbox and mechanical links to an articulation and/or knife system. Another example is the electric motor 754 that operates the displacement member and the articulation driver, for example, of an interchangeable shaft assembly. An outside influence is an unmeasured, unpredictable influence of things like tissue, surrounding bodies and friction on the physical system. Such outside influence can be referred to as drag which acts in opposition to the electric motor 754. The outside influence, such as drag, may cause the operation of the physical system to deviate from a desired operation of the physical system.

Various example aspects are directed to a surgical instrument 750 comprising an end effector 752 with motor-driven surgical stapling and cutting implements. For example, a motor 754 may drive a displacement member distally and proximally along a longitudinal axis of the end effector 752. The end effector 752 may comprise a pivotable anvil 766 and, when configured for use, a staple cartridge 768 positioned opposite the anvil 766. A clinician may grasp tissue between the anvil 766 and the staple cartridge 768, as described herein. When ready to use the instrument 750, the clinician may provide a firing signal, for example by depressing a trigger of the instrument 750. In response to the firing signal, the motor 754 may drive the displacement member distally along the longitudinal axis of the end effector 752 from a proximal stroke begin position to a stroke end position distal of the stroke begin position. As the displacement member translates distally, an I-beam 764 with a cutting element positioned at a distal end, may cut the tissue between the staple cartridge 768 and the anvil 766.

In various examples, the surgical instrument 750 may comprise a control circuit 760 programmed to control the distal translation of the displacement member, such as the I-beam 764, for example, based on one or more tissue conditions. The control circuit 760 may be programmed to sense tissue conditions, such as thickness, either directly or indirectly, as described herein. The control circuit 760 may be programmed to select a firing control program based on tissue conditions. A firing control program may describe the distal motion of the displacement member. Different firing control programs may be selected to better treat different tissue conditions. For example, when thicker tissue is present, the control circuit 760 may be programmed to translate the displacement member at a lower velocity and/or with lower power. When thinner tissue is present, the control circuit 760 may be programmed to translate the displacement member at a higher velocity and/or with higher power.

In some examples, the control circuit 760 may initially operate the motor 754 in an open loop configuration for a first open loop portion of a stroke of the displacement member. Based on a response of the instrument 750 during the open loop portion of the stroke, the control circuit 760 may select a firing control program. The response of the instrument may include, a translation distance of the displacement member during the open loop portion, a time elapsed during the open loop portion, energy provided to the motor 754 during the open loop portion, a sum of pulse widths of a motor drive signal, etc. After the open loop portion, the control circuit 760 may implement the selected firing control program for a second portion of the displacement member stroke. For example, during the closed loop portion of the stroke, the control circuit 760 may modulate the motor 754 based on translation data describing a position of the displacement member in a closed loop manner to translate the displacement member at a constant velocity. Additional details are disclosed in U.S. Patent Application Serial No. 15/720,852, titled SYSTEM AND METHODS FOR CONTROLLING A DISPLAY OF A SURGICAL INSTRUMENT, filed September 29, 2017, which is herein incorporated by reference in its entirety.

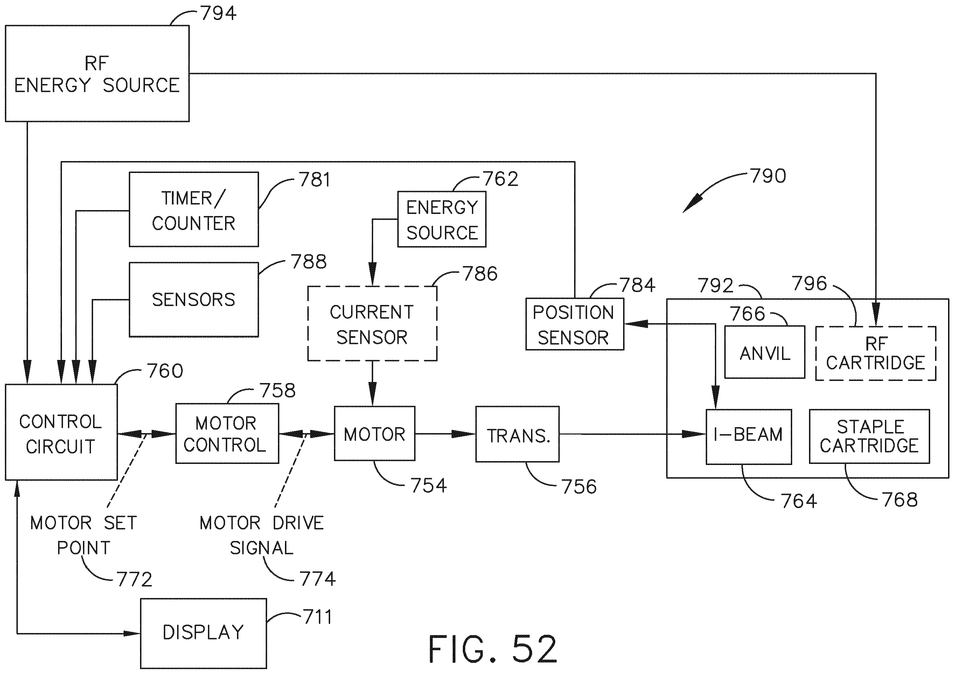

FIG. 52 is a schematic diagram of a surgical instrument 790 configured to control various functions according to one aspect of this disclosure. In one aspect, the surgical instrument 790 is programmed to control distal translation of a displacement member such as the I-beam 764. The surgical instrument 790 comprises an end effector 792 that may comprise an anvil 766, an I-beam 764, and a removable staple cartridge 768 which may be interchanged with an RF cartridge 796 (shown in dashed line).

In one aspect, sensors 788 may be implemented as a limit switch, electromechanical device, solid-state switches, Hall-effect devices, MR devices, GMR devices, magnetometers, among others. In other implementations, the sensors 638 may be solid-state switches that operate under the influence of light, such as optical sensors, IR sensors, ultraviolet sensors, among others. Still, the switches may be solid-state devices such as transistors (e.g., FET, junction FET, MOSFET, bipolar, and the like). In other implementations, the sensors 788 may include electrical conductorless switches, ultrasonic switches, accelerometers, and inertial sensors, among others.

In one aspect, the position sensor 784 may be implemented as an absolute positioning system comprising a magnetic rotary absolute positioning system implemented as an AS5055EQFT single-chip magnetic rotary position sensor available from Austria Microsystems, AG. The position sensor 784 may interface with the control circuit 760 to provide an absolute positioning system. The position may include multiple Hall-effect elements located above a magnet and coupled to a CORDIC processor, also known as the digit-by-digit method and Volder's algorithm, that is provided to implement a simple and efficient algorithm to calculate hyperbolic and trigonometric functions that require only addition, subtraction, bitshift, and table lookup operations.

In one aspect, the I-beam 764 may be implemented as a knife member comprising a knife body that operably supports a tissue cutting blade thereon and may further include anvil engagement tabs or features and channel engagement features or a foot. In one aspect, the staple cartridge 768 may be implemented as a standard (mechanical) surgical fastener cartridge. In one aspect, the RF cartridge 796 maybe implemented as an RF cartridge. These and other sensors arrangements are described in commonly owned U.S. Patent Application Serial No. 15/628,175, titled TECHNIQUES FOR ADAPTIVE CONTROL OF MOTOR VELOCITY OF A SURGICAL STAPLING AND CUTTING INSTRUMENT, filed June 20, 2017, which is herein incorporated by reference in its entirety.

The position, movement, displacement, and/or translation of a linear displacement member, such as the I-beam 764, can be measured by an absolute positioning system, sensor arrangement, and position sensor represented as position sensor 784. Because the I-beam 764 is coupled to the longitudinally movable drive member, the position of the I-beam 764 can be determined by measuring the position of the longitudinally movable drive member employing the position sensor 784. Accordingly, in the following description, the position, displacement, and/or translation of the I-beam 764 can be achieved by the position sensor 784 as described herein. A control circuit 760 may be programmed to control the translation of the displacement member, such as the I-beam 764, as described herein. The control circuit 760, in some examples, may comprise one or more microcontrollers, microprocessors, or other suitable processors for executing instructions that cause the processor or processors to control the displacement member, e.g., the I-beam 764, in the manner described. In one aspect, a timer/counter 781 provides an output signal, such as the elapsed time or a digital count, to the control circuit 760 to correlate the position of the I-beam 764 as determined by the position sensor 784 with the output of the timer/counter 781 such that the control circuit 760 can determine the position of the I-beam 764 at a specific time (t) relative to a starting position. The timer/counter 781 may be configured to measure elapsed time, count external events, or time external events.

The control circuit 760 may generate a motor set point signal 772. The motor set point signal 772 may be provided to a motor controller 758. The motor controller 758 may comprise one or more circuits configured to provide a motor drive signal 774 to the motor 754 to drive the motor 754 as described herein. In some examples, the motor 754 may be a brushed DC electric motor. For example, the velocity of the motor 754 may be proportional to the motor drive signal 774. In some examples, the motor 754 may be a brushless DC electric motor and the motor drive signal 774 may comprise a PWM signal provided to one or more stator windings of the motor 754. Also, in some examples, the motor controller 758 may be omitted, and the control circuit 760 may generate the motor drive signal 774 directly.

The motor 754 may receive power from an energy source 762. The energy source 762 may be or include a battery, a super capacitor, or any other suitable energy source. The motor 754 may be mechanically coupled to the I-beam 764 via a transmission 756. The transmission 756 may include one or more gears or other linkage components to couple the motor 754 to the I-beam 764. A position sensor 784 may sense a position of the I-beam 764. The position sensor 784 may be or include any type of sensor that is capable of generating position data that indicate a position of the I-beam 764. In some examples, the position sensor 784 may include an encoder configured to provide a series of pulses to the control circuit 760 as the I-beam 764 translates distally and proximally. The control circuit 760 may track the pulses to determine the position of the I-beam 764. Other suitable position sensors may be used, including, for example, a proximity sensor. Other types of position sensors may provide other signals indicating motion of the I-beam 764. Also, in some examples, the position sensor 784 may be omitted. Where the motor 754 is a stepper motor, the control circuit 760 may track the position of the I-beam 764 by aggregating the number and direction of steps that the motor has been instructed to execute. The position sensor 784 may be located in the end effector 792 or at any other portion of the instrument.

The control circuit 760 may be in communication with one or more sensors 788. The sensors 788 may be positioned on the end effector 792 and adapted to operate with the surgical instrument 790 to measure the various derived parameters such as gap distance versus time, tissue compression versus time, and anvil strain versus time. The sensors 788 may comprise a magnetic sensor, a magnetic field sensor, a strain gauge, a pressure sensor, a force sensor, an inductive sensor such as an eddy current sensor, a resistive sensor, a capacitive sensor, an optical sensor, and/or any other suitable sensor for measuring one or more parameters of the end effector 792. The sensors 788 may include one or more sensors.

The one or more sensors 788 may comprise a strain gauge, such as a micro-strain gauge, configured to measure the magnitude of the strain in the anvil 766 during a clamped condition. The strain gauge provides an electrical signal whose amplitude varies with the magnitude of the strain. The sensors 788 may comprise a pressure sensor configured to detect a pressure generated by the presence of compressed tissue between the anvil 766 and the staple cartridge 768. The sensors 788 may be configured to detect impedance of a tissue section located between the anvil 766 and the staple cartridge 768 that is indicative of the thickness and/or fullness of tissue located therebetween.

The sensors 788 may be is configured to measure forces exerted on the anvil 766 by the closure drive system. For example, one or more sensors 788 can be at an interaction point between a closure tube and the anvil 766 to detect the closure forces applied by a closure tube to the anvil 766. The forces exerted on the anvil 766 can be representative of the tissue compression experienced by the tissue section captured between the anvil 766 and the staple cartridge 768. The one or more sensors 788 can be positioned at various interaction points along the closure drive system to detect the closure forces applied to the anvil 766 by the closure drive system. The one or more sensors 788 may be sampled in real time during a clamping operation by a processor portion of the control circuit 760. The control circuit 760 receives real-time sample measurements to provide and analyze time-based information and assess, in real time, closure forces applied to the anvil 766.

A current sensor 786 can be employed to measure the current drawn by the motor 754. The force required to advance the I-beam 764 corresponds to the current drawn by the motor 754. The force is converted to a digital signal and provided to the control circuit 760.

An RF energy source 794 is coupled to the end effector 792 and is applied to the RF cartridge 796 when the RF cartridge 796 is loaded in the end effector 792 in place of the staple cartridge 768. The control circuit 760 controls the delivery of the RF energy to the RF cartridge 796.

Additional details are disclosed in U.S. Patent Application Serial No. 15/636,096, titled SURGICAL SYSTEM COUPLABLE WITH STAPLE CARTRIDGE AND RADIO FREQUENCY CARTRIDGE, AND METHOD OF USING SAME, filed June 28, 2017, which is herein incorporated by reference in its entirety.

### Generator Hardware

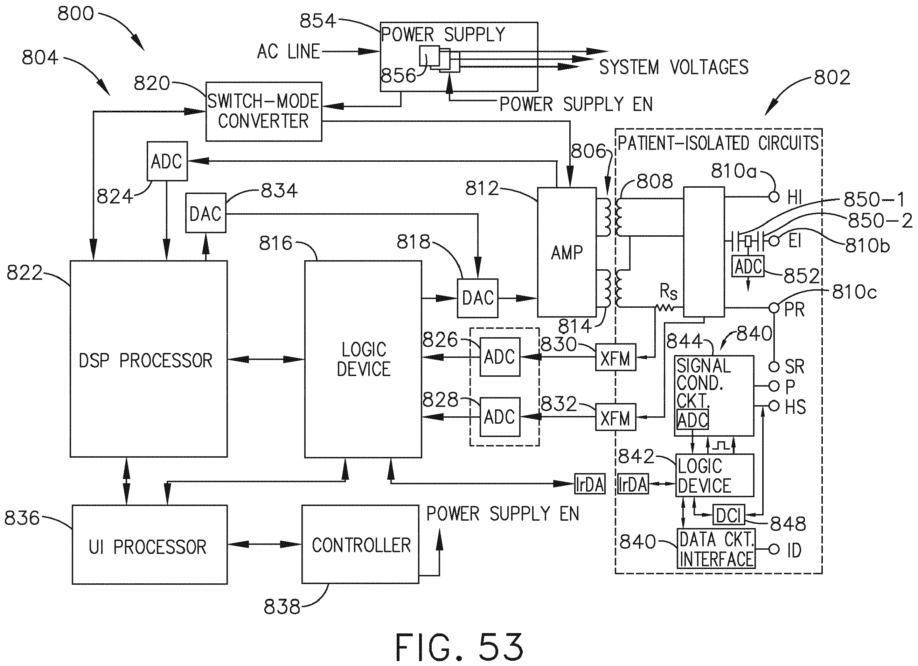

FIG. 53 is a simplified block diagram of a generator 800 configured to provide inductorless tuning, among other benefits. Additional details of the generator 800 are described in U.S. Patent No. 9,060,775, titled SURGICAL GENERATOR FOR ULTRASONIC AND ELECTROSURGICAL DEVICES, which issued on June 23, 2015, which is herein incorporated by reference in its entirety. The generator 800 may comprise a patient isolated stage 802 in communication with a non-isolated stage 804 via a power transformer 806. A secondary winding 808 of the power transformer 806 is contained in the isolated stage 802 and may comprise a tapped configuration (e.g., a center-tapped or a non-center-tapped configuration) to define drive signal outputs 810a, 810b, 810c for delivering drive signals to different surgical instruments, such as, for example, an ultrasonic surgical instrument, an RF electrosurgical instrument, and a multifunction surgical instrument which includes ultrasonic and RF energy modes that can be delivered alone or simultaneously. In particular, drive signal outputs 810a, 810c may output an ultrasonic drive signal (e.g., a 420V root-mean-square (RMS) drive signal) to an ultrasonic surgical instrument, and drive signal outputs 810b, 810c may output an RF electrosurgical drive signal (e.g., a 100V RMS drive signal) to an RF electrosurgical instrument, with the drive signal output 810b corresponding to the center tap of the power transformer 806.

In certain forms, the ultrasonic and electrosurgical drive signals may be provided simultaneously to distinct surgical instruments and/or to a single surgical instrument, such as the multifunction surgical instrument, having the capability to deliver both ultrasonic and electrosurgical energy to tissue. It will be appreciated that the electrosurgical signal, provided either to a dedicated electrosurgical instrument and/or to a combined multifunction ultrasonic/electrosurgical instrument may be either a therapeutic or sub-therapeutic level signal where the sub-therapeutic signal can be used, for example, to monitor tissue or instrument conditions and provide feedback to the generator. For example, the ultrasonic and RF signals can be delivered separately or simultaneously from a generator with a single output port in order to provide the desired output signal to the surgical instrument, as will be discussed in more detail below. Accordingly, the generator can combine the ultrasonic and electrosurgical RF energies and deliver the combined energies to the multifunction ultrasonic/electrosurgical instrument. Bipolar electrodes can be placed on one or both jaws of the end effector. One jaw may be driven by ultrasonic energy in addition to electrosurgical RF energy, working simultaneously. The ultrasonic energy may be employed to dissect tissue, while the electrosurgical RF energy may be employed for vessel sealing.

The non-isolated stage 804 may comprise a power amplifier 812 having an output connected to a primary winding 814 of the power transformer 806. In certain forms, the power amplifier 812 may comprise a push-pull amplifier. For example, the non-isolated stage 804 may further comprise a logic device 816 for supplying a digital output to a digital-to-analog converter (DAC) circuit 818, which in turn supplies a corresponding analog signal to an input of the power amplifier 812. In certain forms, the logic device 816 may comprise a programmable gate array (PGA), a FPGA, programmable logic device (PLD), among other logic circuits, for example. The logic device 816, by virtue of controlling the input of the power amplifier 812 via the DAC circuit 818, may therefore control any of a number of parameters (e.g., frequency, waveform shape, waveform amplitude) of drive signals appearing at the drive signal outputs 810a, 810b, 810c. In certain forms and as discussed below, the logic device 816, in conjunction with a processor (e.g., a DSP discussed below), may implement a number of DSP-based and/or other control algorithms to control parameters of the drive signals output by the generator 800.

Power may be supplied to a power rail of the power amplifier 812 by a switch-mode regulator 820, e.g., a power converter. In certain forms, the switch-mode regulator 820 may comprise an adjustable buck regulator, for example. The non-isolated stage 804 may further comprise a first processor 822, which in one form may comprise a DSP processor such as an Analog Devices ADSP-21469 SHARC DSP, available from Analog Devices, Norwood, MA, for example, although in various forms any suitable processor may be employed. In certain forms the DSP processor 822 may control the operation of the switch-mode regulator 820 responsive to voltage feedback data received from the power amplifier 812 by the DSP processor 822 via an ADC circuit 824. In one form, for example, the DSP processor 822 may receive as input, via the ADC circuit 824, the waveform envelope of a signal (e.g., an RF signal) being amplified by the power amplifier 812. The DSP processor 822 may then control the switch-mode regulator 820 (e.g., via a PWM output) such that the rail voltage supplied to the power amplifier 812 tracks the waveform envelope of the amplified signal. By dynamically modulating the rail voltage of the power amplifier 812 based on the waveform envelope, the efficiency of the power amplifier 812 may be significantly improved relative to a fixed rail voltage amplifier schemes.

In certain forms, the logic device 816, in conjunction with the DSP processor 822, may implement a digital synthesis circuit such as a direct digital synthesizer control scheme to control the waveform shape, frequency, and/or amplitude of drive signals output by the generator 800. In one form, for example, the logic device 816 may implement a DDS control algorithm by recalling waveform samples stored in a dynamically updated lookup table (LUT), such as a RAM LUT, which may be embedded in an FPGA. This control algorithm is particularly useful for ultrasonic applications in which an ultrasonic transducer, such as an ultrasonic transducer, may be driven by a clean sinusoidal current at its resonant frequency. Because other frequencies may excite parasitic resonances, minimizing or reducing the total distortion of the motional branch current may correspondingly minimize or reduce undesirable resonance effects. Because the waveform shape of a drive signal output by the generator 800 is impacted by various sources of distortion present in the output drive circuit (e.g., the power transformer 806, the power amplifier 812), voltage and current feedback data based on the drive signal may be input into an algorithm, such as an error control algorithm implemented by the DSP processor 822, which compensates for distortion by suitably pre-distorting or modifying the waveform samples stored in the LUT on a dynamic, ongoing basis (e.g., in real time). In one form, the amount or degree of pre-distortion applied to the LUT samples may be based on the error between a computed motional branch current and a desired current waveform shape, with the error being determined on a sample-by-sample basis. In this way, the pre-distorted LUT samples, when processed through the drive circuit, may result in a motional branch drive signal having the desired waveform shape (e.g., sinusoidal) for optimally driving the ultrasonic transducer. In such forms, the LUT waveform samples will therefore not represent the desired waveform shape of the drive signal, but rather the waveform shape that is required to ultimately produce the desired waveform shape of the motional branch drive signal when distortion effects are taken into account.

The non-isolated stage 804 may further comprise a first ADC circuit 826 and a second ADC circuit 828 coupled to the output of the power transformer 806 via respective isolation transformers 830, 832 for respectively sampling the voltage and current of drive signals output by the generator 800. In certain forms, the ADC circuits 826, 828 may be configured to sample at high speeds (e.g., 80 mega samples per second (MSPS)) to enable oversampling of the drive signals. In one form, for example, the sampling speed of the ADC circuits 826, 828 may enable approximately 200x (depending on frequency) oversampling of the drive signals. In certain forms, the sampling operations of the ADC circuit 826, 828 may be performed by a single ADC circuit receiving input voltage and current signals via a two-way multiplexer. The use of high-speed sampling in forms of the generator 800 may enable, among other things, calculation of the complex current flowing through the motional branch (which may be used in certain forms to implement DDS-based waveform shape control described above), accurate digital filtering of the sampled signals, and calculation of real power consumption with a high degree of precision. Voltage and current feedback data output by the ADC circuits 826, 828 may be received and processed (e.g., first-in-first-out (FIFO) buffer, multiplexer) by the logic device 816 and stored in data memory for subsequent retrieval by, for example, the DSP processor 822. As noted above, voltage and current feedback data may be used as input to an algorithm for pre-distorting or modifying LUT waveform samples on a dynamic and ongoing basis. In certain forms, this may require each stored voltage and current feedback data pair to be indexed based on, or otherwise associated with, a corresponding LUT sample that was output by the logic device 816 when the voltage and current feedback data pair was acquired. Synchronization of the LUT samples and the voltage and current feedback data in this manner contributes to the correct timing and stability of the pre-distortion algorithm.

In certain forms, the voltage and current feedback data may be used to control the frequency and/or amplitude (e.g., current amplitude) of the drive signals. In one form, for example, voltage and current feedback data maybe used to determine impedance phase. The frequency of the drive signal may then be controlled to minimize or reduce the difference between the determined impedance phase and an impedance phase setpoint (e.g., 0°), thereby minimizing or reducing the effects of harmonic distortion and correspondingly enhancing impedance phase measurement accuracy. The determination of phase impedance and a frequency control signal may be implemented in the DSP processor 822, for example, with the frequency control signal being supplied as input to a DDS control algorithm implemented by the logic device 816.

In another form, for example, the current feedback data may be monitored in order to maintain the current amplitude of the drive signal at a current amplitude setpoint. The current amplitude setpoint may be specified directly or determined indirectly based on specified voltage amplitude and power setpoints. In certain forms, control of the current amplitude may be implemented by control algorithm, such as, for example, a proportional-integral-derivative (PID) control algorithm, in the DSP processor 822. Variables controlled by the control algorithm to suitably control the current amplitude of the drive signal may include, for example, the scaling of the LUT waveform samples stored in the logic device 816 and/or the full-scale output voltage of the DAC circuit 818 (which supplies the input to the power amplifier 812) via a DAC circuit 834.

The non-isolated stage 804 may further comprise a second processor 836 for providing, among other things user interface (UI) functionality. In one form, the UI processor 836 may comprise an Atmel AT91 SAM9263 processor having an ARM 926EJ-S core, available from Atmel Corporation, San Jose, California, for example. Examples of UI functionality supported by the UI processor 836 may include audible and visual user feedback, communication with peripheral devices (e.g., via a USB interface), communication with a foot switch, communication with an input device (e.g., a touch screen display) and communication with an output device (e.g., a speaker). The UI processor 836 may communicate with the DSP processor 822 and the logic device 816 (e.g., via SPI buses). Although the UI processor 836 may primarily support UI functionality, it may also coordinate with the DSP processor 822 to implement hazard mitigation in certain forms. For example, the UI processor 836 may be programmed to monitor various aspects of user input and/or other inputs (e.g., touch screen inputs, foot switch inputs, temperature sensor inputs) and may disable the drive output of the generator 800 when an erroneous condition is detected.

In certain forms, both the DSP processor 822 and the UI processor 836, for example, may determine and monitor the operating state of the generator 800. For the DSP processor 822, the operating state of the generator 800 may dictate, for example, which control and/or diagnostic processes are implemented by the DSP processor 822. For the UI processor 836, the operating state of the generator 800 may dictate, for example, which elements of a UI (e.g., display screens, sounds) are presented to a user. The respective DSP and UI processors 822, 836 may independently maintain the current operating state of the generator 800 and recognize and evaluate possible transitions out of the current operating state. The DSP processor 822 may function as the master in this relationship and determine when transitions between operating states are to occur. The UI processor 836 may be aware of valid transitions between operating states and may confirm if a particular transition is appropriate. For example, when the DSP processor 822 instructs the UI processor 836 to transition to a specific state, the UI processor 836 may verify that requested transition is valid. In the event that a requested transition between states is determined to be invalid by the UI processor 836, the UI processor 836 may cause the generator 800 to enter a failure mode.

The non-isolated stage 804 may further comprise a controller 838 for monitoring input devices (e.g., a capacitive touch sensor used for turning the generator 800 on and off, a capacitive touch screen). In certain forms, the controller 838 may comprise at least one processor and/or other controller device in communication with the UI processor 836. In one form, for example, the controller 838 may comprise a processor (e.g., a Meg168 8-bit controller available from Atmel) configured to monitor user input provided via one or more capacitive touch sensors. In one form, the controller 838 may comprise a touch screen controller (e.g., a QT5480 touch screen controller available from Atmel) to control and manage the acquisition of touch data from a capacitive touch screen.

In certain forms, when the generator 800 is in a "power off' state, the controller 838 may continue to receive operating power (e.g., via a line from a power supply of the generator 800, such as the power supply 854 discussed below). In this way, the controller 838 may continue to monitor an input device (e.g., a capacitive touch sensor located on a front panel of the generator 800) for turning the generator 800 on and off. When the generator 800 is in the power off state, the controller 838 may wake the power supply (e.g., enable operation of one or more DC/DC voltage converters 856 of the power supply 854) if activation of the "on/off' input device by a user is detected. The controller 838 may therefore initiate a sequence for transitioning the generator 800 to a "power on" state. Conversely, the controller 838 may initiate a sequence for transitioning the generator 800 to the power off state if activation of the "on/off" input device is detected when the generator 800 is in the power on state. In certain forms, for example, the controller 838 may report activation of the "on/off" input device to the UI processor 836, which in turn implements the necessary process sequence for transitioning the generator 800 to the power off state. In such forms, the controller 838 may have no independent ability for causing the removal of power from the generator 800 after its power on state has been established.

In certain forms, the controller 838 may cause the generator 800 to provide audible or other sensory feedback for alerting the user that a power on or power off sequence has been initiated. Such an alert may be provided at the beginning of a power on or power off sequence and prior to the commencement of other processes associated with the sequence.

In certain forms, the isolated stage 802 may comprise an instrument interface circuit 840 to, for example, provide a communication interface between a control circuit of a surgical instrument (e.g., a control circuit comprising handpiece switches) and components of the non-isolated stage 804, such as, for example, the logic device 816, the DSP processor 822, and/or the UI processor 836. The instrument interface circuit 840 may exchange information with components of the non-isolated stage 804 via a communication link that maintains a suitable degree of electrical isolation between the isolated and non-isolated stages 802, 804, such as, for example, an IR-based communication link. Power may be supplied to the instrument interface circuit 840 using, for example, a low-dropout voltage regulator powered by an isolation transformer driven from the non-isolated stage 804.

In one form, the instrument interface circuit 840 may comprise a logic circuit 842 (e.g., logic circuit, programmable logic circuit, PGA, FPGA, PLD) in communication with a signal conditioning circuit 844. The signal conditioning circuit 844 may be configured to receive a periodic signal from the logic circuit 842 (e.g., a 2 kHz square wave) to generate a bipolar interrogation signal having an identical frequency. The interrogation signal may be generated, for example, using a bipolar current source fed by a differential amplifier. The interrogation signal may be communicated to a surgical instrument control circuit (e.g., by using a conductive pair in a cable that connects the generator 800 to the surgical instrument) and monitored to determine a state or configuration of the control circuit. The control circuit may comprise a number of switches, resistors, and/or diodes to modify one or more characteristics (e.g., amplitude, rectification) of the interrogation signal such that a state or configuration of the control circuit is uniquely discernable based on the one or more characteristics. In one form, for example, the signal conditioning circuit 844 may comprise an ADC circuit for generating samples of a voltage signal appearing across inputs of the control circuit resulting from passage of interrogation signal therethrough. The logic circuit 842 (or a component of the non-isolated stage 804) may then determine the state or configuration of the control circuit based on the ADC circuit samples.

In one form, the instrument interface circuit 840 may comprise a first data circuit interface 846 to enable information exchange between the logic circuit 842 (or other element of the instrument interface circuit 840) and a first data circuit disposed in or otherwise associated with a surgical instrument. In certain forms, for example, a first data circuit may be disposed in a cable integrally attached to a surgical instrument handpiece or in an adaptor for interfacing a specific surgical instrument type or model with the generator 800. The first data circuit may be implemented in any suitable manner and may communicate with the generator according to any suitable protocol, including, for example, as described herein with respect to the first data circuit. In certain forms, the first data circuit may comprise a non-volatile storage device, such as an EEPROM device. In certain forms, the first data circuit interface 846 may be implemented separately from the logic circuit 842 and comprise suitable circuitry (e.g., discrete logic devices, a processor) to enable communication between the logic circuit 842 and the first data circuit. In other forms, the first data circuit interface 846 may be integral with the logic circuit 842.

In certain forms, the first data circuit may store information pertaining to the particular surgical instrument with which it is associated. Such information may include, for example, a model number, a serial number, a number of operations in which the surgical instrument has been used, and/or any other type of information. This information may be read by the instrument interface circuit 840 (e.g., by the logic circuit 842), transferred to a component of the non-isolated stage 804 (e.g., to logic device 816, DSP processor 822, and/or UI processor 836) for presentation to a user via an output device and/or for controlling a function or operation of the generator 800. Additionally, any type of information may be communicated to the first data circuit for storage therein via the first data circuit interface 846 (e.g., using the logic circuit 842). Such information may comprise, for example, an updated number of operations in which the surgical instrument has been used and/or dates and/or times of its usage.

As discussed previously, a surgical instrument may be detachable from a handpiece (e.g., the multifunction surgical instrument may be detachable from the handpiece) to promote instrument interchangeability and/or disposability. In such cases, conventional generators may be limited in their ability to recognize particular instrument configurations being used and to optimize control and diagnostic processes accordingly. The addition of readable data circuits to surgical instruments to address this issue is problematic from a compatibility standpoint, however. For example, designing a surgical instrument to remain backwardly compatible with generators that lack the requisite data reading functionality may be impractical due to, for example, differing signal schemes, design complexity, and cost. Forms of instruments discussed herein address these concerns by using data circuits that may be implemented in existing surgical instruments economically and with minimal design changes to preserve compatibility of the surgical instruments with current generator platforms.

Additionally, forms of the generator 800 may enable communication with instrument-based data circuits. For example, the generator 800 may be configured to communicate with a second data circuit contained in an instrument (e.g., the multifunction surgical instrument). In some forms, the second data circuit may be implemented in a many similar to that of the first data circuit described herein. The instrument interface circuit 840 may comprise a second data circuit interface 848 to enable this communication. In one form, the second data circuit interface 848 may comprise a tristate digital interface, although other interfaces may also be used. In certain forms, the second data circuit may generally be any circuit for transmitting and/or receiving data. In one form, for example, the second data circuit may store information pertaining to the particular surgical instrument with which it is associated. Such information may include, for example, a model number, a serial number, a number of operations in which the surgical instrument has been used, and/or any other type of information.

In some forms, the second data circuit may store information about the electrical and/or ultrasonic properties of an associated ultrasonic transducer, end effector, or ultrasonic drive system. For example, the first data circuit may indicate a burn-in frequency slope, as described herein. Additionally or alternatively, any type of information may be communicated to second data circuit for storage therein via the second data circuit interface 848 (e.g., using the logic circuit 842). Such information may comprise, for example, an updated number of operations in which the instrument has been used and/or dates and/or times of its usage. In certain forms, the second data circuit may transmit data acquired by one or more sensors (e.g., an instrument-based temperature sensor). In certain forms, the second data circuit may receive data from the generator 800 and provide an indication to a user (e.g., a light emitting diode indication or other visible indication) based on the received data.

In certain forms, the second data circuit and the second data circuit interface 848 may be configured such that communication between the logic circuit 842 and the second data circuit can be effected without the need to provide additional conductors for this purpose (e.g., dedicated conductors of a cable connecting a handpiece to the generator 800). In one form, for example, information may be communicated to and from the second data circuit using a one-wire bus communication scheme implemented on existing cabling, such as one of the conductors used transmit interrogation signals from the signal conditioning circuit 844 to a control circuit in a handpiece. In this way, design changes or modifications to the surgical instrument that might otherwise be necessary are minimized or reduced. Moreover, because different types of communications implemented over a common physical channel can be frequency-band separated, the presence of a second data circuit may be "invisible" to generators that do not have the requisite data reading functionality, thus enabling backward compatibility of the surgical instrument.

In certain forms, the isolated stage 802 may comprise at least one blocking capacitor 850-1 connected to the drive signal output 810b to prevent passage of DC current to a patient. A single blocking capacitor may be required to comply with medical regulations or standards, for example. While failure in single-capacitor designs is relatively uncommon, such failure may nonetheless have negative consequences. In one form, a second blocking capacitor 850-2 may be provided in series with the blocking capacitor 850-1, with current leakage from a point between the blocking capacitors 850-1, 850-2 being monitored by, for example, an ADC circuit 852 for sampling a voltage induced by leakage current. The samples may be received by the logic circuit 842, for example. Based changes in the leakage current (as indicated by the voltage samples), the generator 800 may determine when at least one of the blocking capacitors 850-1, 850-2 has failed, thus providing a benefit over single-capacitor designs having a single point of failure.

In certain forms, the non-isolated stage 804 may comprise a power supply 854 for delivering DC power at a suitable voltage and current. The power supply may comprise, for example, a 400 W power supply for delivering a 48 VDC system voltage. The power supply 854 may further comprise one or more DC/DC voltage converters 856 for receiving the output of the power supply to generate DC outputs at the voltages and currents required by the various components of the generator 800. As discussed above in connection with the controller 838, one or more of the DC/DC voltage converters 856 may receive an input from the controller 838 when activation of the "on/off' input device by a user is detected by the controller 838 to enable operation of, or wake, the DC/DC voltage converters 856.

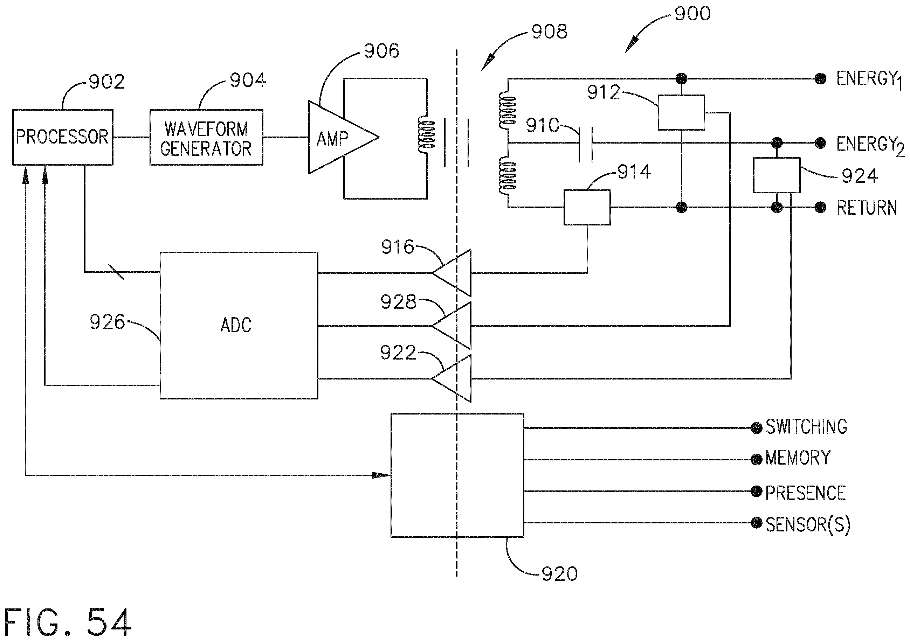

FIG. 54 illustrates an example of a generator 900, which is one form of the generator 800 (FIG. 53). The generator 900 is configured to deliver multiple energy modalities to a surgical instrument. The generator 900 provides RF and ultrasonic signals for delivering energy to a surgical instrument either independently or simultaneously. The RF and ultrasonic signals may be provided alone or in combination and may be provided simultaneously. As noted above, at least one generator output can deliver multiple energy modalities (e.g., ultrasonic, bipolar or monopolar RF, irreversible and/or reversible electroporation, and/or microwave energy, among others) through a single port, and these signals can be delivered separately or simultaneously to the end effector to treat tissue.

The generator 900 comprises a processor 902 coupled to a waveform generator 904. The processor 902 and waveform generator 904 are configured to generate a variety of signal waveforms based on information stored in a memory coupled to the processor 902, not shown for clarity of disclosure. The digital information associated with a waveform is provided to the waveform generator 904 which includes one or more DAC circuits to convert the digital input into an analog output. The analog output is fed to an amplifier 1106 for signal conditioning and amplification. The conditioned and amplified output of the amplifier 906 is coupled to a power transformer 908. The signals are coupled across the power transformer 908 to the secondary side, which is in the patient isolation side. A first signal of a first energy modality is provided to the surgical instrument between the terminals labeled ENERGY 1 and RETURN. A second signal of a second energy modality is coupled across a capacitor 910 and is provided to the surgical instrument between the terminals labeled ENERGY2 and RETURN. It will be appreciated that more than two energy modalities may be output and thus the subscript "n" may be used to designate that up to n ENERGYn terminals may be provided, where n is a positive integer greater than 1. It also will be appreciated that up to "n" return paths RETURNn may be provided without departing from the scope of the present disclosure.

A first voltage sensing circuit 912 is coupled across the terminals labeled ENERGY 1 and the RETURN path to measure the output voltage therebetween. A second voltage sensing circuit 924 is coupled across the terminals labeled ENERGY2 and the RETURN path to measure the output voltage therebetween. A current sensing circuit 914 is disposed in series with the RETURN leg of the secondary side of the power transformer 908 as shown to measure the output current for either energy modality. If different return paths are provided for each energy modality, then a separate current sensing circuit should be provided in each return leg. The outputs of the first and second voltage sensing circuits 912, 924 are provided to respective isolation transformers 916, 922 and the output of the current sensing circuit 914 is provided to another isolation transformer 918. The outputs of the isolation transformers 916, 928, 922 in the on the primary side of the power transformer 908 (non-patient isolated side) are provided to a one or more ADC circuit 926. The digitized output of the ADC circuit 926 is provided to the processor 902 for further processing and computation. The output voltages and output current feedback information can be employed to adjust the output voltage and current provided to the surgical instrument and to compute output impedance, among other parameters. Input/output communications between the processor 902 and patient isolated circuits is provided through an interface circuit 920. Sensors also may be in electrical communication with the processor 902 by way of the interface circuit 920.

In one aspect, the impedance may be determined by the processor 902 by dividing the output of either the first voltage sensing circuit 912 coupled across the terminals labeled ENERGY1/RETURN or the second voltage sensing circuit 924 coupled across the terminals labeled ENERGY2/RETURN by the output of the current sensing circuit 914 disposed in series with the RETURN leg of the secondary side of the power transformer 908. The outputs of the first and second voltage sensing circuits 912, 924 are provided to separate isolations transformers 916, 922 and the output of the current sensing circuit 914 is provided to another isolation transformer 916. The digitized voltage and current sensing measurements from the ADC circuit 926 are provided the processor 902 for computing impedance. As an example, the first energy modality ENERGY 1 may be ultrasonic energy and the second energy modality ENERGY2 may be RF energy. Nevertheless, in addition to ultrasonic and bipolar or monopolar RF energy modalities, other energy modalities include irreversible and/or reversible electroporation and/or microwave energy, among others. Also, although the example illustrated in FIG. 54 shows a single return path RETURN may be provided for two or more energy modalities, in other aspects, multiple return paths RETURNn may be provided for each energy modality ENERGYn. Thus, as described herein, the ultrasonic transducer impedance may be measured by dividing the output of the first voltage sensing circuit 912 by the current sensing circuit 914 and the tissue impedance may be measured by dividing the output of the second voltage sensing circuit 924 by the current sensing circuit 914.

As shown in FIG. 54, the generator 900 comprising at least one output port can include a power transformer 908 with a single output and with multiple taps to provide power in the form of one or more energy modalities, such as ultrasonic, bipolar or monopolar RF, irreversible and/or reversible electroporation, and/or microwave energy, among others, for example, to the end effector depending on the type of treatment of tissue being performed. For example, the generator 900 can deliver energy with higher voltage and lower current to drive an ultrasonic transducer, with lower voltage and higher current to drive RF electrodes for sealing tissue, or with a coagulation waveform for spot coagulation using either monopolar or bipolar RF electrosurgical electrodes. The output waveform from the generator 900 can be steered, switched, or filtered to provide the frequency to the end effector of the surgical instrument. The connection of an ultrasonic transducer to the generator 900 output would be preferably located between the output labeled ENERGY1 and RETURN as shown in FIG. 54. In one example, a connection of RF bipolar electrodes to the generator 900 output would be preferably located between the output labeled ENERGY2 and RETURN. In the case of monopolar output, the preferred connections would be active electrode (e.g., pencil or other probe) to the ENERGY2 output and a suitable return pad connected to the RETURN output.

Additional details are disclosed in U.S. Patent Application Publication No. 2017/0086914, titled TECHNIQUES FOR OPERATING GENERATOR FOR DIGITALLY GENERATING ELECTRICAL SIGNAL WAVEFORMS AND SURGICAL INSTRUMENTS, which published on March 30, 2017, which is herein incorporated by reference in its entirety.

As used throughout this description, the term "wireless" and its derivatives may be used to describe circuits, devices, systems, methods, techniques, communications channels, etc., that may communicate data through the use of modulated electromagnetic radiation through a non-solid medium. The term does not imply that the associated devices do not contain any wires, although in some aspects they might not. The communication module may implement any of a number of wireless or wired communication standards or protocols, including but not limited to Wi-Fi (IEEE 802.11 family), WiMAX (IEEE 802.16 family), IEEE 802.20, long term evolution (LTE), Ev-DO, HSPA+, HSDPA+, HSUPA+, EDGE, GSM, GPRS, CDMA, TDMA, DECT, Bluetooth, Ethernet derivatives thereof, as well as any other wireless and wired protocols that are designated as 3G, 4G, 5G, and beyond. The computing module may include a plurality of communication modules. For instance, a first communication module may be dedicated to shorter range wireless communications such as Wi-Fi and Bluetooth and a second communication module may be dedicated to longer range wireless communications such as GPS, EDGE, GPRS, CDMA, WiMAX, LTE, Ev-DO, and others.

As used herein a processor or processing unit is an electronic circuit which performs operations on some external data source, usually memory or some other data stream. The term is used herein to refer to the central processor (central processing unit) in a system or computer systems (especially systems on a chip (SoCs)) that combine a number of specialized "processors."

As used herein, a system on a chip or system on chip (SoC or SOC) is an integrated circuit (also known as an "IC" or "chip") that integrates all components of a computer or other electronic systems. It may contain digital, analog, mixed-signal, and often radio-frequency functions-all on a single substrate. A SoC integrates a microcontroller (or microprocessor) with advanced peripherals like graphics processing unit (GPU), Wi-Fi module, or coprocessor. A SoC may or may not contain built-in memory.

As used herein, a microcontroller or controller is a system that integrates a microprocessor with peripheral circuits and memory. A microcontroller (or MCU for microcontroller unit) may be implemented as a small computer on a single integrated circuit. It may be similar to a SoC; an SoC may include a microcontroller as one of its components. A microcontroller may contain one or more core processing units (CPUs) along with memory and programmable input/output peripherals. Program memory in the form of Ferroelectric RAM, NOR flash or OTP ROM is also often included on chip, as well as a small amount of RAM. Microcontrollers may be employed for embedded applications, in contrast to the microprocessors used in personal computers or other general purpose applications consisting of various discrete chips.

As used herein, the term controller or microcontroller may be a stand-alone IC or chip device that interfaces with a peripheral device. This may be a link between two parts of a computer or a controller on an external device that manages the operation of (and connection with) that device.

Any of the processors or microcontrollers described herein, may be implemented by any single core or multicore processor such as those known under the trade name ARM Cortex by Texas Instruments. In one aspect, the processor may be an LM4F230H5QR ARM Cortex-M4F Processor Core, available from Texas Instruments, for example, comprising on-chip memory of 256 KB single-cycle flash memory, or other non-volatile memory, up to 40 MHz, a prefetch buffer to improve performance above 40 MHz, a 32 KB single-cycle serial random access memory (SRAM), internal read-only memory (ROM) loaded with StellarisWare® software, 2 KB electrically erasable programmable read-only memory (EEPROM), one or more pulse width modulation (PWM) modules, one or more quadrature encoder inputs (QEI) analog, one or more 12-bit Analog-to-Digital Converters (ADC) with 12 analog input channels, details of which are available for the product datasheet.

In one aspect, the processor may comprise a safety controller comprising two controller-based families such as TMS570 and RM4x known under the trade name Hercules ARM Cortex R4, also by Texas Instruments. The safety controller may be configured specifically for IEC 61508 and ISO 26262 safety critical applications, among others, to provide advanced integrated safety features while delivering scalable performance, connectivity, and memory options.

Modular devices include the modules (as described in connection with FIGS. 3 and 9, for example) that are receivable within a surgical hub and the surgical devices or instruments that can be connected to the various modules in order to connect or pair with the corresponding surgical hub. The modular devices include, for example, intelligent surgical instruments, medical imaging devices, suction/irrigation devices, smoke evacuators, energy generators, ventilators, insufflators, and displays. The modular devices described herein can be controlled by control algorithms. The control algorithms can be executed on the modular device itself, on the surgical hub to which the particular modular device is paired, or on both the modular device and the surgical hub (e.g., via a distributed computing architecture). In some exemplifications, the modular devices' control algorithms control the devices based on data sensed by the modular device itself (i.e., by sensors in, on, or connected to the modular device). This data can be related to the patient being operated on (e.g., tissue properties or insufflation pressure) or the modular device itself (e.g., the rate at which a knife is being advanced, motor current, or energy levels). For example, a control algorithm for a surgical stapling and cutting instrument can control the rate at which the instrument's motor drives its knife through tissue according to resistance encountered by the knife as it advances.

### Situational Awareness

Situational awareness is the ability of some aspects of a surgical system to determine or infer information related to a surgical procedure from data received from databases and/or instruments. The information can include the type of procedure being undertaken, the type of tissue being operated on, or the body cavity that is the subject of the procedure. With the contextual information related to the surgical procedure, the surgical system can, for example, improve the manner in which it controls the modular devices (e.g. a robotic arm and/or robotic surgical tool) that are connected to it and provide contextualized information or suggestions to the surgeon during the course of the surgical procedure.

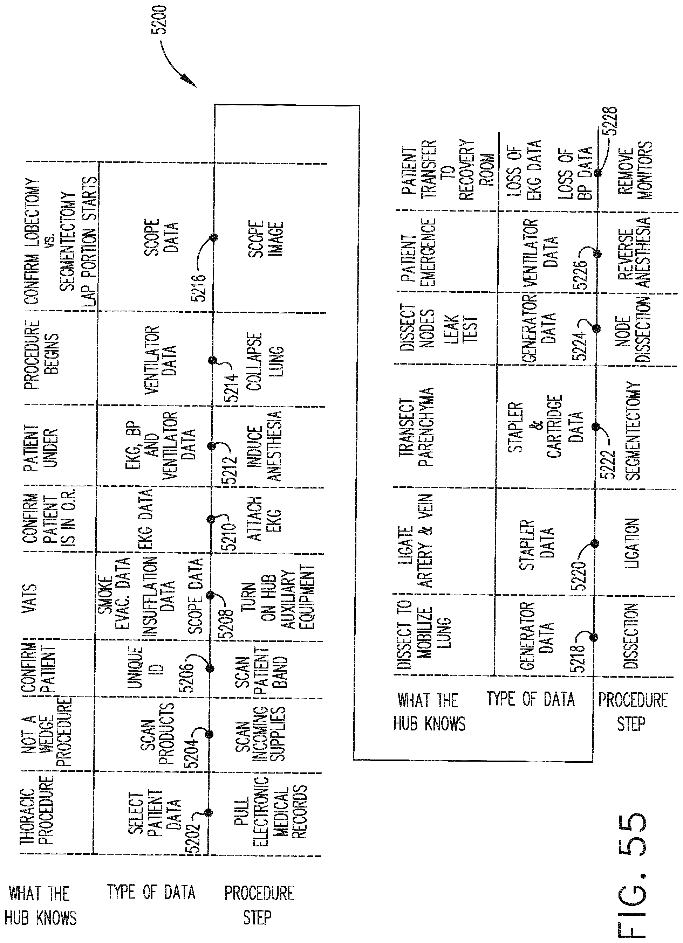

Referring now to FIG. 55, a timeline 5200 depicting situational awareness of a hub, such as the surgical hub 106 or 206, for example, is depicted. The timeline 5200 is an illustrative surgical procedure and the contextual information that the surgical hub 106, 206 can derive from the data received from the data sources at each step in the surgical procedure. The timeline 5200 depicts the typical steps that would be taken by the nurses, surgeons, and other medical personnel during the course of a lung segmentectomy procedure, beginning with setting up the operating theater and ending with transferring the patient to a post-operative recovery room.

The situationally aware surgical hub 106, 206 receives data from the data sources throughout the course of the surgical procedure, including data generated each time medical personnel utilize a modular device that is paired with the surgical hub 106, 206. The surgical hub 106, 206 can receive this data from the paired modular devices and other data sources and continually derive inferences (i.e., contextual information) about the ongoing procedure as new data is received, such as which step of the procedure is being performed at any given time. The situational awareness system of the surgical hub 106, 206 is able to, for example, record data pertaining to the procedure for generating reports, verify the steps being taken by the medical personnel, provide data or prompts (e.g., via a display screen) that may be pertinent for the particular procedural step, adjust modular devices based on the context (e.g., activate monitors, adjust the field of view (FOV) of the medical imaging device, or change the energy level of an ultrasonic surgical instrument or RF electrosurgical instrument), and take any other such action described above.

As the first step 5202 in this illustrative procedure, the hospital staff members retrieve the patient's Electronic Medical Record (EMR) from the hospital's EMR database. Based on select patient data in the EMR, the surgical hub 106, 206 determines that the procedure to be performed is a thoracic procedure.

Second step 5204, the staff members scan the incoming medical supplies for the procedure. The surgical hub 106, 206 cross-references the scanned supplies with a list of supplies that are utilized in various types of procedures and confirms that the mix of supplies corresponds to a thoracic procedure. Further, the surgical hub 106, 206 is also able to determine that the procedure is not a wedge procedure (because the incoming supplies either lack certain supplies that are necessary for a thoracic wedge procedure or do not otherwise correspond to a thoracic wedge procedure).

Third step 5206, the medical personnel scan the patient band via a scanner that is communicably connected to the surgical hub 106, 206. The surgical hub 106, 206 can then confirm the patient's identity based on the scanned data.

Fourth step 5208, the medical staff turns on the auxiliary equipment. The auxiliary equipment being utilized can vary according to the type of surgical procedure and the techniques to be used by the surgeon, but in this illustrative case they include a smoke evacuator, insufflator, and medical imaging device. When activated, the auxiliary equipment that are modular devices can automatically pair with the surgical hub 106, 206 that is located within a particular vicinity of the modular devices as part of their initialization process. The surgical hub 106, 206 can then derive contextual information about the surgical procedure by detecting the types of modular devices that pair with it during this pre-operative or initialization phase. In this particular example, the surgical hub 106, 206 determines that the surgical procedure is a VATS procedure based on this particular combination of paired modular devices. Based on the combination of the data from the patient's EMR, the list of medical supplies to be used in the procedure, and the type of modular devices that connect to the hub, the surgical hub 106, 206 can generally infer the specific procedure that the surgical team will be performing. Once the surgical hub 106, 206 knows what specific procedure is being performed, the surgical hub 106, 206 can then retrieve the steps of that procedure from a memory or from the cloud and then cross-reference the data it subsequently receives from the connected data sources (e.g., modular devices and patient monitoring devices) to infer what step of the surgical procedure the surgical team is performing.

Fifth step 5210, the staff members attach the EKG electrodes and other patient monitoring devices to the patient. The EKG electrodes and other patient monitoring devices are able to pair with the surgical hub 106, 206. As the surgical hub 106, 206 begins receiving data from the patient monitoring devices, the surgical hub 106, 206 thus confirms that the patient is in the operating theater.

Sixth step 5212, the medical personnel induce anesthesia in the patient. The surgical hub 106, 206 can infer that the patient is under anesthesia based on data from the modular devices and/or patient monitoring devices, including EKG data, blood pressure data, ventilator data, or combinations thereof, for example. Upon completion of the sixth step 5212, the pre-operative portion of the lung segmentectomy procedure is completed and the operative portion begins.

Seventh step 5214, the patient's lung that is being operated on is collapsed (while ventilation is switched to the contralateral lung). The surgical hub 106, 206 can infer from the ventilator data that the patient's lung has been collapsed, for example. The surgical hub 106, 206 can infer that the operative portion of the procedure has commenced as it can compare the detection of the patient's lung collapsing to the expected steps of the procedure (which can be accessed or retrieved previously) and thereby determine that collapsing the lung is the first operative step in this particular procedure.

Eighth step 5216, the medical imaging device (e.g., a scope) is inserted and video from the medical imaging device is initiated. The surgical hub 106, 206 receives the medical imaging device data (i.e., video or image data) through its connection to the medical imaging device. Upon receipt of the medical imaging device data, the surgical hub 106, 206 can determine that the laparoscopic portion of the surgical procedure has commenced. Further, the surgical hub 106, 206 can determine that the particular procedure being performed is a segmentectomy, as opposed to a lobectomy (note that a wedge procedure has already been discounted by the surgical hub 106, 206 based on data received at the second step 5204 of the procedure). The data from the medical imaging device 124 (FIG. 33) can be utilized to determine contextual information regarding the type of procedure being performed in a number of different ways, including by determining the angle at which the medical imaging device is oriented with respect to the visualization of the patient's anatomy, monitoring the number or medical imaging devices being utilized (i.e., that are activated and paired with the surgical hub 106, 206), and monitoring the types of visualization devices utilized. For example, one technique for performing a VATS lobectomy places the camera in the lower anterior corner of the patient's chest cavity above the diaphragm, whereas one technique for performing a VATS segmentectomy places the camera in an anterior intercostal position relative to the segmental fissure. Using pattern recognition or machine learning techniques, for example, the situational awareness system can be trained to recognize the positioning of the medical imaging device according to the visualization of the patient's anatomy. As another example, one technique for performing a VATS lobectomy utilizes a single medical imaging device, whereas another technique for performing a VATS segmentectomy utilizes multiple cameras. As yet another example, one technique for performing a VATS segmentectomy utilizes an infrared light source (which can be communicably coupled to the surgical hub as part of the visualization system) to visualize the segmental fissure, which is not utilized in a VATS lobectomy. By tracking any or all of this data from the medical imaging device, the surgical hub 106, 206 can thereby determine the specific type of surgical procedure being performed and/or the technique being used for a particular type of surgical procedure.

Ninth step 5218, the surgical team begins the dissection step of the procedure. The surgical hub 106, 206 can infer that the surgeon is in the process of dissecting to mobilize the patient's lung because it receives data from the RF or ultrasonic generator indicating that an energy instrument is being fired. The surgical hub 106, 206 can cross-reference the received data with the retrieved steps of the surgical procedure to determine that an energy instrument being fired at this point in the process (i.e., after the completion of the previously discussed steps of the procedure) corresponds to the dissection step. In certain instances, the energy instrument can be an energy tool mounted to a robotic arm of a robotic surgical system.

Tenth step 5220, the surgical team proceeds to the ligation step of the procedure. The surgical hub 106, 206 can infer that the surgeon is ligating arteries and veins because it receives data from the surgical stapling and cutting instrument indicating that the instrument is being fired. Similarly to the prior step, the surgical hub 106, 206 can derive this inference by cross-referencing the receipt of data from the surgical stapling and cutting instrument with the retrieved steps in the process. In certain instances, the surgical instrument can be a surgical tool mounted to a robotic arm of a robotic surgical system.

Eleventh step 5222, the segmentectomy portion of the procedure is performed. The surgical hub 106, 206 can infer that the surgeon is transecting the parenchyma based on data from the surgical stapling and cutting instrument, including data from its cartridge. The cartridge data can correspond to the size or type of staple being fired by the instrument, for example. As different types of staples are utilized for different types of tissues, the cartridge data can thus indicate the type of tissue being stapled and/or transected. In this case, the type of staple being fired is utilized for parenchyma (or other similar tissue types), which allows the surgical hub 106, 206 to infer that the segmentectomy portion of the procedure is being performed.

Twelfth step 5224, the node dissection step is then performed. The surgical hub 106, 206 can infer that the surgical team is dissecting the node and performing a leak test based on data received from the generator indicating that an RF or ultrasonic instrument is being fired. For this particular procedure, an RF or ultrasonic instrument being utilized after parenchyma was transected corresponds to the node dissection step, which allows the surgical hub 106, 206 to make this inference. It should be noted that surgeons regularly switch back and forth between surgical stapling/cutting instruments and surgical energy (i.e., RF or ultrasonic) instruments depending upon the particular step in the procedure because different instruments are better adapted for particular tasks. Therefore, the particular sequence in which the stapling/cutting instruments and surgical energy instruments are used can indicate what step of the procedure the surgeon is performing. Moreover, in certain instances, robotic tools can be utilized for one or more steps in a surgical procedure and/or handheld surgical instruments can be utilized for one or more steps in the surgical procedure. The surgeon(s) can alternate between robotic tools and handheld surgical instruments and/or can use the devices concurrently, for example. Upon completion of the twelfth step 5224, the incisions are closed up and the post-operative portion of the procedure begins.

Thirteenth step 5226, the patient's anesthesia is reversed. The surgical hub 106, 206 can infer that the patient is emerging from the anesthesia based on the ventilator data (i.e., the patient's breathing rate begins increasing), for example.

Lastly, the fourteenth step 5228 is that the medical personnel remove the various patient monitoring devices from the patient. The surgical hub 106, 206 can thus infer that the patient is being transferred to a recovery room when the hub loses EKG, BP, and other data from the patient monitoring devices. As can be seen from the description of this illustrative procedure, the surgical hub 106, 206 can determine or infer when each step of a given surgical procedure is taking place according to data received from the various data sources that are communicably coupled to the surgical hub 106, 206.

Situational awareness is further described in U.S. Provisional Patent Application Serial No. 62/611,341, titled INTERACTIVE SURGICAL PLATFORM, filed December 28, 2017, the disclosure of which is herein incorporated by reference in its entirety. In certain instances, operation of a robotic surgical system, including the various robotic surgical systems disclosed herein, for example, can be controlled by the hub 106, 206 based on its situational awareness and/or feedback from the components thereof and/or based on information from the cloud 104.

### Examples

Various aspects of the subject matter described herein are set out in the following numbered examples, which may or may not be claimed.
Example 1: A method for evacuating particulates from a surgical site is disclosed. The method comprises communicatively connecting a particulate evacuation module and a surgical hub, wherein the surgical hub comprises a surgical hub enclosure, and wherein the particulate evacuation module is configured to be received within the surgical hub enclosure. The method further comprises removing a particulate from the surgical site and into the particulate evacuation module, analyzing the removed particulate, and modifying an operation of the particulate evacuation module based on the analysis of the removed particulate.
Example 2: The method of Example 1, further comprising attaching a generator module to the surgical hub enclosure.
Example 3: The method of Example 2, further comprising communicatively connecting the generator module and the particulate evacuation module.
Example 4: The method of any one of Examples 2 and 3, further comprising connecting the generator module to at least one of a monopolar device, a bipolar device, and an ultrasonic device.
Example 5: The method of any one of Examples 2-4, further comprising modifying an operation of the generator module based on the analysis of the removed particulate.
Example 6: The method of any one of Examples 2-5, wherein the generator module comprises a plurality of sub-generator modules, and wherein the surgical hub is configured to facilitate communication between the sub-generator modules to allow for the sub-generator modules to act as a single generator module.
Example 7: The method of any one of Examples 1-6, wherein the particulate evacuation module comprises a fluid line, and wherein an end of the fluid line terminates within the surgical hub enclosure.
Example 8: A method for evacuating particulates from a surgical site is disclosed. The method comprises connecting a particulate evacuation module and a surgical hub, wherein the surgical hub comprises a surgical hub enclosure, and wherein the particulate evacuation module is configured to be received within the surgical hub enclosure. The method further comprises activating a surgical instrument within the surgical site, drawing a particulate from the surgical site into the particulate evacuation module, analyzing the particulate, and modifying an operating parameter of the particulate evacuation module based on the analysis of the particulate.
Example 9: The method of Example 8, wherein the surgical instrument comprises at least one of a monopolar device, a bipolar device, and an ultrasonic device.
Example 10: The method of any one of Examples 8 and 9, further comprising attaching a generator module to the surgical hub enclosure.
Example 11: The method of Example 10, further comprising communicatively connecting the generator module and the smoke evacuation module.
Example 12: The method of any one of Examples 10 and 11, further comprising connecting the generator module to at least one of a monopolar device, a bipolar device, and an ultrasonic device.
Example 13: The method of any one of Examples 10-12, further comprising modifying an operation of the generator module based on the analysis of the particulate.
Example 14: The method of any one of Examples 10-13, wherein the generator module comprises a plurality of sub-generator modules, and wherein the surgical hub is configured to facilitate communication between the sub-generator modules to allow for the sub-generator modules to act as a single generator module.
Example 15: A method for evacuating smoke from a surgical site is disclosed. The method comprises connecting a smoke evacuation module and a surgical hub, wherein the surgical hub comprises a surgical hub enclosure, and wherein the smoke evacuation module is configured to be received within the surgical hub enclosure. The method further comprises inserting a component of the smoke evacuation module into the surgical site, removing smoke from the surgical site, analyzing the removed smoke, and modifying an operation of the smoke evacuation module based on the analysis of the removed smoke.
Example 16: The method of Example 15, further comprising attaching a generator module to the surgical hub enclosure.
Example 17: The method of Example 16, further comprising connecting the generator module and the smoke evacuation module.
Example 18: The method of any one of Examples 16 and 17, further comprising connecting the generator module to at least one of a monopolar device, a bipolar device, and an ultrasonic device.
Example 19: The method of any one of Examples 16-18, further comprising modifying an operation of the generator module based on the analysis of the removed smoke.
Example 20: The method of any one of Examples 16-19, wherein the generator module comprises a plurality of sub-generator modules, and wherein the surgical hub is configured to facilitate communication between the sub-generator modules to allow for the sub-generator modules to act as a single generator module.
Various additional aspects of the subject matter described herein are set out in the following numbered examples, which may or may not be claimed.
Example 1: A method, comprising: acquiring, by a surgical evacuation system, a value of a parameter; transmitting the value of the parameter to at least one of a cloud computing network and a surgical hub; processing, by at least one of the cloud computing network and the surgical hub, the value of the parameter; determining, by at least one of the cloud computing network and the surgical hub, an impact of the value of the parameter on at least one of the surgical evacuation system and a modular device; sending an instruction to adjust operation from at least one of the cloud computing network and the surgical hub to at least one of the surgical evacuation system and the modular device; and adjusting operation by at least one of the surgical evacuation system and the modular device based on the instruction.
Example 2: The method of Example 1, wherein the parameter is sensed by an internal sensor in the surgical evacuation system.
Example 3: The method of Example 2, wherein the parameter is at least one of a particulate concentration, an aerosol percentage, or a chemical analysis and any combination thereof.
Example 4: The method of any one of Examples 1-3, wherein the internal sensor in the surgical evacuation system is at least one of a fluid sensor, a chemical sensor, a laser particle counter, or a pressure sensor and any combination thereof.
Example 5: The method of any one of Examples 1-4, wherein the parameter is an operational parameter.
Example 6: The method of Example 5, wherein the operational parameter is at least one of an airflow, a pressure differential, or an air quality and any combination thereof.
Example 7: The method of any one of Examples 1-6, wherein the operational parameter is sensed by at least one of an air quality particle sensor or an ambient pressure sensor and any combination thereof.
Example 8: The method of any one of Examples 1-7, further comprising: transmitting the value of the parameter from the surgical hub to the cloud computing network.
Example 9: The method of Example 8, wherein the value of the parameter is manipulated by the surgical hub prior to transmitting the value of the parameter to the cloud computing network.
Example 10: The method of any one of Examples 1-9, wherein the value of the parameter is transmitted from the surgical hub to the cloud computing network via a network router.
Example 11: The method of any one of Examples 1-10, further comprising: sending the instruction to adjust operation from the cloud computing network to the surgical hub.
Example 12: The method of Example 11, wherein the surgical hub sends the instruction to adjust operation to at least one of the surgical evacuation system and the modular device.
Example 13: The method of any one of Examples 1-12, wherein the surgical evacuation system acquires values of at least two parameters.
Example 14: The method of any one of Examples 1-13, wherein the modular device is at least one of a visualization circuit; a robotic circuit; an intelligent communications circuit; a imaging circuit; a generator; a suction/irrigation circuit; a communicator; a processor; a storage array; and an operating room mapping circuit.
Example 15: The method of any one of Examples 1-14, wherein at least one of the cloud computing network and the surgical hub is in two way communication to at least one of the surgical evacuation system and the modular device.
Example 16: The method of any one of Examples 1-15, wherein the cloud computing network is in two way communication with the surgical hub.
Example 17: The method of any one of Examples 1-16, wherein at least one of the cloud computing network and the surgical hub include algorithms contained in a memory in order to determine the impact of the value of the parameter on at least one of the surgical evacuation system and the modular device.
Example 18: The method of any one of Examples 1-17, wherein at least one of the cloud computing network and the surgical hub include learning software contained in a memory in order to determine the impact of the value of the parameter on at least one of the surgical evacuation system and the modular device.
Example 19: A computer implemented surgical system, comprising: a surgical hub, the surgical hub including a processor and a memory; a smoke evacuation module in communication with the surgical hub, wherein the smoke evacuation module is configured to: acquire a value of a parameter; transmit the value of the parameter to at least one of a cloud computing network and the surgical hub; wherein at least one of the cloud computing network and the surgical hub is configured to: process the value of the parameter; determine an impact of the value of the parameter on at least one of the smoke evacuation module and a modular device; send an instruction to adjust operation to at least one of the smoke evacuation module and the modular device; wherein at least one of the smoke evacuation module and the modular device is configured to: adjust operation based on the instruction.
Example 20: A non-transitory computer-readable medium storing computer readable instructions, which when executed cause a machine to acquire, by a surgical evacuation system, a value of a parameter; transmit the value of the parameter to at least one of a cloud computing network and a surgical hub; process, by at least one of the cloud computing network and the surgical hub, the value of the parameter; determine, by at least one of the cloud computing network and the surgical hub, an impact of the value of the parameter on at least one of the surgical evacuation system and a modular device; send an instruction to adjust operation from at least one of the cloud computing network and the surgical hub to at least one of the surgical evacuation system and the modular device; and adjust operation by at least one of the surgical evacuation system and the modular device based on the instruction.
Example 21: A method comprising acquiring, by a surgical evacuation system, a value of a parameter; transmitting, via a network router, the value of the parameter to a cloud computing network; processing, by the cloud computing network, the value of the parameter; determining, by the cloud computing network, an impact of the value of the parameter on at least one of the surgical evacuation system and a modular device; sending, via the network router, an instruction to adjust operation from the cloud computing network to the at least one of the surgical evacuation system and the modular device; and adjusting operation by the at least one of the surgical evacuation system and the modular device based on the instruction.
Example 22: The method of Example 21, wherein the parameter is sensed by an internal sensor in the surgical evacuation system.
Example 23: The method of any one of Examples 21-22, wherein the parameter is at least one of a particulate concentration, an aerosol percentage, or a chemical analysis and any combination thereof.
Example 24: The method of any one of Examples 21-23, wherein the internal sensor in the surgical evacuation system is at least one of a fluid sensor, a chemical sensor, a laser particle counter, or a pressure sensor and any combination thereof.
Example 25: The method of any one of Examples 21-24, wherein the parameter is an operational parameter.
Example 26: The method of Example 25, wherein the operational parameter is at least one of airflow, pressure differential, or air quality and any combination thereof.
Example 27: The method of any one of Examples 25-26, wherein the operational parameter is sensed by at least one of an air quality particle sensor or an ambient pressure sensor and any combination thereof.
Example 28: A method comprising acquiring, by a surgical evacuation system, a value of a parameter; transmitting the value of the parameter to a surgical hub; transmitting, via a network router, the value of the parameter from surgical hub to a cloud computing network; processing, by the cloud computing network, the value of the parameter; determining, by the cloud computing network, an impact of the value of the parameter on at least one of the surgical evacuation system and a modular device; sending, via the network router, an instruction to adjust operation from the cloud computing network to the at least one of the surgical evacuation system and the modular device; and adjusting operation by the at least one of the surgical evacuation system and the modular device based on the instruction.
Example 29: The method of Example 28, wherein the parameter is sensed by an internal sensor in the surgical evacuation system.
Example 30: The method of Example 29, wherein the internal sensor in the surgical evacuation system is at least one of a fluid sensor, a chemical sensor, a laser particle counter, or a pressure sensor and any combination thereof.
Example 31: The method of any one of Examples 28-30, wherein the parameter is at least one of a particulate concentration, an aerosol percentage, or a chemical analysis and any combination thereof.
Example 32: The method of any one of Examples 28-31, wherein the parameter is an operational parameter.
Example 33: The method of Example 32, wherein the operational parameter is at least one of airflow, pressure differential, or air quality and any combination thereof.
Example 34: The method of any one of Examples 32-33, wherein the operational parameter is sensed by at least one of an air quality particle sensor or an ambient pressure sensor and any combination thereof.
Example 35: A method comprising acquiring, by a surgical evacuation system, a value of a parameter; transmitting the value of the parameter to a surgical hub; processing, by surgical hub, the value of the parameter; determining, by surgical hub, an impact of the value of the parameter on at least one of the surgical evacuation system and a modular device; sending an instruction to adjust operation from surgical hub to the at least one of the surgical evacuation system and the modular device; and adjusting operation by the at least one of the surgical evacuation system and the modular device based on the instruction.
Example 36: The method of Example 35, wherein the parameter is sensed by an internal sensor in the surgical evacuation system.
Example 37: The method of any one of Examples 35-36, wherein the parameter is at least one of a particulate concentration, an aerosol percentage, or a chemical analysis and any combination thereof.
Example 38: The method of any one of Examples 35-37, wherein the internal sensor in the surgical evacuation system is at least one of a fluid sensor, a chemical sensor, a laser particle counter, or a pressure sensor and any combination thereof.
Example 39: The method of any one of Examples 35-38, wherein the parameter is an operational parameter.
Example 40: The method of any one of Examples 35-39, wherein the operational parameter is at least one of airflow, pressure differential, or air quality and any combination thereof.
Example 41: The method of any one of Examples 35-40, wherein the operational parameter is sensed by at least one of an air quality particle sensor and an ambient pressure sensor.
Example 42: A computer implemented surgical system, comprising a surgical hub; a smoke evacuation module located within surgical hub, wherein the smoke evacuation module is configured to sense, by a sensor, a parameter; transmit, to surgical hub, the parameter; wherein surgical hub is configured to analyze the parameter; modify operation of at least one of the smoke evacuation module and a second module in communication with surgical hub based on the analysis of the parameter.
Example 43: A surgical hub comprising a processor and a memory coupled to the processor, the memory storing instructions executable by the processor to perform the method described in any one of Examples 21-31:
Example 44: A non-transitory computer-readable medium storing computer readable instructions, which when executed cause a machine to perform the method described in any one of Examples 21-31.
Example 45: A method, comprising: acquiring, by a surgical evacuation system, a value of a parameter; transmitting the value of the parameter to at least one of a cloud computing network and a surgical hub; processing, by at least one of the cloud computing network and the surgical hub, the value of the parameter; determining, by at least one of the cloud computing network and the surgical hub, an impact of the value of the parameter on at least one of the surgical evacuation system and a modular device; sending an instruction to adjust operation from at least one of the cloud computing network and the surgical hub to at least one of the surgical evacuation system and the modular device; and adjusting operation by at least one of the surgical evacuation system and the modular device based on the instruction.
Example 46: The method of Example 45, wherein the parameter is sensed by an internal sensor in the surgical evacuation system.
Example 47: The method of any one of Examples 45-46, wherein the parameter is at least one of a particulate concentration, an aerosol percentage, or a chemical analysis and any combination thereof.
Example 48: The method of any one of Examples 45-47, wherein the internal sensor in the surgical evacuation system is at least one of a fluid sensor, a chemical sensor, a laser particle counter, or a pressure sensor and any combination thereof.
Example 49: The method of any one of Examples 45-48, wherein the parameter is an operational parameter.
Example 50: The method of any one of Examples 45-49, wherein the operational parameter is at least one of an airflow, a pressure differential, or an air quality and any combination thereof.
Example 51: The method of any one of Examples 45-50, wherein the operational parameter is sensed by at least one of an air quality particle sensor or an ambient pressure sensor and any combination thereof.
Example 52: The method of any one of Examples 45-51, further comprising: transmitting the value of the parameter from the surgical hub to the cloud computing network.
Example 53: The method of any one of Examples 45-52, wherein the value of the parameter is manipulated by the surgical hub prior to transmitting the value of the parameter to the cloud computing network.
Example 54: The method of any one of Examples 45-53, wherein the value of the parameter is transmitted from the surgical hub to the cloud computing network via a network router.
Example 55: The method of any one of Examples 45-54, further comprising: sending the instruction to adjust operation from the cloud computing network to the surgical hub.
Example 56: The method of any one of Examples 45-55, wherein the surgical hub sends the instruction to adjust operation to at least one of the surgical evacuation system and the modular device.
Example 57: The method of any one of Examples 45-56, wherein the surgical evacuation system acquires values of at least two parameters.
Example 58: The method of any one of Examples 45-57, wherein the modular device is at least one of a visualization circuit; a robotic circuit; an intelligent communications circuit; a imaging circuit; a generator; a suction/irrigation circuit; a communicator; a processor; a storage array; and an operating room mapping circuit.
Example 59: The method of any one of Examples 45-58, wherein at least one of the cloud computing network and the surgical hub is in two way communication to at least one of the surgical evacuation system and the modular device.
Example 60: The method of any one of Examples 45-59, wherein the cloud computing network is in two way communication with the surgical hub.
Example 61: The method of any one of Examples 45-60, wherein at least one of the cloud computing network and the surgical hub include algorithms contained in a memory in order to determine the impact of the value of the parameter on at least one of the surgical evacuation system and the modular device.
Example 62: The method of any one of Examples 45-61, wherein at least one of the cloud computing network and the surgical hub include learning software contained in a memory in order to determine the impact of the value of the parameter on at least one of the surgical evacuation system and the modular device.
Example 63: A computer implemented surgical system, comprising: a surgical hub, the surgical hub including a processor and a memory; a smoke evacuation module in communication with the surgical hub, wherein the smoke evacuation module is configured to: acquire a value of a parameter; transmit the value of the parameter to at least one of a cloud computing network and the surgical hub; wherein at least one of the cloud computing network and the surgical hub is configured to: process the value of the parameter; determine an impact of the value of the parameter on at least one of the smoke evacuation module and a modular device; send an instruction to adjust operation to at least one of the smoke evacuation module and the modular device; wherein at least one of the smoke evacuation module and the modular device is configured to: adjust operation based on the instruction.
Example 64: A non-transitory computer-readable medium storing computer readable instructions, which when executed cause a machine to acquire, by a surgical evacuation system, a value of a parameter; transmit the value of the parameter to at least one of a cloud computing network and a surgical hub; process, by at least one of the cloud computing network and the surgical hub, the value of the parameter; determine, by at least one of the cloud computing network and the surgical hub, an impact of the value of the parameter on at least one of the surgical evacuation system and a modular device; send an instruction to adjust operation from at least one of the cloud computing network and the surgical hub to at least one of the surgical evacuation system and the modular device; and adjust operation by at least one of the surgical evacuation system and the modular device based on the instruction.

Various additional aspects of the subject matter described herein are set out in the following numbered examples, which may or may not be claimed.
Example 1: A surgical hub, comprising: a control circuit, the control circuit including a plurality of segments; the plurality of segments including a first segment, a second segment, and a third segment; the first segment includes a main processor and a safety processor, wherein the main processor is coupled to a memory, and the memory stores instructions executable by the main processor to energize the first segment before the second and third segment, energizing the second and third segments after the first segment performs a first function; the second segment includes a sensing circuit and a display circuit; and the third segment including a motor control circuit and a motor.
Example 2: The surgical hub of Example 1, wherein the first function is checking the first, second, and third segments for at least one of a short circuit and an error.
Example 3: The surgical hub of any one of Examples 1-2, wherein the first function is performing a safety check on the first, second, and third segments.
Example 4: The surgical hub of any one of Examples 1-3, wherein the first function is verifying the sensing circuit, the display circuit, the motor control circuit, and the motor are correct components.
Example 5: The surgical hub of any one of Examples 1-4, wherein the control circuit identifies the main processor, the safety processor, the sensing circuit, the display circuit, the motor control circuit, and the motor, and displays on a display device a status of the main processor, the safety processor, the sensing circuit, the display circuit, the motor control circuit, and the motor.
Example 6: The surgical hub of any one of Examples 1-5, wherein the control circuit identifies the first segment, the second segment and the third segment, and displays on a display device a status to a user of the first segment, the second segment, and the third segment.
Example 7: The surgical hub of any one of Examples 1-6, further comprising a pump coupled to the motor.
Example 8: The surgical hub of anyone of Examples 1-7, wherein the motor operates at a first state or a second state.
Example 9: The surgical hub of any one of Examples 1-8, wherein the first state is activated by a user, and the motor causes the pump to operate at a first rate.
Example 10: The surgical hub of any one of Examples 1-9, wherein the motor causes the pump to operate at a second rate in the second state.
Example 11: A method of operating a segmented control circuit in a surgical evacuation system, comprising: activating a first segment, wherein the first segment includes a main processor and a safety processor; causing the first segment to perform a first function; activating a second segment, wherein the second segment includes a sensing circuit and a display circuit; and activating a third segment, wherein the third segment includes a motor control circuit and a motor.
Example 12: The method of operating the segmented control circuit of Example 11, wherein the first function is checking the first, second, and third segments for at least one of a short circuit and an error.
Example 13: The method of operating the segmented control circuit of any one of Examples 11-12, wherein the first function is performing a safety check on the first, second, and third segments.
Example 14: The method of operating the segmented control circuit of any one of Examples 11-13, wherein the first function is verifying the sensing circuit, the display circuit, the motor control circuit, and the motor are correct components.
Example 15: The method of operating the segmented control circuit of any one of Examples 11-14, further comprising: identifying, by the control circuit, the main processor, the safety processor, the sensing circuit, the display circuit, the motor control circuit, and the motor; and displaying on a display device a status of the main processor, the safety processor, the sensing circuit, the display circuit, the motor control circuit, and the motor.
Example 16: The method of operating the segmented control circuit of any one of Examples 11-15, further comprising: identifying, by the control circuit, the first segment, the second segment, and the third segment; and displaying on a display device a status of the first segment, the second segment, and the third segment.
Example 17: The method of operating the segmented control circuit of any one of Examples 11-16, further comprising: operating the motor at a first state or a second state.
Example 18: The method of operating the segmented control circuit of any one of Examples 11-17, wherein the first state is activated by a user, where the motor causes the pump to operate at a first rate.
Example 19: The method of operating the segmented control circuit of any one of Examples 11-18, wherein the motor causes the pump to operate at a second rate in the second state.
Example 20: A non-transitory computer-readable medium storing computer readable instructions, which when executed cause a machine to: activate a first segment, wherein the first segment includes a main processor and a safety processor; cause the first segment to perform a first function; activate a second segment, wherein the second segment includes a sensing circuit and a display circuit; and activate a third segment, wherein the third segment includes a motor control circuit and a motor.
Example 21: A surgical evacuation system comprising a control circuit, the control circuit including a plurality of segments; the plurality of segments including a first segment, a second segment, and a third segment; the first segment includes a main processor and a safety processor, wherein the main processor is coupled to a memory, and the memory stores instructions executable by the main processor to energize the first segment before the second and third segment, energizing the second and third segments after the first segment performs a first function; the second segment includes a sensing circuit and a display circuit; and the third segment including a motor control circuit and a motor.
Example 22: The surgical evacuation system of Example 21, wherein the first function is checking the first, second, and third segments for at least one of a short circuit and an error.
Example 23: The surgical evacuation system of any one of Examples 21-22, wherein the first function is performing a safety check on the first, second, and third segments.
Example 24: The surgical evacuation system of any one of Examples 21-23, wherein the first function is verifying the sensing circuit, the display circuit, the motor control circuit, and the motor are the correct components.
Example 25: The surgical evacuation system of any one of Examples 21-24, wherein the control circuit identifies the main processor, the safety processor, the sensing circuit, the display circuit, the motor control circuit, and the motor, and displays on a display device a status of the main processor, the safety processor, the sensing circuit, the display circuit, the motor control circuit, and the motor.
Example 26: The surgical evacuation system of any one of Examples 21-25, wherein the control circuit identifies the first segment, the second segment and the third segment, and displays on a display device a status to a user of the first segment, the second segment, and the third segment.
Example 27: The surgical evacuation system of any one of Examples 21-26, further comprising a pump coupled to the motor.
Example 28: The surgical evacuation system of any one of Examples 21-27, wherein the motor operates at a first state or a second state.
Example 29: The surgical evacuation system of Example 28, wherein the first state is activated by a user, and the motor causes the pump to operate at a rapid rate.
Example 30: The surgical evacuation any one of Examples 28-29, wherein the second state is a slow state, where the motor causes the pump to operate at a slower rate.
Example 31: A method of operating a segmented control circuit in a surgical evacuation system, comprising activating a first segment, wherein the first segment includes a main processor and a safety processor; causing the first segment to perform a first function; activating a second segment, wherein the second segment includes a sensing circuit and a display circuit; and activating a third segment, wherein the third segment includes a motor control circuit and a motor.
Example 32: The method of operating the segmented control circuit of Example 31, wherein the first function is checking the first, second, and third segments for at least one of a short circuit and an error.
Example 33: The method of operating the segmented control circuit of any one of Examples 31-32, wherein the first function is performing a safety check on the first, second, and third segments.
Example 34: The method of operating the segmented control circuit of any one of Examples 31-33, wherein the first function is verifying the sensing circuit, the display circuit, the motor control circuit, and the motor are the correct components.
Example 35: The method of operating the segmented control circuit of any one of Examples 31-34, further comprising: identifying, by the control circuit, the main processor, the safety processor, the sensing circuit, the display circuit, the motor control circuit, and the motor; and displaying on a display device a status of the main processor, the safety processor, the sensing circuit, the display circuit, the motor control circuit, and the motor.
Example 36: The method of operating the segmented control circuit of any one of Example 31-35, further comprising identifying, by the control circuit, the first segment, the second segment, and the third segment; and displaying on a display device a status of the first segment, the second segment, and the third segment.
Example 37: The method of operating the segmented control circuit of any one of Examples 31-36, wherein a pump is coupled to the motor.
Example 38: The method of operating the segmented control circuit of Example 37, further comprising operating the motor at a first state or a second state.
Example 39: The method of operating the segmented control circuit of Example 38, wherein the first state is activated by a user, where the motor causes the pump to operate at a rapid rate.
Example 40: The method of operating the segmented control circuit of any one of Examples 38-39, wherein the second state is a slow state, where the motor causes the pump to operate at a slower rate.
Example 41: A surgical hub comprising a processor and a memory coupled to the processor, the memory storing instructions executable by the processor to perform the method described in any one of Examples 31-40.
Example 42: A non-transitory computer-readable medium storing computer readable instructions, which when executed cause a machine to perform the method described in any one of Examples 31-40.
Example 43: The method of operating the segmented control circuit of Example 38, wherein the first state is activated by a user, where the motor causes the pump to operate at a first rate.
Example 44: The method of operating the segmented control circuit of Example 38, wherein the motor causes the pump to operate at a second rate in the second state.
Example 45: A non-transitory computer-readable medium storing computer readable instructions, which when executed cause a machine to: activate a first segment, wherein the first segment includes a main processor and a safety processor; cause the first segment to perform a first function; activate a second segment, wherein the second segment includes a sensing circuit and a display circuit; and activate a third segment, wherein the third segment includes a motor control circuit and a motor.

Various additional aspects of the subject matter described herein are set out in the following numbered examples, which may or may not be claimed.
Example 1: A surgical evacuation system, comprising a smoke evacuation device comprising a pump; and a motor operably coupled to the pump; a communication circuit; and a filter device in communication with the smoke evacuation device through the communication circuit, wherein the filter device comprises a plurality of filter components including a plurality of filter elements comprising at least one of a fluid filter, particulate filter, and a charcoal filter; and a plurality of filter sensors, wherein the communication circuit is configured to authenticate the plurality of filter components; verify a remaining life of at least one of the plurality of filter components; update parameters output from the plurality of filter components; and record errors output from the plurality of filter components.
Example 2: The surgical evacuation system of Example 1, wherein the communication circuit is further configured to limit a number or a type of a filter component that can be identified by the communication circuit.
Example 3: The surgical evacuation system of any one of Examples 1-2, wherein the communication circuit is further configured to enable or disable the plurality of filter components based on a result of the authentication.
Example 4: The surgical evacuation system of any one of Examples 1-3, wherein the communication circuit authenticates the plurality of filter components by using filter component information.
Example 5: The surgical evacuation system of Example 4, wherein the filter component information comprises at least one of a product type, a product name, a unique device identifier, a product trademark, a serial number, and a configuration parameter of the plurality of filter components.
Example 6: The surgical evacuation system of any one of Examples 1-5, wherein the filter device is configured to encrypt the parameters output from the plurality of filter components before sending the parameters to the communication circuit.
Example 7: The surgical evacuation system of Example 6, wherein upon receiving the encrypted parameters, the communication circuit decrypts the encrypted parameters.
Example 8: The surgical evacuation system of any one of Examples 1-7, wherein the communication device acts as a master device and the filter device acts as a slave device, wherein the communication between the master device and the slave device is unidirectional from the master device to the slave device when performing the authentication process.
Example 9: A method for communication between a smoke evacuation device and a filter device through a communication circuit, wherein the method comprises authenticating, by the communication circuit, a plurality of filter components in the filter device, wherein the plurality of filter components include a plurality of filter elements comprising at least one of a fluid filter, particulate filter, and a charcoal filter; and a plurality of filter sensors; verifying, by the communication circuit, a remaining life of at least one of the plurality of filter components; updating, by the communication circuit, parameters output from the plurality of filter components; and recording, by the communication circuit, errors output from the plurality of filter components, wherein the smoke evacuation device comprises a pump; and a motor operably coupled to the pump.
Example 10: The method of Example 9, further comprising limiting a number or a type of a filter component that can be identified by the communication circuit.
Example 11: The method of Example 9, further comprising enabling or disabling, by the communication circuit, the plurality of filter components based on a result of the authentication.
Example 12: The method of any one of Examples 9-11, wherein the communication circuit authenticates the plurality of filter components by using filter component information.
Example 13: The method of Example 12, wherein the filter component information comprises at least one of a product type, a product name, a unique device identifier, a product trademark, a serial number, and a configuration parameter of the plurality of filter components.
Example 14: The method of any one of Example 9-13, further comprising encrypting, by the filter device, the parameters output from the plurality of filter components before sending the parameters to the communication circuit.
Example 15: The method of Example 14, further comprising upon receiving the encrypted parameters, decrypting, by the communication circuit, the encrypted parameters.
Example 16: The method of any one of Examples 9-15, wherein the communication device acts as a master device and the filter device acts as a slave device, wherein the communication between the master device and the slave device is unidirectional from the master device to the slave device when performing the authentication step.
Example 17: A surgical hub comprising a processor and a memory coupled to the processor, the memory storing instructions executable by the processor to perform the method described in anyone of Examples 9-16.
Example 18: A non-transitory computer-readable medium storing computer readable instructions, which when executed cause a machine to perform the method described in any one of Examples 9-16.
Example 19: A surgical hub comprising a processor and a memory coupled to the processor, the memory storing instructions executable by the processor to authenticate a plurality of filter components in a filter device, wherein the plurality of filter components include a plurality of filter elements comprising at least one of a fluid filter, particulate filter, and a charcoal filter; and a plurality of filter sensors; verify a remaining life of at least one of the plurality of filter components; update parameters output from the plurality of filter components; and record errors output from the plurality of filter components.
Example 20: The surgical hub of Example 19, wherein the memory stores further instructions executable by the processor to limit a number or a type of a filter component that can be identified.
Example 21: The surgical hub of any one of Examples 19-20, wherein the memory stores further instructions executable by the processor to enable or disable the plurality of filter components based on a result of the authentication.
Example 22: The surgical hub of any one of Examples 19-21, wherein the plurality of filter components are authenticated by using filter component information comprising at least one of a product type, a product name, a unique device identifier, a product trademark, a serial number, and a configuration parameter of the plurality of filter components.

Various additional aspects of the subject matter described herein are set out in the following numbered examples, which may or may not be claimed.
Example 1: A surgical evacuation system, comprising a pump; a motor operably coupled to the pump; and a flow path fluidically coupled to the pump, wherein the flow path comprises a first fluid filter configured to extract a large droplet in a fluid moving through the flow path; and a second fluid filter configured to extract a small droplet in the fluid moving through the flow path, wherein the first fluid filter is coupled in series with the second fluid filter, wherein the first fluid filter is positioned upstream of the second fluid filter, wherein an outlet port of the second fluid filter is coupled to an inlet port of a non-fluid filter.
Example 2: The surgical evacuation system of Example 1, further comprising a first recirculation channel, wherein an inlet port of the first recirculation channel is positioned between the second fluid filter and the non-fluid filter, wherein the first recirculation channel is configured to recirculate the fluid output from the second fluid filter.
Example 3: The surgical evacuation system of Example 2, further comprising a first recirculation valve configured to close and open the first recirculation channel, wherein when the first recirculation valve is opened, the fluid output from the second fluid filter is recirculated through the first recirculation channel.
Example 4: The surgical evacuation system of Example 3, further comprising a first sensor positioned near the first recirculation valve, wherein the first sensor is configured to detect a parameter of the fluid, wherein the first recirculation valve opens the first recirculation channel when the parameter detected by the first sensor is equal to or greater than a first predetermined threshold value.
Example 5: The surgical evacuation system of any one of Examples 2-4, wherein the fluid directed into the first recirculation channel is injected into the fluid path upstream of the second fluid filter.
Example 6: The surgical evacuation system of Example 5, wherein the fluid directed into the first recirculation channel is injected into the first fluid filter.
Example 7: The surgical evacuation system of any one of Examples 2-6, wherein the fluid directed into the first recirculation channel is injected into an upstream portion of the second fluid filter.
Example 8: The surgical evacuation system of any one of Examples 2-7, wherein the first recirculation channel extends downward from the inlet port of the first recirculation channel, which allows the large droplet or the small droplet in the fluid output from the second fluid filter to be directed to the first recirculation channel via gravity.
Example 9: The surgical evacuation system of any one of Examples 2-8, further comprising a second recirculation channel, wherein an inlet port of the second recirculation channel is positioned between the first fluid filter and the second fluid filter, wherein the second recirculation channel is configured to recirculate the fluid output from the first fluid filter.
Example 10: The surgical evacuation system of Example 9, further comprising a second recirculation valve configured to close and open the second recirculation channel, wherein when the second recirculation valve is opened, the fluid output from the first fluid filter is recirculated through the second recirculation channel.
Example 11: The surgical evacuation system of Example 10, further comprising a second sensor positioned near the second recirculation valve, wherein the second sensor is configured to detect the parameter of the fluid, wherein the second recirculation valve opens the second recirculation channel when the parameter detected by the second sensor is equal to or greater than a second predetermined threshold value.
Example 12: The surgical evacuation system of any one of Example 9-11, wherein the fluid directed into the second recirculation channel is injected into the fluid path upstream of the first fluid filter.
Example 13: The surgical evacuation system of any one of Examples 9-12, wherein the fluid directed into the second recirculation channel is injected into an upstream portion of the first fluid filter.
Example 14: The surgical evacuation system of any one of Examples 9-13, wherein the second recirculation channel extends downward from the inlet port of the second recirculation channel, which allows the large droplet or the small droplet in the fluid output from the first fluid filter to be directed to the second recirculation channel via gravity.
Example 15: The surgical evacuation system of any one of Examples 1-14, wherein the first fluid filter comprises at least one baffle.
Example 16: The surgical evacuation system of any one of Examples 1-15, wherein the first fluid filter comprises a diverter valve.
Example 17: The surgical evacuation system of any one of Examples 1-16, wherein the second fluid filter comprises a filter selected from the group consisting of a membrane filter, a honeycomb filter, and a porous structure filter, and combinations thereof.
Example 18: The surgical evacuation system of any one of Example 1-17, wherein the non-fluid filter comprises a particulate filter.
Example 19: The surgical evacuation system of any one of Example 1-17, wherein at least one of the first fluid filter and the second fluid filter is disabled when it is determined that the number of recirculation processes performed through a recirculation channel is equal to or greater than a third predetermined threshold value.
Example 20: A surgical evacuation system, comprising: a pump; a motor operably coupled to the pump; and a flow path fluidically coupled to the pump, wherein the flow path comprises: a first fluid filter configured to extract a large droplet in a fluid moving through the flow path, wherein the first fluid filter comprises at least one baffle; and a second fluid filter configured to extract a small droplet in the fluid moving through the flow path, wherein the second fluid filter comprises a filter selected from the group consisting of a membrane filter, a honeycomb filter, and a porous structure filter, and combinations thereof, wherein the first fluid filter is coupled in series with the second fluid filter, wherein the first fluid filter is positioned upstream of the second fluid filter, wherein an outlet port of the second fluid filter is coupled to an inlet port of a non-fluid filter.
Example 21: The surgical evacuation system of Example 20, further comprising a first recirculation channel, wherein an inlet port of the first recirculation channel is positioned between the second fluid filter and the non-fluid filter, wherein the first recirculation channel is configured to recirculate the fluid output from the second fluid filter.
Example 22: The surgical evacuation system of any one of Examples 20-21, further comprising a second recirculation channel, wherein an inlet port of the second recirculation channel is positioned between the first fluid filter and the second fluid filter, wherein the second recirculation channel is configured to recirculate the fluid output from the first fluid filter.

Various additional aspects of the subject matter described herein are set out in the following numbered examples, which may or may not be claimed.
Example 1: A return pad of an electrosurgical system, the return pad comprising: a plurality of conductive members configured to receive radio frequency current applied to a patient; and a plurality of sensing devices configured to detect at least one of the following: a nerve control signal applied to the patient; and a movement of an anatomical feature of the patient resulting from application of the nerve control signal.
Example 2: The return pad of Example 1, wherein the plurality of conductive members is further configured for capacitive coupling with the patient.
Example 3: The return pad of any one of Examples 1-2, further comprising a switching device configured for selectively decoupling at least one of the plurality of conductive members from another one of the plurality of conductive members.
Example 4: The return pad of any one of Examples 1-3, further comprising a switching device configured for selectively decoupling each of the conductive members from one another.
Example 5: The return pad of any one of Examples 1-4, further comprising a switching device configured for selectively decoupling the plurality of conductive members from the patient.
Example 6: The return pad of any one of Examples 1-5, further comprising a switching device configured for selectively decoupling the plurality of conductive members from a generator.
Example 7: The return pad of any one of Examples 1-6, further comprising a switching device configured for selectively coupling each of the plurality of conductive members to a generator.
Example 8: The return pad of any one of Examples 1-7, wherein the plurality of sensing devices are configured as an array of sensing devices.
Example 9: The return pad of any one of Examples 1-8, wherein each of the plurality of sensing devices is coupled to a corresponding one of the plurality of conductive members.
Example 10: The return pad of any one of Examples 1-9, wherein each of the plurality of conductive members is coupled to a corresponding one of the plurality of sensing devices.
Example 11: The return pad of any one of Examples 1-10, wherein at least one of the plurality of sensing devices comprises one of the following: a pressure sensor; an accelerometer; and a pressure sensor and an accelerometer.
Example 12: The return pad of any one of Examples 1-11, wherein each of the plurality of sensing devices is further configured to output a signal indicative of the detection.
Example 13: An electrosurgical system, comprising: a generator configured to supply an alternating current at a radio frequency; an instrument configured to apply the alternating current to a patient; a return pad capacitively couplable to the patient, wherein the return pad comprises: a plurality of conductive members configured to conduct radio frequency current, wherein the plurality of conductive members are capacitively couplable to a patient and are selectively couplable to the generator; a plurality of sensing devices configured to detect at least one of the following: a nerve control signal applied to the patient; and a movement of an anatomical feature of the patient resulting from application of the nerve control signal; and a conductor coupled to the return pad and the generator.
Example 14: The electrosurgical system of Example 13, further comprising a multi input: single output switching device coupled to each of the plurality of sensing devices.
Example 15: The electrosurgical system of Example 14, further comprising an analog-to-digital converter coupled to the multi input: single output switching device.
Example 16: The electrosurgical system of any one of Examples 1-15, wherein the generator is further configured to: generate the alternating current as a high frequency waveform; generate a low frequency waveform which is configured to stimulate a nerve of the patient; utilize the high frequency waveform to modulate the low frequency waveform to form a composite waveform; and supply the composite waveform to the instrument.
Example 17: The electrosurgical system of any one of Examples 1-16, wherein the generator is further configured to amplitude modulate the low frequency waveform to form the composite waveform.
Example 18: A return pad of an electrosurgical system, the return pad comprising: a plurality of electrodes configured for capacitive coupling with a patient; and an array of sensing devices configured to detect at least one of the following: a nerve control signal applied to the patient; and a movement of an anatomical feature of the patient resulting from application of the nerve control signal.
Example 19: The return pad of Example 18, wherein each sensing device of the array of sensing devices is configured to output a signal indicative of the detection.
Example 20: The return pad of any one of Examples 18-19, wherein each sensing device of the array of sensing devices is positioned on a corresponding one of the plurality of electrodes.

While several forms have been illustrated and described, it is not the intention of the applicant to restrict or limit the scope of the appended claims to such detail. Numerous modifications, variations, changes, substitutions, combinations, and equivalents to those forms may be implemented and will occur to those skilled in the art without departing from the scope of the present disclosure. Moreover, the structure of each element associated with the described forms can be alternatively described as a means for providing the function performed by the element. Also, where materials are disclosed for certain components, other materials may be used. It is therefore to be understood that the foregoing description and the appended claims are intended to cover all such modifications, combinations, and variations as falling within the scope of the disclosed forms. The appended claims are intended to cover all such modifications, variations, changes, substitutions, modifications, and equivalents.

The foregoing detailed description has set forth various forms of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, and/or examples can be implemented, individually and/or collectively, by a wide range of hardware, software, firmware, or virtually any combination thereof. Those skilled in the art will recognize that some aspects of the forms disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as one or more program products in a variety of forms, and that an illustrative form of the subject matter described herein applies regardless of the particular type of signal bearing medium used to actually carry out the distribution.

Instructions used to program logic to perform various disclosed aspects can be stored within a memory in the system, such as dynamic random access memory (DRAM), cache, flash memory, or other storage. Furthermore, the instructions can be distributed via a network or by way of other computer readable media. Thus a machine-readable medium may include any mechanism for storing or transmitting information in a form readable by a machine (e.g., a computer), but is not limited to, floppy diskettes, optical disks, compact disc, read-only memory (CD-ROMs), and magneto-optical disks, read-only memory (ROMs), random access memory (RAM), erasable programmable read-only memory (EPROM), electrically erasable programmable read-only memory (EEPROM), magnetic or optical cards, flash memory, or a tangible, machine-readable storage used in the transmission of information over the Internet via electrical, optical, acoustical or other forms of propagated signals (e.g., carrier waves, infrared signals, digital signals, etc.). Accordingly, the non-transitory computer-readable medium includes any type of tangible machine-readable medium suitable for storing or transmitting electronic instructions or information in a form readable by a machine (e.g., a computer).

As used in any aspect herein, the term "control circuit" may refer to, for example, hardwired circuitry, programmable circuitry (e.g., a computer processor comprising one or more individual instruction processing cores, processing unit, processor, microcontroller, microcontroller unit, controller, digital signal processor (DSP), programmable logic device (PLD), programmable logic array (PLA), or field programmable gate array (FPGA)), state machine circuitry, firmware that stores instructions executed by programmable circuitry, and any combination thereof. The control circuit may, collectively or individually, be embodied as circuitry that forms part of a larger system, for example, an integrated circuit (IC), an application-specific integrated circuit (ASIC), a system on-chip (SoC), desktop computers, laptop computers, tablet computers, servers, smart phones, etc. Accordingly, as used herein "control circuit" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

As used in any aspect herein, the term "logic" may refer to an app, software, firmware and/or circuitry configured to perform any of the aforementioned operations. Software may be embodied as a software package, code, instructions, instruction sets and/or data recorded on non-transitory computer readable storage medium. Firmware may be embodied as code, instructions or instruction sets and/or data that are hard-coded (e.g., nonvolatile) in memory devices.

As used in any aspect herein, the terms "component," "system," "module" and the like can refer to a computer-related entity, either hardware, a combination of hardware and software, software, or software in execution.

As used in any aspect herein, an "algorithm" refers to a self-consistent sequence of steps leading to a desired result, where a "step" refers to a manipulation of physical quantities and/or logic states which may, though need not necessarily, take the form of electrical or magnetic signals capable of being stored, transferred, combined, compared, and otherwise manipulated. It is common usage to refer to these signals as bits, values, elements, symbols, characters, terms, numbers, or the like. These and similar terms may be associated with the appropriate physical quantities and are merely convenient labels applied to these quantities and/or states.

A network may include a packet switched network. The communication devices may be capable of communicating with each other using a selected packet switched network communications protocol. One example communications protocol may include an Ethernet communications protocol which may be capable permitting communication using a Transmission Control Protocol/Internet Protocol (TCP/IP). The Ethernet protocol may comply or be compatible with the Ethernet standard published by the Institute of Electrical and Electronics Engineers (IEEE) titled "IEEE 802.3 Standard", published in December, 2008 and/or later versions of this standard. Alternatively or additionally, the communication devices may be capable of communicating with each other using an X.25 communications protocol. The X.25 communications protocol may comply or be compatible with a standard promulgated by the International Telecommunication Union-Telecommunication Standardization Sector (ITU-T). Alternatively or additionally, the communication devices may be capable of communicating with each other using a frame relay communications protocol. The frame relay communications protocol may comply or be compatible with a standard promulgated by Consultative Committee for International Telegraph and Telephone (CCITT) and/or the American National Standards Institute (ANSI). Alternatively or additionally, the transceivers may be capable of communicating with each other using an Asynchronous Transfer Mode (ATM) communications protocol. The ATM communications protocol may comply or be compatible with an ATM standard published by the ATM Forum titled "ATM-MPLS Network Interworking 2.0" published August 2001, and/or later versions of this standard. Of course, different and/or after-developed connection-oriented network communication protocols are equally contemplated herein.

Unless specifically stated otherwise as apparent from the foregoing disclosure, it is appreciated that, throughout the foregoing disclosure, discussions using terms such as "processing," "computing," "calculating," "determining," "displaying," or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

One or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Those skilled in the art will recognize that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to claims containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

With respect to the appended claims, those skilled in the art will appreciate that recited operations therein may generally be performed in any order. Also, although various operational flow diagrams are presented in a sequence(s), it should be understood that the various operations may be performed in other orders than those which are illustrated, or may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "related to," or other past-tense adjectives are generally not intended to exclude such variants, unless context dictates otherwise.

It is worthy to note that any reference to "one aspect," "an aspect," "an exemplification," "one exemplification," and the like means that a particular feature, structure, or characteristic described in connection with the aspect is included in at least one aspect. Thus, appearances of the phrases "in one aspect," "in an aspect," "in an exemplification," and "in one exemplification" in various places throughout the specification are not necessarily all referring to the same aspect. Furthermore, the particular features, structures or characteristics may be combined in any suitable manner in one or more aspects.

Any patent application, patent, non-patent publication, or other disclosure material referred to in this specification and/or listed in any Application Data Sheet is incorporated by reference herein, to the extent that the incorporated materials is not inconsistent herewith. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

In summary, numerous benefits have been described which result from employing the concepts described herein. The foregoing description of the one or more forms has been presented for purposes of illustration and description. It is not intended to be exhaustive or limiting to the precise form disclosed. Modifications or variations are possible in light of the above teachings. The one or more forms were chosen and described in order to illustrate principles and practical application to thereby enable one of ordinary skill in the art to utilize the various forms and with various modifications as are suited to the particular use contemplated. It is intended that the claims submitted herewith define the overall scope.

## Claims

1. A method for evacuating particulates from a surgical site, the method comprising:
communicatively connecting a particulate evacuation module and a surgical hub, wherein the surgical hub comprises a surgical hub enclosure, and wherein the particulate evacuation module is configured to be received within the surgical hub enclosure;
removing a particulate from the surgical site and into the particulate evacuation module;
analyzing the removed particulate; and
modifying an operation of the particulate evacuation module based on the analysis of the removed particulate.

2. The method of Claim 1, further comprising attaching a generator module to the surgical hub enclosure.

3. The method of Claim 2, further comprising communicatively connecting the generator module and the particulate evacuation module.

4. The method of Claim 2 or Claim 3, further comprising connecting the generator module to at least one of a monopolar device, a bipolar device, and an ultrasonic device.

5. The method of any one of Claims 2 to 4, further comprising modifying an operation of the generator module based on the analysis of the removed particulate.

6. The method of any one of Claims 2 to 5, wherein the generator module comprises a plurality of sub-generator modules, and wherein the surgical hub is configured to facilitate communication between the sub-generator modules to allow for the sub-generator modules to act as a single generator module.

7. The method of any preceding Claim, wherein the particulate evacuation module comprises a fluid line, and wherein an end of the fluid line terminates within the surgical hub enclosure.

8. A method for evacuating particulates from a surgical site, the method comprising:
connecting a particulate evacuation module and a surgical hub, wherein the surgical hub comprises a surgical hub enclosure, and wherein the particulate evacuation module is configured to be received within the surgical hub enclosure;
activating a surgical instrument within the surgical site;
drawing a particulate from the surgical site into the particulate evacuation module;
analyzing the particulate; and
modifying an operating parameter of the particulate evacuation module based on the analysis of the particulate.

9. The method of Claim 8, wherein the surgical instrument comprises at least one of a monopolar device, a bipolar device, and an ultrasonic device.

10. The method of Claim 8 of Claim 9, further comprising attaching a generator module to the surgical hub enclosure.

11. The method of Claim 10, further comprising communicatively connecting the generator module and the smoke evacuation module.

12. The method of Claim 10 or Claim 11, further comprising connecting the generator module to at least one of a monopolar device, a bipolar device, and an ultrasonic device.

13. The method of any one of Claims 10 to 12, further comprising modifying an operation of the generator module based on the analysis of the particulate.

14. The method of any one of Claims 10 to 13, wherein the generator module comprises a plurality of sub-generator modules, and wherein the surgical hub is configured to facilitate communication between the sub-generator modules to allow for the sub-generator modules to act as a single generator module.

15. A method for evacuating smoke from a surgical site, the method comprising:
connecting a smoke evacuation module and a surgical hub, wherein the surgical hub comprises a surgical hub enclosure, and wherein the smoke evacuation module is configured to be received within the surgical hub enclosure;
inserting a component of the smoke evacuation module into the surgical site;
removing smoke from the surgical site;
analyzing the removed smoke; and
modifying an operation of the smoke evacuation module based on the analysis of the removed smoke.

16. The method of Claim 15, further comprising attaching a generator module to the surgical hub enclosure.

17. The method of Claim 16, further comprising connecting the generator module and the smoke evacuation module.

18. The method of Claim 16 or Claim 17, further comprising connecting the generator module to at least one of a monopolar device, a bipolar device, and an ultrasonic device.

19. The method of any one of Claims 16 to 18, further comprising modifying an operation of the generator module based on the analysis of the removed smoke.

20. The method of any one of Claims 16 to 19, wherein the generator module comprises a plurality of sub-generator modules, and wherein the surgical hub is configured to facilitate communication between the sub-generator modules to allow for the sub-generator modules to act as a single generator module.
